(19) Europäisches Patentamt
European Patent Office
Office européen des brevets

(11) Publication number: **0 103 381**
**B1**

(12) # EUROPEAN PATENT SPECIFICATION

(45) Date of publication of patent specification: **23.09.87**

(51) Int. Cl.$^4$: **C 07 D 231/54,** A 61 K 31/415

(21) Application number: **83304264.1**

(22) Date of filing: **22.07.83**

(54) **Substituted anthra(1,9-cd)pyrazol-6(2H)-ones.**

(30) Priority: **23.07.82 US 401157**
**28.06.83 US 507961**

(43) Date of publication of application:
**21.03.84 Bulletin 84/12**

(45) Publication of the grant of the patent:
**23.09.87 Bulletin 87/39**

(84) Designated Contracting States:
**AT BE CH DE FR GB IT LI LU NL SE**

(58) References cited:
**CH-A- 191 142**
**GB-A- 470 475**
**US-A-2 518 150**

(73) Proprietor: **WARNER-LAMBERT COMPANY**
**201 Tabor Road**
**Morris Plains New Jersey 07950 (US)**

(72) Inventor: **Showalter, Howard Daniel Hollis**
**900 Patricia**
**Ann Arbor Michigan 48103 (US)**
Inventor: **Werbel, Leslie M.**
**1570 Covington**
**Ann Arbor Michigan 48104 (US)**
Inventor: **Johnson, Judith L.**
**2779 Ellis Road**
**Ypsilanti Michigan 48103 (US)**
Inventor: **Elslager, Edward F.**
**4081 Thornoaks**
**Ann Arbor Michigan 48104 (US)**

(74) Representative: **Jones, Michael Raymond et al**
**HASELTINE LAKE & CO. Hazlitt House**
**28 Southampton Buildings Chancery Lane**
**London WC2A 1AT (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Background of the Invention

Several 2,5 and 2,7-disubstituted anthra[1,9-*cd*]-pyrazol-6(2*H*)-ones are disclosed in the prior literature. See for example J. Chem. Soc., 1630 (1952); J. Chem. Soc., 1984 (1954). Neither reference discloses any utility for these compounds.

Summary of the Invention

The invention in its first generic chemical compound aspect is a compound having the structural formula

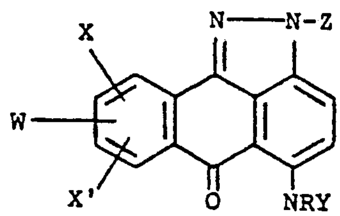

wherein X, X' and W may be the same or different and are hydrogen, hydroxy, alkoxy having one to four carbon atoms and chlorine; R is H or alkyl of from one to six carbon atoms; Y is H, alkyl of from one to six carbon atoms which may be substituted with an $OR_1$ group wherein $R_1$ is H or alkyl of from one to six carbon atoms, or $ANR_2R_3$ wherein A is straight or branched alkylene of from two to eight carbon atoms, $R_2$ and $R_3$ may be the same or different and are H, alkyl of from one to six carbon atoms which may be substituted with OH or an NRaRa wherein Ra may be the same or different and is H or alkyl of from one to three carbon atoms which may be substituted with OH, or $NR_bR_b$ wherein $R_b$ is the same or different and is H or alkyl of from one to three carbon atoms, or $R_2$ and $R_3$ when taken together may be ethylene or may form

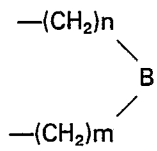

wherein n and m may be the same of different and are one, two, or three provided that the sum of n and m is an integer of from three to six, and B is a direct bond, O, S, or N—$R_4$ wherein $R_4$ is H or alkyl of from one to six carbon atoms; R and Y when taken together may be ethylene or may form

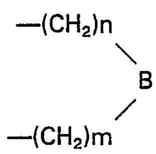

wherein n and m and B are defined above; Z is H, alkyl of from one to six carbon atoms which may be substituted with an $N(R_1)_2$, $SR_1$, or $OR_1$ group wherein $R_1$ is the same or different and is defined above, or $DNR_2R_3$ wherein D is straight or branched alkylene of from two to eight carbon atoms which may be substituted with an OH group and $R_2$ and $R_3$ are as defined above; and the pharmaceutically acceptable salts thereof; with the following provisos, 1) when X, X' and W are H and Z is H, R and Y when taken together do not complete a piperdine ring, 2) when X, X', and W are H and Z is $CH_3$, R and Y when taken together do not complete a piperidine ring or a morpholine ring, and 3) when both of R and Y are H, Z is neither H nor alkyl of from 1 to 6 carbon atoms.

The compounds excluded by proviso 3) can be used for the treatment of, or for the preparation of a medicament for the treatment of, microbial infections in a mammal, leukemia in a mammal, or solid tumors in a mammal.

The invention sought to be patented in its second generic chemical compound aspect is a compound having the structure formula

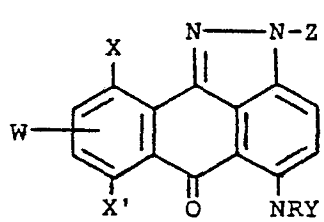

wherein x, X' and W may be the same or different and are H or OH, alkoxy having one to four carbon atoms or chlorine; R is H or alkyl of from one to six carbon atoms; Y is H, alkyl of from one to six carbon atoms which may be substituted with an $OR_1$ group wherein $R_1$ is H or alkyl of from one to six carbon atoms, or $ANR_2R_3$ wherein A is straight or branched alkylene of from two to eight carbon atoms, $R_2$ and $R_3$ may be the same or different and are H, alkyl of from one to six carbon atoms which may be substituted with OH or an NRaRa wherein Ra may be the same or different and is H or alkyl of from one to three carbon atoms which may be substituted with OH, or $NR_bR_b$ wherein $R_b$ is the same or different and is H or alkyl of from one to three carbon atoms, or $R_2$ and $R_3$ when taken together may be ethylene or may form

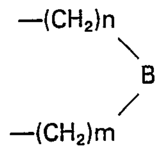

wherein n and m may be the same or different and are one, two, or three provided that the sum of n and m is an integer of from three to six, and B is a direct bond, O, S, or N—$R_4$ wherein $R_4$ is H or alkyl of from one to six carbon atoms; R and Y when taken together may be ethylene or may form

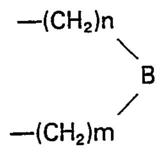

wherein n, m, and B are defined above; Z is H, alkyl of from one to six carbon atoms which may be substituted with an $N(R_1)_2$, $SR_1$, or $OR_1$ group wherein $R_1$ is the same or different and is defined above, or $DNR_2R_3$ wherein D is straight or branched alkylene of from two to eight carbon atoms which may be substituted with an OH group and $R_2$ and $R_3$ are as defined above; and the pharmaceutically acceptable salts thereof; with the following provisos, 1) when X, X' and W are H and Z is H, R and Y when taken together do not complete a piperidine ring, 2) when X, X' and W are H and Z is $CH_3$, R and Y when taken together do not complete a piperidine ring or a morpholine ring, and 3) when both of R and Y are H, Z is neither H nor alkyl having from 1 to 6 carbon atoms.

The invention sought to be patented in its third generic chemical compound aspect is a compound having the structural formula

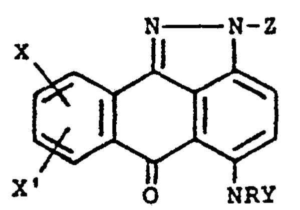

wherein X and X' may be the same or different and are H, OH, alkoxy of one to four carbon atoms or chlorine; R is H or alkyl of from one to six carbon atoms; Y is H, alkyl of from one to six carbon atoms which may be substituted with an $OR_1$ group wherein $R_1$ is H or alkyl of from one to six carbon atoms, or $ANR_2R_3$ wherein A is alkylene of from two to 11 carbon atoms, $R_2$ and $R_3$ may be the same or different and are H, alkyl of from one to six carbon atoms which may be substituted with OH or an NRaRa wherein Ra is H or alkyl of from one to three carbon atoms which may be substituted with OH, or $NR_bR_b$ wherein $R_b$ is the same of different and is H or alkyl of from one to three carbon atoms, or $R_2$ and $R_3$ when taken together may be ethylene or may form

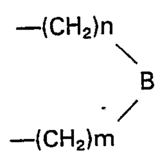

wherein n and m are the same or different and are 1, 2 or 3 provided that the sum of n and m is an integer of from 3 to 6, and B is a direct bond, O, S, or N—$R_4$ wherein $R_4$ is H or alkyl of from 1 to 6 carbon atoms; R and Y when taken together may be ethylene or may form

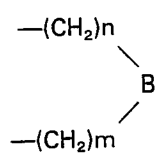

wherein n, m, and B are defined above; Z is H, alkyl of from one to six carbon atoms which may be substituted with an $N(R_1)_2$, $SR_1$, or $OR_1$ group wherein $R_1$ is defined above, or $DNR_2R_3$ wherein D is alkylene of from two to 11 carbon atoms which may be substituted with an OH group and $R_2$ and $R_3$ are as defined above; and the pharmaceutically acceptable salts thereof; with the following provisos, 1) when X is H and Z is H, R and Y when taken together do not complete a piperidine ring, 2) when X is H and Z is $CH_3$, R and Y when taken together do not complete a piperidine ring or a morpholine ring, and 3) when both of R and Y are H, Z is neither H nor alkyl of from 1 to 6 carbon atoms.

The invention in its fourth generic chemical compound aspect is a compound having the structural formula I

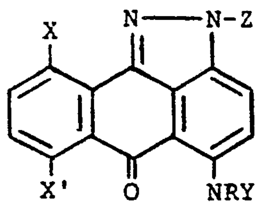

(I)

wherein X and X′ may be the same or different and are H or OH; r is H or alkyl of from one to six carbon atoms; Y is H, alkyl of from one to six carbon atoms which may be substituted with an $OR_1$ group wherein $R_1$ is H or alkyl of from one to six carbon atoms, or $ANR_2R_3$ wherein A is straight or branched alkylene of from two to eight carbon atoms, $R_2$ and $R_3$ may be the same or different and are H, alkyl of from one to six carbon atoms which may be substituted with OH or an NRaRa wherein Ra is the same or different and as H or alkyl of from one to three carbon atoms which may be substituted with OH, or $NR_bR_b$ wherein $R_b$ is the same or different and is H or alkyl of from one to three carbon atoms, or $R_2$ and $R_3$ when taken together may be ethylene or may form

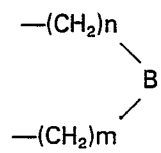

wherein n and m are the same or different and are one, two or three, provided that the sum of n and m is an integer of from three to six, and B is a direct bond, O, S, or N—$R_4$ wherein $R_4$ is H or alkyl of from one to six carbon atoms; R and Y when taken together may be ethylene or may form

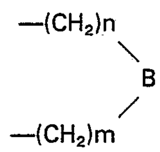 .

wherein n, m, and B are defined above; Z is H, alkyl of from one to six carbon atoms which may be substituted with an $N(R_1)_2$, $SR_1$, or $OR_1$ group wherein $R_1$ is the same of different and is as defined above, or $DNR_2R_3$ wherein D is straight or branched alkylene of from two to eight carbon atoms which may be substituted with an OH group and $R_2$ and $R_3$ are as defined above; and the pharmaceutically acceptable salts thereof; with the following provisos, 1) when X and X′ are H and Z is H, R and Y when taken together do not complete a piperidine ring, 2) when X and X′ are H and Z is $CH_3$, R and Y when taken together do not complete a piperidine ring or a morpholine ring, and 3) when both or R and Y are H, Z is neither H nor alkyl of from 1 to 6 carbon atoms.

The invention in a first subgeneric aspect of its fourth chemical compound aspect is is a chemical compound having structural formula I wherein X and X′ are OH; and the pharmaceutically acceptable salts thereof.

The invention is a second subgeneric aspect of its fourth chemical compound aspect is a chemical compound having structural formula I wherein X and X′ are H; and the pharmaceutically acceptable salts thereof.

The invention is a third subgeneric aspect of its fourth chemical compound aspect is a chemical compound having structural formula I wherein A and D are the same or different and are ethylene or propylene; and the pharmaceutically acceptable salts thereof.

The invention in a fourth subgeneric aspect of its fourth chemical compound aspect is a compound having structural formula I′

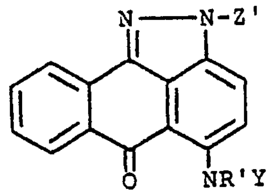

(I′)

wherein R′ is H or alkyl of from 1 to 6 carbon atoms; Y′ is $CH_2CH_2NHCH_2CH_2OH$ when Z′ is alkyl of from one to four carbon atoms which may be substituted with an $SR_1'$ or $OR_1'$ group wherein $R_1'$ is H or alkyl of from one to four carbon atoms or $D'NR_2'R_3'$ wherein D′ is straight or branched alkylene of from two to four carbon atoms which may be substituted with an OH group and $R_2'$ and $R_3'$ may be the same or different and are H, alkyl of from one to six carbon atoms which may be substituted with an OH or $R_2'$ and $R_3'$ when taken together may be ethylene of may form

$$\begin{matrix} -(CH_2)n' & \\ & \diagdown \\ & B' \\ & \diagup \\ -(CH_2)m' & \end{matrix}$$

wherein n′ and m′ may be the same of different and are one or two provided that the sum of n′ and m′ is three or four and B′ is a direct bond, O, S, or N—$R_4'$ wherein $R_4'$ is H or alkyl of from one to four carbon atoms; or Z′ is $CH_2CH_2NHCH_2CH_2OH$ when Y′ is H, alkyl of from one to six carbon atoms which may be substituted with an $OR_1'$ group wherein $R_1'$ is defined above or $A'NR_1'R_2'$ wherein A′ is alkylene of from two to four carbon atoms and $R_1'$ and $R_2'$ are as defined above; and the pharmaceutically acceptable salts thereof.

The invention in a fifth subgeneraic aspect of its fourth chemical compound aspect is a compound having structural formula I″

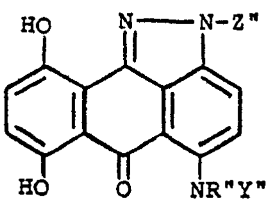

(I″)

wherein R″ is H or alkyl of from one to six carbon atoms; Y″ is H, alkyl of from one to six carbon atoms which may be substituted with an $OR_1''$ group wherin $R_1''$ is H or alkyl of from one to four carbon atoms, or $A''NR_2''R_2''$ wherein A″ is alkylene of from two to four carbon atoms, $R_2''$ and $R_3''$ may be the same or different and are H, alkyl of from one to six carbon atoms which may be substituted with an OH or an NRa″Ra″ wherein Ra″ is the same or different and is H or alkyl of from one to three carbon atoms which may be substituted with an OH or $R_2''$ and $R_3''$ when taken together may be ethylene or may form

$$\begin{matrix} -(CH_2)n'' & \\ & \diagdown \\ & B'' \\ & \diagup \\ -(CH_2)m'' & \end{matrix}$$

wherein n″ and m″ may be the same or different and are one or two provided that the sum of n″ and m″ is three or four, and B″ is a direct bond, O, S, or N—$R_4''$ wherein $R_4''$ is H or alkyl of from one to four carbon atoms; Z″ is alkyl of from one to four carbon atoms which may be substituted with an $SR_1''$, or $OR_1''$ group wherein $R_1''$ is defined above, or $D''NR_2''R_3''$ wherein D″ is alkylene of from two to four carbon atoms which may be substituted with an OH group and $R_2''$ and $R_3''$ are as defined above; and the pharmaceutically acceptable salts thereof.

The invention in a sixth subgeneric aspect of its second chemical compound aspect is a compound having the structural formula I‴

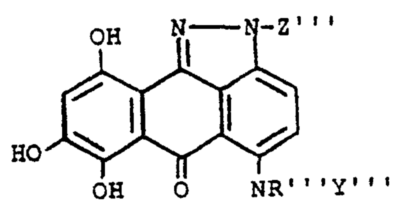

(I‴)

wherein $R'''$ is H or alkyl of from one to six carbon atoms; $Y'''$ is H, alkyl of from one to six carbon atoms which may be substituted with an $OR_1'''$ group wherein $R_1'''$ is H or alkyl of from one to four carbon atoms, or $A'''NR_2'''R_3'''$ wherein $A'''$ is alkylene of from two to four carbon atoms, $R_2'''$ and $R_3'''$ may be the same or different and are H, alkyl of from one to six carbon atoms which may be substituted with an OH or an $NRa'''Ra'''$ wherein $Ra'''$ is the same or different and is H or alkyl of from one to three carbon atoms which may be substituted with an OH or $R_2'''$ when taken together may be ethylene or may form

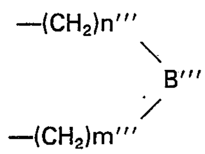

wherein $n'''$ and $m'''$ be the same or different and are one or two provided that the sum of $n'''$ and $m'''$ is three or four, and $B'''$ is a direct bond, O, S, or $N—R_4'''$ wherein $R_4'''$ is H or alkyl of from one to four carbon atoms; $Z'''$ is alkyl of from one to four carbon atoms which may be substituted with an $SR_1'''$ or $OR_1'''$ group wherein $R_1'''$ is defined above, or $D'''NR_2'''R_3'''$ wherein $D'''$ is alkylene of from two to four carbon atoms which may be substituted with an OH group and $R_2'''$ and $R_3'''$ are as defined above; and the pharmaceutically acceptable salts thereof.

The invention is a seventh subgeneric aspect of its fourth chemical compound aspect is a compound having the structural formula $I''''$

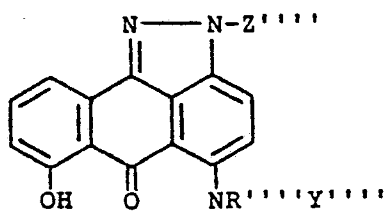

$(I'''')$

wherein $R''''$ is H or alkyl of from one to six carbon atoms; $Y''''$ is H, alkyl of from one to six carbon atoms which may be substituted with an $OR_1''''$ group wherein $R_1''''$ is H or alkyl of from one to four carbon atoms, or $A''''NR_2''''R_3''''$ wherein $A''''$ is alkylene of from two to four carbon atoms, $R_2''''$ and $R_3''''$ may be the same or different and are H, alkyl of from one to six carbon atoms which may be substituted with an OH or an $NRa''''Ra''''$ wherein $Ra''''$ is the same or different and is H or alkyl of from one to three carbon atoms which may be substituted with an OH or $R_2''''$ and $R_3''''$ when taken together may be ethylene or may form

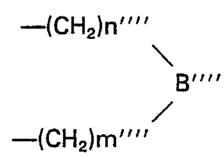

wherein $n''''$ and $m''''$ may be the same of different and are one or two provided that the sum of $n''''$ and $m''''$ is three or four, and $B''''$ is a direct bond, O, s, or $R—R_4''''$ wherein $R_4''''$ is H or alkyl of from one to four carbon atoms; $Z''''$ is alkyl of from one to four carbon atoms which may be substituted with an $SR_1''''$, or $OR_1''''$ group wherein $R_1''''$ is defined above, or $D''''NR_2''''R_3''''$ wherein $D''''$ is alkylene of from two to four carbon atoms which may be substituted with an OH group and $R_2''''$ and $R_3''''$ are as defined above; and the pharmaceutically acceptable salts thereof.

The invention in an eighth subgeneric aspect of its fourth chemical compound aspect is a compound having the structural formula $I^V$

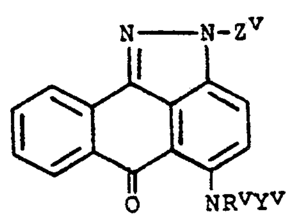

$(I^V)$

wherein $R^V$ is H or alkyl of from one to six carbon atoms; $Y^V$ is H, alkyl of from one to four carbon atoms or $A^VNR_2^VR_3^V$ wherein $A^V$ is alkylene of from two to four carbon atoms, $R_2$ and $R_3$ may be the same of different and are H or alkyl of from one to six carbon atoms which may be substituted with an OH or $R_2^V$ and $R_3^V$ when taken together may be ethylene or may form

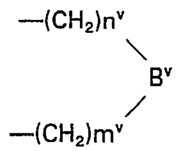

wherein $n^v$ and $m^v$ may be the same or different and are one or two provided that the sum of $n^v$ and $m^v$ is three or four, and $B^v$ is a direct bond, O, S, or $NR_4{}^v$ wherein $R_4{}^v$ is H or alkyl of from one to four carbon atoms; $Z^v$ is alkyl of from one to four carbon atoms, which may be substituted with an $SR_1{}^v$, or $OR_1{}^v$ group wherein $R_1{}^v$ is H or alkyl of from one to four carbon atoms, or $D^vNR_2{}^vR_3{}^v$ wherein $D^v$ is alkylene of from two to four carbon atoms which may be substituted with an OH group and $R_2{}^v$ and $R_3{}^v$ are defined above; and the pharmaceutically acceptable salts thereof.

The invention is a ninth subgeneric aspect of its fourth chemical compound aspect is a compound having the structural formula $I^{vi}$

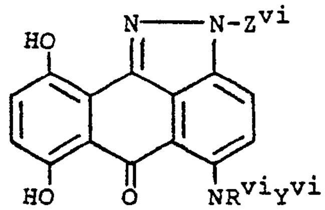

($I^{vi}$)

wherein $R^{vi}$ is H or alkyl of from one to six carbon atoms; $Y^{vi}$ is H, alkyl of from one to four carbon atoms which may be substituted with an $OR_1{}^{vi}$ group wherein $R_1{}^{vi}$ is H or alkyl of from one to four carbon atoms or $A^{vi}NR_2{}^{vi}R_3{}^{vi}$ wherein $A^{vi}$ is alkylene of from two to four carbon atoms $R_2{}^{vi}$ and $R_3{}^{vi}$ may be the same or different and are alkyl of from one to six carbon atoms which may be substituted with an OH, or $R_2{}^{vi}$ and $R_3{}^{vi}$ when taken together may be ethylene or may form

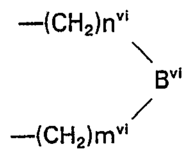

wherein $n^{vi}$ and $m^{vi}$ may be the same or different and are one or two provided that the sum of $n^{vi}$ and $m^{vi}$ is three or four, and $B^{vi}$ is a direct bond, O, S, or $NR_4{}^{vi}$ wherein $R_4{}^{vi}$ is H or alkyl of from one to four carbon atoms; $Z^{vi}$ is $D^{vi}NR_2{}^{vi}R_3{}^{vi}$ wherein $D^{vi}$ is alkylene of from two to four carbon atoms, $R_2{}^{vi}$ and $R_3{}^{vi}$ are defined above; and the pharmaceutically acceptable salts thereof.

The invention as species of the first generic chemical compound aspect of the invention are the chemical compounds having the following names:

·2-[2-(diethylamino)ethyl)]-5-[[2-[(2-hydroxyethyl)amino]ethyl]amino]anthra[1,9-*cd*]pyrazol-6(2*H*-one;

2-[2-[(2-hydroxyethyl)amino]ethyl]-5-[[2-[(2-hydroxyethyl)amino]ethyl]amino]anthra[1,9-*cd*]pyrazol-6(2*H*)-one;

5-[(2-aminoethyl)amino]-2-[2-[(2-hydroxyethyl)amino]ethyl]amino]anthra[1,9-*cd*]pyrazol-6(2*H*)-one;

2-[2-(diethylamino)ethyl]-7,10-dihydroxy-5-[[2-[(2-hydroxyethyl)amino]ethyl]amino]anthra[1,9-*cd*]-pyrazol-6(2*H*)-one;

5-[[2-[(2-hydroxyethyl)amino]ethyl]amino]-7,10-dihydroxy-2-(2-hydroxyethyl)anthra[1,9-*cd*]pyrazol-6(2*H*)-one;

2-[2-[[2-(dimethylamino)ethyl)amino]ethyl]-5-[[2-[(2-hydroxyethyl)amino)ethyl)amino]-7,10-dihydroxyanthra[1,9-*cd*]pyrazol-6(2*H*)-one;

2-[2-(diethylamino)ethyl]-[[2-(4-morpholinyl)ethyl)]amino]anthra[1,9-*cd*]pyrazol-6(2*H*)-one;

2-(2-aminoethyl)-5-[[2-[(2-hydroxyethyl)amino)ethyl)amino]anthra[1,9-*cd*]pyrazol-6(2*H*)-one;

5-[(2-aminoethyl)amino]-2-[2-(diethylamino)ethyl]-7,10-dihydroxyanthra[1,9-*cd*]pyrazol-6(2*H*)-one;

2-[2-(diethylamino)ethyl]-7,10-dihydroxy-5-[[2-[(methylamino)ethyl)amino]anthra[1,9-*cd*]pyrazol-6(2*H*)-one;

2-[2-(dimethylamino)ethyl]-7,10-dihydroxy-5-[[2-[(2-hydroxyethyl)amino)ethyl)amino]anthra[1,9-*cd*]-pyrazol-6(2*H*)-one;

5-[(2-aminoethyl)amino]-2-[2-(dimethylamino)ethyl]7,10-dihydroxyanthra[1,9-*cd*]pyrazol-6(2*H*)-one;

5-[(3-aminopropyl)amino]-2-[2-(dimethylamino)ethyl]-7,10-dihydroanthra[1,9-*cd*]pyrazol-6(2*H*)-one;

5-[(2-aminoethyl)amino]-7,10-dihydroxy-2-(2-hydroxyethyl)anthra[1,9-*cd*]pyrazol-6(2*H*)-one;

5-[[2-(dimethylamino)ethyl]amino]-7,10-dihydroxy-2-(2-hydroxyethyl)anthra[1,9-*cd*]pyrazol-6(2*H*)-one;

2-[3-(diethylamino)-2-hydroxypropyl]-7,10-dihydroxy-5[[2-[(2-hydroxyethyl)amino]ethyl]amino]-anthra[1,9-*cd*]pyrazol-6(2*H*)-one;

5-[(2-aminoethyl)amino]-2-[3-(diethylamino)-2-hydroxypropyl]-7,10-dihydroxyanthra[1,9-*cd*]pyrazol-6(2*H*)-one;

2-[3-(dimethylamino)propyl]-7,10-dihydroxy-5-[[2-[(2-hydroxyethyl)amino]ethyl]amino]anthra[1,9-*cd*]-pyrazol-6(2*H*)-one;

7,10-dihydroxy-5-[[2-[(2-hydroxyethyl)amino]ethyl]amino]-2-[2-[(2-hydroxyethyl)methylamino]ethyl]-anthra[1,9-*cd*]pyrazol-6(2*H*)-one;

7,10-dihydroxy-5-[[2-[(2-hydroxyethyl)amino]ethyl]amino]-2-[3-[(2-hydroxyethyl)amino]propyl]anthra-[1,9-*cd*]pyrazol-6(2*H*)-one;

5-[(2-aminoethyl)amino]-7,10-dihydroxy-2-[2-[(2-hydroxyethyl)amino]ethyl]anthra[1,9-*cd*]pyrazol-6(2*H*)-one;

5-[[2-(dimethylamino]ethyl]amino]-7,10-dihydroxy-2[2-[(2-hydroxyethyl)amino]ethyl]anthra[1,9-*cd*]-pyrazol-6(2*H*)-one;

5-[[2-(diethylamino)ethyl]amino]-7,10-dihydroxy-2-[2-[(2-hydroxyethyl)amino]ethyl]anthra[1,9-*cd*]-pyrazol-6(2*H*)-one;

5-[(3-aminopropyl)amino]-7,10-dihydroxy-2-[2-[(2-hydroxyethyl)amino]ethyl]anthra[1,9-*cd*]pyrazol-6(2*H*)-one;

7,10-dihydroxy-2-[2-[(2-hydroxyethyl)amino]ethyl]-5-[[3-[(2-hydroxyethyl)amino]propyl]amino]anthra-[1,9-*cd*]pyrazol-6(2*H*)-one;

5-[[2-[[2-(dimethylaminoethyl]amino]ethyl]amino]-7,10-dihydroxy-2-[2-[(2-hydroxyethyl)amino]ethyl]-anthra[1,9-*cd*]pyrazol-6(2*H*)-one;

5-[[2-[(2-aminoethyl)amino]ethyl]amino]-7,10-dihydroxy-2-[2-[(2-hydroxyethyl)amino]ethyl]anthra-[1,9-*cd*]pyrazol-6(2*H*)-one;

5-[[2-[bis(2-hydroxyethyl)amino]ethyl]amino]-7,10-dihydroxy-2-[2-[(2-hydroxyethyl)amino]ethyl]-anthra[1,9-*cd*]pyrazol-6(2*H*)-one;

7,10-dihydroxy-2-[2-[(2-hydroxyethyl)amino]ethyl-5-[[2-(methylamino)ethyl]amino]anthra[1,9-*cd*]-pyrazol-6(2*H*)-one;

2-(2-aminoethyl)-7,10-dihydroxy-5-[[2-[(2-hydroxyethyl)amino]ethyl]amino]anthra[1,9-*cd*]pyrazol-6(2*H*)-one;

2-(2-aminoethyl)-5-[(2-aminoethyl)amino]-7,10-dihydroxyanthra[1,9-*cd*]pyrazol-6(2*H*)-one;

2-[2-aminoethyl)-5-[[2-[[2-(dimethylamino)ethyl]amino]ethyl)amino]-7,10-dihydroxyanthra[1,9-*cd*]-pyrazol-6(2*H*)-one;

2-(2-aminoethyl)-5-[[3-[(2-hydroxyethyl)amino]propyl]amino]-7,10-dihydroxy anthra[1,9-*cd*]pyrazol-6(2*H*)-one;

2-(2,3-dihydroxypropyl)-7,10-dihydroxy-5-[[2-[(2-hydroxyethyl)amino]ethyl)amino]anthra[1,9-*cd*]-pyrazol-6(2*H*)-one;

7-hydroxy-2-[2-[(2-hydroxyethyl)amino]ethyl]-5-[[2-[(2-hydroxyethyl)amino]ethyl)amino]anthra-[1,9-*cd*]pyrazol-6(2*H*)-one;

7-hydroxy-2-[2-[(2-hydroxyethyl)amino]ethyl)-5-[[2-(methylamino)ethyl]amino]anthra[1,9-*cd*]pyrazol-6(2*H*)-one;

10-Hydroxy-2-[2-[(2-hydroxyethyl)amino]ethyl]-5-[[2-[(2-hydroxyethyl)amino]ethyl)amino]anthra-[1,9-*cd*]pyrazol-6(2*H*)-one;

7,8,10-trihydroxy-2-[2-[(2-hydroxyethyl)amino]ethyl]5-[[2-[(2-hydroxyethyl)amino]ethyl)amino]anthra-[1,9-*cd*]pyrazol-6(2*H*)-one;

7,8,10-trihydroxy-2-[2-[(2-hydroxyethyl)amino)]ethyl]-5-[[2-(methylamino)ethyl]amino]anthra[1,9-*cd*]-pyrazol-6(2*H*)-one;

5-[[2-[(2-aminoethyl)amino]ethyl]amino]-7,10-dihydroxy-2-(2-hydroxyethyl)anthra[1,9-*cd*]pyrazol-6(2*H*)-one;

2-(3-aminopropyl)-7,10-dihydroxy-5-[[2-[(2-hydroxyethyl)amino]ethyl]amino]anthra[1,9-*cd*]pyrazol-6(2*H*)-one;

2-(3-aminopropyl)-5-[[2-[[2-(dimethylamino)ethyl]amino]ethyl]amino]-7,10-dihydroxy anthra[1,9-*cd*]-pyrazol-6(2*H*)-one;

2-(2-aminoethyl)-7,10-dihydroxy-5-[[2-(methylamino)ethyl]amino]anthra[1,9-*cd*]pyrazol-6(2*H*)-one;

5-[(2-aminoethyl)amino]-2-[3-(dimethylamino)propyl]-7,10-dihydroxyanthra[1,9-*cd*]pyrazol-6(2*H*)-one;

7,8-dihydroxy-2-[2-[(2-hydroxyethyl)amino]ethyl]-5-[[2-[(2-hydroxyethyl)amino)ethyl]amino]anthra-[1,9-*cd*]pyrazol-6(2*H*)-one; and

the pharmaceutically acceptable salts thereof.

The invention is its fifth generic chemical compound aspect is a chemical compound having the structural formula

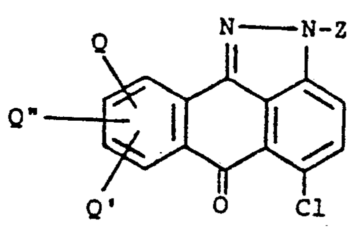

wherein Q, Q', and Q'' may be the same or different and are hydrogen, OH, alkoxy of one to four carbon atoms, chlorine, benzyloxy, *p*-chlorobenzyloxy and *p*-methoxybenzyloxy; and the pharmaceutically acceptable salts thereof; Z is defined above; and the pharmaceutically acceptable salts thereof; provided that when Q = Q' = Q'' = H, Z may not be H or $CH_3$.

The invention in its sixth generic chemical compound aspect is a chemical compound having the structural formula III

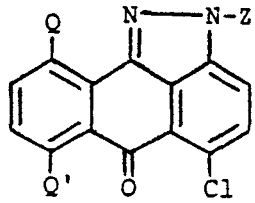

(III)

wherein Q and Q' may be the same or different and are H, OH, benzyloxy, *p*-chlorobenzyloxy, or *p*-methoxybenzyloxy and Z is defined above; and the pharmaceutically acceptable salts thereof; provided that when Q = Q' = H, Z may not be H or $CH_3$.

The invention is a first subgeneric aspect of its sixth chemical compound aspect is a chemical compound having structural formula III wherein Q and Q' are H;
and the pharmaceutically acceptable salts thereof.

The invention in a second subgeneric aspect of its sixth chemical compound aspect is a chemical compound having the structural formula III wherein Q and Q' are benzyloxy, *p*-chlorobenzyloxy, or *p*-methoxybenzyloxy; and the pharmaceutically acceptable salts thereof.

The invention is a third subgeneric aspect of its sixth chemical compound aspect is a chemical compound having structural formula III wherein Q and Q' are OH;
and the pharmaceutically acceptable salts thereof.

The invention as species of the fifth generic chemical compound aspect of the invention are the chemical compounds having the following names:

5-chloro-2-[2-(diethylamino)ethyl]anthra[1,9-*cd*]pyrazol-6(2*H*)-one;
5-chloro-2-[2-[(2-hydroxyethyl)amino]ethyl]anthra[1,9-*cd*]pyrazol-6(2*H*)-one;
5-chloro-2-[2-(diethylamino)ethyl]-7,10-dihydroxyanthra[1,9-*cd*]pyrazol-6(2*H*)-one;
5-chloro-7,10-dihydroxy-2-(2-hydroxyethyl)anthra[1,9-*cd*]pyrazol-6(2*H*)-one;
5-chloro-2-[2-(diethylamino)ethyl]-7,10-bis(phenylmethoxy)anthra[1,9-*cd*]pyrazol-6(2*H*)-one;
5-chloro-2-[2-[2-[[(4-methylphenyl)sulfonyl]oxy]ethyl]-7,10-bis(phenylmethoxy)anthra[1,9-*cd*]pyrazol-6(2*H*)-one;
5-chloro-7,10-dihydroxy-2-[2-[(2-hydroxyethyl)amino]ethyl]anthra[1,9-*cd*]pyrazol-6(2*H*)-one;
5-chloro-2-[2-[(2-hydroxyethyl)amino]ethyl]-7,10-bis(phenylmethoxy)anthra[1,9-*cd*]pyrazol-6(2*H*)-one;
5-chloro-2-[2-[[2-(dimethylamino)ethyl]amino]ethyl]-7,10-dihydroxyanthra[1,9-*cd*]pyrazol-6(2*H*)-one;
2-(2-aminoethyl)-5-chloroanthra[1,9-*cd*]pyrazol-6(2*H*)-one;
5-chloro-2-[2-(dimethylamino)ethyl]-7,10-dihydroxyanthra[1,9-*cd*]pyrazol-6(2*H*)-one;
5-chloro-2-[3-(diethylamino)-2-hydroxypropyl]-7,10-dihydroxyanthra[1,9-*cd*]pyrazol-6(2*H*)-one;
5-chloro-2-[3-(dimethylamino)propyl]-7,10-dihydroxyanthra[1,9-*cd*]pyrazol-6(2*H*)-one;
5-chloro-2-(2-hydroxyethyl)-7,10-bis(phenylmethoxy)anthra[1,9-*cd*]pyrazol-6(2*H*)-one;
5-chloro-7,10,dihydroxy-2-[2-[(2-hydroxyethyl)methylamino]ethyl]anthra[1,9-*cd*]pyrazol-6(2*H*)-one;
5-chloro-2-[2-[(2-hydroxyethyl)methylamino]ethyl]7,10-bis(phenylmethoxy)anthra[1,9-*cd*]pyrazol-6(2*H*)-one;
5-chloro-7,10-dihydroxy-2-[3-[(2-hydroxyethyl)amino]propyl]anthra[1,9-*cd*]pyrazol-6(2*H*)-one;
5-chloro-2-[3-[(2-hydroxyethyl)amino]propyl]7,10-bis(phenylmethoxy)anthra[1,9-*cd*]pyrazol-6(2*H*)-one;
5-chloro-2-[3-[[4-methylphenyl)sulfonyl]oxy]propyl]-7,10-bis(phenylmethoxy)anthra[1,9-*cd*]pyrazol-6(2*H*)-one;
5-chloro-2-(3-hydroxypropyl)-7,10-bis(phenylmethoxy)anthra[1,9-*cd*]pyrazol-6(2*H*)-one;
2-(2-aminoethyl)-5-chloro-7,10-dihydroxyanthra[1,9-*cd*]pyrazol-6(2*H*)-one;
2-(2-aminoethyl)-5-chloro-7,10-bis(phenylmethoxy)-anthra[1,9-*cd*]pyrazol-6(2*H*)-one;
5-chloro-2-[(2,2-dimethyl-1,3-dioxolan-4-yl)-methyl]-7,10-bis(phenylmethoxy)anthra[1,9-*cd*]pyrazol-6(2*H*)-one;
5-chloro-2-[2-[(2-hydroxyethyl)amino]ethyl]-7-(phenylmethoxy)anthra[1,9-*cd*]pyrazol-6(2*H*)-one;
5-chloro-2-[2-[(2-hydroxyethyl)amino]ethyl]-10-(phenylmethoxy)anthra[1,9-*cd*]pyrazol-6(2*H*)-one;
5-chloro-2-[2-[(2-hydroxyethyl)amino]ethyl]7,8,10-tris(phenylmethoxy)anthra[1,9-*cd*]pyrazol-6(2*H*)-one;
2-(3-aminopropyl)-5-chloro-7,10-bis(phenylmethoxy)-anthra[1,9-*cd*]pyrazol-6(2*H*)-one;
5-chloro-2-[2-[(2-hydroxyethyl)amino]ethyl]-7,8-bis(phenylmethoxy)anthra[1,9-*cd*]pyrazol-6(2*H*)-one;
5-chloro-2-[2-[[2-(dimethylamino)ethyl]amino]ethyl]7,10-bis(phenylmethoxy)anthra[1,9-*cd*]pyrazol-6(2*H*)-one;

and the pharmaceutically acceptable salts thereof.

The invention in its seventh generic chemical compound aspect is a compound having the structural formula IV

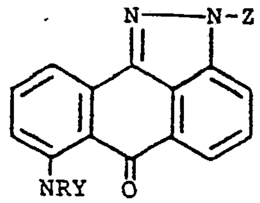

(IV)

wherein R is H or alkyl of from one to six carbon atoms; Y is H, alkyl of from one to six carbon atoms which may be substituted with an $OR_1$ group wherein $R_1$ is H or alkyl of from one to six carbon atoms, or $ANR_2R_3$ wherein A is alkylene of from two to eight carbon atoms, $R_2$ and $R_3$ may be the same or different and are H, alkyl of from one to six carbon atoms which may be substituted with OH or an NRaRa wherein Ra may be the same or different and is H or alkyl of from one to three carbon atoms which may be substituted with OH, or $R_2$ and $R_3$ when taken together may be ethylene or may form

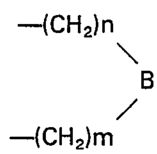

wherein n and m may be the same of different and are one, two, or three provided that the sum of n and m is an integer of from three to six, and B is a direct bond, O, S, or N—$R_4$ wherein $R_4$ is H or alkyl of from one to six carbon atoms; R and Y when taken together may be ethylene or may form

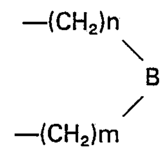

wherein n and m and B are defined above; Z is H, alkyl of from one to six carbon atoms which may be substituted with an $N(R_1)_2$, $SR_1$, or $OR_1$ group wherein $R_1$ is the same or different and is defined above, or $DNR_2R_3$ wherein D is alkylene of from two to eight carbon atoms which may be substituted with an OH group and $R_2$ and $R_3$ are as defined above; and the pharmaceutically acceptable salts thereof; with the following provisos, 1) when Z is H, R and Y when taken together do not complete a piperidine ring, 2) when Z is $CH_3$, R and Y when taken together do not complete a piperidine ring or a morpholine ring, and 3) when both of R and Y are H, Z is neither H nor alkyl of from one to six carbon atoms.

The compounds excluded by proviso 3) can be used for the treatment of, or for the preparation of a medicament for the treatment of, microbial infections in a mammal, leukemia in a mammal, or solid tumors in a mammal.

The invnetion as species of the seventh generic chemical compound aspect of the invention are the chemical compounds having the following names:

2-[2-(diethylamino)ethyl]-7-[[2-[(hydroxyethyl)amino)ethyl)amino]anthra[1,9-*cd*]pyrazol-6(2*H*)-one;

2-[2-(diethylamino)ethyl-7-[[2-(diethylamino)ethyl]amino]anthra[1,9-*cd*]pyrazol-6(2*H*)-one;

2-[2--[(2-hydroxyethyl)amino]ethyl]-7-[[2-[(2-hydroxyethyl)amino]ethyl]amino]anthra[1,9-*cd*]pyrazol-6(2*H*)-one;

and the pharmaceuctically acceptable salts thereof.

The invention is in its eighth generic chemical compound aspect is a compound having the structural formula VII

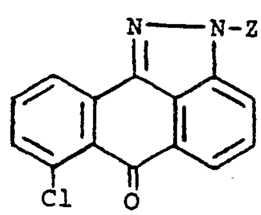

(VII)

wherein Z is defined above, provided it is not H or $CH_3$.

The invention as a species of the eighth generic chemical compound aspect of the invention is the chemical compound having the following names:

7-chloro-2-[2-(diethylamino)ethyl]anthra[1,9-*cd*]pyrazol-6(2*H*)-one;

7-chloro-2-[2-[(2-hydroxyethyl)amino]ethyl]anthra[1,9-*cd*]pyrazol-6(2*H*)-one;
and the pharmaceutically acceptable salts thereof.

The invention in its ninth chemical compound aspect is 5,8-dichloro-1,4,9,.10-anthracenetetrone.

The invention in its tenth chemical compound aspect is the compound 2-[(hydrazinoethyl)amino]-ethanol and the acid addition salts thereof.

The invention in its first generic chemical process aspect is a procedure for preparing a compound having the structural formula

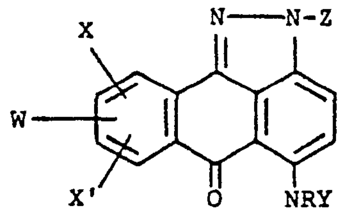

which comprises reacting a compound having the structural formula

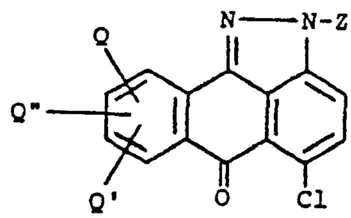

with an amine having the formula HNRY wherein W, X, X′, Q, Q′, Q″, Y, Z, and R arre defined above and, when necessary, removing by catalytic hydrogenation or by treatment with boron tribromide or trichloride any benzyl groups.

The invention in its second generic chemical process aspect is a process for preparing a compound having structural formula I

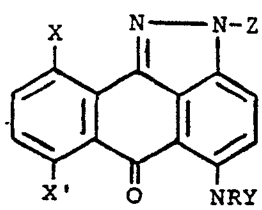

(I)

which comprises reacting a compound having structural formula II

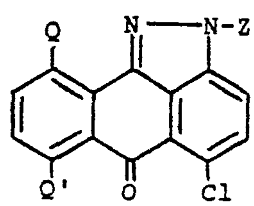

(II)

with an amine having the formula HNRY wherein X, X′, Q, Q′, Y, Z, and R are defined above and, when necessary, removing by catalytic hydrogenation or by treatment with boron tribromide or boron trichloride any benzyl groups.

The invention in a first subgeneric aspect of its second chemical process aspect is the process defined above wherein X and X′ are OH.

The invention in a second subgeneric aspect of its second chemical process aspect is the process defined above wherein X and X′ are H.

The invention in its third generic chemical process aspect is a process for preparing a compound having structural formula III

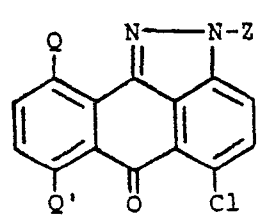

(III)

which comprises reacting a compound having formula V

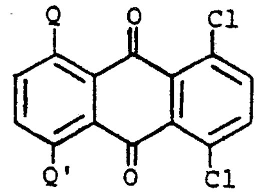

(V)

with a hydrazine having the formula $H_2N—NHZ$, wherein Q, Q′ and Z are defined above.

The invention in a first subgeneric aspect of its third chemical process aspect is the process defined abvoe wherein Q and Q′ are benzyloxy, *p*-chlorobenzyloxy, or *p*-methoxybenzyloxy.

The invention in a second subgeneric aspect of its third chemical process aspect is the process defined above wherein Q and Q′ are OH.

The invention in a third subgeneric aspect of its third chemical process aspect is the process defined above wherein Q and Q′ are H.

The invention in its fourth generic chemical process aspect is a process for preparing a compound having structural formula IV

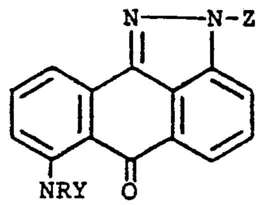

(IV)

which comprises reacting a compound having structural formula VII

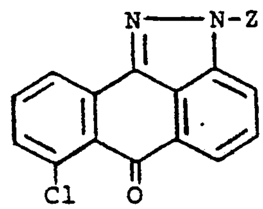

(VII)

with an amine having the formula HNRY, wherein Y, Z, and R are defined above.

The invention in its fifth chemical process aspect is a process for preparing 5,8-dichloro-1,4,9,10-anthracenetetrone which comprises reacting 1,4-dichloro-5,8-dihydroxy-9,10-anthracenedione with lead tetracetate.

The invention in its sixth chemical process aspect is a process for preparing 2-[(hydrazinoethyl)amino]ethanol which comprises reacting hydrazine with N-(2-hydroxyethyl)aziridine.

The invention in its first pharmaceutical composition aspect is a pharmaceutical composition comprising a compound having structural formula I and the pharmaceutically acceptable salt thereof in combination with a pharmaceutically acceptable carrier.

The invention in its second pharmaceutical composition aspect is a pharmaceutical composition comprising a compound having structural formula I′ and the pharmaceutically acceptable salts thereof in combination with a pharmaceutically acceptable carrier.

The invention in its third pharmaceutical composition aspect is a pharmaceutical composition comprising a compound having structural formula I″ and the pharmaceutically acceptable salts thereof in combination with a pharmaceutically acceptable carrier.

The invention in its fourth pharmaceutical composition aspect is a pharmaceutical composition comprising a compound having structural formula I‴ and the pharmaceutically acceptable salts thereof in combination with a pharmaceutically acceptable carrier.

The invention in its fifth pharmaceutical composition aspect is a pharmaceutical composition comprising a compound having structural formula I⁗ and the pharmaceutically acceptable salts thereof in combination with a pharmaceutically acceptable carrier.

The invention in its sixth pharmaceutical composition aspect is a pharmaceutical composition comprising a compound having structural formula $I^{v}$ and the pharmaceutically acceptable salts thereof in combination with a pharmaceutically acceptable carrier.

The invention in its seventh pharmaceutical composition aspect is a pharmaceutical composition comprising a compound having structural fomula $I^{vi}$ and the pharmaceutically acceptable salts thereof in combination with a pharmaceutically acceptable carrier.

The invention in its eighth pharmaceutical composition aspect is a pharmaceutical composition comprising a compound having structural formula IV and the pharmaceutically acceptable salts thereof in combination with a pharmaceutically acceptable carrier.

The invention in its first pharmaceutical use aspect relates to a compound having structural formula I and the pharmaceutically acceptable salts thereof for treating microbial infections in a mammal.

The invention in its second pharmaceutical use aspect relates to a compound having structural fomula I′ and the pharmaceutically acceptable salts therof for treating leukemia in a mammal.

The invention in its third pharmaceutical use aspects relates to a compound having structural formula I″ and the pharmaceutically acceptable salts thereof for treating leukemia in a mammal.

The invention in its fourth pharmaceutical use aspect relates to compound having structural formula I‴ and the pharmaceutically acceptable salts thereof for treating solid tumors in a mammal.

The invention in its fifth pharmaceutical use aspect relates to a compound having structural formula I⁗ and the pharmaceutically acceptable salts thereof for treating solid tumors in a mammal.

The invention in its sixth pharmaceutical use aspect relates to a compound having structural formula $I^{v}$ and the pharmaceutically acceptable salts thereof for treating solid tumors in a mammal.

The invention in its seventh pharmaceutical use aspect relates to a compound having structural formula $I^{vi}$ and the pharmaceutically acceptable salts thereof for treating solid tumors in a mammal.

The invention in its eighth pharmaceutical use aspect relates to a compound having structural formula IV and the pharmaceutically acceptable salts thereof for treating solid tumors in a mammal.

Description of the Preferred Embodiments

The compound of the invention may be prepared conveniently by the following reaction sequence

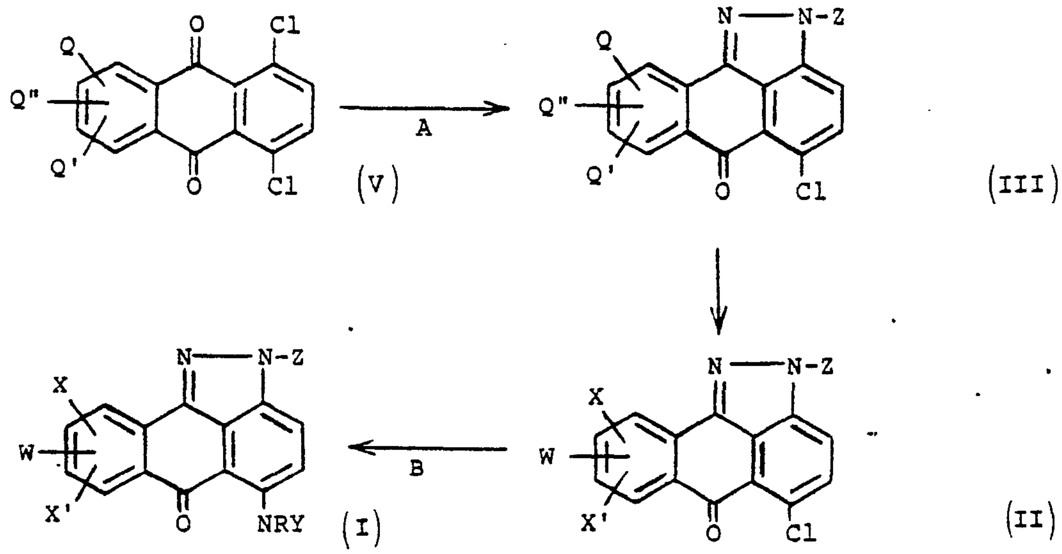


(V) (III) (II) (I)

The reaction step "A," involves the reaction of compound V and a suitably substituted hydrazine, $NH_2NHZ$ wherein Q, Q′ and Q″, and Z are defined hereinabove. This reaction may be accomplished in any of a variety of reaction inert solvents by mixing approximately equimolar amounts of compound V and the desired hydrazine in the chosen solvent at elevated temperature. Use of a catalyst such as potassium fluoride or of a slight molar excess of the hydrazine reactant may improve a particular yield. Examples of suitable solvents are *N,N*-dimethylformamide, dimethylsulfoxide, pyridine, acetonitrile or the cellosolves. Pyridine is the preferred solvent, suitable reaction temperatures are from about 30—85°C. In general, the reaction is allowed to proceed for about six to about 24 hours at which time the reaction is substantially complete. The completeness of a particular reaction may be measured by known procedures such as thin layer chromatography for example. It is generally observed that increasing the reaction temperature will decrease the time necessary for completing the reaction. The proper choice of the reaction variables is within the skill of the art. The products of the reaction are isolated and purified by standard procedures. For example, the reaction mixture may be concentrated by evaporating the solvent and the residue may be partitioned between water and a convenient nonwater-miscible organic solvent such as chloroform, benzene or dichloromethane. The solvent may then be evaporated and the residue may be chromatographed, for example, on silica gel. Choice of the proper chromatography solvent is within the skill of the art. After chromatography, the product may be recrystallized, if desired. When the Q, Q′ and Q″ substituents of the so produced compound III comprise benzyloxy, *p*-chlorobenzyloxy, or *p*-methoxybenzyloxy, the benzyl substituents may be removed, for example, by treatment with boron trichloride or boron tribromide in a chlorinated hydrocarbon solvent such as dichloromethane at about 0°C to produce compound II wherein the corresponding X substituents represent hydroxyl. Acid addition salts may also be prepared by standard procedures. For example, a hydrochloride salt may be prepared by dissolving the free base in a convenient solvent such as 2-propanol and treating this solution with a solution of hydrogen chloride in 2-propanol. The acid addition salts may be reconverted to the respective free base by treatment with a dilute solution of sodium hydroxide or potassium carbonate for example.

The reaction step "B" involves the reaction of compound II with a suitably substituted amine HNRY wherein R, W, X, X', Y' and Z are defined hereinabove. This reaction may be accomplished in any of a variety of reaction inert solvents by mixing approximately equimolar amounts of compound II and the desired amine in the chosen solvent at elevated temperature. The use of a slight molar excess of the amine reactant, an inert atmosphere and a catalyst such as anhydrous cuprous chloride may improve a particular yield. The use of these variations for a particular reaction is optional and is within the skill of the art. Examples of the suitable solvents are *N,N'*-dimethylformamide, dimethylsulfoxide, pyridine, acetonitrile or the cellosolves. Suitable reaction temperatures are from about 85—130°C. This reaction has been observed to proceed particularly efficiently in refluxing pyridine. In general, the reaction is allowed to proceed for about 6 to about 24 hours at which time it is substantially complete. The completeness of a particular reaction may be measured by known procedures such as thin layer chromatography for example. It is generally observed that increasing the reaction temperature will decrease the time necessary for completing the reaction. The proper choice of the reaction variables is within the skill of the art. The products of the reaction are isolated and purified by standard procedures which are substantially identicial to those described above for compound II. Likewise, acid addition salts of compound I may be prepared by standard procedures such as that described hereinabove for compound II.

Alternatively, the compound of formula III may be treated directly with an amine of formula HNRY to produce a compound of the formula

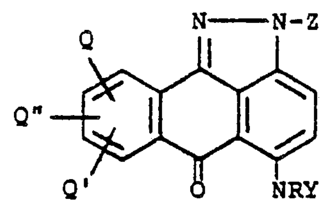

This compound may then be debenzylated by a standard procedure to produce the corresponding compound having structural formula I.

In an alternate process the compounds of formula I wherein X and X' are hydroxy may be prepared by the reaction of compound VI (compound V wherein Q and Q' are dihydroxy

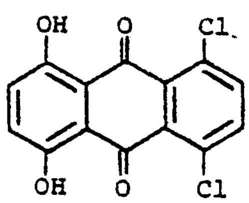

(VI)

with a suitably substituted hydrazine $NH_2NHZ$ to produce a compound of structural formula II wherein X is OH; Z is defined hereinabove. The reaction of VI and the hydrazine may be accomplished by mixing approximately equimolar amounts of the reactants in a solvent such as *N,N'*-dimethylformamide dimethylsulfoxide or pyridine at temperatures about 30—90°C preferably 30—60°C, in the presence of a base such as potassium bicarbonate. Pyridine is the preferred solvent and when utilized does not require an additional base. The use of catalyst such as potassium fluoride may improve the yield of a particular reaction. The subsequent conversion of the so produced compound II wherein X and X' are hydroxy to the corresponding compound I is carried out as already described hereinabove as reaction step "B".

In an alternate method for preparing the compounds of formula III, a compound of formula V is reacted with a hydroxyalkylhydrazine of the formula $NH_2—NH(CH_2)_{2-11}—OH$, preferably $NH_2—NH(CH_2)_{2-3}—OH$ to produce a compound of formula III wherein Z is $—(CH_2)_{2-11}—OH$ and is preferably $—(CH_2)_{2-3}—OH$. This reaction is carried out substantially as described hereinabove as reaction step "A". The OH group of the Z substituent is then derivatized to produce an easily displaceable substituent known to those skilled in the art as a "leaving group". For example, the OH group may be converted to a tosyloxy or mesyloxy group by reaction with respectively *p*-toluenesulphonylchloride or methanesulphonylchloride in pyridine by procedures known to those skilled in the art. The leaving group, so produced, may be subsequently displaced with, for example, an amine such as diethylamine to produce a Z substituent of the structure $—(CH_2)_{2-11}—NEt_2$. In the preferred procedure the substituent Z so produced is $—(CH_2)_3NEt_2$. The benzyl groups or substituted benzyl groups of compound III, if present, are removed as described above to produce a compound of formula II, which may be converted to a compound of formula I as already described hereinabove as rection step "B".

The compounds having structural formula I wherein X and X′ are chloro are prepared starting from compound VI by first converting VI to the corresponding di-*p*-toluenesulfonic acid ester VI′.

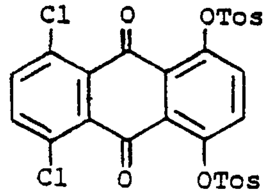

(VI′)

This conversion is conveniently carried out by treating VI with *p*-toluenesulfonyl chloride in a nonreactive solvent such as acetonitrile at reflux temperature. The diester VI′ is the treated with a substituted hydrazine $NH_2NHZ$ substantially as described above for the conversion of compound V to compound III. The product of this reaction, VI″

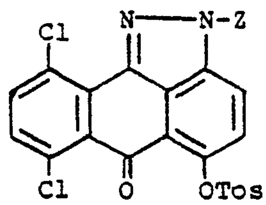

(VI″)

is then treated with an amine having the formula HNYR substantially as described above for the conversion of compound II to compound I. The product of this reaction has the following structural formula

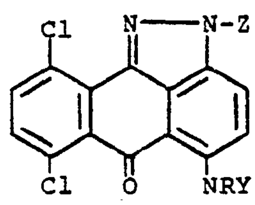

wherein R, Y, and Z are as defined hereinabove.

The compounds having structural formula IV are prepared by reacting a compound having structural formula VII with an amine having the formula HNRY using substantially the same reaction conditions described above for the conversion of compound II to compound I, i.e., reaction step "B". A particular Z substituent, for example, $CH_2CH_2OH$ may also be derivatized and converted to another particular Z substituent, for example $CH_2CH_2NEt_2$ in a similar manner to the procedure already described hereinabove.

The compounds of structural formula VII ar prepared by reacting a suitably substituted hydrazine, $NH_2$—NHZ, wherein Z is defined hereinabove, with 1,5-dichloro-9,10-anthracenedione in a manner substantially identical to that described above for converting compound V to compound III, i.e., reaction sequence "A".

The present invention also contemplates the novel hydrazine, 2-[(hydrazinoethyl)amino]ethanol, $NH_2NHCH_2CH_2NHCH_2CH_2OH$. This novel hydrazine is a useful intermediate for the preparation of a variety of final compounds of the invention. This novel hydrazine may be prepared by a variety of procedures which are considered equivalent for purposes of the invention. One such procedure involves the reaction of hydrazine and N-(2-hydroxyethyl)aziridine in an aqueous medium at reflux temperature. The novel 2-[(hydroazinoethyl)amino]ethanol so produced is isolated by standard procedures as a clear liquid which has bp 120°C at 0.035 mmHg. The novel 2-[[(hydrazinoethyl)amino]ethanol forms acid addition salts with organic and inorganic acids such as hydrochloric, hydrobromic, sulfonic, phosphonic, methanolsulfonic, acetic, benzoic acid. For purposes of the invention, such salts are considered equivalent to the free base form of the novel hydrazine.

The benzylated ethers V wherein any of the Q substituents represent benzyloxy, *p*-chlorobenzyloxy or *p*-methoxybenzyloxy may be prepared by treating compound V wherein any of the Q substituents represent OH with the corresponding benzyl bromide or benzyl chloride in a convenient nonreactive solvent such as acetone, dimethylsulfoxide or *N,N*-dimethylformamide. The use of a hydrogen halide acceptor such as an alkali metal carbonate (e.g., potassium carbonate) for this reaction is preferred.

The novel intermediate, 5,8-dichloro-1,4,9,10-anthracenetetrone may be prepared by oxidation of 1,4-dichloro-5,8-dihydroxy-9,10-anthracenedione. The reaction may be carried out with lead tetracetate in glacial acetic acid at or near room temperature.

The 1,4-dichloro-9,10-anthracenedione, compound V wherein Q = Q′ = Q″ = hydrogen, may be prepared by known methods, see for example *J. Am. Chem. Soc., 48*; 3198 (1926).

The 1,4-dichloro-5,8-dihydroxy-9,10-anthracenedione, compound VI, may be prepared by known methods, see for example US Patent No. 3,631,074.

The compound 1,5-dichloro-9,10-anthracenedione, which is utilized to prepare the compound of formula VII is commercially available or may be prepared by methods known to those skilled in the art, see for example Beilstein 7 787.

The compounds of the invention form pharmaceuceutically acceptable salts with both organic and inorganic acids. Examples of suitable acids for salt formation are hydrochloric, sulfuric, phosphoric, acetic, citric, oxalic, malonic, salicylic, malic, fumaric, succinic, ascorbic, maleic, methanesulfonic, isethionic, lactic, gluconic, glucuronic, sulfamic, benzoic, tartaric, pamoic acid. The salts are prepared by contacting the free base form with an equivalent amount of the desired acid in the conventional manner. The free base forms may be regenerated by treating the salt form with a base. For example, dilute aqueous base solutions may be utilized. Dilute aqueous sodium hydroxide, potassium carbonate, ammonia, and sodium bicarbonate solutions are suitable for this purpose. The free base forms differ from their respective salts forms somewhat in certain physical properties such as solubility in polar solvents, but the salts are otherwise equivalent to their respective free base forms for purposes of the invention.

The compounds of the invention can exist in unsolvated as well as solvated forms, including hydrated forms. In general, the solvated forms, with pharmaceutically acceptable solvents such as water or ethanol are equivalent to the unsolvated forms for purposes of the invention.

The term halogen is intended to include fluorine, chlorine, bromine, and iodine.

The alkyl and alkoxy groups contemplated by the invention, unless specified otherwise, comprise both straight and branched carbon chains of from one to six carbon atoms. Representative of such groups are methyl, ethyl, isopropyl, butyl, pentyl, 3-methylpentyl, methoxy, ethoxy, *i*-propoxy, *t*-butoxy, *n*-hexoxy and 3-methylpentoxy.

The alkylene groups contemplated by the invention, unless specified otherwise, comprise both straight and branched carbon chains of from two to 11 carbon atoms. Representative of such groups are ethylene, *n*-propylene, *n*-butylene, *n*-heptalene, *i*-propylene, 3-ethyl-1,5-pentalene and 3-propyl-1,6-hexalene. The preferred alkylene groups of the invention have the following structural formulas:

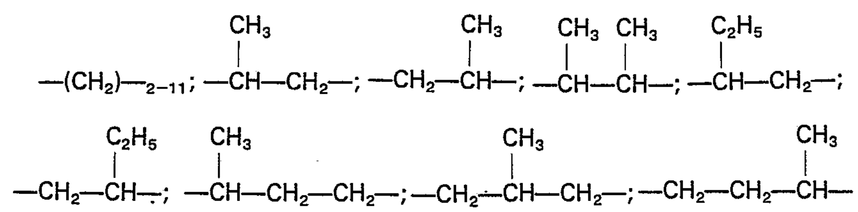

Certain substituents, such as alkyl or alkylene substituents comtemplated by the invention are defined as possibly being substituted with additonal substituents, e.g., $NH_2$. Those skilled in the art will recognize that certain combinations of such substituents are most probably unstable and these are not intended to be included within the scope of the definitions. For example, an α-aminoalkyl or alkylene group of the general formula $=N—CH—NH_2$ would not be expected to be stable whereas the corresponding dialkylated substituent $=N—CH—N(Alk)_2$ is expected to be stable and is intended to be included within the definitions. It is within the skill of the art to recognize these and other such substituents which are possibly unstable.

The compounds of the invention are new chemical substances of value as pharmacological agents for the treatment of bacterial and fungal infections in warm-blooded animals. They may also be utilized as antiseptic agents such as for use in the sterilization of laboratory glassware etc. The antibacterial and antifungal activity of representative compounds of the invention was established by the screening procedure described below.

1. *Preparation of inocula*

(A) Bacteria and yeast:

The bacterial and yeast isolates are maintained in agar slants or in liquid media, hereby designated as inoculum media. The cultures are transferred at regular intervals in such media. (See Table for the corresponding inoculum media of each culture.) The organisms are generally transferred on to agar slants or liquid inoculum media and incubated overnight (18—20 hours): 37°C for the bacterial isolates and 28°C for the fungal cultures.

The microbial cells from the overnight agar slants are then scraped off and suspended in saline solution (0.85% NaCl). The microbial concentrations are adjusted to a light transmittancy of 20—35%, Junior Coleman Spectrophotometer (555 M). For the organisms that are maintained in liquid media, an aliquot of the culture suspension is simply diluted with saline to 20—35% light transmittancy.

The above microbial suspension serve as inocula for the assay plates. Thus, 0.16—10 ml (see Table for exact amount) are used to inoculate 100 ml of the molten-agar assay medium.

(B) Mycelial fungi:

The *Penicillium avellaneum* is grown for six days, at 28°C, on an agar medium. This is to allow sporulation of the culture. The organism is then harvested by scraping off the cells from the agar surface (mycelia and spores) and suspending them in saline solution containing 0.05% Tween 80.

The suspension is adjusted to a light transmittancy of 20%. One ml of this suspension is used to inoculate 100 ml of the molten-agar assay medium.

2. *Preparation of assay plates*

Stainless steel frames, 12.3 × 25.3 cm (ID) and glass plates, 15.3 × 31.7 cm are used to make the test trays. The frames are attached to the plates with tape at each end and the inner edges sealed with 2% agar. Twenty five ml of inoculated assay medium is spread evenly on each tray and allowed to solidify. The trays are covered, inverted, and refrigerated until used.

3. *Disking of samples*

The compounds or samples to be tested are dissolved in suitable solvents, e.g., alcohols, dimethylsulfoxide, or N,N-dimethylformamide. The samples are generally dissolved so that the final concentration of the solvent is <10%.* The compounds are tested at different concentrations: 3,000; 1,000; 500; 100; and 10 mcg/ml. Paper discs (12.7 mm diameter) are placed on the agar trays with forceps, then 0.08 ml of the dissolved compound is pipetted onto each disc using a 0.2 ml pipette. (*If the compound does not stay in solution at <10% alcohol, then the full strength alcohol is used. However, the impregnated discs are air-dried before they are laid on to the seeded agar plates.)

4. *Interpretation of results*

The disked agar trays are incubated overnight (18—20 hours) at 37°C for the bacterial cultures and 28°C for the yeasts. The *Penicillium avellaneium* tray is incubated for at least 20—24 hours since it is a slower-growing organism.

Active compounds show a zone of inhibition around the disc. The diameter of the zone is measured in mm. The zone diameter of active compounds ranges from a minimum of 13.5 mm to as high as 60 mm. The size of the zone diameter generally reflects the activity of the compound: the larger the zone the greater the activity.

TABLE

| Culture | Number | Inoculum Medium | Innoculum Level ml/100 ml | Assay Medium |
|---|---|---|---|---|
| *Aerobacter aerogenes* | 0126 | Veal Infusion Broth | 1 | Mycin Agar |
| *Escherichia coli* | 04863 | AM—08 Agar | 1 | AM—08 |
| *Bacillus subtilis* | 04555 | AM—08 Broth | 0.5 | AM—08 |
| *Streptococcus faecalis* | 05045 | Folic Acid Assay Broth | 2 | AM—09 |
| *Penicillium avellaneum* | M2988 | AM—25 Agar | 1 | AM—25 |

5. *Culture media*

The composition of the various culture media, except for the commercially available media, are shown below. The commercial ready-made Veal Infusion Medium is obtained from Difco Laboratories, Detroit, Michigan, USA. Add 1.5% agar to these media for use as agar plates.

| AM—08 | % |
|---|---|
| Glucose | 0.2 |
| Sodium Glutamate | 1.04 |
| $KH_2PO_4$ | 0.03 |
| $Na_2HPO_4$ | 0.07 |
| Salts #1[a] | 1 ml |
| Salts #2[b] | 10 ml |
| $H_2O$ (distilled) | |

| aSalts # 1 | % | bSalts # 2 | % |
|---|---|---|---|
| $MgSO_4$ | 1.0 | $MnSO_4$ | 1.0 |
| $CaCl_2$ | 5.0 | $ZnSO_4.7H_2O$ | 1.0 |
| NaCl | 5.0 | $FeSO_4.7H_2O$ | 1.0 |
| $CuSO_4.5H_2O$ | 0.01 | $H_2O$ (distilled) | |
| $H_2O$ (distilled) | | | |

| AM—09 | |
|---|---|
| $K_2HPO_4$ | 3.9 g |
| Dextrose | 25 g |
| Na-citrate·$2H_2O$ | 34.4 g |
| Casein hydrolysate | 6.2 g |
| Asparagine | 375 mg |
| L-tryptophan | 125 mg |
| Cysteine | 312.5 mg |
| Glutathione | 3.1 mg |
| Thiamine HCl | 250 g |
| Riboflavin | 625 g |
| Ca pantothenate | 500 g |
| Nicotinic acid | 500 g |
| *p*-aminobenzoic acid | 625 g |
| Biotin | 12.5 g |
| Pyridoxine HCl | 2.5 g |
| Folic Acid | 500 g |
| NaCl | 12.5 g |
| $MgSO_4$ | 250 g |
| $FeSO_4$ | 12.5 g |
| $MnSO_4$·$H_2O$ | 125 g |
| Tween 80 | 62.5 mg |
| $H_2O$ (distilled) | 1000 ml |

| AM—25 | % |
|---|---|
| $Na_2HPO_4 \cdot H_2O$ | 0.35 |
| $KH_2PO_4$ | 0.05 |
| Yeast Extract (Difco) | 0.5 |
| Dextrose | 1.0 |
| Distilled Water | |

Utilizing the above described procedure, the following results were obtained for representative compounds of the invention.

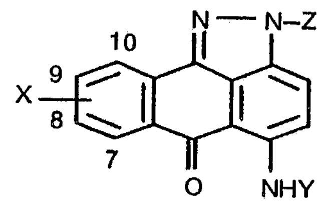

Inhibition Zone Diameter mm (Dose mg/ml)

| X | Y | Z | A. Aerogenes | E. Coli | B. Subtilis | S. Faecalis | P. Avellaneum |
|---|---|---|---|---|---|---|---|
| H | $CH_2CH_2NEt_2$ | $CH_2CH_2NEt_2$ | 19 (0.5) | 15 (1.0) | 15 (0.1) | 14 (0.5) | 19 (3.0) |
| H | $CH_2CH_2NHCH_2CH_2OH$ | $CH_2CH_2NEt_2$ | 18 (0.5) | 14 (0.1) | 16 (0.1) | 15 (0.5) | 0 (3.0) |
| H | $CH_2CH_2NEt_2$ | $CH_3$ | 17 (0.5) | 15 (3.0) | 15 (0.1) | 16 (1.0) | 16 (0.5) |
| H | $CH_2CH_2NHCH_2CH_2OH$ | $CH_2CH_2OH$ | 14 (0.5) | 14 (3.0) | 14 (0.1) | 16 (3.0) | 0 (3.0) |
| H | $CH_2CH_2NEt_2$ | $CH_2CH_2OH$ | 20 (0.5) | 15 (1.0) | 15 (0.1) | 14 (1.0) | 16 (3.0) |
| H | $CH_2CH_2NHCH_2CH_2OH$ | H | 18 (0.5) | 0 (3.0) | 19 (0.5) | 17 (3.0) | 0 (3.0) |
| H | $CH_2CH_2NHCH_2CH_2OH$ | $CH_3$ | 17 (0.5) | 14 (1.0) | 14 (0.1) | 14 (1.0) | 0 (3.0) |
| H | $CH_2CH_2NH_2$ | $CH_2CH_2NEt_2$ | 14 (0.5) | 14 (1.0) | 15 (0.1) | 17 (0.5) | 16 (3.0) |
| H | $CH_2CH_2NHCH_2CH_2OH$ | $CH_2CH_2NHCH_2CH_2OH$ | 14 (1.0) | 16 (3.0) | 18 (0.5) | 0 (3.0) | 0 (3.0) |

| X | Y | Z | A. Aerogenes | E. Coli | B. Subtilis | S. Faecalis | P. Avellaneum |
|---|---|---|---|---|---|---|---|
| H | $CH_2CH_2NH_2$ | $CH_2CH_2NHCH_2CH_2OH$ | 16 (0.5) | 14 (0.1) | 15 (0.1) | 0 (3.0) | 0 (3.0) |
| H | $CH_2CH_2NEt_2$ | $CH_2CH_2NHCH_2CH_2OH$ | 18 (0.5) | 14 (0.1) | 14 (0.1) | 15 (0.5) | 16 (3.0) |
| H | $CH_2CH_2OH$ | $CH_2CH_2NHCH_2CH_2OH$ | 14 (1.0) | 15 (0.5) | 18 (0.5) | 14 (1.0) | 0 (3.0) |
| H | $CH_2CH_2NMe_2$ | $CH_2CH_2NHCH_2CH_2OH$ | 16 (0.5) | 15 (1.0) | 20 (0.5) | 15 (0.5) | 0 (3.0) |
| H | $CH_3$ | $CH_2CH_2NHCH_2CH_2OH$ | 15 (0.1) | 14 (0.5) | 14 (0.1) | 15 (0.5) | 0 (3.0) |
| H | H $CH_2CH_2$– 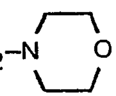 | $CH_2CH_2OH$ | 0 (3.0) | 16 (3.0) | 16 (1.0) | 0 (3.0) | 0 (3.0) |
| H | $(CH_2)_3NEt_2$ | $CH_2CH_2OH$ | 15 (1.0) | 0 (3.0) | 22 (1.0) | 14 (3.0) | 0 (3.0) |
| H | H $CH_2CH_2$– 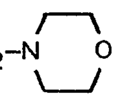 | $CH_2CH_2NEt_2$ | 15 (1.0) | 14 (1.0) | 25 (1.0) | 14 (0.5) | 14 (1.0) |
| H | $(CH_2)_3NEt_2$ | $CH_2CH_2NEt_2$ | 20 (0.1) | 15 (3.0) | 25 (1.0) | 14 (1.0) | 15 (3.0) |
| H | $(CH_2)_4NEt_2$ | $CH_2CH_2NEt_2$ | 16 (0.1) | 15 (3.0) | 17 (1.0) | 14 (1.0) | 14 (3.0) |
| H | $(CH_2)_7NEt_2$ | $CH_2CH_2NEt_2$ | 0 (3.0) | 14 (1.0) | 17 (0.5) | 14 (3.0) | 14 (1.0) |

| X | Y | Z | A. Aerogenes | E. Coli | B. Subtilis | S. Faecalis | P. Avellaneum |
|---|---|---|---|---|---|---|---|
| H | $(CH_2)_4NEt_2$ | $CH_2CH_2OH$ | 0 (3.0) | 0 (3.0) | 25 (1.0) | 0 (3.0) | 0 (3.0) |
| H | $(CH_2)_7NEt_2$ | $CH_2CH_2OH$ | 0 (3.0) | 0 (3.0) | 20 (1.0) | 0 (3.0) | 0 (3.0) |
| H | $(CH_2)_5CH_3$ | $CH_2CH_2NEt_2$ | 0 (3.0) | 0 (3.0) | 16 (1.0) | 0 (3.0) | 16 (3.0) |
| H | 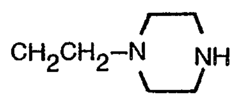 | $CH_2CH_2NEt_2$ | 19 (0.1) | 0 (3.0) | 24 (1.0) | 14 (3.0) | 0 (1.0) |
| H | $CH_2CH_2NHCH_2CH_2OH$ | $CH_2CH_2NH_2$ | 17 (0.1) | 14 (0.5) | 14 (3.0) | 0 (3.0) | 0 (3.0) |
| 7,10-$(OH)_2$ | $CH_2CH_2NHCH_2CH_2OH$ | $CH_2CH_2NEt_2$ | 14 (1.0) | 14 (0.5) | 15 (1.0) | 14 (1.0) | 0 (3.0) |
| 7,10-$(OH)_2$ | $CH_2CH_2NEt_2$ | $CH_2CH_2NEt_2$ | 0 (3.0) | 14 (0.5) | 14 (0.5) | 14 (0.5) | 16 (3.0) |

| X | Y | Z | A. Aerogenes | E. Coli | B. Subtilis | S. Faecalis | P. Avellaneum |
|---|---|---|---|---|---|---|---|
| 7,10-$(OH)_2$ | $CH_2CH_2NH_2$ | $CH_3$ | 14 (3.0) | 14 (3.0) | 0 (3.0) | 14 (3.0) | 0 (3.0) |
| 7,10-$(OH)_2$ | $CH_2CH_2NHCH_2CH_2OH$ | $CH_2CH_2OH$ | 0 (3.0) | 0 (3.0) | 17 (3.0) | 0 (3.0) | 0 (3.0) |
| 7,10-$(OH)_2$ | $CH_2CH_2NH_2$ | $CH_2CH_2OH$ | 15 (1.0) | 0 (3.0) | 15 (1.0) | 0 (3.0) | 0 (3.0) |
| 7,10-$(OH)_2$ | $CH_2CH_2NH_2$ | $CH_2CH_2NEt_2$ | 16 (0.5) | 15 (1.0) | 16 (1.0) | 0 (3.0) | 14 (3.0) |
| 7,10-$(OH)_2$ | $CH_2CH_2NHMe$ | $CH_2CH_2NEt_2$ | 16 (0.5) | 16 (0.5) | 17 (1.0) | 14 (3.0) | 0 (3.0) |
| 7,10-$(OH)_2$ | $CH_2CH_2NEt_2$ | $CH_2CHOHCH_2NEt_2$ | 14 (3.0) | 15 (0.5) | 15 (3.0) | 14 (3.0) | 0 (3.0) |
| 7,10-$(OH)_2$ | $CH_2CH_2NH_2$ | $CH_2CHOHCH_2NEt_2$ | 15 (0.5) | 15 (0.5) | 14 (3.0) | 0 (3.0) | 0 (3.0) |
| 7,10-$(OH)_2$ | $(CH_2)_3NH(CH_2)_2OH$ | $(CH_2)_2NH(CH_2)_2OH$ | 14 (0.5) | 14 (0.5) | 16 (1.0) | 14 (3.0) | 0 (3.0) |
| 7,10-$(OH)_2$ | $(CH_2)_2NH(CH_2)_2NMe_2$ | $(CH_2)_2NH(CH_2)_2OH$ | 14 (1.0) | 15 (1.0) | 18 (3.0) | 16 (3.0) | 0 (3.0) |
| 7,10-$(OH)_2$ | $(CH_2)_2NH(CH_2)_2OH$ | $(CH_2)_2NMe_2$ | 14 (1.0) | 15 (0.5) | 15 (1.0) | 0 (3.0) | 0 (3.0) |
| 7,10-$(OH)_2$ | $(CH_2)_2NMe_2$ | $(CH_2)_2OH$ | 15 (0.5) | 0 (3.0) | 0 (3.0) | 14 (3.0) | 0 (3.0) |

| X | Y | Z | A. Aerogenes | E. Coli | B. Subtilis | S. Faecalis | P. Avellaneum |
|---|---|---|---|---|---|---|---|
| 7,10-$(OH)_2$ | $(CH_2)_2NH_2$ | $(CH_2)_2NME_2$ | 14 (0.5) | 14 (0.5) | 14 (1.0) | 16 (3.0) | 0 (3.0) |
| 7,10-$(OH)_2$ | $(CH_2)_2NH_2$ | $(CH_2)_2NH(CH_2)_2OH$ | 15 (3.0) | 14 (1.0) | 14 (3.0) | 0 (3.0) | 0 (3.0) |
| 7,10-$(OH)_2$ | $(CH_2)_2NH(CH_2)_2OH$ | $(CH_2)_2NH(CH_2)_2OH$ | 15 (3.0) | 14 (1.0) | 14 (3.0) | 0 (3.0) | 0 (3.0) |
| 7,10-$(OH)_2$ | $(CH_2)_2NH(CH_2)_2OH$ | $(CH_2)_2OCH_2$ | 14 (3.0) | 14 (1.0) | 14 (3.0) | 0 (3.0) | 0 (3.0) |
| 7,10-$(OH)_2$ | $(CH_2)_2NMe_2$ | $(CH_2)_2NH(CH_2)_2OH$ | 14 (0.5) | 14 (0.5) | 14 (1.0) | 14 (1.0) | 0 (3.0) |
| 7,10-$(OH)_2$ | $(CH_2)_2NEt_2$ | $(CH_2)_2NH(CH_2)_2OH$ | 14 (0.5) | 14 (0.5) | 15 (0.5) | 14 (1.0) | 14 (3.0) |
| 7,10-$(OH)_2$ | $(CH_2)_2NH_2$ | $(CH_2)_2OMe$ | 14 (3.0) | 16 (3.0) | 16 (3.0) | 0 (3.0) | 0 (3.0) |
| 7,10-$(OH)_2$ | $(CH_2)_3NH_2$ | $(CH_2)_2NH(CH_2)_2OH$ | 14 (3.0) | 15 (3.0) | 0 (3.0) | 0 (3.0) | 0 (3.0) |
| 7,10-$(OH)_2$ | $(CH_2)_4NH_2$ | $(CH_2)_2NH(CH_2)_2OH$ | 16 (3.0) | 15 (3.0) | 0 (3.0) | 0 (3.0) | 0 (3.0) |

| X | Y | Z | A. Aerogenes | E. Coli | B. Subtilis | S. Faecalis | P. Avellaneum |
|---|---|---|---|---|---|---|---|
| 7,10-$(OH)_2$ | $(CH_2)_5NH_2$ | $(CH_2)_2NH(CH_2)_2OH$ | 0 (3.0) | 14 (3.0) | 0 (3.0) | 0 (3.0) | 0 (3.0) |
| 7,10-$(OH)_2$ | $(CH_2)_2$ 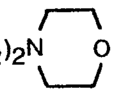 | $(CH_2)_2NH(CH_2)_2OH$ | 0 (3.0) | 14 (3.0) | 15 (1.0) | 0 (3.0) | 0 (3.0) |
| 7,10-$(OH)_2$ | 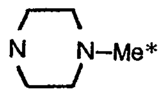 | $(CH_2)_2NH(CH_2)_2OH$ | 17 (0.5) | 14 (1.0) | 0 (3.0) | 0 (3.0) | 0 (3.0) |
| 7,10-$(OH)_2$ | $N(CH_2)_2NMe_2$* (N–Me) | $(CH_2)_2NH(CH_2)_2OH$ | 16 (0.5) | 15 (3.0) | 0 (3.0) | 0 (3.0) | 0 (3.0) |
| 7,10-$(OH)_2$ | $(CH_2)_2NH_2$ | $(CH_2)_2NH_2$ | 14 (3.0) | 0 (3.0) | 0 (3.0) | 0 (3.0) | 0 (3.0) |
| 7,10-$(OH)_2$ | $(CH_2)_3NH_2$ | $(CH_2)_2NMe_2$ | 14 (3.0) | 15 (3.0) | 0 (3.0) | 0 (3.0) | 0 (3.0) |
| 7,10-$(OH)_2$ | $(CH_2)_2NH(CH_2)_2OH$ | $(CH_2)_3NMe_2$ | 16 (3.0) | 0 (3.0) | 0 (3.0) | 0 (3.0) | 0 (3.0) |
| 7,10-$(OH)_2$ | $(CH_2)_3NH_2$ | $(CH_2)_3NMe_2$ | 14 (3.0) | 14 (3.0) | 14 (3.0) | 0 (3.0) | 0 (3.0) |
| 7,10-$(OH)_2$ | $(CH_2)_2NH_2$ | $(CH_2)_3NMe_2$ | 15 (3.0) | 15 (3.0) | 0 (3.0) | 0 (3.0) | 0 (3.0) |
| 7,10-$(OH)_2$ | $(CH_2)_2NEt_2$ | $CH_2CH_2OH$ | 0 (3.0) | 0 (3.0) | 14 (0.5) | 14 (3.0) | 0 (3.0) |

*Substituent bonded directly to aromatic ring.

| X | Y | Z | A. Aerogenes | E. Coli | B. Subtilis | S. Faecalis | P. Avellaneum |
|---|---|---|---|---|---|---|---|
| 7,10-$(OH)_2$ | $CH_2CH_2NHCH_2CH_2OH$ | $CH_2CHOHCH_2NEt_2$ | 16 (0.5) | 16 (0.5) | 14 (3.0) | 15 (3.0) | 0 (3.0) |
| 7,10-$(OH)_2$ | $CH_2CH_2NHCH_2CH_2NMe_2$ | $CH_2CH_2NH_2$ | — | — | 16 (3.0) | 0 (3.0) | 0 (3.0) |
| 7,10-$(OH)_2$ | $(CH_2)_3NHCH_2CH_2OH$ | $CH_2CH_2NH_2$ | — | — | 14 (0.5) | 0 (3.0) | 0 (3.0) |
| 7,10-$(OH)_2$ | $CH_2CH_2NHCH_2CH_2OH$ | $CH_2CH_2CH_2NH_2$ | — | — | 16 (3.0) | 0 (3.0) | 0 (3.0) |
| 7,10-$(OH)_2$ | $CH_2CH_2NHCH_2CH_2NMe_2$ | $CH_2CH_2CH_2NH_2$ | — | — | 15 (3.0) | 0 (3.0) | 0 (3.0) |
| 7-OH | $CH_2CH_2NHCH_2CH_2OH$ | $CH_2CH_2NHCH_2CH_2OH$ | — | — | 14 (3.0) | 0 (3.0) | 0 (3.0) |
| 10-OH | $CH_2CH_2NHCH_2CH_2OH$ | $CH_2CH_2NHCH_2CH_2OH$ | — | — | 14 (3.0) | 0 (3.0) | 0 (3.0) |
| 7-OH | $CH_2CH_2NHCH_3$ | $CH_2CH_2NHCH_2CH_2OH$ | — | — | 15 (3.0) | 14 (3.0) | 0 (3.0) |
| H | Cl* | $(CH_2)_2NEt_2$ | 14 (0.5) | 14 (0.5) | 16 (0.1) | 16 (1.0) | 16 (0.5) |
| H | Cl* | $(CH_2)_2NH_2$ | 25 (0.1) | 16 (0.1) | 14 (3.0) | 0 (3.0) | 16 (3.0) |
| H | Cl* | $(CH_2)_2NH(CH_2)_2OH$ | 18 (0.5) | 16 (0.1) | 16 (0.1) | 15 (3.0) | 0 (3.0) |
| H | Cl* | $CH_2CHOHCH_2NEt_2$ | 17 (3.0) | 14 (0.5) | 14 (1.0) | 15 (1.0) | 16 (1.0) |
| 7,10-$(OH)_2$ | Cl* | $CH_2CHOHCH_2NEt_2$ | 0 (3.0) | 14 (0.5) | 14 (3.0) | 14 (3.0) | 14 (3.0) |
| 7,10-$(OH)_2$ | Cl* | $(CH_2)_2NEt_2$ | 0 (3.0) | 0 (3.0) | 14 (1.0) | 0 (3.0) | 16 (3.0) |
| 7,10-$(OH)_2$ | Cl* | $(CH_2)_2NMe_2$ | 15 (3.0) | 14 (1.0) | 0 (3.0) | 0 (3.0) | 15 (0.5) |

*Substituent bonded directly to aromatic ring.

| X | Y | Z | A. Aerogenes | E. Coli | B. Subtilis | S. Faecalis | P. Avellaneum |
|---|---|---|---|---|---|---|---|
| 7,10-$(OH)_2$ | Cl* | $(CH_2)_2NH(CH_2)_2OH$ | 14 (1.0) | 14 (0.5) | 0 (3.0) | 0 (3.0) | 0 (3.0) |
| 7,10-$(OH)_2$ | Cl* | $(CH_2)_2NH(CH_2)_2NMe_2$ | 0 (3.0) | 0 (3.0) | 14 (3.0) | 0 (3.0) | 0 (3.0) |
| 7,10-$(OH)_2$ | Cl* | $(CH_2)_2NH_2$ | 14 (1.0) | 15 (3.0) | 0 (3.0) | 0 (3.0) | 0 (3.0) |
| 7,10-$(OH)_2$ | Cl* | $(CH_2)_2N(Me)(CH_2)_2OH$ | 0 (3.0) | 14 (3.0) | 0 (3.0) | 0 (3.0) | 0 (3.0) |
| 7,10-$(OH)_2$ | Cl* | $(CH_2)_3NH_2$ | | | | | |

*Substituent bonded directly to aromatic ring.

Inhibition Zone Diameter mm (Dose mg/ml)

| Y | Z | A. Aerogenes | E. Coli | B. Subtilis | S. Faecalis | P. Avellaneum |
|---|---|---|---|---|---|---|
| $(CH_2)_2NH(CH_2)_2OH$ | $CH_2CH_2NEt_2$ | 18 (0.5) | 15 (0.5) | 20 (0.1) | 16 (0.5) | 15 (3.0) |
| $(CH_2)_2NEt_2$ | $CH_2CH_2NEt_2$ | 16 (0.5) | 14 (0.5) | 19 (0.1) | 15 (0.5) | 16 (1.0) |
| $(CH_2)_2NH(CH_2)_2OH$ | $(CH_2)_2NH(CH_2)_2OH$ | 17 (0.5) | 20 (1.0) | 16 (0.5) | 0 (3.0) | 0 (3.0) |

In addition to their usefulness as antibiotic and antifungal agents, certain of the compounds of the invention display in vivo antileukemic activity when tested by the following procedure.

The in vivo lymphocytic leukemia P388 test is carried out by the United States National Cancer Institute. The animals used are either male or female $CD_2F_1$ mice. There are six to seven animals per test group. The tumor transplant is by intraperitoneal injection of dilute ascitic fluid containing cells of lymphocytic leukemia P388. The test compounds are administered intraperitoneally in two single doses with a four-day interval between doses at various dose levels following tumor inoculation. The animals are weighed and survivors are recorded on a regular basis for 30 days. A ratio of survival time for treated (T)/control (C) animals is calculated. The criterion for efficacy is T/C × 100 > 125%. The positive control compound in this test is 1,4-dihydroxy-5,8-[bis[[2-[(2-hydroxyethyl)amino]-ethyl]amino]-9,10-anthracenedione given at dosages ranging from 12.0 to 0.075 mg/kg. See *Cancer Chemotherapy Reports,* Part 3, *3,* 1 (1972) for a comprehensive discussion of the protocol.

Utilizing this procedure, the following results were obtained for representative compounds of the invention.

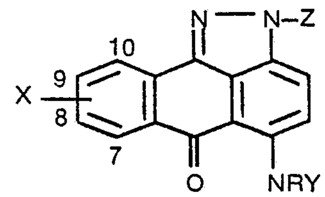

| X | Z | NRY | Dose mg/kg | T/C × 100 (Percent) |
|---|---|---|---|---|
| 7,10-$(OH)_2$ | $(CH_2)_2N(Me)_2\cdot 2HCl$ | $NH(CH_2)_2NH(CH_2)_2OH$ | 12.5 | 242, 182 |
| | | | 6.25 | 214, 214 |
| | | | 3.12 | 192, 171 |
| | | | 1.56 | 163 |
| | | | 0.78 | 161 |
| 7,10-$(OH)_2$ | $(CH_2)_2N(Me)_2\cdot 2HCl$ | $NH(CH_2)_2NH_2$ | 12.5 | 283, 245 Cures |
| | | | 6.25 | 182, 192 |
| | | | 3.12 | 182, 173 |
| | | | 1.56 | 194, 149 |
| | | | 0.78 | 194, 146 |
| 7,10-$(OH)_2$ | $(CH_2)_2N(Et)_2\cdot 2HCl$ | $NH(CH_2)_2N(Et)_2$ | 100 | 180, 174 |
| | | | 50 | 168, 154, 146 |
| | | | 25 | 140, 143, 149 |
| | | | 12.5 | 132, 114, 140 |
| H | $(CH_2)_2N(Et)_2\cdot 2HCl$ | $NH(CH_2)_2N[CH_2)_2]_2O$ | 400 | 211 |
| | | | 200 | 190, 148 |
| | | | 100 | 145, 126 |
| H | $(CH_2)_2N(Et)_2\cdot 2HCl$ | $NH(CH_2)_2NH(CH_2)_2OH$ | 25 | 197 |
| | | | 12.5 | 161, 152, 178 |
| | | | 6.25 | 145, 146, 161, 178 |
| | | | 3.12 | 128, 142, 164, 151 |
| | | | 1.56 | 142, 142, 146 |
| | | | 0.78 | 135 |
| 7,10-$(OH)_2$ | $(CH_2)_2N(Et)_2\cdot 2HCl$ | $NH(CH_2)_2NH(CH_2)_2OH$ | 12.5 | 264, 212 |
| | | | 6.25 | 180, 180, 164 |
| | | | 3.12 | 164, 164, 166 |
| | | | 1.56 | 148, 149, 155 |
| | | | 0.78 | 140, 151, 159 |
| | | | 0.39 | 152, 126 |
| 7,10-$(OH)_2$ | $(CH_2)_2N(Et)_2\cdot 2HBr$ | $NH(CH_2)_2NHMe$ | 50 | 172 |
| | | | 25 | 219 Cures |
| | | | 12.5 | 191 |
| | | | 6.25 | 163 |
| | | | 3.12 | 154 |

| X | Z | NRY | Dose mg/kg | T/C × 100 (Percent) |
|---|---|---|---|---|
| 7,10-$(OH)_2$ | $(CH_2)_2N(Et)_2 \cdot 2HCl$ | $NH(CH_2)_2NH_2$ | 25 | 179 Cures |
| | | | 12.5 | 280, 189 Cures |
| | | | 6.25 | 189, 191 |
| | | | 3.12 | 154, 160 |
| | | | 1.56 | 172 |
| 7,10-$(OH)_2$ | $(CH_2)_2NH(CH_2)_2N(Me)_2$ | $NH(CH_2)_2NH(CH_2)_2OH$ | 50 | 226 |
| | | | 25 | 226, 169 Cures |
| | | | 12.5 | 179, 219 |
| | | | 6.25 | 188, 191 |
| | | | 3.12 | 207 |
| 7,10-$(OH)_2$ | $(CH_2)_2NH(CH_2)_2OH \cdot 2HCl$ | $NCH_3(CH_2)_2N(Me)_2$ | 12.5 | 188 |
| | | | 6.25 | 179 |
| 7,10-$(OH)_2$ | $(CH_2)_2NH(CH_2)_2OH \cdot 2HCl$ | $N[(CH_2)_2]_2NMe$ | 25 | 150 |
| | | | 12.5 | 169 |
| | | | 6.25 | 163 |
| H | $(CH_2)_2NH(CH_2)_2OH \cdot 2HCl$ | $NH(CH_2)_2N(Me)_2$ | 25 | 162 |
| | | | 12.5 | 166, 168 |
| | | | 6.25 | 163, 174 |
| | | | 3.12 | 158 |
| 7,10-$(OH)_2$ | $(CH_2)_2NH(CH_2)_2OH \cdot 2HCl$ | $NH(CH_2)_2N(Me)_2$ | 25 | 88 Cures |
| | | | 12.5 | 220, 250 Cures |
| | | | 6.25 | 165, 182 |
| | | | 3.12 | 165, 165 |
| | | | 1.56 | 161, 165 |
| | | | 0.78 | 152 |
| H | $(CH_2)_2NH(CH_2)_2OH \cdot 2HCl$ | $NH(CH_2)_2N(Et)_2$ | 25 | 149 |
| | | | 12.5 | 150, 155, 158, 160 |
| | | | 6.25 | 138, 146, 150, 142 |
| | | | 3.12 | 143, 143, 144, 151 |
| | | | 1.56 | 138, 133 |
| 7,10-$(OH)_2$ | $(CH_2)_2NH(CH_2)_2OH \cdot 2HCl$ | $NH(CH_2)_2N(Et)_2$ | 50 | 241 |
| | | | 25 | 190, 209 Cures |
| | | | 12.5 | 177, 190 |
| | | | 6.25 | 154, 168 |
| | | | 3.12 | 148, 150 |
| | | | 1.56 | 131, 135 |
| 7,10-$(OH)_2$ | $(CH_2)_2NH(CH_2)_2OH \cdot 2HCl$ | $NH(CH_2)_2NH(CH_2)_2N(Me)_2$ | 50 | 200, 254 Cures |
| | | | 25 | 207, 228, 129 Cures |
| | | | 12.5 | 157, 179, 188 |
| | | | 6.25 | 169, 172 |
| | | | 3.12 | 152, 163 |
| H | $(CH_2)_2NH(CH_2)_2OH \cdot CH_3CO_2H$ | $NH(CH_2)_2NH(CH_2)_2OH$ | 50 | 199 |
| | | | 25 | 171, 185, 185 |
| | | | 12.5 | 148, 189, 166 |
| | | | 6.25 | 147, 157, 166 |
| | | | 3.12 | 138, 150, 166 |
| | | | 1.56 | 132, 142 |

| X | Z | NRY | Dose mg/kg | T/C × 100 (Percent) |
|---|---|---|---|---|
| 7,10-$(OH)_2$ | $(CH_2)_2NH(CH_2)_2OH \cdot 2HCl$ | $NH(CH_2)_2NH(CH_2)_2OH$ | 50 | 139 |
| | | | 25 | 158, 168 |
| | | | 12.5 | 153, 177 |
| | | | 6.25 | 206 Cures |
| | | | 3.12 | 187, 187 |
| | | | 1.56 | 158, 168 |
| H | $(CH_2)_2NH(CH_2)_2OH \cdot 2HCl$ | $NH(CH_2)_2NH_2$ | 25 | 189 |
| | | | 12.5 | 175, 180 |
| | | | 6.25 | 146, 180 |
| | | | 3.12 | 162, 173 |
| | | | 1.56 | 150 |
| 7,10-$(OH)_2$ | $(CH_2)_2NH(CH_2)_2OH \cdot 2HCl$ | $NH(CH_2)_2NH_2$ | 3.12 | 158, 196 Cures |
| | | | 1.56 | 173, 187 Cures |
| | | | 0.78 | 182 |
| | | | 0.39 | 175 |
| 7,10-$(OH)_2$ | $(CH_2)_2NH(CH_2)_2OH \cdot 2HCl$ | $NH(CH_2)_3NH(CH_2)_2OH$ | 6.25 | 169, 254 Cures |
| | | | 3.12 | 173, 186 |
| | | | 1.56 | 201, 166 |
| 7,10-$(OH)_2$ | $(CH_2)_2NH(CH_2)_2OH \cdot 2HCl$ | $NH(CH_2)_3NH_2$ | 100 | 131 Cures |
| | | | 50 | 254, 272 Cures |
| | | | 25 | 207, 250 Cures |
| | | | 12.5 | 177, 190 |
| | | | 6.25 | 165, 172 |
| | | | 3.12 | 165, 165 |
| 7,10-$(OH)_2$ | $(CH_2)_2NH(CH_2)_2OH \cdot HCl$ | $NH(CH_2)_4NH_2$ | 100 | 172, 173 |
| | | | 50 | 139, 165 |
| | | | 25 | 150, 152 |
| | | | 12.5 | 139, 145 |
| | | | 6.25 | 131 |
| | | | 3.12 | 130 |
| 7,10-$(OH)_2$ | $(CH_2)_2NH(CH_2)_2OH \cdot 2HCl$ | $NH(CH_2)_5NH_2$ | 100 | 150 |
| | | | 50 | 127, 139 |
| | | | 25 | 127, 127 |
| | | | 12.5 | 127 |
| H | $(CH_2)_2NH_2 \cdot 2HCl$ | $NH(CH_2)_2NH(CH_2)_2OH$ | 25 | 187, 1187 |
| | | | 12.5 | 173, 177 |
| | | | 6.25 | 158, 173 |
| | | | 3.12 | 149, 158 |
| | | | 1.56 | 139 |
| 7,10-$(OH)_2$ | $(CH_2)_2NH_2 \cdot 2HCl$ | $NH(CH_2)_2NH(CH_2)_2OH$ | 25 | 257 Cures |
| | | | 12.5 | 182 |
| | | | 6.25 | 163 |
| | | | 3.12 | 160 |
| | | | 1.56 | 153 |
| 7,10-$(OH)_2$ | $(CH_2)_2OMe \cdot HCl$ | $NH(CH_2)_2NH(CH_2)_2OH$ | 100 | 139, 140 |
| | | | 50 | 135 |
| | | | 25 | 127, 131 |
| | | | 12.5 | 127 |

| X | Z | NRY | Dose mg/kg | T/C × 100 (Percent) |
|---|---|---|---|---|
| 7,10-$(OH)_2$ | $(CH_2)_2OH \cdot 2HCl$ | $NH(CH_2)_2N(Me)_2$ | 200 | 157, 283 Cures |
| | | | 100 | 225, 242 |
| | | | 50 | 180, 182, 182 |
| | | | 25 | 153, 157, 173 |
| | | | 12.5 | 124, 135, 139 |
| 7,10-$(OH)_2$ | $(CH_2)_2OH \cdot 2HCl$ | $NH(CH_2)_2N(Et)_2$ | 400 | 190 |
| | | | 300 | 177 |
| | | | 200 | 167 |
| | | | 150 | 155 |
| | | | 100 | 149 |
| | | | 75 | 135 |
| | | | 37.5 | 135 |
| | | | 25 | 128 |
| 7,10-$(OH)_2$ | $(CH_2)_2OH \cdot HCl$ | $NH(CH_2)_2NH(CH_2)_2OH$ | 200 | 196, 271 |
| | | | 100 | 172, 184 |
| | | | 50 | 163, 165 |
| | | | 25 | 165, 187 |
| | | | 12.5 | 158, 163 |
| | | | 6.25 | 143, 163 |
| 7,10-$(OH)_2$ | $(CH_2)_2OH \cdot 2HCl$ | $NH(CH_2)_2NH_2$ | 100 | 172, 196 |
| | | | 50 | 162, 172 |
| | | | 25 | 146, 154 |
| | | | 12.5 | 145, 151 |
| | | | 6.25 | 135 |
| 7,10-$(OH)_2$ | $(CH_2)_2SMe \cdot 2HCl$ | $NH(CH_2)_2NH(CH_2)_2OH$ | 12.5 | 132 |
| | | | 6.25 | 140 |
| 7,10-$(OH)_2$ | $CH_2CHOHCH_2N(Et)_2 \cdot 2HCl$ | $NH(CH_2)_2N(Et)_2$ | 100 | 157 |
| | | | 50 | 131, 135 |
| | | | 25 | 131, 135 |
| | | | 12.5 | 128 |
| 7,10-$(OH)_2$ | $CH_2CHOHCH_2N(Et)_2 \cdot 2HCl$ | $NH(CH_2)_2NH(CH_2)_2OH$ | 12.5 | 182, 223 Cures |
| | | | 6.25 | 128, 203 |
| | | | 3.12 | 128, 188, 189 |
| | | | 1.56 | 139 |
| | | | 0.78 | 132 |
| 7,10-$(OH)_2$ | $CH_2CHOHCH_2N(Et)_2 \cdot 2HCl$ | $NH(CH_2)_2NH_2$ | 12.5 | 142, 210 |
| | | | 6.25 | 166, 189, 262, Cures |
| | | | 3.12 | 166, 182, 202 |
| | | | 1.56 | 157, 146 |
| | | | 0.78 | 132, 179 |
| | | | 0.39 | 135 |
| 7,10-$(OH)_2$ | $Me \cdot HCl$ | $NH(CH_2)_2NH(CH_2)_2OH$ | 100 | 174 |
| | | | 50 | 162, 164 |
| | | | 25 | 150, 164 |
| | | | 12.5 | 140, 149 |
| | | | 6.25 | 133, 155 |
| | | | 3.12 | 138 |
| 7,10-$(OH)_2$ | $CH_2CH_2NH_2 \cdot 2HCl$ | $NHCH_2CH_2NH_2$ | 25 | 156, 218 |
| | | | 12.5 | 144, 203, 221 |
| 7,10-$(OH)_2$ | $CH_2CH_2NMe_2 \cdot 2HCl$ | $NHCH_2CH_2CH_2NH_2$ | 12.5 | 205, 208 |
| | | | 6.25 | 196 Cures |

| X | Z | NRY | Dose mg/kg | T/C × 100 (Percent) |
|---|---|---|---|---|
| 7,10-$(OH)_2$ | $CH_2CH_2CH_2NMe_2 \cdot 2HCl$ | $NHCH_2CH_2NHCH_2CH_2OH$ | 12.5<br>6.25 | 218, 203<br>185, 167 |
| 7,10-$(OH)_2$ | $CH_2CH(OH)CH_2OH \cdot HCl$ | $NHCH_2CH_2NHCH_2CH_2OH$ | 400<br>200<br>100<br>50<br>25 | 281, 255<br>218, 213<br>208, 194 Cures<br>177<br>184, 166 |
| 7,10-$(OH)_2$ | $CH_2CH_2CH_2NMe_2 \cdot 2HCl$ | $NHCH_2CH_2CH_2NH_2$ | 25<br>12.5<br>6.25 | 194, 212<br>173, 180<br>167, 173, 190 |
| 7,10-$(OH)_2$ | $CH_2CH_2CH_2NMe_2 \cdot 2HCl$ | $NHCH_2CH_2NH_2$ | 12.5<br>6.25<br>3.12 | 224 Cures<br>186, 200<br>191, 180 |
| 7,10-$(OH)_2$ | $CH_2CH_2NMeCH_2CH_2OH$<br>$\cdot 1.6HCl$ | $NHCH_2CH_2NHCH_2CH_2OH$ | 12.5<br>6.25<br>3.12 | 203<br>182<br>184 |
| 7,10-$(OH)_2$ | $CH_2CH_2NHCH_2CH_2OH$<br>$\cdot 2.1HCl$ | $NH(CH_2)_3M(CH_2CH_2OH)_2$ | 100<br>50<br>25 | 192<br>167<br>163 |
| 7,10-$(OH)_2$ | $CH_2CH_2NHCH_2CH_2OH$<br>$\cdot HCl$ | $NHCH_2CH_2NHCH_2CH_2NH_2$ | 25<br>12.5<br>6.25 | 221, 227 Cures<br>233, 231 Cures<br>194, 157 |
| 7,10-$(OH)_2$ | $CH_2CH_2NHCH_2CH_2OH$<br>$\cdot 2.3HCl$ | $NHCH_2CH_2N(CH_2CH_2OH)_2$ | 100<br>50<br>25<br>12.5 | 223, 254<br>203, 169<br>187, 194<br>158, 166 |
| 7,10-$(OH)_2$ | $CH_2CH_2NHCH_2CH_2OH$<br>$\cdot 2.75HCl$ | $NH(CH_2)_3NH(CH_2)_4NH(CH_2)_3NH_2$ | 12.5<br>6.25 | 145, 117<br>127, 109 |
| 7,10-$(OH)_2$ | $(CH_2)_3NHCH_2CH_2OH$ | $NHCH_2CH_2NHCH_2CH_2OH$ | 100<br>50<br>25<br>12.5 | 259 Cures<br>222, 183<br>183, 174<br>157, 146 |
| 7,10-$(OH)_2$ | $(CH_2)_3NHCH_2CH_2OH$<br>$\cdot 0.1HCl$ | $NHCH_2CH_2CH_2NH_2$ | 100<br>50 | 185, 155<br>160, 146 |
| 7,10-$(OH)_2$ | $CH_2CH_2NHCH_2CH_2OH$<br>°$CH_3CO_2H \cdot HBr$ | $NHCH_2CH_2NHCH_3$ | 12.5<br>6.25<br>3.12 | 277, 275 Cures<br>277, 275 Cures<br>268, 177 Cures |
| 7,10-$(OH)_2$ | $CH_2CH_2NH_2 \cdot 3.3HCl$ | $NHCH_2CH_2NHCH_2CH_2NMe_2$ | 50<br>25<br>12.5 | 220 Cures<br>192, 177<br>186, 167 |
| 7,10-$(OH)_2$ | $CH_2CH_2NH_2 \cdot 2.1HCl$ | $NHCH_2CH_2CH_2NHCH_2CH_2OH$ | 6.25<br>3.12<br>1.56 | 184, 194<br>177, 184<br>181, 172 |
| 7,10-$(OH)_2$ | $CH_2CH_2CH_2NH_2 \cdot 2HCl$ | $NHCH_2CH_2NHCH_2CH_2OH$ | 50<br>25<br>12.5<br>6.25 | 285 Cures<br>285 Cures<br>247, 163 Cures<br>193, 134 |

| X | Z | NRY | Dose mg/kg | T/C × 100 (Percent) |
|---|---|---|---|---|
| 7,10-$(OH)_2$ | $CH_2CH_2CH_2NH_2 \cdot 3HCl$ | $NHCH_2CH_2NHCH_2CH_2NMe_2$ | 25<br>12.5 | 228, 130<br>219, 123 |
| 7,10-$(OH)_2$ | $CH_2CH_2NHCH_3 \cdot 2HCl$ | $NHCH_2CH_2NHCH_2CH_2OH$ | 12.5 | 147 |
| 7-OH | $CH_2CH_2NHCH_2CH_2OH$ $\cdot 2HCl$ | $NHCH_2CH_2NHCH_2CH_2OH$ | 25<br>12.5<br>6.25 | 285, 184 Cures<br>228, 142 Cures<br>183, 134 |
| 10-OH | $CH_2CH_2NHCH_2CH_2OH$ $\cdot 2.1HCl$ | $NHCH_2CH_2NHCH_2CH_2OH$ | 100<br>50<br>25 | 221, 184<br>172, 162<br>163, 155 |
| 7,8,10-$(OH)_3$ | $CH_2CH_2NHCH_2CH_2OH$ $\cdot 2.1HCl$ | $NHCH_2CH_2NHCH_2CH_2OH$ | 25<br>12.5 | 265 Cures<br>257 Cures |
| 7,9,10-$(OH)_3$ | $CH_2CH_2NHCH_2CH_2OH$ $\cdot 2.1HCl$ | $NHCH_2CH_2NHCH_2CH_2OH$ | 3.12<br>1.56 | 165<br>155 |

Antileukemic Activity of Chloroanthrapyrazoles
Against Leukemias

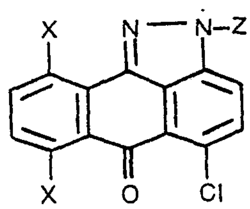

| X | Z | L1210 Leukemia In Vitro* $ID_{50}$ Molar | P388 Leukemia in Mice Dose (mg/kg) | P388 Leukemia in Mice T/C × 100 (Percent) |
|---|---|---|---|---|
| H | $(CH_2)_2NH(CH_2)_2OH \cdot CH_3CO_2H$ | $3.9 \times 10^{-7}$ | 200<br>100 | 148, 126<br>124 |
| OH | $(CH_2)_2NH(CH_2)_2OH \cdot HCl$ | $6.6 \times 10^{-7}$ | 50<br>25<br>12.5<br>6.25 | 161, 161<br>143, 145<br>138, 140<br>131 |
| OH | $(CH_2)_2NH_2 \cdot HCl$ | $6.3 \times 10^{-7}$ | 50 | 125 |

*Procedure described in Europ. J. Cancer *17*, 671 (1981)

Broad Spectrum Antitumor Activity* of 2-[2-(Diethylamino)ethyl]-7,10-dihydroxy-5-[[2-[(2-hydroxyethyl)amino]ethyl]amino]anthra[1-9-cd]pyrazol-6(2H)-one Dihydrochloride in Mice

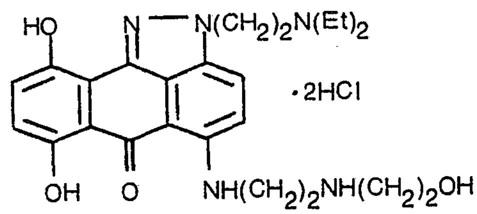

| Tumor | Route Tumor/Drug | Regimen | Dose (mg/kg | T/C × 100 (Percent) | % Tumor Reduction |
|---|---|---|---|---|---|
| ADJ—PC6 Plasmacytoma | IP/IP | Q04D × 03 | 16 | 163 | |
| | | | 8 | 235 | |
| | | | 4 | 216 | |
| | | | 2 | 207 | |
| B16 Melanoma (BDF1) | IP/IP | Q01D × 09 | 8 | 176 | |
| | | | 4 | 151 | |
| B16 Melanoma (B6C3F1) | I-/IP | Q01D × 09 | 6 | 189 | |
| | | | 3 | 148 | |
| Colon 38 (BDF1) | SC/IP | Q01D × 09 | 16 | | 71 |
| L1210 Leukemia (CDF1) | IP/IP | Q01D × 09 | 16 | 133 | |
| | | | 8 | 240 | |
| | | | 4 | 178 | |
| | | | 2 | 144 | |
| | | | 1 | 137 | |
| M5076 Ovary | IP/IP | Q04D × 04 | 10 | 260 | |
| | | | 5 | 186 | |
| | | | 2.5 | 233 | |
| | | | 1.25 | 143 | |

*Procedure described in Cancer Chemotherapy Reviews, *7,* 167 (1980) and references cited therein.

In addition to their usefulness as antibiotic and antifungal agents and as antileukemic agents, certain of the compounds of the invention display in vitro activity against solid tumors when tested by the following procedure.

HCT—8 (human colon adenocarcinoma) cells are trypsinized using Trypsin-EDTA. A single cell suspension is achieved by passing the cells through a 26 gauge needle with a 20 cc syringe. A cell suspension is prepared using RPMI 1640 growth medium (available from Gibco Laboratories) +10% fetal calf serum +50 µg/ml garamycin with a cell concentration of approximately 30,000 cells/ml. The cell suspension is dispensed in Linbro 24-well plates; 1 ml/well. The plates are incubated for approximately 48 hrs at 37°C in a 5% $CO_2$ atmosphere. At this time test compounds are added in the appropriate concentration. Five µl of the 200 µg/ml stock solution is added to each well in a primary test. Ten µl of the appropriate dilution is added to each well for a titration test. The plates are reincubated an additional 60—65 hrs at 37°C in a 5% $CO_2$ atmosphere. The test is read by lysing the cells using a mix of cationic surfactant, glacial acetic acid and sodium chloride. Two ml of the lysed cell suspension from each well is added to 8 ml of diluent. Each sample is read with a Coulter counter (ZBI model). The activity of each sample is measured as a percentage of the controls and the data is reported as $ID_{50}$, that is the molar quantity of drug required to kill 50% of the tumor cells.

Utilizing this procedure, the following results were obtained for representative compounds of the invention.

In Vitro Activity of Aminoanthrapyrazoles
Against Human Colon Adenocarcinoma

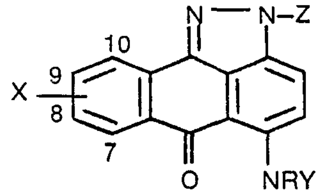

| X | $Z_s$ | $NRY_s$ | $ID_{50}$ Molar |
|---|---|---|---|
| H | $(CH_2)_2NEt_2 \cdot 2HCl$ | $NH(CH_2)_2NEt_2$ | $1.4 \times 10^{-7}$ |
| H | $CH_3 \cdot 2HCl$ | $NH(CH_2)_2NEt_2$ | $4.1 \times 10^{-7}$ |
| H | $(CH_2)_2OH \cdot 2HCl$ | $NH(CH_2)_2NEt_2$ | $1.8 \times 10^{-6}$ |
| H | $H \cdot HCl$ | $NH(CH_2)_2NH(CH_2)_2OH$ | $1.5 \times 10^{-6}$ |
| H | $CH_3 \cdot HCl$ | $NH(CH_2)_2NH(CH_2)_2OH$ | $4.0 \times 10^{-7}$ |
| H | $(CH_2)_2NEt_2 \cdot 2HCl$ | $NH(CH_2)_2NH_2$ | $5.2 \times 10^{-8}$ |
| H | $(CH_2)_2NH(CH_2)_2OH \cdot CH_3CO_2H$ | $NH(CH_2)_2NH(CH_2)OH$ | $9.6 \times 10^{-7}$ |
| H | $(CH_2)_2NH(CH_2)_2OH \cdot 2HCl$ | $NH(CH_2)_2NH_2$ | $4.2 \times 10^{-8}$ |
| H | $(CH_2)_2NH(CH_2)_2OH \cdot 2HCl$ | $NH(CH_2)_2NEt_2$ | $1.2 \times 10^{-7}$ |
| H | $(CH_2)_2NH(CH_2)_2OH \cdot 2HCl$ | $NH(CH_2)_2NMe_2$ | $2.3 \times 10^{-7}$ |
| H | $(CH_2)_2NH(CH_2)_2OH \cdot HCl$ | $NHCH_3$ | $2.8 \times 10^{-7}$ |
| H | $(CH_2)_2OH \cdot 2HCl$ | $NH(CH_2)_3NEt_2$ | $4.8 \times 10^{-7}$ |
| H | $(CH_2)_2NEt_2 \cdot 2HCl$ | $NH(CH_2)_2$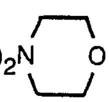 | $1.2 \times 10^{-7}$ |
| H | $(CH_2)_2NEt_2 \cdot 2HCl$ | $NH(CH_2)_3NEt_2$ | $1.8 \times 10^{-7}$ |
| H | $(CH_2)_2NEt_2 \cdot 2HCl$ | $NH(CH_2)_4NEt_2$ | $2.2 \times 10^{-7}$ |
| H | $(CH_2)_2NEt_2 \cdot 2HCl$ | $NH(CH_2)_7NEt_2$ | $2.2 \times 10^{-6}$ |
| H | $(CH_2)_2NEt_2 \cdot 3HBr$ | $NH(CH_2)_2$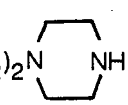 | $3.8 \times 10^{-7}$ |
| H | $(CH_2)_2NH_2 \cdot 2HCl$ | $NH(CH_2)_2NH(CH_2)_2OH$ | $6.8 \times 10^{-8}$ |
| $7,10\text{-}(OH)_2$ | $(CH_2)_2NEt_2 \cdot 2HCl$ | $NH(CH_2)_2NH(CH_2)_2OH$ | $2.7 \times 10^{-7}$ |
| $7,10\text{-}(OH)_2$ | $(CH_2)_2NEt_2 \cdot 2HCl$ | $NH(CH_2)_2NEt_2$ | $6.4 \times 10^{-7}$ |
| $7,10\text{-}(OH)_2$ | $CH_3 \cdot HCl$ | $NH(CH_2)_2NH(CH_2)_2OH$ | $7.9 \times 10^{-7}$ |
| $7,10\text{-}(OH)_2$ | $(CH_2)_2NEt_2 \cdot 2HCl$ | $NH(CH_2)_2NH_2$ | $3.8 \times 10^{-7}$ |

| X | Z | NRY | ID$_{50}$ Molar |
|---|---|---|---|
| 7,10-$(OH)_2$ | $(CH_2)_2NEt_2 \cdot 2HBr$ | $NH(CH_2)_2NHMe$ | $1.2 \times 10^{-7}$ |
| 7,10-$(OH)_2$ | $(CH_2)_2NMe_2 \cdot 2HCl$ | $NH(CH_2)_2NH(CH_2)_2OH$ | $1.8 \times 10^{-7}$ |
| 7,10-$(OH)_2$ | $CH_2CHOHCH_2NEt_2 \cdot 2HCl$ | $NH(CH_2)_2NH_2$ | $4.6 \times 10^{-7}$ |
| 7,10-$(OH)_2$ | $CH_2CHOHCH_2NEt_2 \cdot 2HCl$ | $NH(CH_2)_2NH(CH_2)_2OH$ | $6.9 \times 10^{-7}$ |
| 7,10-$(OH)_2$ | $(CH_2)_2OH \cdot HCl$ | $NH(CH_2)_2NMe_2$ | $1.7 \times 10^{-7}$ |
| 7,10-$(OH)_2$ | $(CH_2)_2NMe_2 \cdot 2HCl$ | $NH(CH_2)_2NH_2$ | $1.4 \times 10^{-7}$ |
| 7,10-$(OH)_2$ | $(CH_2)_2NH_2 \cdot 2HCl$ | $NH(CH_2)_2NH(CH_2)_2OH$ | $1.7 \times 10^{-6}$ |
| 7,10-$(OH)_2$ | $(CH_2)_2NH(CH_2)_2OH \cdot 2HCl$ | 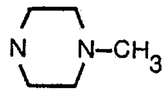 | $5.4 \times 10^{-7}$ |
| 7,10-$(OH)_2$ | $(CH_2)_2NH_2 \cdot 2HCl$ | $NH(CH_2)_2NH_2$ | $9.1 \times 10^{-7}$ |
| 7,10-$(OH)_2$ | $(CH_2)_2NMe_2 \cdot 2HCl$ | $NH(CH_2)_3NH_2$ | $1.1 \times 10^{-6}$ |
| 7,10-$(OH)_2$ | $(CH_2)_3NMe_2 \cdot 2HCl$ | $NH(CH_2)_3NH_2$ | $3.5 \times 10^{-7}$ |
| 7,10-$(OH)_2$ | $CH_2CH_2CH_2NMe_2 \cdot 2HCl$ | $NHCH_2CH_2NHCH_2CH_2OH$ | $9.2 \times 10^{-8}$ |
| 7,10-$(OH)_2$ | $CH_2CH_2NMeCH_2CH_2OH \cdot 1.6HCl$ | $NHCH_2CH_2NHCH_2CH_2OH$ | $4.5 \times 10^{-7}$ |
| 7,10-$(OH)_2$ | $CH_2CH_2NHCH_2CH_2OH \cdot 1.8HCl$ | $NHCH_2CH_2NHCH_3$ | $4.0 \times 10^{-7}$ |
| 7,10-$(OH)_2$ | $CH_2CH_2NH_2 \cdot 3.3HCl$ | $NHCH_2CH_2NHCH_2CH_2NMe_2$ | $1.2 \times 10^{-6}$ |
| 7,10-$(OH)_2$ | $CH_2CH_2NH_2 \cdot 2.1HCl$ | $NH(CH_2)_3NHCH_2CH_2OH$ | $2.8 \times 10^{-7}$ |
| 7,10-$(OH)_2$ | $CH_2CH_2NH_2 \cdot 2.9HCl$ | $NHCH_2CH_2CH_2NH_2$ | $8.5 \times 10^{-7}$ |
| 7,10-$(OH)_2$ | $CH_2CH_2CH_2NH_2 \cdot 2HCl$ | $NHCH_2CH_2NHCH_2CH_2OH$ | $7.7 \times 10^{-7}$ |
| 7,10-$(OH)_2$ | $CH_2CH_2NHCH_3 \cdot 2HCl$ | $NHCH_2CH_2NHCH_2CH_2OH$ | $2.3 \times 10^{-7}$ |
| 7-OH | $CH_2CH_2NHCH_2CH_2OH \cdot 2HCl$ | $NHCH_2CH_2NHCH_2CH_2OH$ | $6.1 \times 10^{-8}$ |
| 10-OH | $CH_2CH_2NHCH_2CH_2OH \cdot 2.1HCl$ | $NHCH_2CH_2NHCH_2CH_2OH$ | $1.1 \times 10^{-6}$ |
| 7-OH | $CH_2CH_2NHCH_2CH_2OH \cdot 1.8HCl$ | $NHCH_2CH_2NHMe$ | $2.7 \times 10^{-8}$ |

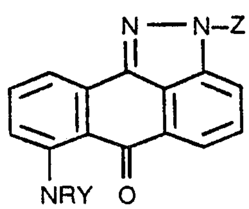

| Z | NRY | ID$_{50}$ Molar |
|---|---|---|
| $(CH_2)_2NEt_2 \cdot 2HCl$ | $NH(CH_2)_2NEt_2$ | $2.2 \times 10^{-7}$ |
| $(CH_2)_2NEt_2 \cdot 2HCl$ | $NH(CH_2)_2NH(CH_2)_2OH$ | $9.3 \times 10^{-8}$ |

When being utilized as antibiotics and antifungal agents, the compounds of the invention can be prepared and administered in a wide variety of topical, oral, and parenteral dosage forms. It will be clear to those skilled in the art that the following dosage forms may comprise as the active component, either a compound of formula I, certain of the compounds of formula II or a corresponding pharmaceutically acceptable salt of one of said compounds or a mixture of such compounds and/or salts.

For preparing pharmaceutical compositions from the compounds described by this invention, inert, pharmaceutically acceptable carriers can be either solid or liquid. Solid form preparations include powders, tablets, dispersible granules, capsules, cachets, and suppositories. A solid carrier can be one or more substances which may also act as diluents, flavoring agents, solubilizers, lubricants, suspending agents, binders, or tablet disintegrating agents; it can also be encapsulating material. In powders, the carrier is a finely divided solid which is in admixture with the finely divided active compound. In the tablet the active compound is mixed with carrier having the necessary binding properties in suitable proportions and compacted in the shape and size desired. The powders and tablets preferably contain from 5 or 10 to about 70 percent of the active ingredient. Suitable solid carrriers are magnesium carbonate, magnesium stearate, talc, sugar, lactose, pectin, dextrin, starch, gelatin, tragacanth, methyl cellulose, sodium carboxymethyl cellulose, a low melting wax or cocoa butter. The term "preparation" is intended to include the formulation of the active compound with encapsulating material as carrier providing a capsule in which the active component (with or without other carriers) is surrounded by carrier, which is thus in association with it. Similarly, cachets are included. Tablets, powders, cachets, and capsules can be used as solid dosage forms suitable for oral administration.

Liquid form preparations include solutions, suspensions, and emulsions. As an example may be mentioned water or water-propylene glycol solutions for parenteral injection. Liquid preparations can also be formulated in solution in aqueous polyethylene glycol solution. Aqueous solutions suitable for oral use can be prepared by dissolving the active component in water and adding suitable colorants, flavors, stabilizing, and thickening agents as desired. Aqueous suspensions suitable for oral use can be made by dispersing the finely divided active component in water with viscous material, i.e., natural or synthetic gums, resins, methyl cellulose, sodium carboxymethyl cellulose, and other well-known suspending agents.

Topical preparations include dusting powders, creams, lotions, gels, and sprays. These various topical preparations may be formulated by well known procedures. See for example Remington's Pharmaceutical Sciences, Chapter 43, 14th ed. 1970, Mack Publishing Co., Easton Pennsylvania 18042, USA.

Preferably, the pharmaceutical preparation is in unit dosage form. In such form, the preparation is subdivided into unit doses containing appropriate quantities of the active component. The unit dosage form can be a packaged preparation, the package containing discrete quantities of preparation, for example, packeted tablets, capsules, and powders in vials or ampoules. The unit dosage form can also be a capsule, cathet, or tablet itself or it can be the appropriate number of any of these packaged form.

The quantity of active compound in a unit dose of preparation may be varied or adjusted from 50 mg to 500 mg according to the particular application and the potency of the active ingredient.

In therapeutic use as antibiotic and antifungal agents the compounds of this invention are administered at the initial dosage of about 0.1 mg to about 50 mg per kilogram. A dose range of about 0.5 mg to about 10 mg per kilogram is preferred. The dosages, however, may be varied depending upon the requirements of the patient, the severity of the condition being treated, and the compound being employed. Determination of the proper dosage for a particular situation is within the skill of the art. Generally, treatment is initiated with smaller dosages which are less than the optimum dose of the compound. Thereafter, the dosage is increased by small increments until the optimum effect under the circumstances is reached. For convenience, the total daily dosage may be divided and administered in portions during the day if desired.

The active compounds may also be administered parenterally or intraperitoneally. Solutions of the active compound as a free base or pharmaceutically acceptable salt can be prepared in water suitably mixed with a surfactant such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol), suitable mixtures thereof and vegetable oils. The proper fluidity can be maintained, for example, by the use of a coating such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid or thimerosal. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active compound in the required amount in the appropriate solvent with various of the other ingredients enumerated above, as required, followed by filtered sterilization. Generally, dispersions are prepared by incorporating the various sterilized active ingredient into a sterile vehicle which contains the basic dispersion medium and the required other ingredients from those enumerated above. In the case of the sterile powders for the preparation of sterile injectable solutions, the preferred methods of preparation are vacuum drying and the freeze-drying technique which yield a powder of the active ingredient plus any additional desired ingredient from a previously sterile-filtered solution thereof.

As used herein, "pharmaceutically acceptable carrier" includes any and all solvents, dispersion media, coatings, antibacterial and antifungal agents, isotonic and absorption delaying agents. The use of such media and agents for pharmaceutically active substances is well known in the art. Except insofar as any conventional media or agent is incompatible with the active ingredient, its use in the therapeutic compositions is contemplated. Supplementary active ingredients can also be incorporated into the compositions.

It is especially advantageous to formulate parenteral compositions in dosage unit form for ease of administration and uniformity of dosage. Dosage unit form as used herein refers to physically discrete units suitable as unitary dosages for the mammalian subjects to be treated; each unit containing a predetermined quantity of active material calculated to produce the desired therapeutic effect in association with the required pharmaceutical carrier. The specification for the novel dosage unit forms of the invention are dictated by and directly dependent on (a) the unique characteristics of the active material and the particular therapeutic effect to be achieved, and (b) the limitation inherent in the art of compounding such an active material for the treatment of disease in living subjects having a diseased condition in which bodily health is impaired as herein disclosed in detail.

The principal active ingredient is compounded for convenient and effective administration in effective amounts with a suitable pharmaceutically-acceptable carrier in dosage unit form as hereinbefore disclosed. A unit dosage form can, for example, contain the principal active compound in amounts ranging from about 0.1 to about 500 mg, with from about 0.5 to about 250 mg being preferred. Expressed in proportions, the active compound is generally present in from about 0.1 to about 500 mg/ml of carrier. In the case of compositions containing supplementary active ingredients, the dosages are determined by reference to the usual dose and the manner of administration of the said ingredients. The daily perenteral doses for mammalian subjects to be treated ranges from 0.1 mg/kg to 100 mg/kg. The preferred daily dosage range is 0.3 mg/kg to 10 mg/kg.

The following nonlimiting examples illustrate the preferred methods for preparing the compounds of the invention.

## Example 1

2-[2-(Diethylamino)ethyl)]-5-[[2-(diethylamino)ethyl]amino]anthra[1,9-*cd*]pyrazol-6(2H)-one

A mixture of 1.2 g (3.4 mmol) of 5-chloro-2-[2-(diethylamino)ethyl]anthra[1,9-*cd*]pyrazol-6(2*H*)-one, 1.0 g (8 mmol) of *N,N*-diethylethylenediamine, about 1 mg of anhydrous cuprous chloride and 30 ml of anhydrous 2-ethoxyethanol is heated at reflux under argon. After seven hours, an additional 0.5 g (4 mmol) of the diamine and about 1 mg of catalyst is added and the mixture is refluxed for 23 hours, cooled, and concentrated. The residue is dissolved in dichloromethane, washed successively with water, dilute ammonium hydroxide, and brine. Chromatography of the dried dichloromethane layer over silica gel with 10:1:89 methanol:triethylamine:dichloromethane provides the purified product. Dissolution in hot 2-propanol followed by treatment with excess hydrogen chloride in 2-propanol affords 1.2 g of the dried product as a salt with 2.1 equivalents of hydrogen chloride solvated with 1.2 equivalents of water; mp 262—276°C (decomposition).

5-Chloro-2-[2-(diethylamino)ethyl]anthra[1,9-*cd*]pyrazol-6(2*H*)-one is prepared as follows:

A mixture of 4.15 g (15 mmol) of 1,4-dichloro-9,10-anthracenedione [*J. Amer. Chem. Soc. 48;* 3198 (1926)] 2.6 g (20 mmol) of (2-diethylaminoethyl)hydrazine [*J. Med. Chem.,* 1; 493, (1964)], and 35 ml of pyridine is heated at reflux for ten hours, cooled, and concentrated. The residue is dissolved in dichloromethane and washed with water. Chromatography of the dried dichloromethane layer over silica gel with ethyl acetate and then 95:5 ethyl acetate:methanol affords 3.8 g of a solid whose crystallization from 2-propanol gives 2.9 g of pure material; mp 90—92°C.

Dissolution of 0.89 g of the product in hot 2-propanol followed by treatment with excess hydrogen chloride in 2-propanol affords 0.9 g of the hydrochloride salt; mp 263—266°C (decomposition).

## Example 2

2-[2-(Diethylamino)ethyl)]-5-[[2-[(2-hydroxyethyl)amino]ethyl]amino]anthra[1,9-*cd*]pyrazol-6(2*H*)-one

A mixture of 2.5 g (7.1 mmol) of 5-chloro-2-[2-(diethylamino)ethyl]anthra[1,9-*cd*]pyrazol-6(2*H*)-one, 1 g (9 mmol) of 2-(2-aminoethylamino)ethanol and catalytic amounts of anhydrous cuprous chloride and potassium iodide in 25 ml of anhydrous 2-ethoxyethanol is heated at reflux under argon. Additional 0.5—1.0 g portions of the amine and catalytic amounts of the halide salts are added after six and 12 hours, respectively. After a total reflux time of 30 hours, the mixture is worked up as described for Example 1, with purification on silica gel utilizing first 10:1:89 and then 15:1:84 methanol:triethylamine:ethyl acetate.

Following treatment with hydrogen chloride, there is obtained 1.1 g of the dried product as a salt with 2.0 equivalents of hydrogen chloride solvated with 2.4 equivalents of water; mp 239—241°C (decomposition).

Example 3

5-[(2-Aminoethyl)amino]-2-[2-(diethylamino)ethyl]anthra[1,9-*cd*]pyrazol-6(2*H*)-one

A mixture of 1.6 g (4.5 mmol) of 5-chloro-2-[2-(diethylamino)ethyl]anthra[1,9-*cd*]pyrazol-6(2*H*)-one, 2.5 ml of anhydrous ethylenediamine, and 25 ml of anhydrous pyridine is heated at reflux under argon for seven hours, cooled, diluted with toluene, and concentrated. The solid residue is dissolved in dichloromethane, washed with water, and then brine. Chromatography of the dried dichloromethane layer over silica gel with 1:9 methanol:dichloromethane provides 0.8 g of the product. Dissolution in hot 2-propanol followed by treatment with excess hydrogen chloride in 2-propanol affords 1.0 g of dried product as a salt with 2.0 equivalents of hydrogen chloride solvated with 1.8 equivalents of water; mp 276—279°C (decomposition).

The following compounds are prepared as described in Example 3 from 5-chloro-2-[2-(diethylamino)-ethyl]anthra[1,9-*cd*]pyrazol-6(2*H*)-one and the corresponding amine:

Example 4

2-[2-(Diethylamino)ethyl]-[[2-(4-morpholinyl)ethyl)]amino]anthra[1,9-*cd*]pyrazol-6(2*H*)-one

Reaction with 4-(2-aminoethyl)morpholine gives the product as a salt with 2.0 equivalents of hydrogen chloride solvated with 1.2 equivalents of water; mp 288—290°C (decomposition).

Example 5

2-[2-(Diethylamino)ethyl]-5-[[3-(diethylamino)propyl]amino]anthra[1,9-*cd*]pyrazol-6(2*H*)-one

Reaction with *N,N*-diethyl-1,3-propanediamine gives the product as a salt with 2.0 equivalents of hydrogen chloride solvated with 0.2 equivalent of water; mp 270—272°C (decomposition).

Example 6

2-[2-(Diethylamino)ethyl]-5-[[7-(diethylamino)heptyl]amino]anthra[1,9-*cd*]pyrazol-6(2*H*)-one

Reaction with *N,N*-diethyl-1,7-heptanediamine gives the product as a salt with 2.0 equivalents of hydrogen chloride solvated with 0.3 equivalent of water; mp 190—192°C (decomposition).

Example 7

5-[[4-(Diethylamino)butyl]amino]-2-[2-(diethylaminoethyl]anthra[1,9-*cd*]pyrazol-6(2*H*)-one

Reaction with *N,N*-diethyl-1,4-butanediamine gives the product as a salt with 2.0 equivalents of hydrogen chloride solvated with 0.7 equivalent of water; mp 243—246°C (decomposition).

Example 8

2-[2-(Diethylamino)ethyl]-5-(hexylamino)anthra[1,9-*cd*]pyrazol-6(2*H*)-one

Reaction with *n*-hexylamine gives the product as a salt with 1.0 equivalent of hydrogen chloride solvated with 0.1 equivalent of water; mp 176—179°C (decomposition).

Example 9

2-[2-(Diethylamino)ethyl]-5-[[2-(1-piperazinyl)ethyl]amino]anthra[1,9-*cd*]pyrazol-6(2*H*)-one

Reaction with 4-(2-aminoethyl)-1-piperazine carboxylic acid, benzyl ester, then hydrolysis of the isolated intermediates with hot 48% hydrobromic acid in acetic acid gives the product as a salt with 3.3 equivalents of hydrogen bromide solvated with 1.4 equivalents of water and 0.1 equivalent of acetic acid; mp 284—287°C (decomposition).

4-(2-Aminoethyl)-1-piperazine carboxylic acid, benzyl ester, is prepared from 4-(2-aminoethyl)-1-piperazine by a procedure analogous to that described for the preparation of (2-aminoethyl)-methylcarbamic acid, benzyl ester, in US Patent 3,931,268; $^1$H NMR (deuteriochloroform): δ 2.78 (triplet), 5.08 (singlet), 7.30 (singlet).

Example 10

5-[[2-[Diethylamino)ethyl]amino]-2-methylanthra[1,9-*cd*]pyrazol-6(2*H*)-one

A mixture of 1.88 g (7 mmol) of 5-chloro-2-methyleanthra[1,9-*cd*]pyrazol-6(2*H*)-one [*J. Chem. Soc.,* 1630 (1952)], 1.2 g (10 mmol) of *N,N*-diethylethylenediamine, 0.14 g of anhydrous potassium fluoride, and 10 ml of dimethylsulfoxide is heated at reflux under argon for four hours, cooled, diluted with water, and extracted with dichloromethane. The dichloromethane extract is washed twice with brine and then with 5% aqueous hydrochloric acid. The acid solution is washed with dichloromethane, made basic with sodium carbonate, and extracted with dichloromethane. The dried dichloromethane layer is clarified with charcoal, filtered, and concentrated to a residue. The salt was made as described in Example 3 to afford 1.1 g of the dried product, after thorough washing with ether, as a salt with 1.8 equivalents of hydrogen chloride solvated with 1.8 equivalents of hydrogen chloride solvated with 0.7 equivalent of water; mp 260—264°C (decomposition).

Example 11

5-[[2-[(2-Hydroxyethyl)amino]ethyl]amino]-2-methylanthra[1,9-*cd*]pyrazol-6(2*H*)-one

A mixture of 1.75 g (6.5 mmol) of 5-chloro-2-methylanthra[1,9-*cd*]pyrazol-6(2*H*)-one, 6 ml (59 mmol) of 2-(2-aminoethylamino)ethanol, catalytic amounts of anhydrous cuprous chloride and potassium iodide, and 25 ml of 2-methoxyethanol is heated at reflux under argon for four hours, cooled, and concentrated. The residue is dissolved in dichloromethane, washed with water, and then with 5% aqueous hydrochloric acid. The acid solution is washed with dichloromethane, made basic, and extracted into dichloromethane. Chromatography of the dried dichloromethane extract over silica gel with gradient elution employing 5—15% methanol in dichloromethane provides the purified product. The salt was made as described in Example 3 to afford 0.69 g of the dried product as a salt with 1.0 equivalent of hydrogen chloride solvated with 0.1 eqivalent of water; mp 270—272°C (decomposition).

Example 12

2-(2-Hydroxyethyl)-5-[[2-[(2-hydroxyethyl)amino]ethyl]amino]anthra[1,9-*cd*]pyrazol-6(2*H*)-one

A mixture of 896 mg (3 mmol) of 5-chloro-2-(2-hydroxyethyl)anthra[1,9-*cd*]pyrazol-6(2*H*)-one, 3.1 ml (30 mmol) of 2-(2-aminoethylamino)ethanol, and 6 ml of anhydrous pyridine is heated at reflux under argon for 8.5 hours, cooled, and concentrated to leave a residue. Trituration from ether:2-propanol leaves a gummy solid which upon further trituration from methanol-ether provides 851 mg of the product. Dissolution in chloroform followed by treatment with excess hydrogen chloride in 2-propanol affords 923 mg of the dried product as a salt with 1.6 equivalents of hydrogen chloride solvated with 0.5 equivalent of water; mp 267—272°C (decomposition).

5-Chloro-2-(2-hydroxyethyl)anthra[1,9-*cd*]pyrazol-6(2*H*)-one is prepared as follows:

A mixture of 5.54 g (20 mmol) of 1,4-dichloro-9,10-anthracenedione, 2.2 ml (33.3 mmol) of (2-hydroxyethyl)hydrazine and 20 ml of dry pyridine is stirred at 60°C for 32 hours and concentrated. A solid residue is triturated with ether and then crystallized from chloroform to give 3.58 g of product; mp 209—211°C. Processing of the mother liquor affords 0.21 g of additional product; mp 208—210°C.

Example 13

5-[[2-(Diethylamino)ethyl]amino]-2-(2-hydroxyethyl)anthra[1,9-*cd*]pyrazol-6(2*H*)-one

Reaction of 896 mg (3 mmol) of 5-chloro-2-(2-hydroxyethyl)anthra[1,9-*cd*]pyrazol-6(2*H*)-one, 4.2 ml (30 mmol) of *N,N*-diethylethylenediamine, and 6 ml of anhydrous pyridine as described in Example 12 gives 1.02 g of the dried product as a salt with 1.75 equivalents of hydrogen chloride solvated with 0.5 equivalent of water; mp 199—205°C (decomposition).

The following compounds are prepared as described in Example 12 from 5-chloro-2-(2-hydroxyethyl)-anthra[1,9-*cd*]pyrazol-6(2*H*)-one and the corresponding amine:

Example 14

2-(2-Hydroxyethyl)-5-[[2-(4-morpholinyl)ethyl]amino][1,9-*cd*]pyrazol-6(2*H*)-one

Reaction with 4-(2-aminoethyl)morpholine gives the product as a salt with 1.9 equivalents of hydrogen chloride solvated with 0.5 equivalent of water; mp 260°C (decomposition).

Example 15

5-[[3-(Diethylamino)propyl]amino]-2-(2-hydroxyethyl)anthra[1,9-*cd*]pyrazol-6(2*H*)-one

Reaction with *N,N*-diethyl-1-1,3-propanediamine gives the product as a salt with 2.0 equivalents of hydrogen chloride solvated with 0.5 equivalent of water; mp 201—210°C (decomposition).

Example 16

5-[[4-(Diethylamino)butyl]amino]-2-(2-hydroxyethyl)anthra[1,9-*cd*]pyrazol-6(2*H*)-one

Reaction with *N,N*-diethyl-1,4-propanediamine gives the product as a salt with 1.9 equivalents of hydrogen chloride solvated with 1.0 equivalent of water; mp 155—185°C (decomposition).

Example 17

5-[[7-(Diethylamino)heptyl]amino]-2-(2-hydroxyethyl)anthra[1,9-*cd*]pyrazol-6(2*H*)-one

Reaction with *N,N*-diethyl-1,7-heptanediamine gives the product as a salt with 1.0 equivalent of hydrogen chloride; mp 206—208°C (decomposition).

Example 18

2-(2-Hydroxyethyl)-5-[[2-(1-piperazinyl)ethyl]amino]anthra[1,9-*cd*]pyrazol-6(2*H*)-one

Reaction with 4-(2-aminoethyl)-1-piperazine carboxylic acid, benzyl ester, then hydrolysis of the isolated intermediate with refluxing 48% hydrobromic acid in acetic acid and salt formation gives the product as a salt with 2.0 equivalents of hydrogen chloride solvated with 0.5 equivalent of water; mp 292—297°C (decomposition).

Example 19

5-[[2-[(2-Hydroxyethyl)amino]ethyl]amino]anthra[1,9-*cd*]pyrazol-6-(2*H*)-one

A mixture of 2.54 g (10 mmol) of 5-chloroanthra-[1,9-*cd*]pyrazol-6(2*H*)-one [*J. Chem. Soc.,* 1630 (1952)], 10 ml (100 mmol) of 2-(2-aminoethylamino)ethanol, and 25 ml of anhydrous pyridine is heated at reflux under argon for 24 hours, cooled, and concentrated. The residue is triturated with 2-propanol to give a solid whose dissolution in methanol:dichloromethane followed by salt formation as described in Example 3 affords 1.5 g of the dried product as a salt with 1.5 equivalents of hydrogen chloride solvated with 0.6 equivalent of water; mp 251—254°C (decomposition).

Example 20

5-[[2-(Diethylamino)ethyl]amino]anthra[1,9-*cd*]pyrazol-6(2*H*)-one

A mixture of 1.6 g (6.3 mmol) of 5-chloroanthra-[1,9-*cd*]pyrazol-6(2*H*)-one, 3.5 g (30 mmol) of *N,N*-diethylethylenediamine, and 20 ml of anhydrous pyridine is heated at reflux for 20 hours, cooled, and concentrated. The residue is dissolved in dichloromethane, washed with water, and then extracted with 1% aqueous hydrochloric acid. The acid solution is washed with dichloromethane, then made basic with aqueous sodium hydroxide. The aqueous solution is extracted with dichloromethane and the dried dichloromethane layer is concentrated to a residue which is converted into a salt as described for Example 3 to give 0.7 g of the dried product as a salt with 1.4 equivalents of hydrogen chloride solvated with 0.1 equivalent of water; mp 120—130°C.

Example 21

2-[2-[(2-Hydroxyethyl)amino]ethyl]-5-[[2-[(2-hydroxyethyl)amino]ethyl]amino]anthra[1,9-*cd*]pyrazol-6(2*H*)-one

A mixture of 1.91 g (5 mmol) of 5-chloro-2-[2-[(2-hydroxyethyl)amino]ethyl]anthra[1,9-*cd*]pyrazol-6(2*H*)-one, hydrochloride, 2.6 ml of 2-(2-aminoethylamino)ethanol, and 5 ml of anhydrous pyridine is heated at reflux under argon for 6.5 hours, cooled, and concentrated. Trituration of the solid residue with cold 2-propanol gives 1.43 g of the dried product; mp 154—156°C. Crystallization from glacial acetic acid: 2-propanol gives 1.35 g of the dried product as a salt with 1.0 equivalent of acetic acid solvated with 0.5 equivalent of water; mp 146—148°C.

5-Chloro-2-[2-[(2-hydroxyethyl)amino]ethyl]anthra[1,9-*cd*]pyrazol-6(2*H*)-one is prepared as follows:

To a refluxing mixture of 832 mg (3 mmol) of 1,4-dichloro-9,10-anthracenedione in 8 ml of dry acetonitrile is added dropwise over 40 minutes, 450 mg (3.8 mmol) of 2-[(hydrazinoethyl)amino]ethanol in 3 ml of acetonitrile. The mixture is refluxed for one hour, cooled, and triturated with cold 2-propanol to give 602 mg of product; mp 140—142°C. Processing of the mother liquor affords 71 mg of additional product; mp 124—126°C. Crystallization of the free base from glacial acetic acid gives the diacetate salt; mp 125—130°C. The hydrochloride salt is prepared as described in Example 3; mp 260—263°C (decomposition).

2-[(Hydrazinoethyl)amino]ethanol is prepared as follows:

A solution of 86.8 g (1.0 mol) of *N*-(2-hydroxyethyl)ethyleneimine and 400 ml (about 6 mol) of 54% aqueous hydrazine is heated at reflux for two days. Excess water and hydrazine is distilled at 40—50°C/13 mm, then the pot residue is distilled at 142°C/0.10 mm to yield 80.9 g of product with an 88% purity. Careful redistillation of a small sample gives analytically pure material; bp 120°C/0.035 mm.

Example 22

5-[(2-Aminoethyl)amino]-2-[2-[(2-hydroxyethyl)amino]ethyl]anthra[1,9-*cd*]pyrazol-6(2*H*)-one

Reaction of 1.91 g of 5-chloro-2-[2-[(2-hydroxyethyl)amino]ethyl]anthra[1,9-*cd*]pyrazol-6(2*H*)-one hydrochloride with 1.6 ml (25 mmol) of 1,2-ethylenediamine, as described in Example 21, followed by concentration affords a solid which is washed with ether, 2-propanol, and a little dichloromethane, then triturated with methanol to remove a solid impurity. The concentrated filtrate is dissolved in water and purified over a column of HP-20 resin eluting first with water and then with methanol. Concentration of the methanol eluate followed by salt formation as described in Example 3 affords 1.0 g of the dried product as a salt with 2.0 equivalents of hydrogen chloride solvated with 0.9 equivalent of water; mp 263—267°C (decomposition).

Example 23

5-[[2-(Diethylamino)ethyl]amino]-2-[2-[(2-hydroxyethyl)amino]ethyl]anthra[1,9-*cd*]pyrazol-6(2*H*)-one

Reaction of 1.91 g of 5-chloro-2-[2-[(2-hydroxyethyl)amino]ethyl]anthra[1,9-*cd*]pyrazol-6(2*H*)-one, hydrochloride, with 3.5 ml (25 mmol) of *N,N*-diethylenediamine as described in Example 21 affords 1.4 g of product; mp 132—133. Processing of the mother liquor affords 0.3 g of additional product; mp 130—131°C. Salt formation as described for Example 3 gives 1.6 g of the dried product as a salt with 2.0 equivalents of hydrogen chloride solvated with 1.0 equivalent of water; mp 272—274°C (decomposition).

Example 24

5-[(2-Hydroxyethyl)amino]-2-[2-[(2-hydroxyethyl)amino]ethyl]anthra[1,9-*cd*]pyrazol-6(2*H*)-one

A mixture of 2.5 g (6.6 mmol) of 5-chloro-2-[2-[(2-hydroxyethyl)amino]ethyl]anthra[1,9-*cd*]pyrazol-6(2*H*)-one, hydrochloride, 2 ml (33 mmol) of 2-aminoethanol and 13 ml of anhydrous pyridine is reacted

and worked up as described in Example 21 to afford a solid precipitate whose salt formation as described in Example 3 affords 1.4 g of the dried product as a salt with 1.1 equivalents of hydrogen chloride solvated with 0.6 equivalent of water; mp 260—261°C (decomposition).

Example 25

2-[2-[(2-Hydroxyethyl)amino]ethyl]-5-[[2-(dimethylamino)ethyl]amino]anthra[1,9-*cd*]pyrazol-6(2*H*)-one

Reaction of 2.72 g (7 mmol) of 5-chloro-2-[2-[(2-hydroxyethyl)amino]ethyl]anthra[1,9-*cd*]pyrazol-6(2*H*)-one, hydrochloride, 1.2 g (14 mmol) of *N,N*-dimethylethylenediamine, and 20 ml of pyridine for 42 hours at reflux followed by workup as described in Example 21 gives a solid residue whose dissolution in hot methanol followed by salt formation as described in Example 3 gives 1.0 g of the dried product as a salt with 2.1 equivalents of hydrogen chloride solvated with 0.9 equivalent of water; mp 286—288°C (decomposition).

Example 26

2-[2-[(2-Hydroxyethyl)amino]ethyl]-5-(methylamino)anthra[1,9-*cd*]pyrazol-6(2*H*)-one

Reaction of 5-chloro-2-[2-[(2-hydroxyethyl)amino]ethyl]anthra[1,9-*cd*]pyrazol-6(2*H*)-one, hydrochloride, with excess methylamine as described in Example 21 gives the product as a salt with 1.0 equivalent of hydrogen chloride; mp 285—288°C (decomposition).

Example 27

2-(2-Aminoethyl)-5-[[2-[(2-hydroxyethyl)amino]ethyl]amino]anthra[1,9-*cd*]pyrazol-6(2*H*)-one

A mixture of 3.0 g (8.9 mmol) of 2-(2-aminoethyl)-5-chloroanthra[1,9-*cd*]pyrazol-6(2*H*)-one, 2.0 ml of 2-(2-aminoethylamino)ethanol, and 15 ml of anhydrous pyridine is heated at reflux for 30 hours, cooled, and filtered. The filtrate is concentrated and chromatographed over silica gel with 99:2:1 dichloromethane:methanol:triethylamine, then gradient elution to 99:20:1 to provide the purified product. Salt formation as described in Example 3 gives 0.8 g of the dried product as a salt with 1.7 equivalents of hydrogen chloride solvated with 1.0 equivalent of water and 0.2 equivalent of 2-propanol; mp 270—272°C (decomposition).

2-(2-Aminoethyl)-5-chloroanthra[1,9-*cd*]pyrazol-6(2*H*)-one is prepared as follows:

To a solution of 1.0 g (3.6 mmole) of 1,4-dichloro-9,10-anthracenedione in 10 ml of pyridine at 35° is added dropwise 1.9 ml of (2-aminoethyl)hydrazine [British Patent 880,332]. The mixture is stirred for four hours, concentrated, and purified on silica gel utilizing 94:5:1 dichloromethane:methanol:triethylamine. Salt formation as described in Example 3 gives 0.45 g of the product as a salt with 1.0 equivalent of hydrogen chloride solvated with 1.2 equivalents of water and 0.1 equivalent of 2-propanol; mp 284—285°C (decomposition).

Example 28

2-[2-(Diethylamino)ethyl]-7,10-dihydroxy-5-[[2-[(2-hydroxyethyl)amino]ethyl]amino]anthra[1,9-*cd*]pyrazol-6(2*H*)-one

Reaction of a mixture of 2.9 g (7.5 mmol) of 5-chloro-2-[2-(diethylamino)ethyl]-7,10-dihydroxy-anthra[1,9-*cd*]pyrazol-6(2*H*)-one, 7.5 ml (75 mmol) of 2-(2-aminoethylamino)ethanol, and 35 ml of pyridine for four hours at reflux followed by workup as described in Example 21 and salt formation as described in Example 3 gives 2.8 g of the dried product as a salt with 2.0 equivalents of hydrogen chloride solvated with 0.7 equivalent of water; mp 198—202°C (decomposition).

5-Chloro-2-[2-(diethylamino)ethyl]-7,10-dihydroxyanthra[1,9-*cd*]pyrazol-6(2*H*)-one is prepared as follows:

Reaction of a mixture of 12.7 g (41 mmol) of 1,4-dichloro-5,8-dihydroxy-9,10-anthracenedione, 12 g (90 mmol) of (2-diethylaminoethyl)hydrazine, and 65 ml of pyridine at 50°C for four hours followed by workup as described in Example 22 gives a residue that is dissolved in dichloromethane. Chromatography over silica gel with dichloromethane and then with 3% methanol in dichloromethane affords crude material whose crystallization from 2-propanol gives 6.5 g of a purified solid; mp 136—140°C. Salt formation as described in Example 3 on 1.5 g of this material gives 1.3 g of a dried solid as a salt with 1.0 equivalent of hydrogen chloride solvated with 0.3 equivalent of water; mp 280—282°C (decomposition).

Example 29

2-[2-(Diethylamino)ethyl]-5-[[2-(diethylamino)ethyl]amino]-7,10-dihydroxyanthra[1,9-*cd*]pyrazol-6(2*H*)-one

Reaction of a mixture of 1.93 g (5 mmol) of 5-chloro-2-[2-(diethylamino)ethyl]-7,10-dihydroxyanthra[1,9-*cd*]pyrazol-6(2*H*)-one, 2.9 g (24 mmol) of *N,N*-diethylethylenediamine, and 25 ml of pyridine for five hours at reflux followed by workup as described in Example 21 gives a crude solid which is dissolved in dichloromethane. Chromatography over silica gel with 3%, 6%, and 10% solutions of methanol in dichloromethane affords 1.6 g of pure material. Salt formation as described in Example 3 gives 1.4 g of the dried product as a salt with 2.0 equivalents of hydrogen chloride solvated with 0.3 equivalent of water; mp 290—292°C (decomposition).

Example 30

5-[(2-Aminoethyl)amino]-2-[2-(diethylamino)ethyl]-7,10-dihydroxyanthra[1,9-*cd*]pyrazol-6(2*H*)-one

Reaction of 5-chloro-2-[2-(diethylamino)ethy]-7,10-dihydroxyanthra[1,9-*cd*]pyrazol-6(2*H*)-one with ethylenediamine as described in Example 28 gives the product as a salt with 2.0 equivalents of hydrogen

chloride solvated with 1.7 equivalents of water and 0.1 equivalent of 2-propanol; mp 277—281°C (decomposition).

Example 31

2-[2-(Diethylamino)ethyl]-7,10-dihydroxy-5-[[2-(methylamino)ethyl]amino]anthra[1,9-*cd*]pyrazol-6(2*H*)-one

Reaction of 5-chloro-2-[2-(diethylamino)ethyl]-7,10-dihydroxyanthra[1,9-*cd*]pyrazol-6(2*H*)-one with (2-aminoethyl)-methylcarbamic acid, benzyl ester [US Patent 3,931,268] followed by isolation of the intermediate as described in Example 29 then hydrolysis with hot 48% hydrobromic acid in acetic acid gives the product as a salt with 2.3 equivalents of hydrogen bromide solvated with 2.7 equivalents of water; mp 217—220°C (decomposition).

Example 32

2-[2-(Dimethylamino)ethyl]-7,10-dihydroxy-5-[[2-[(2-hydroxyethyl)amino]ethyl]amino]anthra[1,9-*cd*]pyrazol-6(2*H*)-one

Reaction of 2.0 g (5.6 mmol) of 5-chloro-2-[2-(dimethylamino)ethyl]-7,10-dihydroxyanthra[1,9-*cd*]pyrazol-6(2*H*)-one, 5.6 ml of 2-(2-aminoethylamino)ethanol, and 20 ml of pyridine at 70°C for 24 hours followed by workup as described in Example 21 and salt formation as described in Example 3 gives 2.4 g of the dried product as a salt with 2.4 equivalents of hydrogen chloride solvated with 2.0 equivalents of water; mp 310—313°C (decomposition).

5-Chloro-2-[2-(dimethylamino)ethyl]-7,10-dihydroxyanthra[1,9-*cd*]pyrazol-6(2*H*)-one is prepared as follows:

Reaction of a mixture of 15.5 g (50 mmol) of 1,4-dichloro-5,8-dihydroxy-9,10-anthracenedione, 10.3 g (100 mmol) of (2-dimethylaminoethyl)hydrazine. [*J. Med. Chem., 1;* 493 (1964)] and 60 ml of pyridine at 35°C overnight followed by workup as described in Example 28 gives 3.8 g of product; mp 143—146°C. Salt formation as described in Example 3 gives the product as a salt with 1.1 equivalents of hydrogen chloride solvated with 1.2 equivalents of water; mp 295—300°C (decomposition).

The following compounds are prepared as described in Example 32 from 5-chloro-2-[2-(dimethylamino)ethyl]-7,10-dihydroxyanthra[1,9-*cd*]pyrazol-6(2*H*)-one and the corresponding amine:

Example 33

5-[(2-Aminoethyl)amino]-2-[2-(dimethylamino)ethyl]-7,10-dihydroxyanthra[1,9-*cd*]pyrazol-6(2*H*)-one

Reaction with ethylenediamine gives the product as a salt with 1.9 equivalents of hydrogen chloride solvated with 2.4 equivalents of water; mp 300—302°C (decomposition).

Example 34

5-[(3-Aminopropyl)amino]-2-[2-(dimethylamino)ethyl]-7,10-dihydroxyanthra[1,9-*cd*]pyrazol-6(2*H*)-one

Reaction with 1,3-propanediamine gives the product as a salt with 1.9 equivalents of hydrogen chloride solvated with 1.4 equivalents of water; mp 281—285°C (decomposition).

Example 35

5-[[2-(Diethylamino)ethyl]amino]-7,10-dihydroxy-2-(2-hydroxyethyl)anthra[1,9-*cd*]pyrazol-6(2*H*)-one

Reaction of a mixture of 3.3 g (10 mmol) of 5-chloro-7,10-dihydroxy-2-(2-hydroxyethyl)anthra[1,9-*cd*]pyrazol-6(2*H*)-one, 14.5 ml (100 mmol) of *N,N*-diethylethylenediamine, and 20 ml of pyridine for three hours at reflux followed by workup as described in Example 21 gives 2.47 g of a solid, mp 197—200°C. Salt formation as described in Example 12 affords 2.21 g of the dried product as a salt with 1.6 equivalents of hydrogen chloride solvated with 0.6 equivalent of water; mp 215—219°C (decomposition).

5-Chloro-7,10-dihydroxy-2-(2-hydroxyethyl)anthra[1,9-*cd*]pyrazol-6(2*H*)-one is prepared as follows:

A mixture of 12 g (40 mmol) of 1,4-dichloro-5,8-dihydroxy-9,10-anthracenedione, 4.5 g (60 mmol) of (2-hydroxyethyl)hydrazine, and 40 ml of pyridine is stirred at 50°C overnight, cooled, and concentrated. The residue is triturated successively with chloroform and hot methanol to give 1.1 g of the dried product; mp 231—234°C.

Example 36

5-[[2-[(2-Hydroxyethyl)amino]ethyl]amino]-7,10-dihydroxy-2-(2-hydroxyethyl)anthra[1,9-*cd*]pyrazol-6(2*H*)-one

A mixture of 3.3 g (10 mmol) of 5-chloro-7,10-dihydroxy-2-(2-hydroxyethyl)anthra[1,9-*cd*]pyrazol-6(2*H*)-one, 10.4 g (100 mmol) of 2-(2-aminoethylamino)ethanol, and 20 ml of pyridine is heated at reflux for four hours, cooled, and concentrated. Successive trituration of the residue with acetonitrile, 2-propanol, and methanol gives 1.35 g of a powder. Salt formation as described in Example 12 affords 1.06 g of the dried product as a salt with 1.0 equivalent of hydrogen chloride solvated with 0.5 equivalent of water; mp 196—203°C (decomposition).

The following compounds are prepared as described in Example 35 from 5-chloro-7,10-dihydroxy-2-(2-hydroxyethyl)anthra[1,9-*cd*]pyrazol-6(2*H*)-one and the corresponding amine:

Example 37

5-[(2-Aminoethyl)amino]-7,10-dihydroxy-2-(2-hydroxyethyl)anthra[1,9-*cd*]pyrazol-6(2*H*)-one

Reaction with ethylenediamine gives the product as a salt with 1.8 equivalents of hydrogen chloride solvated with 0.5 equivalent of water; mp >195°C (decomposition).

Example 38

7,10-Dihydroxy-2-(2-hydroxyethyl)-5-[[2-(4-morpholinyl)ethyl]amino]anthra[1,9-*cd*]pyrazol-6(2*H*)-one

Reaction with 4-(2-aminoethyl)morpholine gives the product as a salt with 0.4 equivalent of hydrogen chloride and 0.3 equivalent of water; mp 240—251°C (decomposition)

Example 39

5-[[2-(Dimethylamino)ethyl]amino]-7,10-dihydroxy-2-(2-hydroxyethyl)anthra[1,9-*cd*]pyrazol-6(2*H*)-one

Reaction with *N,N*-dimethylethylenediamine gives the product as a salt with 1.5 equivalents of hydrogen chloride solvated with 2.0 equivalents of water; mp 250°C (decomposition).

Example 40

5-[(2-Aminoethyl)amino]-7,10-dihydroxy-2-methylanthra[1,9-*cd*]pyrazol-6(2*H*)-one

Reaction of a mixture of 3.2 g (10.6 mmol) of 5-chloro-7,10-dihydroxy-2-methylanthra[1,9-*cd*]pyrazol-6(2*H*)-one, 5 ml (74 mmol) of ethylenediamine, and 55 ml of pyridine for seven hours at reflux followed by workup as described in Example 21 gives a solid residue. Dissolution of the solid in hot methanol and *N,N*-dimethylformamide followed by salt formation as described in Example 3 affords 1.5 g of the dried product as a salt with 1.0 equivalent of hydrogen chloride solvated with 0.2 equivalent of water and 0.1 equivalent of *N,N*-dimethylformamide; mp 323—326°C (decomposition).

5-Chloro-7,10-dihydroxy-2-methylanthra[1,9-*cd*]pyrazol-6(2*H*)-one is prepared as follows:

A mixture of 12.4 g (40 mmol) of 1,4-dichloro-5,8-dihydroxy-9,10-anthracenedione, 2.7 ml (50 mmol) of methylhydrazine, and 250 ml of pyridine is heated at 35°C for seven hours, treated with an additional 1 ml of methylhydrazine, heated for seven hours at 35°C, and cooled. The solids are filtered and recrystallized from *N,N*-dimethylformamide to give 8.85 g of the dried product as a salt with 0.1 equivalent of hydrogen chloride, mp 298—305°C (decomposition).

Example 41

7,10-Dihydroxy-5-[[2-[(2-hydroxyethyl)amino]ethyl]amino]-2-methylanthra[1,9-*cd*]pyrazol-6(2*H*)-one

Reaction of a mixture of 3.25 g (10.8 mmol) of 5-chloro-7,10-dihydroxy-2-methylanthra[1,9-*cd*]pyrazol-6(2*H*)-one, 3 ml (30 mmol) of 2-(2-aminoethylamino)ethanol, and 50 ml of pyridine for seven hours at reflux followed by workup as described in Example 21 and salt formation as described in Example 3 gives 1.8 g of the dried product as a salt with 1.0 equivalent of hydrogen chloride solvated with 0.6 equivalent of water; mp 280—284°C (decomposition).

Example 42

5-[[2-(Diethylamino)ethyl]amino]-7,10-dihydroxy-2-methylanthra[1,9-*cd*]pyrazol-6(2*H*)-one

Reaction of a mixture of 2.0 g (6.7 mmol) of 5-chloro-7,10-dihydroxy-2-methylanthra[1,9-*cd*]pyrazol-6(2*H*)-one, 3.5 ml (20 mmol) of *N,N*-diethylenediamine, and 45 ml of pyridine for seven hours at reflux followed by workup as described in Example 21 and salt formation as described in Example 3 gives 1.7 g of the dried product as a salt with 1.5 equivalents of hydrogen chloride solvated with 0.8 equivalent of water; mp 298°C (decomposition).

Example 43

2-[3-(Diethylamino)-2-hydroxypropyl]-7,10-dihydroxy-5-[[2-[(2-hydroxyethyl)amino]ethyl]amino]-anthra[1,9-*cd*]pyrazol-6(2*H*)-one

Reaction of a mixture of 1.3 g (3 mmol) of 5-chloro-2-[3-(diethylamino)-2-hydroxypropyl]-7,10-dihydroxyanthra[1,9-*cd*]pyrazol-6(2*H*)-one, 2.1 ml (21 mmol) of 2-(2-aminoethylamino)ethanol, and 10 ml of pyridine at reflux for six hours followed by workup as described for Example 21 and salt formation as described in Example 3 gives the product as a salt with 2.2 equivalents of hydrogen chloride solvated with 2.8 equivalents of water and 0.2 equivalent of 2-propanol; mp 105—120°C.

5-Chloro-2-[3-(diethylamino)-2-hydroxypropyl]-7,10-dihydroxyanthra[1,9-*cd*]pyrazol-6(2*H*)-one is prepared as follows:

A mixture of 6.2 g (20 mmol) of 1,4-dichloro-5,8-dihydroxy-9,10-anthracenedione, 9.7 g (60 mmol) of 1-(diethylamino)-3-hydrazino-2-propanol (German Patent 1,126,877) and 35 ml of pyridine is stirred at 40°C for one hour then at room temperature overnight. The mixture is concentrated and purified on silica gel utilizing 97:2:1 dichloromethane:methanol:triethylamine to give 1.7 g of product. Salt formation as described in Example 3 gives 1.4 g of the product as a salt with 1.0 equivalent of hydrogen chloride solvated with 0.7 equivalent of water; mp 264—267°C (decomposition).

Example 44

5-[[2-(Diethylamino)ethyl]amino]-2-[3-(diethylamino)-2-hydroxypropyl]-7,10-dihydroxyanthra[1,9-*cd*]pyrazol-6(2*H*)-one

Reaction of 5-chloro-2-[3-(diethylamino)-2-hydroxypropyl]-7,10-dihydroxyanthra[1,9-*cd*]pyrazol-6(2*H*)-one with *N,N*-diethylethylenediamine as described in Example 43 gives the product as a salt with 2.0 equivalents of hydrogen chloride solvated with 1.9 equivalents of water; mp 253—255°C (decomposition).

Example 45

5-[(2-Aminoethyl)amino]-2-[3-(diethylamino)-2-hydroxypropyl]-7,10-dihydroxyanthra[1,9-*cd*]pyrazol-6(2*H*)-one

Reaction of 5-chloro-2-[3-(diethylamino)-2-hydroxypropyl]-7,10-dihydroxyanthra[1,9-*cd*]pyrazol-6(2*H*)-one with ethylenediamine as described in Example 43 gives the product as a salt with 2.0 equivalents of hydrogen chloride solvated with 2.8 equivalents of water; mp 148—152°C.

Example 46

2-[3-(Dimethylamino)propyl]-7,10-dihydroxy-5-[[2-[(2-hydroxyethyl)amino]ethyl]amino]anthra[1,9-*cd*]pyrazol-6(2*H*)-one

Reaction of a mixture of 2.5 g (6 mmol) of 5-chloro-2-[3-(dimethylamino)propyl]-7,10-dihydroxyanthra[1,9-*cd*]pyrazol-6(2*H*)-one, hydrochloride, 2.8 ml (28 mmol) of 2-(2-aminoethylamino)-ethanol, and 20 ml of pyridine at reflux for 24 hours followed by workup as described for Example 21 and salt formation as described in Example 3 gives the product as a salt with 2.0 equivalents of hydrogen chloride solvated with 1.0 equivalent of water; mp 311°C (decomposition).

5-Chloro-2-[3-(dimethylamino)propyl]-7,10-dihydroxyanthra[1,9-*cd*]pyrazol-6(2*H*)-one is prepared as follows:

To a suspension of 30.9 g (100 mmol) of 1,4-dichloro-5,8-dihydroxy-9,10-anthracenedione in 200 ml of pyridine at 37°C is added dropwise 14 g (120 mmol) of (3-dimethylaminopropyl)hydrazine [*J. Med. Chem., 1;* 493 (1964)]. The mixture is diluted with 50 ml of *N,N*-dimethylformamide, stirred for ten hours, and concentrated. The residue is distributed between dichloromethane and 5% aqueous sodium bicarbonate. Purification of the dried organic layer on silica gel utilizing 95.5:4:0.5 dichloromethane:methanol:triethylamine gives 8 g of product. Salt formation as described in Example 3 gives 7.6 g of the product as a salt with 0.8 equivalent of hydrogen chloride solvated with 0.1 equivalent of 2-propanol; mp 267—271°C (decomposition).

Example 47

5-[(3-Aminopropyl)amino]-2-[3-(dimethylamino)propyl]-7,10-dihydroxyanthra[1,9-*cd*]pyrazol-6(2*H*)-one

Reaction of 5-chloro-2-[3-(dimethylamino)propyl]-7,10-dihydroxyanthra[1,9-*cd*]pyrazol-6(2*H*)-one, hydrochloride, with 1,3-propanediamine as described in Example 46 gives the product as a salt with 2.0 equivalents of hydrogen chloride solvated with 0.5 equivalent of water; mp >300°C.

Example 48

7,10-Dihydroxy-5-[[2-[(2-hydroxyethyl)amino]ethyl]amino-2-[2-(methylthio)ethyl]anthra[1,9-*cd*]pyrazol-6(2*H*)-one

Reaction of a mixture of 0.66 g (3.6 mol) of 5-chloro-2-(2-thiomethylethyl)-7,10-dihydroxyanthra[1,9-*cd*]pyrazol-6(2*H*)-one, 1.8 ml (18 mmol) of 2-(2-aminoethylamino)ethanol, and 16 ml of pyridine at reflux overnight followed by workup as described in Example 21 and salt formation as described in Example 3 gives 0.7 g of the dried product as a salt with 1.6 equivalents of hydrogen chloride solvated with 0.5 equivalent of water; mp >133°C (decomposition).

5-Chloro-2-(2-thiomethylethyl)-7,10-dihydroxyanthra[1,9-*cd*]pyrazol-6(2*H*)-one is prepared as follows:

An ice-cold mixture of 2.64 g (5 mmol) of 5-chloro-2-(2-thiomethylethyl)-7,10-bis(phenylmethoxy)-anthra[1,9-*cd*]pyrazol-6(2*H*)-one in 15 ml dichloromethane is treated dropwise during 30 minutes with 30 ml of a 1M solution of boron trichloride in dichloromethane. The mixture is stirred for one hour then treated carefully with 30 ml of methanol. The mixture is warmed to room temperature overnight then concentrated to a residue which is triturated with 2-propanol to give a red solid. Further trituration with 75 ml of boiling methanol gives 0.9 g of pure product; mp 186—190°C.

5-Chloro-2-(2-thiomethylethyl)-7,10-bis(phenylmethoxy)anthra[1,9-*cd*]pyrazol-6(2*H*)-one is prepared as follows:

A mixture of 5.1 g (10 mmol) of 5-chloro-2-(2-hydroxyethyl)-7,10-bis(phenylmethoxy)anthra[1,9-*cd*]pyrazol-6(2*H*)-one, 9.4 g (100 mmol) of methyldisulfide, 20.2 g (100 mmol) of tri-*n*-butylphosphine, and 50 ml of N,N-dimethylformamide is stirred overnight at room temperature. The mixture is cooled and treated carefully with 75 ml of water. The orange solid is collected and washed successively with water, 2-propanol, and diethyl ether to give 5.1 g of the dried product; mp 155—160°C.

5-Chloro-2-(2-hydroxyethyl)-7,10-bis(phenylmethoxy)anthra[1,9-*cd*]pyrazol-6(2*H*)-one is prepared as follows:

Reaction of a mixture of 35.4 g (72 mmol) of 1,4-dichloro-5,8-bis(phenylmethoxy)-9,10-anthracenedione, 11.2 g (147 mmol) of (2-hydroxyethyl)hydrazine, 2.1 g (37 mmol) of anhydrous potassium fluoride, 7.4 g (74 mmol) of anhydrous potassium bicarbonate, and 220 ml of dry dimethylsulfoxide as described in Example 54 gives 33.1 g of the dried product; mp 178—184°C. Crystallization from chloroform raises the melting point to 201—204°C.

1,4-Dichloro-5,8-bis(phenylmethoxy)-9,10-anthracenedione is prepared as follows:

A mixture of 51.3 g (160 mmol) of 1,4-dichloro-5,8-dihydroxy-9,10-anthracenedione (US Patent Number 3,631,074), 46 g (330 mmol) of powdered anhydrous potassium carbonate, 44 ml (380 mmol) of benzyl bromide, and 670 ml of dry acetone are heated at reflux for five days. The mixture is cooled, the

solids are filtered, then washed sequentially with water, methanol, and diethyl ether to give 63.6 g of the dried product; mp 190—194°C. Processing of the acetone filtrate gives 9.4 g of a second crop; mp 142—155°C.

Example 49

5-Chloro-2-[2-(diethylamino)ethyl]-7,10-dihydroxyanthra[1,9-*cd*]pyrazol-6(2*H*)-one

An ice-cold mixture of 9.1 g (16 mmol) of 5-chloro-2-[2-(diethylamino)ethyl]-7,10-bis(phenylmethoxy)anthra[1,9-*cd*]pyrazol-6(2*H*)-one in 30 ml of dichloromethane is treated dropwise during two hours with 96 ml of a 1M solution of boron trichloride. Following addition, the mixture is treated carefully with 30 ml of methanol. The mixture is warmed to room temperature overnight and the solid residue is collected, washed sequentially with 2-propanol, methanol, and diethyl ether to give 5.5 g of the dried product as a salt with 1.0 equivalent of hydrogen chloride, and solvated with 0.2 equivalent of water; mp 280—282°C (decomposition).

5-Chloro-2-[2-(diethylamino)ethyl]-7,10-bis-(phenylmethoxy)anthra[1,9-*cd*]pyrazol-6(2*H*)-one is prepared as follows:

A mixture of 4.2 g (6.3 mmol) of 5-chloro-2-[2-[[(4-methylphenyl)sulfonyl]oxy]ethyl]-7,10-bis(phenylmethoxy)anthra[1,9-*cd*]pyrazol-6(2*H*)-one, 4.4 g (60 mmol) of diethylamine, 17 g (12.6 mmol) of powdered potassium carbonate, and 35 ml of dimethylsulfoxide is stirred overnight at 50°C. The mixture is cooled and diluted with 50 ml of water. The solid is collected and washed with water. The solid is crystallized from chloroform:2-propanol (3:1) to give 2.1 g of product; mp 209—211°C.

5-Chloro-2-[2-[[(4-methylphenyl)sulfonyl]oxy]ethyl]-7,10-bis(phenylmethoxy)anthra[1,9-*cd*]pyrazol-6(2*H*)-one is prepared as follows:

An ice-cold mixture of 22 g (43 mmol) of 5-chloro-2-(2-hydroxyethyl)-7,10-bis(phenylmethoxy)anthra[1,9-*cd*]pyrazol-6(2*H*)-one, 12.3 g (65 mmol) of *p*-toluenesulfonyl chloride and 170 ml of pyridine is stirred for 50 hours. The solid is filtered, washed with methanol and diethyl ether, and dried to give 10.5 g of the product; mp 203—206°C (decomposition). Processing of the filtrate gives 9.3 g of additional product; mp 182—188°C (decomposition).

Example 50

2-[2-[[2-(Dimethylamino)ethyl]amino]ethyl]-5-[[2-[(2-hydroxyethyl)amino]ethyl]amino]-7,10-dihydroxy-anthra[1,9-*cd*]pyrazol-6(2*H*)-one

Reaction of a mixture of 3.6 g (7.4 mmol) 5-chloro-2-[2-[[2-(dimethylamino)ethyl]amino]ethyl]-7,10-dihydroxyanthra[1,9-*cd*]pyrazol-6(2*H*)-one, dihydrochloride, 4.5 ml (45 mmol) of 2-(2-aminoethylamino)ethanol, and 35 ml of pyridine at 80°C overnight followed by workup as described in Example 21 gives 0.5 g of product as a salt with 0.25 equivalent of hydrogen chloride and solvated with 0.75 equivalent of water; mp 110—117°C.

5-Chloro-2-[2-[[2-(dimethylamino)ethyl]amino]ethyl]-7,10-dihydroxyanthra[1,9-*cd*]pyrazol-6(2*H*)-one is prepared as follows:

An ice-cold mixture of 9.3 g (16 mmol) of 5-chloro-2-[2-[[2-(dimethylamino)ethyl]amino]ethyl]-7,10-bis-(phenylmethoxy)anthra[1,9-*cd*]pyrazol-6(2*H*)-one in 30 ml of dichloromethane is treated dropwise during two hours with 96 ml of a 1M solution of boron trichloride. Following addition, the mixture is treated carefully with 30 ml of methanol. The mixture is warmed to room temperature overnight and the solid residue is collected, washed sequentially with 2-propanol, methanol, and diethyl ether to give 3.68 g of the dried product as a salt with 1.8 equivalents of hydrogen chloride and solvated with 0.2 equivalent of 2-propanol and 0.8 equivalent of water; mp 260—268°C (decomposition).

5-Chloro-2-[2-[[2-(dimethylamino)ethyl]amino]ethyl]-7,10-bis(phenylmethoxy)anthra[1,9-*cd*]pyrazol-6(2*H*)-one is prepared as follows:

A mixture of 4.2 g (6.3 mmol) of 5-chloro-2-[2-[[(4-methylphenyl)sulfonyl]oxy]ethyl]-7,10-bis(phenylmethoxy)anthra[1,9-*cd*]pyrazol-6(2*H*)-one, 5.3 g (60 mmol) of *N,N*-dimethylethylenediamine, 17 g (12.6 mmol) of powdered potassium carbonate, and 35 ml of dimethylsulfoxide is stirred overnight at 50°C. The mixture is cooled and diluted with 50 ml of water. The solid is collected and washed with water. The solid is heated in dichloromethane, the solution filtered, then concentrated. Trituration of the residue with hot ethyl acetate gives 1.7 g of the dried product; mp 148—153°C.

Example 51

7,10-Dihydroxy-5-[[2-[(2-hydroxyethyl)amino]ethyl]amino]-2-[2-[(2-hydroxyethyl)-methylamino]ethyl]anthra[1,9-*cd*]pyrazol-6(2*H*)-one

Reaction of a mixture of 3.3 g (7.3 mmol) of 5-chloro-7,10-dihydroxy-2-[2-[(2-hydroxyethyl)-methylamino]ethyl]anthra[1,9-*cd*]pyrazol-6(2*H*)-one, hydrochloride, 3.6 ml (36 mmol) of 2-(2-aminoethylamino)ethanol, and 30 ml of pyridine overnight at 80°C followed by workup as described in Example 21 and salt formation as described in Example 3 gives 1.4 g of the dried product as a salt with 1.6 equivalents of hydrogen chloride solvated with 0.4 equivalent of water; mp 240°C (decomposition).

5-Chloro-7,10-dihydroxy-2-[2-[(2-hydroxyethyl)methylamino]ethyl]anthra[1,9-*cd*]pyrazol-6(2*H*)-one is prepared as follows:

Reaction of a mixture of 8.3 g (15 mmol) of 5-chloro-2-[2-[(2-hydroxyethyl)methylamino]ethyl]-7,10-

bis(phenylmethoxy)anthra[1,9-*cd*]pyrazol-6(2*H*)-one, 87 ml of a 1M solution of boron trichloride in dichloromethane, and 60 ml of dichloromethane as described for Example 49 gives 3.5 g of the dried product as a salt with 1.0 equivalent of hydrogen chloride solvated with 1.75 equivalents of water; mp 279—282°C (decomposition).

5-Chloro-2-[2-[(2-hydroxyethyl)methylamino]ethyl]-7,10-bis(phenylmethoxy)anthra[1,9-*cd*]pyrazol-6(2*H*)-one is prepared as follows:

Reaction of a mixture of 10.0 g (15 mmol) of 5-chloro-2-[2-[[4-methylphenyl)sulfonyl]oxy]ethyl]-7,10-bis(phenyl)methoxy)anthra[1,9-*cd*]pyrazol-6(2*H*)-one, 12.1 ml (150 mmol) of 2-methylaminoethanol, 4.1 g (30 mmol) of potassium carbonate, and 90 ml of dimethylsulfoxide as described in Example 49 gives 8.5 g of the product; mp 191—194°C.

Example 52

7,10-Dihydroxy-5-[[2-[(2-hydroxyethyl)amino]ethyl]amino]-2-[3-[(2-hydroxyethyl)amino]propyl]anthra[1,9-*cd*]pyrazol-6(2*H*)-one

Reaction of a mixture of 1.4 g (3.6 mmol) of 5-chloro-7,10-dihydroxy-2-[3-[(2-hydroxyethyl)amino]-propyl]anthra[1,9-*cd*]pyrazol-6(2*H*)-one, hydrochloride, 3.7 ml (37 mmol) of 2-(2-aminoethylamino)ethanol and 15 ml of pyridine overnight at 80°C followed by workup as described in Example 21 gives 0.9 g of the dried product solvated with 0.6 equivalent of water; mp 100—105°C.

5-Chloro-7,10-dihydroxy-2-[3-[(2-hydroxyethyl)amino]propyl]anthra[1,9-*cd*]pyrazol-6(2*H*)-one is prepared as follows:

Reaction of a mixture of 9.0 g (16 mmol) of 5-chloro-2-[3-[(2-hydroxyethyl)amino]propyl]-7,10-bis(phenylmethoxy)anthra[1,9-*cd*]pyrazol-6(2*H*)-one, 63 ml of a 1M solution of boron trichloride in dichloromethane, and 30 ml of dichloromethane as described for Example 49 gives 6.0 g of the dried product as a salt with 0.8 equivalent of hydrogen chloride solvated with 0.7 equivalent of water; mp 255—265°C (decomposition).

5-Chloro-2-[3-[(2-hydroxyethyl)amino]propyl]-7,10-bis(phenylmethoxy)anthra[1,9-*cd*]pyrazol-6(2*H*)-one is prepared as follows:

Reaction of a mixture of 13.4 g (19.7 mmol) of 5-chloro-2-[3-[[4-methylphenyl)sulfonyl]oxy]propyl]-7,10-bis(phenylmethoxy)anthra[1,9-*cd*]pyrazol-6(2*H*)-one, 12 ml (197 mmol) of 2-aminoethanol, 5.5 g (39.4 mmol) of potassium carbonate, and 120 ml of dimethylsulfoxide as described in Example 49 gives 9.85 g of the product; mp 174—176°C. Crystallization from chloroform gives material of mp 180—185°C.

5-Chloro-2-[3-[[4-methylphenyl)sulfonyl]oxy]propyl]-7,10-bis(phenylmethoxy)anthra[1,9-*cd*]pyrazol-6(2*H*)-one is prepared as follows:

Reaction of a mixture of 13.1 g (25 mmol) of 5-chloro-2-(3-hydroxypropyl)-7,10-bis(phenylmethoxy)-anthra[1,9-*cd*]pyrazol-6(2*H*)-one, 9.5 g (50 mmol) of *p*-toluenesulfonyl chloride, 9 ml (65 mmol) of triethylamine, 150 mg of 4-dimethylaminopyridine, and 125 ml of dichloromethane at 5°C for one day then at room temperature for five hours followed by workup as described for Example 49 gives 14.3 g of the product; mp 137—139°C.

5-Chloro-2-(3-hydroxypropyl)-7,10-bis(phenylmethoxy)anthra[1,9-*cd*]pyrazol-6(2*H*)-one is prepared as follows:

A mixture of 48.9 g (100 mmol) of 1,4-dichloro-5,8-bis(phenylmethoxy)-9,10-anthracenedione, 18.0 g (200 mmol) of (3-hydroxypropyl)hydrazine [*J. Amer. Chem. Soc. 76*; 1283 (1954)], 2.9 g (50 mmol) of anhydrous potassium fluoride, 10.0 g (100 mmol) of anhydrous potassium bicarbonate, and 300 ml of dry dimethylsulfoxide is stirred at 80°C overnight. The warm mixture is diluted with 1.5 ml of water, then allowed to cool. The solids are collected by filtration, washed sequentially with water, 2-propanol, and diethyl ether to afford 31.0 g of the dried product; mp 159—163°C. Processing of the filtrate gives 4.7 g of additional product; mp 150—154°C.

Example 53

5-[(3-Aminopropyl)amino]-7,10-dihydroxy-2-[3-[(2-hydroxyethyl)amino]propyl]anthra[1,9-*cd*]pyrazol-6(2*H*)-one

Reaction of a mixture of 1.3 g (3.3 mmol) of 5-chloro-7,10-dihydroxy-2-[3-[(2-hydroxyethyl)amino]propyl]anthra[1,9-*cd*]pyrazol-6(2*H*)-one, 2.9 ml (35 mmol) of 1,3-propanediamine, and 15 ml of pyridine at 80°C overnight followed by workup as described in Example 21 gives 1.0 g of the dried product as a salt with 0.1 equivalent of hydrogen chloride solvated with 0.3 equivalent of water and 0.1 equivalent of 2-propanol; mp 120—130°C (decomposition).

Example 54

7,10-Dihydroxy-2-[2-[(2-hydroxyethyl)amino]ethyl]-5-[[2-[(2-hydroxyethyl)amino]ethyl]amino]anthra[1,9-*cd*]pyrazol-6(2*H*)-one

Reaction of mixture of 1.28 g (3 mmol) of 5-chloro-7,10-dihydroxy-2-[2-[(2-hydroxyethyl)-amino]ethyl]anthra[1,9-*cd*]pyrazol-6(2*H*)-one, hydrochloride, 1.5 ml (15 mmol) of 2-(2-aminoethylamino)ethanol and 6 ml of pyridine at 80°C overnight followed by workup as described in Example 21 and salt formation as described in Example 3 gives 675 mg of the product as a salt with 2.0 equivalents of hydrogen chloride solvated with 0.9 equivalent of water; mp 215—225°C (decomposition).

5-Chloro-7,10-dihydroxy-2-[2-[(2-hydroxyethyl)amino]ethyl]anthra[1,9-*cd*]pyrazol-6(2*H*)-one is prepared as follows:

To an ice-cold mixture of 26.8 g (48 mmol) of 5-chloro-2-[2-[hydroxyethyl)amino]ethyl]-7,10-bis(phenylmethoxy)anthra[1,9-*cd*]pyrazol-6(2*H*)-one and 60 ml of dry dichloromethane is added dropwise during 2¼ hours 794 ml of a 1M solution of boron trichloride. The mixture is stirred for an additional 0.5 hours, then 200 ml of methanol is added dropwise during 1.5 hours. The mixture is allowed to warm to room temperature overnight and the solids are filtered, washed sequentially with methanol, dichloromethane, diethyl ether, and 2-propanol to give 14.7 g of the dried product; mp 175°C (decomposition). Processing of the filtrate affords 5.4 g of additional product; mp 125—135°C (decomposition). Crystallization of the solid from methanol gives a salt with 1.0 equivalent of hydrogen chloride solvated with 0.7 equivalent of water; mp 180—200°C (decomposition).

5-Chloro-2-[2-[(2-hydroxyethyl)amino]ethyl]-7,10-bis(phenylmethoxy)anthra[1,9-*cd*]pyrazol-6(2*H*)-one is prepared as follows:

A mixture of 58.8 g (120 mmol) of 1,4-dichloro-5,8-bis(phenylmethoxy)-9,10-anthracenedione, 28.6 g (240 mmol) of 2-[(hydrazinoethyl)amino]ethanol, 3.5 g (60 mmol) of anhydrous potassium fluoride, 12 g (120 mmol) of anhydrous potassium bicarbonate, and 360 ml of dry dimethylsulfoxide is stirred at 80°C overnight. The mixture is diluted with 400 ml of water and the orange solids are filtered, washed sequentially with water, 2-propanol, and diethyl ether to give 51.2 g of the dried product; mp 164—168°C.

The following compounds are prepared as described in Example 54 from 5-chloro-7,10-dihydroxy-2-[2-[(2-hydroxyethyl)amino]ethyl]anthra[1,9-*cd*]pyrazol-6(2*H*)-one hydrochloride and the corresponding amine:

Example 55

5-[(2-Aminoethyl)amino]-7,10-dihydroxy-2-[2-[(2-hydroxyethyl)amino]ethyl]anthra[1,9-*cd*]pyrazol-6(2*H*)-one

Reaction with ethylenediamine gives the product as a salt with 2.0 equivalents of hydrogen chloride solvated with 0.9 equivalent of water; mp 272—278°C (decomposition).

Example 56

5-[[2-(Dimethylamino)ethyl]amino]-7,10-dihydroxy-2-[2-[(2-hydroxyethyl)amino]ethyl]anthra[1,9-*cd*]pyrazol-6(2*H*)-one

Reaction with *N,N*-dimethylethylenediamine gives the product as a salt with 1.9 equivalents of hydrogen chloride solvated with 1.7 equivalents of water; mp 278—280°C (decomposition).

Example 57

5-[[2-(Diethylamino)ethyl]amino]-7,10-dihydroxy-2-[2-[(2-hydroxyethyl)amino]ethyl]anthra[1,9-*cd*]pyrazol-6(2*H*)-one

Reaction with *N,N*-diethylethylenediamine gives the product as a salt with 1.9 equivalents of hydrogen chloride solvated with 1.5 equivalents of water; mp 228—231°C.

Example 58

5-[(3-Aminopropyl)amino]-7,10-dihydroxy-2-[2-[(2-hydroxyethyl)amino]ethyl]anthra[1,9-*cd*]pyrazol-6(2*H*)-one

Reaction with 1,3-propanediamine gives the product as a salt with 1.7 equivalents of hydrogen chloride solvated with 1.0 equivalent of water; mp 222°C (decomposition).

Example 59

5-[(4-Aminobutyl)amino]-7,10-dihydroxy-2-[2-[(2-hydroxyethyl)amino]ethyl]anthra[1,9-*cd*]pyrazol-6(2*H*)-one

Reaction with 1,4-butanediamine gives the product as a salt with 1.0 equivalent of hydrogen chloride solvated with 0.5 equivalent of water; mp 240—245°C (decomposition).

Example 60

5-[(5-Aminopentyl)amino]-7,10-dihydroxy-2-[2-[(2-hydroxyethyl)amino]ethyl]anthra[1,9-*cd*]pyrazol-6(2*H*)-one

Reaction with 1,5-pentanediamine gives the product as a salt with 1.9 equivalents of hydrogen chloride solvated with 0.7 equivalent of water; mp 270—275°C (decomposition).

Example 61

7,10-Dihydroxy-2-[2-[(2-hydroxyethyl)amino]ethyl]-5-[[2-(4-morpholinyl)ethyl]amino]anthra[1,9-*cd*]pyrazol-6(2*H*)-one

Reaction with 4-(2-aminoethyl)morphine gives the product as a salt with 2.4 equivalents of hydrogen chloride solvated with 0.8 equivalent of water; mp 280°C (decomposition).

Example 62

7,10-Dihydroxy-2-[2-[(2-hydroxyethyl)amino]ethyl]-5-[[3-[(2-hydroxyethyl)amino]propyl]amino]anthra[1,9-*cd*]pyrazol-6(2*H*)-one

Reaction with 2-(3-aminopropylamino)ethanol gives the product as a salt with 2.1 equivalents of hydrogen chloride solvated with 0.8 equivalent of water and 0.1 equivalent of 2-propanol; mp 170—180°C (decomposition).

Example 63

5-[[2-[[2-(Dimethylaminoethyl]amino]ethyl]amino]-7,10-dihydroxy-2-[2-[(2-hydroxyethyl)amino]lethyl]-anthra[1,9-*cd*]pyrazol-6(2*H*)-one

Reaction with *N,N*-dimethyldiethylenediamine gives the product as a salt with 2.4 equivalents of hydrogen chloride solvated with 1.4 equivalents of water and.0.2 equivalent of 2-propanol; mp 80—90°C (decomposition).

Example 64

7,10-Dihydroxy-2-[2-[(2-hydroxyethyl)amino]ethyl]-5-(4-methyl-1-piperazinyl)anthra[1,9-*cd*]pyrazol-6(2*H*)-one

Reaction with *N*-methylpiperazine gives the product as a salt with 2.2 equivalents of hydrogen chloride solvated with 0.4 equivalent of water and 0.2 equivalent of 2-propanol; mp >123°C (decomposition).

Example 65

5-[[2-(Dimethylamino)ethyl]methylamino]-7,10-dihydroxy-2-[2-[(2-hydroxyethyl)amino]ethyl]anthra[1,9-*cd*]pyrazol-6(2*H*)-one

Reaction with *N,N,N*-trimethylethylenediamine gives the product as a salt with 2.1 equivalents of hydrogen chloride solvated with 1.9 equivalents of water and 0.2 equivalents of 2-propanol; mp >91°C (decomposition).

Example 66

5-[[2-[(2-Aminoethyl)amino]ethyl]amino]-7,10-dihydroxy-2-[2-[(2-hydroxyethyl)amino]ethyl]anthra[1,9-*cd*]pyrazol-6(2*H*)-one

Reaction with diethylenetriamine gives the product as a salt with 1.0 equivalent of hydrogen chloride solvated with 1.0 equivalent of water; mp 210—215°C (decomposition).

Example 67

5-[[2-[Bis(2-hydroxyethyl)amino]ethyl]amino]-7,10-dihydroxy-2-[2-[(2-hydroxyethyl)-amino]ethyl]anthra[1,9-*cd*]pyrazol-6(2*H*)-one

Reaction with *N,N*-bis(2-hydroxyethyl)ethylenediamine gives the product as a salt with 2.3 equivalents of hydrogen chloride solvated with 0.8 equivalent of water; mp 230°C (decomposition).

Example 68

5-[[3-[Bis(2-hydroxyethyl)amino]propyl]amino]-7,10-dihydroxy-2-[2-hydroxyethyl)amino]ethyl]anthra[1,9-*cd*]pyrazol-6(2*H*)-one

Reaction with *N,N*-bis(2-hydroxyethyl)-1,3-propanediamine gives the product as a salt with 2.1 equivalents of hydrogen chloride solvated with 0.4 equivalent of water; mp 198—215°C (decomposition).

Example 69

5-[[3-[[4-[(3-Aminopropyl)amino]butyl]amino]propyl]amino]-7,10-dihydroxy-2-[2-[(2-hydroxyethyl)-amino]ethyl]anthra[1,9-*cd*]pyrazol-6(2*H*)-one

Reaction with spermine gives the product as a salt with 2.75 equivalents of hydrogen chloride solvated with 0.6 equivalent of water and 0.1 equivalent of 2-propanol; mp 185—200°C (decomposition).

Example 70

7,10-Dihydroxy-2-[2-[(2-hydroxyethyl)amino]ethyl]-5-[[2-(methylamino)ethyl]amino]anthra[1,9-*cd*]pyrazol-6(2*H*)-one

Reaction with (2-aminoethyl)-methylcarbamic acid, benzyl ester followed by isolation of the intermediate then hydrolysis with refluxing 48% hydrobromic acid in acetic acid gives the product as a salt with 2.1 equivalents of hydrogen bromide solvated with 2.3 equivalents of water and 0.5 equivalent of acetic acid; mp 222—228°C (decomposition).

Example 71

2-(2-Aminoethyl)-7,10-dihydroxy-5-[[2-[(2-hydroxyethyl)amino]ethyl]amino]anthra[1,9-*cd*]pyrazol-6(2*H*)-one

Reaction of a mixture of 2.0 g (6 mmol) of 2-(2-aminoethyl)-5-chloro-7,10-dihydroxyanthra[1,9-*cd*]pyrazol-6(2*H*)-one, 3 ml (30 mmol) of 2-(2-aminoethylamino)ethanol, and 25 ml of pyridine at reflux overnight followed by workup as described in Example 21 and salt formation as described in Example 3

gives 1.3 g of the product as a salt with 2.0 equivalents of hydrogen chloride solvated with 0.5 equivalent of water; mp 275—280°C (decomposition).

2-(2-Aminoethyl)-5-chloro-7,10-dihydroxyanthra[1,9-*cd*]pyrazol-6(2*H*)-one is prepared from 2-(2-aminoethyl)-5-chloro-7,10-bis(phenylmethoxy)anthra[1,9-*cd*]pyrazol-6(2*H*)-one and boron trichloride as described in Example 54 to give the product as a salt with 1.0 equivalent of hydrogen chloride solvated with 0.7 equivalent of water; mp 265—268°C (decomposition).

2-(2-Aminoethyl)-5-chloro-7,10-bis(phenylmethoxy)anthra[1,9-*cd*]pyrazol-6(2*H*)-one is prepared from 1,4-dichloro-5,8-bis(phenylmethoxy)-9,10-anthracenedione and (2-aminoethyl)hydrazine as described in Example 54 to give the product; mp 176—178°C.

Example 72

2-(2-Aminoethyl)-5-[(2-aminoethyl)amino]-7,10-dihydroxyanthra[1,9-*cd*]pyrazol-6(2*H*)-one

Reaction of 2-(2-aminoethyl)-5-chloro-7,10-dihydroxyanthra[1,9-*cd*]pyrazol-6(2*H*)-one with ethylenediamine as described in Example 71 gives the product as a salt with 1.9 equivalents of hydrogen chloride solvated with 1.0 equivalent of water; mp >230°C (decomposition).

Example 73

2-(2-Aminoethyl)-5-[(3-aminopropyl)amino]-7,10-dihydroxyanthra[1,9-*cd*]pyrazol-6(2*H*)-one

Reaction of 2-(2-aminoethyl)-5-chloro-7,10-dihydroxyanthra[1,9-*cd*]pyrazol-6(2*H*)-one with 1,3-propanediamine as described in Example 71 gives the product as a salt with 2.9 equivalents of hydrogen chloride solvated with 3.5 equivalents of water; mp >310°C (decomposition).

Example 74

2-[2-Aminoethyl)-5-[[2-[[2-(dimethylamino)ethyl]amino]ethyl]amino]-7,10-dihydroxyanthra[1,9-*cd*]pyrazol-6(2*H*)-one

Reaction of 2-(2-aminoethyl)-5-chloro-7,10-dihydroxyanthra[1,9-*cd*]pyrazol-6(2*H*)-one with *N,N*-dimethyldiethylenetriamine as described in Example 71 gives the product as a salt with 3.3 equivalents of hydrogen chloride solvated with 1.0 equivalent of water and 0.2 equivalent of 2-propanol; mp 245—260°C (decomposition).

Example 75

2-(2-Aminoethyl)-5-[[3-[(2-hydroxyethyl)amino]propyl]amino]-7,10-dihydroxyanthra[1,9-*cd*]pyrazol-6(2*H*)-one

Reaction of 2-(2-aminoethyl)-5-chloro-7,10-dihydroxyanthra[1,9-*cd*]pyrazol-6(2*H*)-one with 2-(3-aminopropylamino)ethanol as described in Example 71 gives the product as a salt with 2.1 equivalents of hydrogen chloride solvated with 1.0 equivalent of water and 0.2 equivalent of 2-propanol; mp 175°C (decomposition).

Example 76

7,10-Dihydroxy-5-[[2-[(2-hydroxyethyl)amino]ethyl]amino]-2-(2-methoxyethyl)anthra[1,9-*cd*]pyrazol-6(2*H*)-one

Reaction of a mixture of 2.0 g (5.8 mmol) of 5-chloro-7,10-dihydroxy-2-(2-methoxyethyl)anthra[1,9-*cd*]pyrazol-6(2*H*)-one, 5.8 ml (58 mmol) of 2-(2-aminoethylamino)ethanol, and 25 ml of pyridine at 85°C overnight followed by workup as described in Example 21 gives 1.75 g of product. Salt formation as described in Example 3 gives 1.91 g of product as a salt with 1.1 equivalents of hydrogen chloride solvated with 0.3 equivalent of water and 0.2 equivalent of 2-propanol; mp 68—72°C.

5-Chloro-7,10-dihydroxy-2-(2-methoxyethyl)anthra[1,9-*cd*]pyrazol-6(2*H*)-one is prepared as follows:

Reaction of 11.9 g (23 mmol) of 5-chloro-2-(2-methoxyethyl)-7,10-bis(phenylmethoxy)anthra[1,9-*cd*]pyrazol-6(2*H*)-one, 91 ml of a 1M solution of boron trichloride, and 46 ml of dry dichloromethane as described in Example 24 gives 6.25 g of the dried product; mp 137—145°C (decomposition).

5-Chloro-2-(2-methoxyethyl)-7,10-bis(phenylmethoxy)anthra[1,9-*cd*]pyrazol-6(2*H*)-one is prepared as follows:

An ice-cold mixture of 3.2 g (6 mmol) of 5-chloro-2-(2-hydroxyethyl)-7,10-bis(phenylmethoxy)anthra[1,9-*cd*]pyrazol-6(2*H*)-one, 1.23 ml (20 mmol) of iodomethane, and 20 ml of *N,N*-dimethylformamide is treated gradually with 0.18 g (8 mmol) of sodium hydride. The ice bath is removed and the mixture is stirred for two hours, treated with five drops of glacial acetic acid, then diluted with water. The solids are filtered, washed sequentially with 2-propanol and diethyl ether to give 2.8 g of the dried product; mp 174—178°C.

Example 77

5-[(2-Aminoethyl)amino]-7,10-dihydroxy-2-(2-methoxyethyl)anthra[1,9-*cd*]pyrazol-6(2*H*)-one

Reaction of 5-chloro-7,10-dihydroxy-2-(2-methoxyethyl)anthra[1,9-*cd*]pyrazol-6(2*H*)-one with ethylenediamine as described in Example 76 gives the product as a salt with 1.0 equivalent of hydrogen chloride solvated with 0.3 equivalent of water; mp 263—268°C (decomposition).

Example 78

2-(2,3-Dihydroxypropyl)-7,10-dihydroxy-5-[[2-[(2-hydroxyethyl)amino]ethyl]amino]anthra[1,9-*cd*]pyrazol-6(2*H*)-one

A mixture of 1.3 g (2 mmol) of 2-[(2,2-dimethyl-1,3-dioxolan-4-yl)methyl]-5-[[2-[(2-hydroxyethyl)-amino]ethyl]amino-7,10-bis(phenylmethoxy)anthra[1,9-*cd*]pyrazol-6(2*H*)-one, 260 mg of 20% palladium hydroxide on carbon, and 25 ml of glacial acetic acid is stirred under an atmosphere of hydrogen for two hours. The mixture is filtered and concentrated to a residue which is dissolved in methanolic hydrogen chloride. The mixture is stirred at room temperature for two hours and concentrated to a solid which is crystallized from 1:1 methanol:ethanol to give 0.7 g of the product as a salt with 1.1 equivalents of hydrogen chloride solvated with 1.0 equivalent of water; mp >110°C.

2-[(2,2-Dimethyl-1,3-dioxolan-4-yl)methyl]-5-[[2-[(2-hydroxyethyl)amino]ethyl]amino-7,10-bis(phenylmethoxy)anthra[1,9-*cd*]pyrazol-6(2*H*)-one is prepared as follows:

A mixture of 1.2 g (2 mmol) of 5-chloro-2-[(2,2-dimethyl-1,3-dioxolan-4-yl)methyl]-7,10-bis-(phenylmethoxy)anthra[1,9-*cd*]pyrazol-6(2*H*)-one, 2.2 ml (22 mmol) of 2-(2-aminoethylamino)ethanol, 17 ml of pyridine, and 0.3 g of anhydrous potassium carbonate is stirred at reflux for 42 hours. The mixture is diluted with water and filtered to give a solid that is purified by silica gel chromatography utilizing 94:5:1 dichloromethane:methanol:triethylamine. Concentration of the product fractions followed by trituration with 2-propanol gives 730 mg of pure product; mp 206°C.

5-Chloro-2-[(2,2-dimethyl-1,3-dioxolan-4-yl)methyl]-7,10-bis(phenylmethoxy)anthra[1,9-*cd*]pyrazol-6(2*H*)-one is prepared as follows:

A mixture of 19.6 g (40 mmol) of 1,4-dichloro-5,8-bis(phenylmethoxy)-9,10-anthracenedione, 10 g (68 mmol) of 4-(hydrazinomethyl)-2,2-dimethyl-1,3-dioxolan [*Ann. 448*; 121 (1926)], 4 g (69 mmol) of anhydrous potassium fluoride, 5.5 g (40 mmol) of anhydrous potassium carbonate, and 150 ml of dry dimethylsulfoxide is stirred at 80°C for six hours. The mixture is diluted with water and the solids are filtered, then dissolved in dichloromethane. Chromatography of the dried dichloromethane layer over silica gel with gradient elution utilizing 0.5 to 1% methanol in dichloromethane gives 6 g of a solid that is triturated from 2-propanol, then crystallized from toluene to afford 1.8 g of pure product; mp 184—188°C.

Example 79

2-[2-(Diethylamino)ethyl]-7-[[2-[(2-hydroxyethyl)amino]ethyl]amino]anthra[1,9-*cd*]pyrazol-6(2*H*)-one

Reaction of 2.5 g (7 mmol) of 7-chloro-2-[2-(diethylamino)ethyl]anthra[1,9-*cd*]pyrazol-6(2*H*)-one, 7 ml (70 mmol) of 2-(2-aminoethylamino)ethanol, and 20 ml of pyridine for 20 hours at reflux followed by workup as described in Example 9 gives 1.6 g of a solid, mp 104—107°C, after recrystallization from toluene. Salt formation as described in Example 3 gives 1.6 g of the dried product as a salt with 2.0 equivalents of hydrogen chloride solvated with 1.1 equivalents of water; mp 212—216°C (decomposition).

7-Chloro-2-[2-(diethylamino)ethyl]anthra[1,9-*cd*]pyrazol-6(2*H*)-one is prepared as follows:

A mixture of 13.85 g (50 mmol) of 1,5-dichloro-9,10-anthracenedione, 13.1 g (100 mmol) of (2-diethylaminoethyl)hydrazine, and 100 ml of pyridine is stirred at 50°C for five hours, treated with an additional 10 ml of the substrate hydrazine, stirred at 35°C for 48 hours, cooled, filtered, and concentrated. Trituration of the residue with 2-propanol:ethanol gives 8 g of a solid powder; mp 129—132°C.

Dissolution of a 0.9 g sample in hot methanol followed by salt formation as described in Example 3 gives 0.8 g of the dried product as a salt with 1.0 equivalent of hydrogen chloride; mp 272—275°C (decomposition).

Example 80

2-[2-(Diethylamino)ethyl]-7-[[2-(diethylamino)ethyl]amino]anthra[1,9-*cd*]pyrazol-6(2*H*)-one

Reaction of a mixture of 2.1 g (6 mmol) of 7-chloro-2-[2-(diethylamino)ethyl]anthra[1,9-*cd*]pyrazol-6(2*H*)-one, 5 ml (36 mmol) of *N,N*-diethylethylenediamine, and 20 ml of pyridine for 28 hours at reflux followed by workup as described for Example 21 gives 1.9 g of the dried product as a salt with 2.0 equivalents of hydrogen chloride solvated with 0.2 equivalent of water; mp 292—294°C (decomposition).

Example 81

2-[2-[(2-Hydroxyethyl)amino]ethyl]-7-[[2-[(2-hydroxyethyl)amino]ethyl]amino]anthra[1,9-*cd*]pyrazol-6(2*H*)-one

A mixture of 1.9 g (5 mmol) of 7-chloro-2-[2-[(2-hydroxyethyl)amino]ethyl]anthra[1,9-*cd*]pyrazol-6(2*H*)-one, 2.0 ml (20 mmol) of 2-(2-aminoethylamino)ethanol, and 20 ml of pyridine is heated at reflux for 72 hours. The mixture is cooled, concentrated, and chromatographed over silica gel with 0.5% triethylamine in dichloromethane, utilizing a gradient elution of 2—10% methanol, to give the product. Salt formation as described in Example 3 gives 500 mg of the product as a salt with 2.0 equivalents of hydrogen chloride solvated with 0.4 equivalent of water; mp 285—287°C (decomposition).

7-Chloro-2-[2-[(2-hydroxyethyl)amino]ethyl]anthra[1,9-*cd*]pyrazol-6(2*H*)-one is prepared as follows:

A mixture of 11.1 g (40 mmol) of 1,5-dichloro-9,10-anthracenedione, 13.1 g (110 mmol) of 2-[(hydrazinoethyl)amino]ethanol, 4 g of anhydrous potassium bicarbonate, 1 g of anhydrous potassium fluoride, and 110 ml of dimethyl sulfoxide is stirred at 70°C overnight. The mixture is chilled and the solids are collected by filtration, washed with water, then thoroughly with acetonitrile to give a residue that is

crystallized from 2-propanol to leave 2.6 g of product. The hydrochloride salt is prepared as described in Example 3; mp 272—273°C (decomposition).

Example 82

7,10-Dichloro-2-[2-[(2-hydroxyethyl)amino]ethyl]-5-[[2-[(2-hydroxyethyl)amino]ethyl]amino]anthra[1,9-*cd*]pyrazol-6(2*H*)-one

Reaction of 7,10-dichloro-2-[2-[(2-hydroxyethyl)amino]ethyl]-5-[[(4-methylphenyl)sulfonyl]oxy]-anthra[1,9-*cd*]pyrazol-6(2*H*)-one, hydrochloride, with 2-(2-aminoethylamino)ethanol gives the product.

7,10-Dichloro-2-[2-[(2-hydroxyethyl)amino]ethyl]-5-[[(4-methylphenyl)sulfonyl]oxy]anthra[1,9-*cd*]pyrazol-6(2*H*)-one is prepared as follows:

To a suspension of 30.9 g (50 mmol) of 1,4-dichloro-5,8-bis[[(4-methylphenyl)sulfonyl]oxy]-9,10-anthracenedione, 13 ml (75 mmol) of *N,N*-diisopropylethylamine, and 130 ml of *N,N*-dimethylformamide at 5°C is added dropwise 14.9 g (125 mmol) of 2-[(hydrazinoethyl)amino]ethanol in 70 ml of *N,N*-dimethylformamide. The mixture is allowed to reach 10°C during five hours, then is diluted with 20 ml of acetone. After warming to room temperature, the solution is concentrated to an oil that is distributed between water and dichloromethane. Concentration of the dried dichloromethane layer followed by salt formation as described in Example 3 gives 18.1 g of the dried product as the hydrochloride salt; mp 158—160°C.

1,4-Dichloro-5,8-bis[[(4-methylphenyl)sulfonyl]oxy]-9,10-anthracenedione is prepared as follows:

A mixture of 9.3 g (30 mmol) of 1,4-dichloro-5,8-dihydroxy-9,10-anthracenedione, 12.6 g (66 mmol) of *p*-toluenesulfonyl chloride, 12.2 ml (70 mmol) of *N,N*-diisopropylethylamine, and 120 ml of acetonitrile is heated at 70°C for one hour, then cooled. The crystals are collected by filtration to leave 14.4 g of dried product; mp 195.5—196.5°C. Processing of the filtrates gives 2.2 g of additional product; mp 190—192°C.

Example 83

7-Hydroxy-2-[2-[(2-hydroxyethyl)amino]ethyl]-5-[[2-[(2-hydroxyethyl)amino]ethyl]amino]anthra[1,9-*cd*]pyrazol-6(2*H*)-one

Reaction of a mixture of 2-[2-[(2-hydroxyethyl)amino]ethyl]-5-[[2-[(2-hydroxyethyl)amino]ethyl]-amino]-7-(phenylmethoxy)anthra[1,9-*cd*]pyrazol-6(2*H*)-one with hydrogen and 20% palladium hydroxide on carbon as described in Example 78 gives the product as a salt with 2.0 equivalents of hydrogen chloride solvated with 2.3 equivalents of water; mp 265—270°C (decomposition).

2-[2-[(2-hydroxyethyl)amino]ethyl]-5-[[2-[(2-hydroxyethyl)amino]ethyl]amino-7-(phenylmethoxy)anthra[1,9-*cd*]pyrazol-6(2*H*)-one is prepared as follows:

Reaction of 5-chloro-2-[2-[(2-hydroxyethyl)amino]ethyl]-7-(phenylmethoxy)anthra[1,9-*cd*]pyrazol-6(2*H*)-one with 2-(2-aminoethylamino)ethanol as described in Example 78 gives the product; mp 157—159°C.

5-Chloro-2-[2-[(2-hydroxyethyl)amino]ethyl]-7-(phenylmethoxy)anthra[1,9-*cd*]pyrazol-6(2*H*)-one is prepared as follows:

A mixture of 4.2 g (11.0 mmol) of 1,4-dichloro-5-(phenylmethoxy)-9,10-anthracenedione, 2.6 g (22.0 mmol) of 2-[(hydrazinoethyl)amino]ethanol, 320 mg (5.5 mmol) of anhydrous potassium fluoride, 1.1 g (11.0 mmol) of anhydrous potassium bicarbonate, and 33 ml of dimethylsulfoxide is stirred overnight at 80°C. The mixture is cooled and poured into water. The aqueous mixture is centrifuged and the aqueous phase is decanted to leave an oil that is dried and purified on silica gel utilizing 4:1 dichloromethane:methanol as eluting solvent. Concentration of the product fractions followed by trituration from methanol gives 840 mg of the dried product; mp 141—145°C.

1,4-Dichloro-5-(phenylmethoxy)-9,10-anthracenedione is prepared as follows:

A mixture of 5.33 g (18 mmol) of 1,4-dichloro-5-hydroxy-9,10-anthracenedione (British Patent 1,029,448), 2.6 g (19 mmol) of powdered anhydrous potassium carbonate, 2.5 ml (21 mmol) of benzyl bromide, and 75 ml of dry acetone is heated at reflux overnight. The mixture is cooled and the solids are washed well with acetone. Concentration of the filtrates gives a solid which is triturated with ether to afford 5.8 g of the dried product; mp 118—122°C.

Prepared in a fashion similar to Example 83 is the following:

Example 84

7-Hydroxy-2-[2-[(2-hydroxyethyl)amino]ethyl]-5-[[2-(methylamino)ethyl]amino]anthra[1,9-*cd*]pyrazol-6(2*H*)-one as a salt with 1.8 equivalents of hydrogen chloride solvated with 1.2 equivalents of water; mp 280—282°C (decomposition) is prepared from 2-[2-[(2-hydroxyethyl)amino]ethyl]-5-[[2-[methyl(phenylmethyl)amino]ethyl]amino]-7-(phenylmethoxy)anthra[1,9-*cd*]pyrazol-6(2*H*)-one; mp 110—113°C, which is prepared from the reaction of *N*-methyl-*N*-(phenylmethyl)-1,2-ethanediamine (US Patent 3,201,459) with 5-chloro-2-[2-[(2-hydroxyethyl)amino]ethyl]-7-(phenylmethoxy)anthra[1,9-*cd*]pyrazol-6(2*H*)-one.

Example 85

10-Hydroxy-2-[2-[(2-hydroxyethyl)amino]ethyl]-5-[[2-[(2-hydroxyethyl)amino]ethyl]amino]anthra[1,9-*cd*]pyrazol-6(2*H*)-one

Reaction of a mixture of 2-[2-[(2-hydroxyethyl)amino]ethyl]-5-[[2-[(2-hydroxyethyl)amino]ethyl]-

amino]-10-(phenylmethoxy)anthra[1,9-*cd*]pyrazol-6(2*H*)-one with hydrogen and 20% palladium hydroxide on carbon as described in Example 78 gives the product as a salt with 2.1 equivalents of hydrogen chloride solvated with 0.8 equivalent of water; mp 260—267°C (decomposition).

2-[2-[(2-hydroxyethyl)amino]ethyl]-5-[[2-[(2-hydroxyethyl)amino]ethyl]amino-10-(phenylmethoxy)-anthra[1,9-*cd*]pyrazol-6(2*H*)-one is prepared as follows:

Reaction of 5-chloro-2-[2-[(2-hydroxyethyl)amino]-ethyl]-10-(phenylmethoxy)anthra[1,9-*cd*]pyrazol-6(2*H*)-one with 2-(2-aminoethylamino)ethanol as described in Example 78 gives the product; mp 178—180°C.

5-Chloro-2-[2-[(2-hydroxyethyl)amino]ethyl]-10-(phenylmethoxy)anthra[1,9-*cd*]pyrazol-6(2*H*)-one is prepared as follows:

Reaction of 1,4-dichloro-5-(phenylmethoxy)-9,10-anthracenedione with 2-[(hydrazinoethyl)amino]-ethanol as described in Example 83 gives the product as the minor isomer; mp 165—167°C.

Example 86

7,9,10-Trihydroxy-2-[2-[(2-hydroxyethyl)amino]ethyl]-5-[[2-[(2-hydroxyethyl)amino]lethyl]amino]-anthra[1,9-*cd*]pyrazol-6(2*H*)-one

Reaction of a solution of 2-[2-[(2-hydroxyethyl)amino]ethyl]-5-[[2-[(2-hydroxyethyl)-amino]ethyl]amino]-7,9,10-tris(phenylmethoxy)anthra[1,9-*cd*]pyrazol-6(2*H*)-one in glacial acetic acid with hydrogen and 20% palladium hydroxide on carbon as described in Example 78 gives the product as a salt with 2.1 equivalents of hydrogen chloride solvated with 0.8 equivalent of water; mp >235°C (decomposition).

2-[2-[(2-Hydroxyethyl)amino]ethyl]-5-[[2-[(2-hydroxyethyl)amino]ethyl]amino]-7,9,10-tris(phenyl-methoxy)anthra[1,9-*cd*]pyrazol-6(2*H*)-one is prepared as follows:

A mixture of 660 mg (1 mmol) of 5-chloro-2-[2-[(2-hydroxyethyl)amino]ethyl]-7,9,10-tris(phenyl-methoxy)anthra[1,9-*cd*]pyrazol-6(2*H*)-one, 1 ml (10 mmol) of 2-(2-aminoethylamino)ethanol, and 2 ml of pyridine is heated at reflux for 28 hours. Workup as described in Example 21 gives a solid whose crystallization from acetonitrile:chloroform affords 308 mg of product; mp 158—159°C.

5-Chloro-2-[2-[(2-hydroxyethyl)amino]ethyl]-7,9,10-tris(phenylmethoxy)anthra[1,9-*cd*]pyrazol-6(2*H*)-one is prepared as follows:

A suspension of 7.2 g (12 mmol) of 5,8-dichloro-1,2,4-tris(phenylmethoxy)-9,10-anthracenedione, 2.9 g (24 mmol) of 2-[(hydrazinoethyl)amino]ethanol, 350 mg (6 mmol) of anhydrous potassium fluoride, 1.2 g (12 mmol) of anhydrous potassium bicarbonate, and 25 ml of dimethylsulfoxide is stirred overnight at 75°C. The mixture is cooled, then triturated with 2-propanol. The solids are filtered, washed well with water, 2-propanol, than crystallized from chloroform to give 2.3 g of orange solid; mp 172—173°C.

Processing of the nonaqueous filtrates gives 640 mg of additional product corresponding to ca. 1:1 mixture of isomers by $^{1}H$ NMR; mp 135—140°C.

5,8-Dichloro-1,2,4-tris(phenylmethoxy)-9,10-anthracenedione is prepared as follows:

A suspension of 7.5 g (23 mmol) of 5,8-dichloro-1,2,4-trihydroxy-9,10-anthracenedione, 9.6 ml (81 mmol) of benzyl bromide, 9.9 g (72 mmol) of powdered anhydrous potassium carbonate, 0.4 ml of methanol, 92 ml of acetone, and 46 ml of *N,N*-dimethylformamide is heated at reflux under argon for two days. An additional 2.7 ml of benzyl bromide is added and the mixture is heated for three days. The suspension is filtered and the filtrate is concentrated to an oil which is distributed between dichloromethane and 10% aqueous acetic acid. The dried dichloromethane layer is concentrated to a solid whose crystallization from ethyl acetate affords 7.2 g of product; mp 174—175°C.

5,8-Dichloro-1,2,4-trihydroxy-9,10-anthracenedione is prepared as follows:

A suspension of 451 mg (1 mmol) of 1,2,4-tris(acetyloxy)-5,8-dichloro-9,10-anthracenedione, 5 ml of glacial acetic acid, and 5 ml of 6N aqueous hydrochloric acid is heated at 70°C for one hour. The suspension is cooled and the solids are filtered off. After washing with water and drying, there remains 287 mg of the dried product; mp 290—295°C (decomposition).

1,2,4-Tris(acetyloxy)-5,8-dichloro-9,10-anthracenedione is prepared as follows:

A suspension of 307 mg (1 mmol) of 5,8-dichloro-1,4,9,10-anthracenetetrone, 0.05 ml of 72% perchloric acid, and 10 ml of acetic anhydride is stirred at room temperature for 30 minutes. The solution is diluted with water, the organic layer is separated, and dried, then concentrated to a solid residue. Trituration of the solid from ethyl acetate leaves 235 mg of product; mp 205—206°C.

5,8-Dichloro-1,4,9,10-anthracenetetrone is prepared as follows:

A suspension of 618 mg (2 mmol) of 1,4-dichloro-5,8-dihydroxy-9,10-anthracenedione, 1.06 g (2.4 mmol) of lead tetraacetate, and 25 ml of glacial acetic acid is stirred at room temperature for 45 minutes. The mixture is treated with 0.5 ml ethylene glycol, and after 15 minutes is diluted with dichloromethane. The mixture is washed with water and the dried organic layer is evaporated to a solid. Trituration of the solid from diethyl ether gives 569 mg of product; mp 255—257°C (decomposition).

Example 87

7,8,10-Trihydroxy-2-[2-[(2-hydroxyethyl)amino]ethyl]-5-[[2-[(2-hydroxyethyl)amino]ethyl]amino]-anthra[1,9-*cd*]pyrazol-6(2*H*)-one

Reaction of a solution of 2-[2-[(2-hydroxyethyl)amino]ethyl]-5-[[2-[(2-hydroxyethyl)amino]-

ethyl]amino]-7,8,10-tris(phenylmethoxy)anthra[1,9-*cd*]pyrazol-6(2*H*)-one in glacial acetic acid with hydrogen and 20% palladium hydroxide on carbon as described in Example 78 gives the product as a salt with 2.1 equivalents of hydrogen chloride solvated with 0.6 equivalent of water; mp >210°C (decomposition).

2-[2-[(2-Hydroxyethyl)amino]ethyl]-5-[[2-[(2-hydroxyethyl)amino]ethyl]amino]-7,8,10-tris(phenylmethoxy)anthra[1,9-*cd*]pyrazol-6(2*H*)-one is prepared as follows:

Reaction of 5-chloro-2-[2-[(2-hydroxyethyl)amino]ethyl]-7,8,10-tris(phenylmethoxy)anthra[1,9-*cd*]pyrazol-6(2*H*)-one with 2-(2-aminoethylamino)ethanol as described in Example 86 gives the product; mp 186—188°C.

5-Chloro-2-[2-[(2-hydroxyethyl)amino]ethyl]-7,8,10-tris(phenylmethoxy)anthra[1,9-*cd*]pyrazol-6(2*H*)-one is prepared as follows:

Reaction of 5,8-dichloro-1,2,4-tris(phenylmethoxy)-9,10-anthracenedione with 2-[(hydrazinoethyl)-amino]ethanol as described in Example 86 gives the product as the minor isomer; mp 164—167°C.

Prepared in a fashion similar to Example 87 is the following:

Example 88

7,8,10-Trihydroxy-2-[2-[(2-hydroxyethyl)amino]ethyl]-5-[[2-(methylamino)ethyl]amino]anthra[1,9-*cd*]pyrazol-6(2*H*)-one as a salt with 2.0 equivalents of hydrogen chloride solvated with 0.7 equivalent of water; mp >220°C (decomposition), which is prepared from 2-[2-[(2-hydroxyethyl)amino]ethyl]-5-[2-[methyl(phenylmethyl)amino]ethyl]amino]-7,8,10-tris(phenylmethoxy)anthra[1,9-*cd*]pyrazol-6(2*H*)-one; mp 104—108°C which is prepared from the reaction *N*-methyl-*N*(phenylmethyl)-1,2-ethanediamine with 5-chloro-2-[2-[(2-hydroxyethyl)amino]ethyl]-7,8,10-tris(phenylmethoxy)anthra[1,9-*cd*]pyrazol-6(2*H*)-one.

Example 89

5-[[2-[(2-Aminoethyl)amino]ethyl]amino]-7,10-dihydroxy-2-(2-hydroxyethyl)anthra[1,9-*cd*]pyrazol-6(2*H*)-one

Reaction of a solution of 5-[[2-[(2-aminoethyl)amino]ethyl]amino-2-(2-hydroxyethyl)-7,10-bis(phenylmethoxy)anthra[1,9-*cd*]pyrazol-6(2*H*)-one in glacial acetic acid with hydrogen and 20% palladium hydroxide on carbon as described in Example 78 gives the product as a salt with 2.4 equivalents of hydrogen chloride solvated with 0.8 equivalent of water; mp 170—185°C (decomposition).

5-[[2-[(2-Aminoethyl)amino]ethyl]amino-2-(2-hydroxyethyl)-7,10-bis(phenylmethoxy)anthra[1,9-*cd*]pyrazol-6(2*H*)-one is prepared as follows:

A mixture of 5.1 g (10 mmol) of 5-chloro-2-(2-hydroxyethyl)-7,10-bis(phenylmethoxy)anthra[1,9-*cd*]pyrazol-6(2*H*)-one, 10 g (100 mmol) of diethylenetriamine, 1.4 g (10 mmol) of anhydrous potassium carbonate, and 60 ml of pyridine is heated at reflux for 28 hours. The mixture is cooled, the solids are collected by filtration then washed sequentially with water and 2-propanol to give 3.1 g of the product; mp 185—190°C.

Example 90

2-(3-Aminopropyl)-7,10-dihydroxy-5-[[2-[(2-hydroxyethyl)amino]ethyl]amino]anthra[1,9-*cd*]pyrazol-6(2*H*)-one

Reaction of a solution of 1.2 g (2 mmol) of 2-(3-aminopropyl)-5-[[2-[(2-hydroxyethyl)amino]ethyl]amino]-7,10-bis(phenylmethoxy)anthra[1,9-*cd*]pyrazol-6(2*H*)-one in glacial acetic acid with hydrogen and 20% palladium hydroxide on carbon as described in Example 78 followed by salt formation as described in Example 3 gives 850 mg of the product a salt with 2.0 equivalents of hydrogen chloride solvated with 1.1 equivalents of water; mp 292—294°C (decomposition).

2-(3-Aminopropyl)-5-[[2-[(2-hydroxyethyl)amino]ethyl]amino-7,10-bis(phenylmethoxy)anthra[1,9-*cd*]pyrazol-6(2*H*)-one is prepared as follows:

A mixture of 530 mg (1 mmol) of 2-(3-aminopropyl)-5-chloro-7,10-bis(phenylmethoxy)anthra-1,9-*cd*]pyrazol-6(2*H*)-one, 1 ml (10 mmol) of 2-(2-aminoethylamino)ethanol, 140 mg (1 mmol) of anhydrous potassium carbonate, and 8 ml of pyridine is heated at reflux for 22 hours. Workup as described in Example 89 gives 400 mg of the product; mp 191—195°C.

2-(3-Aminopropyl)-5-chloro-7,10-bis(phenylmethoxy)anthra[1,9-*cd*]pyrazol-6(2*H*)-one is prepared as follows:

Reaction of a mixture of 1,4-dichloro-5,8-bis-(phenylmethoxy)-9,10-anthracenedione and (3-aminopropyl)hydrazine [*Helvetica Chimica Acta 42;* 533 (1959)] as described in Example 54 gives the product; mp 180—184°C.

Prepared in a fashion similar to Example 90 is the following:

Example 91

2 - (3 - Aminopropyl - 5 - [[2 - [[2 - (dimethylamino)ethyl]amino]ethyl]amino] - 7,10 - dihydroxy-anthra[1,9 - *cd*]pyrazol - 6(2*H*) - one as a salt with 3.0 equivalents of hydrogen chloride solvated with 2.0 equivalents of water; mp 294°C (decomposition), which is prepared from 2 - (3 - aminopropyl) - 5 - [[2 - [[2 - (dimethylamino)ethyl]amino]ethyl]amino] - 7,10 - bis(phenylmethoxy)anthra[1,9 - *cd*]pyrazol - 6(2*H*) - one; mp 143—160°C, which is prepared from the reaction of *N,N*-dimethyldiethylenetriamine with 2 - (3 - aminopropyl) - 5 - chloro - 7,10 - bis(phenylmethoxy)anthra[1,9 - *cd*]pyrazol - 6(2*H*) - one.

Example 92

2-(2-Aminoethyl-7,10-dihydroxy-5-[[2-(methylamino)ethyl]amino]anthra[1,9-*cd*]pyrazol-6(2*H*)-one

Reaction of a mixture of 2-(2-aminoethyl)-5-[[2-methyl(phenylmethyl)amino]ethyl]amino]-7,10-bis(phenylmethoxy)anthra[1,9-*cd*]pyrazol-6(2*H*)-one with hydrogen and 20% palladium hydroxide on carbon as described in Example 78 gives the product as a salt with 2.2 equivalents of hydrogen chloride solvated with 0.5 equivalent of water; mp 259—264°C (decomposition).

2 - (2 - Aminoethyl) - 5 - [[2 - [methyl)phenylmethylamino]ethyl]amino] - 7,10 - bis(phenylmethoxy)anthra[1,9 - *cd*]pyrazol - 6(2*H*) - one is prepared as follows:

Reaction of 2-(2-aminothyl)-5-chloro-7,10-bis(phenylmethoxy)anthra[1,9-*cd*]pyrazol-6(2*H*)-one with *N*-methyl-*N*-(phenylmethyl)-1,2-ethanediamine as described in Example 78 gives the product; mp 169—172°C.

Example 93

7,10-Dihydroxy-5-[[2-[(2-hydroxyethyl)amino]ethyl]amino]-2-[2-(methylamino)ethyl]anthra[1,9-*cd*]pyrazol-6(2*H*)-one

Reaction of a mixture of 5-[[2-[(2-hydroxyethyl)amino]ethyl]amino]-2-[2-(methylamino)ethyl]-7,10-bis-(phenylmethoxy)anthra[1,9-*cd*]pyrazol-6(2*H*)-one with hydrogen and 20% palladium hydroxide on carbon as described in Example 78 gives the product as a salt with 2.0 equivalents of hydrogen chloride solvated with 1.8 equivalents of water; mp 180—185°C (decomposition).

5 - [[2 - [(2 - hydroxyethyl)amino]ethyl]amino] - 2 - [2 - (methylamino)ethyl] - 7,10 - bis(phenylmethoxy)anthra[1,9 - *cd*]pyrazol - 6(2*H*) - one is prepared as follows:

Reaction of 5 - chloro - 2 - (2 - (methylamino)ethyl] - 7,10 - bis(phenylmethoxy)anthra[1,9 - *cd*]-pyrazol - 6(2*H*) - one with 2 - (2 - aminoethylamino)ethanol as described in Example 78 gives the product; mp 186—189°C.

5 - Chloro - 2 - [2 - (methylamino)ethyl - 7,10 - bis(phenylmethoxy)anthra[1,9 - *cd*]pyrazol - 6(2*H*) - one is prepared as follows:

Reaction of 5 - chloro - 2 - [3 - [[4 - methylphenyl)sulfonyl]oxy]ethyl - 7,10 - bis(phenylmethoxy)-anthra[1,9 - *cd*]pyrazol - 6(2*H*) - one with methylamine as described in Example 51 gives the product; mp 171—176°C.

Example 94

5-[(2-Aminoethyl)amino]-2-[3-(dimethylamino)propyl]-7,10-dihydroxyanthra[1,9-*cd*]pyrazol-6(2*H*)-one

Reaction of 5 - chloro - 2 - [3 - (dimethylamino)propyl] - 7,10 - dihydroxyanthra[1,9 - *cd*]pyrazol - 6(2*H*) - one, hydrochloride, with ethylenediamine as described in Example 46 gives the product as a salt with 2.0 equivalents of hydrogen chloride solvated with 0.5 equivalent of water and 0.1 equivalent of 2-propanol; mp 316°C (decomposition).

Example 95

7,8-Dihydroxy-2-[2-[(2-hydroxyethyl)amino]ethyl]-5-[[2-[(2-hydroxyethyl)amino]ethyl]amino]anthra[1,9-*cd*]pyrazol-6(2*H*)-one

Reaction of a mixture of 2 - [2 - [(2 - hydroxyethyl)amino]ethyl] - 5 - [[2 - [(2 - hydroxyethyl)amino]-ethyl]amino] - 7,8 - bis(phenylmethoxy)anthra[1,9 - *cd*]pyrazol - 6(2*H*) - one with hydrogen and 20% palladium hydroxide on carbon as described in Example 78 gives the product.

2 - [2 - [(2 - hydroxyethyl)amino]ethyl] - 5 - [[2 - [(2 - hydroxyethyl)amino]ethyl]amino] - 7,8 - bis(phenylmethoxy)anthra[1,9 - *cd*]pyrazol - 6(2*H*) - one is prepared as follows:

Reaction of 5 - chloro - 2 - [2 - [(2 - hydroxyethyl)amino]ethyl] - 7,8 - bis(phenylmethoxy)anthra-[1,9 - *cd*]pyrazol - 6(2*H*) - one with 2 - (2 - aminoethylamino)ethanol as described in Example 78 gives the product.

5 - Chloro - 2 - [2 - [(2 - hydroxyethyl)amino]ethyl] - 7,8 - bis(phenylmethoxy)anthra[1,9 - *cd*]-pyrazol - 6(2*H*) - one is prepared as follows:

Reaction of 1,4-dichloro-5,6-bis(phenylmethoxy-9,10-anthracenedione with 2-[(hydrazinoethyl)amino]-ethanol as described in Example 54 gives the product.

1,4-Dichloro-5,6-bis(phenylmethoxy)-9,10-anthracenedione is prepared as follows:

Reaction of 1,4-dichloro-5,6-dihydroxy-9,10-anthracenedione with benzyl bromide as described in Example 48 gives the product.

1,4-Dichloro-5,6-dihydroxy-9,10-anthracenedione is prepared as follows:

Reaction of nitrosyl sulfuric acid and 5,6-diamino-1,4-dichloro-9,10-anthracenedione [*Khim. Geterotsikl. Soedin.* 808 (1968)] gives the product.

**Claims for the Contracting States: BE CH DE FR GB IT LI LU NL SE**

1. A compound having the structural formula

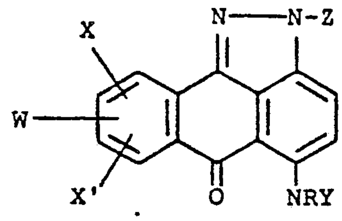

wherein X, X′, and W may be the same or different and are H, OH, alkoxy of one to four carbon atoms or chlorine; R is H or alkyl of from one to six carbon atoms; Y is H, alkyl of from one to six carbon atoms which may be substituted with an $OR_1$ group wherein $R_1$ is H or alkyl of from one to six carbon atoms, or $ANR_2R_3$ wherein A is straight or branched alkylene of from two to eight carbon atoms, $R_2$ and $R_3$ may be the same or different and are H, alkyl of from one to six carbon atoms which may be substituted with OH or an NRaRa wherein Ra may be the same or different and is H or alkyl of from one to three carbon atoms which may be substituted with OH, or $NR_bR_b$ wherein $R_b$ is the same or different and is H or alkyl of from one to three carbon atoms, or $R_2$ and $R_3$ when taken together may be ethylene or may form

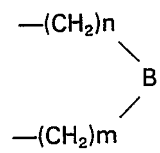

wherein n and m may be the same or different and are one, two, or three provided that the sum of n and m is an integer of from three to six, and B is a direct bond, O, S, or N—$R_4$ wherein $R_4$ is H or alkyl of from one to six carbon atoms; R and Y when taken together may be ethylene or may form

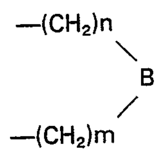

wherein n, m, and B are defined above; Z is H, alkyl of from one to six carbon atoms which may be substituted with an $N(R_1)_2$, $SR_1$, or $OR_1$ group wherein $R_1$ is the same or different and is defined above, or $DNR_2R_3$ wherein D is straight or branched alkylene of from two to eight carbon atoms which may be substituted with an OH group and $R_2$ and $R_3$ are as defined above; and the pharmaceutically acceptable salts thereof; with the following provisos, 1) when all of X, X′ and W are H and Z is H, R and Y when taken together do not complete a piperidine ring, 2) when all of X, X′ and W are H and Z is $CH_3$, R and Y when taken together do not complete a piperidine ring or a morpholine ring, and 3) when both of R and Y are H, Z is neither H nor alkyl of from 1 to 6 carbon atoms.

2. A compound having the structural formula

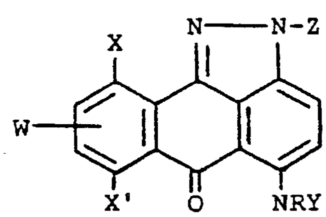

wherein X, X′, and W may be the same or different and are H or OH, alkoxy of one to four carbon atoms or chlorine; R is H or alkyl of from one to six carbon atoms; Y is H, alkyl of from one to six carbon atoms which may be substituted with an $OR_1$ group wherein $R_1$ is H or alkyl of from one to six carbon atoms, or $ANR_2R_3$ wherein A is straight or branched alkylene of from two to eight carbon atoms, $R_2$ and $R_3$ may be the same or different and are H, alkyl of from one to six carbon atoms which may be substituted with OH or an NRaRa wherein Ra may be the same or different is H or alkyl of from one to three carbon atoms which may be substituted with OH, or $NR_bR_b$ wherein $R_b$ is the same or different and is H or alkyl of from one to three carbon atoms, or $R_2$ and $R_3$ when taken together may be ethylene or may form

$$-(CH_2)_n \backslash B / -(CH_2)_m$$

wherein n and m may be the same or different and are one, two, or three provided that the sum of n and m is an integer of from three to six, and B is a direct bond, O, S, or N—$R_4$ wherein $R_4$ is H or alkyl of from one to six carbon atoms; R and Y when taken together may be ethylene or may form

$$-(CH_2)_n \backslash B / -(CH_2)_m$$

wherein n, m, and B are defined above; Z is H, alkyl of from one to six carbon atoms which may be substituted with an $N(R_1)_2$, $SR_1$, or $OR_1$ group wherein $R_1$ is the same or different and is as defined above, or $DNR_2R_3$ wherein D is straight or branched alkylene of from two to eight carbon atoms which may be substituted with an OH group and $R_2$ and $R_3$ are as defined above; and the pharmaceutically acceptable salts thereof; with the following provisos, 1) when X and X′ are H and Z is H, R and Y when taken together do not complete a piperidine ring, 2) when X and X′ are H and Z is $CH_3$, R and Y when taken together do not complete a piperidine ring or a morpholine ring, and 3) when both of R and Y are H, Z is neither H nor alkyl of from one to six carbon atoms.

3. A compound having the structural formula

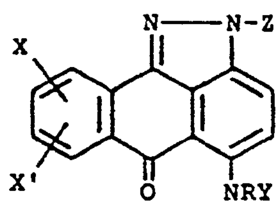

wherein X and X′ may be the same or different and are H, OH, alkoxy of one to four carbon atoms or chlorine; R is H or alkyl of from one to six carbon atoms; Y is H, alkyl of from one to six carbon atoms which may be substituted with an $OR_1$ group wherein $R_1$ is H or alkyl of from one to six carbon atoms, or $ANR_2R_3$ wherein A is straight or branched alkylene of from two to eight carbon atoms, $R_2$ and $R_3$ may be the same or different and are H, alkyl of from one to six carbon atoms which may be substituted with OH or an NRaRa wherein Ra may be the same or different is H or alkyl of from one to three carbon atoms which may be substituted with OH, or $NR_bR_b$ wherein $R_b$ is the same or different and is H or alkyl of from one to three carbon atoms, or $R_2$ and $R_3$ when taken together may be ethylene or may form

$$-(CH_2)_n \backslash B / -(CH_2)_m$$

wherein n and m may be the same or different and are one, two, or three provided that the sum of n and m is an integer of from three to six, and B is a direct bond, O, S, or N—$R_4$ wherein $R_4$ is H or alkyl of from one to six carbon atoms; R and Y when taken together may be ethylene or may form

$$-(CH_2)_n \backslash B / -(CH_2)_m$$

wherein n, m, and B are defined above; Z is H, alkyl of from one to six carbon atoms which may be substituted with an $N(R_1)_2$, $SR_1$, or $OR_1$ group wherein $R_1$ is the same or different and is as defined above, or $DNR_2R_3$ wherein D is straight or branched alkylene of from two to eight carbon atoms which may be substituted with an OH group and $R_2$ and $R_3$ are as defined above; and the pharmaceutically acceptable salts thereof; with the following provisos, 1) when X and X′ are H and Z is H, R and Y when taken together do not complete a piperidine ring, 2) when X and X′ are H and Z is $CH_3$, R and Y when taken together do not

complete a piperidine ring or a morpholine ring, and 3) when both of R and Y are H, Z is neither H nor alkyl of from 1 to 6 carbon atoms.

4. A compound having the structural formula I

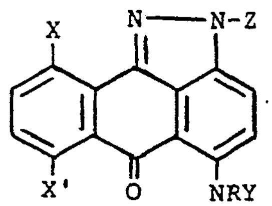

(I)

wherein X and X′ may be the same or different and are H or OH; R is H or alkyl of from one to six carbon atoms; Y is H, alkyl of from one to six carbon atoms which may be substituted with an $OR_1$ group wherein $R_1$ is H or alkyl of from one to six carbon atoms, or $ANR_2R_3$ wherein A is straight or branched alkylene of from two to eight carbon atoms, $R_2$ and $R_3$ may be the same or different and are H, alkyl of from one to six carbon atoms which may be substituted with OH or an NRaRa wherein Ra may be the same or different is H or alkyl of from one to three carbon atoms which may be substituted with OH, or $NR_bR_b$ wherein $R_b$ is the same or different and is H or alkyl of from one to three carbon atoms, or $R_2$ and $R_3$ when taken together may be ethylene or may form

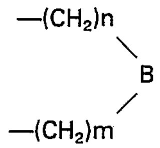

wherein n and m may be the same or different and are one, two, or three provided that the sum of n and m is an integer of from three to six, and B is a direct bond, O, S, or N—$R_4$ wherein $R_4$ is H or alkyl of from one to six carbon atoms; R and Y when taken together may be ethylene or may form

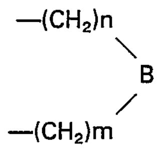

wherein n, m, and B are defined above; Z is H, alkyl of from one to six carbon atoms which may be substituted with an $N(R_1)_2$, $SR_1$, or $OR_1$ group wherein $R_1$ is the same or different and is as defined above, or $DNR_2R_3$ wherein D is straight or branched alkylene of from two to eight carbon atoms which may be substituted with an OH group and $R_2$ and $R_3$ are as defined above; and the pharmaceutically acceptable salts thereof; with the following provisos, 1) when X and X′ are H and Z is H, R and Y when taken together do not complete a piperidine ring, 2) when X and X′ are H and Z is $CH_3$, R and Y when taken together do not complete a piperidine ring or a morpholine ring, and 3) when both of R and Y are H, Z is neither H nor alkyl of from 1 to 6 carbon atoms.

5. The chemical compounds defined in Claim 4 wherein X and X′ are OH; and the pharmaceutically acceptable salts thereof.

6. The chemical compounds defined in Claim 4 wherein X and X′ are H; and the pharmaceutically acceptable salts thereof.

7. The chemical compounds defined in Claim 4 wherein A and D are the same or different and are ethylene or propylene; and the pharmaceutically acceptable salts thereof.

8. The chemical compounds defined in Claim 4 having the structural formula I′

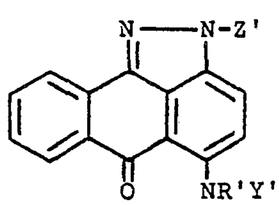

(I′)

wherein R′ is H or alkyl of from 1 to 6 carbon atoms, Y′ is $CH_2CH_2NHCH_2CH_2OH$ when Z′ is alkyl of from one to four carbon atoms which may be substituted with an $SR_1'$, or $OR_1'$ group wherein $R_1'$ is H or alkyl of from one to four carbon atoms or $D'NR_2'R_3'$ wherein D′ is straight or branched alkylene of from two to four carbon

atoms which may be substituted with an OH group and $R_2'$ and $R_3'$ may be the same or different and are H, alkyl of from one to six carbon atoms which may be substituted with an OH or $R_2'$ and $R_3'$ when taken together may be ethylene or may form

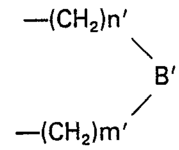

wherein n' and m' may be th same or different and are one or two provided that the sum of n' and m' is three of four, and B' is a direct bond, O, S, or N—$R_4'$ wherein $R_4'$ is H or alkyl of from one to four carbon atoms; or Z' is $CH_2CH_2NHCH_2CH_2OH$ when Y' is H, alkyl of from one to six carbon atoms which may be substituted with an $OR_1'$ group wherein $R_1'$ is defined above or $A'NR_1'R_2'$ wheein A' is alkylene of from two to four carbon atoms and $R_1'$ and $R_2'$ are as defined above; and the pharmaceutically acceptable salts thereof.

9. The compounds defined in Claim 4 having structural formula I''

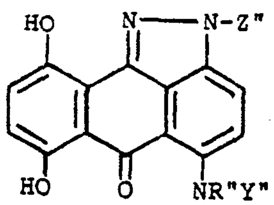

(I'')

wherein R'' is H or alkyl of from one to six carbon atoms; Y'' is H, of from one to six carbon atoms which may be substituted with an $OR_1''$ group wherein $R_1''$ is H or alkyl of from one to four carbon atoms, or $A''NR_2''R_3''$ wherein A'' is alkylene of from two to four carbon atoms, $R_2''$ and $R_3''$ may be the same or different and are H, alkyl of from one to six carbon atoms which may be substituted with an OH or an NRa''Ra'' wherein Ra'' may be the same or different and is H or alkyl of from one to three carbon atoms which may be substituted with an OH or $R_2''$ and $R_3''$ when taken together may be ethylene or may form

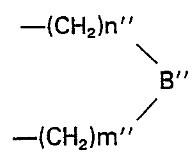

wherein n'' and m'' may be the same or different and are one or two provided that the sum of n'' and m'' is three or four, and B'' is a direct bond, O, S, or N—$R_4''$ wherein $R_4''$ is H or alkyl of from one to four carbon atoms; Z'' is alkyl of from one to four carbon atoms which may be substituted with an $SR_1''$, or $OR_1''$ group wherein $R_1''$ is defined above, or $D''NR_2''R_3''$ wherein D'' is alkylene of from two to four carbon atoms which may be substituted with an OH group and $R_2''$ and $R_3''$ are as defined above; and the pharmaceutically acceptable salts thereof.

10. The compounds defined in Claim 2 have structural formula I'''

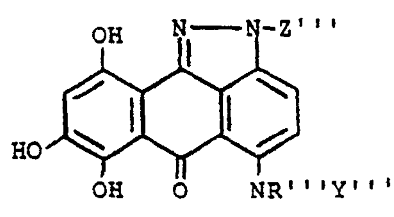

(I''')

wherein R''' is H or alkyl of from one to six carbon atoms; Y''' is H, alkyl of from one to six carbon atoms which may be substituted with an $OR_1'''$ group wherein $R_1'''$ is H or alkyl of from one to four carbon atoms, or $A'''NR_2'''R_3'''$ wherein A''' is alkylene of from two to four carbon atoms, $R_2'''$ and $R_3'''$ may be the same or different and are H, alkyl of from one to six carbon atoms which may be substituted with an OH or an NRa'''Ra''' wherein Ra''' may be the same or different and is H or alkyl of from one to three carbon atoms which may be substituted with an OH or $R_2'''$ and $R_3'''$ when taken together may be ethylene or may form

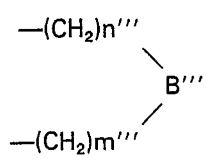

wherein n''' and m''' may be the same or different and are one or two provided that the sum of n''' and m''' is three or four, and B''' is a direct bond, O, S, or N—$R_4'''$ wherein $R_4'''$ is H or alkyl of from one to four carbon atoms; Z''' is alkyl of from one to four carbon atoms which may be substituted with an $SR_1'''$, or $OR_1'''$ group wherein $R_1'''$ is defined above, or $D'''NR_2'''R_3'''$ wherein D''' is alkylene of from two to four carbon atoms which may be substituted with an OH group and $R_2'''$ and $R_3'''$ are as defined above; and the pharmaceutically acceptable salts thereof.

11. The compounds defined in Claim 4 having structural formula I''''

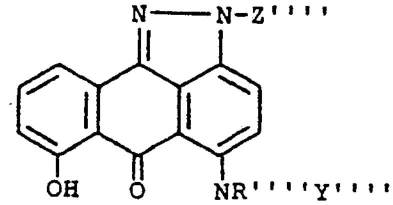

(I'''')

wherein R'''' is H or alkyl of from one to six carbon atoms; Y'''' is H, or from one to six carbon atoms which may be substituted with an $OR_1''''$ group wherein $R_1''''$ is H or alkyl of from one to four carbon atoms, or $A''''NR_2''''R_3''''$ wherein A'''' is alkylene of from two to four carbon atoms, $R_2''''$ and $R_3''''$ may be the same or different and are H, alkyl of from one to six carbon atoms which may be substituted with an OH or an NRa''''Ra'''' wherein Ra'''' may be the same or different and is H or alkyl of from one to three carbon atoms which may be substituted with an OH or $R_2''''$ and $R_3''''$ when taken together may be ethylene or may form

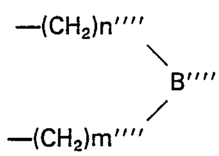

wherein n'''' and m'''' may be the same or different and are one or two provided that the sum of n'''' and m'''' is three or four, and B'''' is a direct bond, O, S, or N—$R_4''''$ wherein $R_4''''$ is H or alkyl of from one to four carbon atoms; Z'''' is alkyl of from one to four carbon atoms which may be substituted with an $SR_1''''$, or $OR_1''''$ group wherein $R_1''''$ is defined above, or $D''''NR_2''''R_3''''$ wherein D'''' is alkylene of from two to four carbon atoms which may be substituted with an OH group and $R_2''''$ and $R_3''''$ are as defined above; and the pharmaceutically acceptable salts thereof.

12. The compounds defined in Claim 4 having the structural formula $I^v$

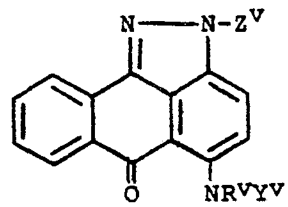

($I^V$)

wherein $R^v$ is H or alkyl of from one to six carbon atoms; $Y^v$ is H, alkyl of from one to four carbon atoms or $A^vNR_2^vR_3^v$ wherein $A^v$ is alkylene of from two to four carbon atoms, $R_2^v$ and $R_3^v$ may be the same or different and are H or alkyl of from one to six carbon atoms which may be substituted with an OH or $R_2^v$ and $R_3^v$ when taken together may be ethylene or may form

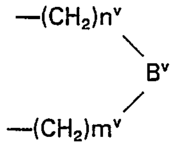

wherein $n^v$ and $m^v$ may be the same or different and are one or two provided that the sum of $n^v$ and $m^v$ is three or four, and $B^v$ is a direct bond, O, S, or $NR_4^v$ wherein $R_4^v$ is H or alkyl of from one to four carbon atoms; $Z^v$ is alkyl of from one to four carbon atoms, which may be substituted with an $SR_1^v$, or $OR_1^v$ group wherein $R_1^v$ is H or alkyl of from one to four carbon atoms, or $D^vNR_2^vR_3^v$ wherein $D^v$ is alkylene of from two to four carbon atoms which may be substituted with an OH group and $R_2^v$ and $R_3^v$ are defined above; and the pharmaceutically acceptable salts thereof.

13. The compounds defined in Claim 4 have the structural formula $I^{vi}$

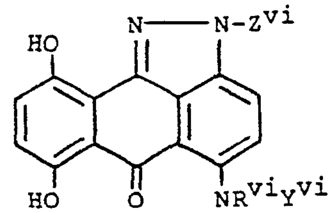

$(I^{vi})$

wherein $R^{vi}$ is H or alkyl of from one to six carbon atoms; $Y^{vi}$ is H, alkyl of form one to four carbon atoms which may be substituted with an $OR_1{}^{vi}$ group wherein $R_1{}^{vi}$ is H or alkyl of from one to four carbon atoms or $A^{vi}NR_2{}^{vi}R_3{}^{vi}$ wherein $A^{vi}$ is alkylene of from two to four carbon atoms $R_2{}^{vi}$ and $R_3{}^{vi}$ may be the same or different and are alkyl of from one to six carbon atoms which may be substituted with an OH, or $R_2{}^{vi}$ and $R_3{}^{vi}$ when taken together may be ethylene or may form

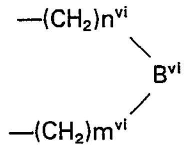

wherein $n^{vi}$ and $m^{vi}$ may be the same or different and are one or two provided that the sum of $n^{vi}$ and $m^{vi}$ is three or four, and $B^{vi}$ is a direct bond, O, S, or $NR_4{}^{vi}$ wherein $R_4{}^{vi}$ is H or alkyl of from one to four carbon atoms; $Z^{vi}$ is $D^{vi}NR_2{}^{vi}R_3{}^{vi}$ wherein $D^{vi}$ is alkylene of from two to four carbon atoms, $R_2{}^{vi}$ and $R_3{}^{vi}$ are defined above; and the pharmaceutically acceptable salts thereof.

14. The chemical compounds defined in Claim 2 having the following names:

2-[2-[(2-hydroxyethyl)amino]ethyl]-5-[[2-[(2-hydroxyethyl)amino]ethyl]amino]anthra[1,9-*cd*]pyrazol-6(2*H*)-one;

2-[2-[(2-diethylamino)ethyl]-7,10-dihydroxy-5-[[2-[(2-hydroxyethyl)amino]ethyl]amino]anthra[1,9-*cd*]-pyrazol-6(2*H*)-one;

2-[2-[[2-(dimethylamino)ethyl]amino]ethyl]-5-[[2-[(2-hydroxyethyl)amino]ethyl]amino]-7,10-dihydroxyanthra[1,9-*cd*]pyrazol-6(2*H*)-one;

5-[(2-aminoethyl)amino]-2-[2-(diethylamino)ethyl]-7,10-dihydroxyanthra[1,9-*cd*]pyrazol-6(2*H*)-one; 2-[2-(dimethylamino)ethyl]-7,10-dihydroxy-5-[[2-[(2-hydroxyethyl)amino]ethyl]amino]anthra[1,9-*cd*]pyrazol-6(2*H*)-one;

5-[[2-[dimethylamino)ethyl]amino]-7,10-dihydroxy-2-(2-hydroxyethyl)anthra[1,9-*cd*]pyrazol-6(2*H*)-one;

5-[(2-aminoethyl)amino]-7,10-dihydroxy-2-[2-[(2-hydroxyethyl)amino]ethyl]anthra[1,9-*cd*]pyrazol-6(2*H*)-one;

5-[(3-aminopropyl)amino]-7,10-dihydroxy-2-[2-[(2-hydroxyethyl)amino]ethyl]anthra[1,9-*cd*]pyrazol-6(2*H*)-one;

5-[[2-[[2-(dimethylaminoethyl]amino]ethyl]amino]-7,10-dihydroxy-2-[2-[(2-hydroxyethyl)amino]ethyl]-anthra[1,9-*cd*]pyrazol-6(2*H*)-one;

5-[[2-[bis(2-hydroxyethyl)amino]ethyl]amino]-7,10-dihydroxy-2-[2-[(2-hydroxyethyl)amino]thyl]-anthra[1,9-*cd*]pyrazol-6(2*H*)-one;

7,10-dihydroxy-2-(2-[(2-hydroxyethyl)amino]ethyl]-5-[[2-(methylamino)ethyl]amino]anthra[1,9-*cd*]-pyrazol-6(2*H*)-one;

2-(2-aminomethyl)-7,10-dihydroxy-5-[[2-[(2-hydroxyethyl)amino]ethyl]amino]anthra[1,9-*cd*]pyrazol-6(2*H*)-one;

2-(2,3-dihydroxypropyl)-7,10-dihydroxy-5-[[2-[(2-hydroxyethyl)amino]ethyl]amino]anthra[1,9-*cd*]-pyrazol-6(2*H*)-one;

7-hydroxy-2-[2-[(2-hydroxyethyl)amino]ethyl]-5-[[2-[(2-hydroxyethyl)amino]ethyl]amino]anthra[1,9-*cd*]pyrazol-6(2*H*)-one;

7-hydroxy-2-[2-[(2-hydroxyethyl)amino]ethyl)-5-[[2-(methylamino)ethyl]amino]anthra[1,9-*cd*]pyrazol-6(2*H*)-one;

7,8,10-trihydroxy-2-[2-[(2-hydroxethyl)amino]ethyl]-5-[[2-[(2-hydroxyethyl)amino]ethyl]amino]anthra-[1,9-*cd*]pyrazol-6(2*H*)-one;

7,8,10-trihydroxy-2-[2-[(2-hydroxyethyl)amino)]ethyl]-5-[[2-(methylamino)ethyl]amino]anthra[1,9-*cd*]-pyrazol-6(2*H*)-one;

2-(3-aminopropyl)-7,10-dihydroxy-5-[[2-[(2-hydroxyethyl)amino]ethyl]amino]anthra[1,9-*cd*]pyrazol-6(2*H*)-one;

2-(2-aminoethyl)-7,10-dihydroxy-5-[[2-(methylamino)ethyl]amino]anthra[1,9-*cd*]pyrazol-6(2*H*)-one;

5-[(2-aminoethyl)amino)-2-[3-(dimethylamino)propyl]-7,10-dihydroxyanthra[1,9-*cd*]pyrazol-6(2*H*)-one;

7,8-dihydroxy-2-[2-[(2-hydroxyethyl)amino]ethyl]-5-[[2-[(2-hydroxyethyl)amino]ethyl]amino]anthra-[1,9-*cd*]pyrazol-6(2*H*)-one;
and the pharmaceutically acceptable salts thereof.

15. A chemical compound having the structural formula:

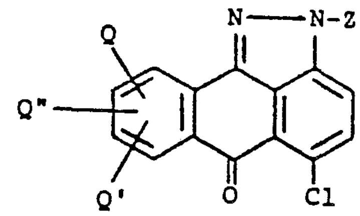

wherein Q, Q′ and Q″ may be the same or different and are H, OH, benzyloxy, *p*-chlorobenzyloxy and *p*-methoxybenzyloxy; and the pharmaceutically acceptable salts thereof; Z is defined in Claim 1; and the pharmaceutically acceptable salts thereof; provided that when Q = Q′ = Q″ = H, Z may not be H or alkyl of from 1 to 6 carbon atoms.

16. A chemical compound having the structural formula III

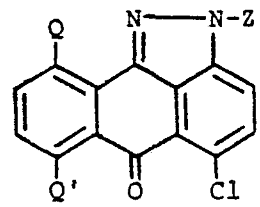

(III)

wherein Q and Q′ may be the same or different and are H, OH, benzyloxy, *p*-chlorobenzyloxy, or *p*-methoxybenzyloxy and Z is defined in Claim 1; and the pharmaceutically acceptable salts thereof; provided that when Q = Q′ = H, Z may not be H or alkyl of from 1 to 6 carbon atoms.

17. The chemical compounds defined in Claim 16 wherein Q and Q′ are H; and the pharmaceutically acceptable salts thereof.

18. The chemical compounds defined in Claim 16 wherein Q and Q′ are benzyloxy, *p*-chlorobenzyloxy, or *p*-methoxybenzyloxy; and the pharmaceutically acceptable salts thereof.

19. The chemical compounds defined in Claim 16 wherein Q and Q′ are OH; and the pharmaceutically acceptable salts thereof.

20. The chemical compounds defined in Claim 15 having the following names:
5-chloro-2-[2-[(2-hydroxyethyl)amino]ethyl]anthra[1,9-*cd*]pyrazol-6(2*H*)-one;
5-chloro-2-[2-(diethylamino)ethyl]-7,10-dihydroxyanthra[1,9-*cd*]pyrazol-6(2*H*)-one;
5-chloro-7,10-dihydroxy-2-(2-hydroxyethyl)anthra[1,9-*cd*]pyrazol-6(2*H*)-one;
5-chloro-2-[2-(diethylamino)ethyl]-7,10-bis(phenylmethoxy)anthra[1,9-*cd*]pyrazol-6(2*H*)-one;
5-chloro-2-[2-[[(4-methylphenyl)sulfonyl]oxy]ethyl]-7,10-bis(phenylmethoxy)anthra[1,9-*cd*]pyrazol-6(2*H*)-one;
5-chloro-7,10-dihydroxy-2-[2-[(2-hydroxyethyl)amino]ethyl]anthra[1,9-*cd*]pyrazol-6(2*H*)-one;
5-chloro-2-[2-[(2-hydroxyethyl)amino]ethyl-7,10-bis(phenylmethoxy)anthra[1,9-*cd*]pyrazol-6(2*H*)-one;
5-chloro-2-[2-[[2-(dimethylamino)ethyl]amino]ethyl]-7,10-dihydroxyanthra[1,9-*cd*]pyrazol-6(2*H*)-one;
5-chloro-2-[2-(dimethylamino)ethyl]-7,10-dihydroxyanthra[1,9-*cd*]pyrazol-6(2*H*)-one;
5-chloro-2-[3-(dimethylamino)propyl]-7,10-dihydroxyanthra[1,9-*cd*]pyrazol-6(2*H*)-one;
5-chloro-2-(2-hydroxyethyl)-7,10-bis(phenylmethoxy)anthra[1,9-*cd*]pyrazol-6(2*H*)-one;
5-chloro-2-[3-[[4-methylphenyl)sulfonyl]oxy]propyl]-7,10-bis(phenylmethoxy)anthra[1,9-*cd*]pyrazol-6(2*H*)-one;
5-chloro-2-(3-hydroxypropyl)-7,10-bis(phenylmethoxy)anthra[1,9-*cd*]pyrazol-6(2*H*)-one;
2-(2-aminoethyl)-5-chloro-7,10-dihydroxyanthra[1,9-*cd*]pyrazol-6(2*H*)-one;
2-(2-aminoethyl)-5-chloro-7,10-bis(phenylmethoxy)-anthra[1,9-*cd*]pyrazol-6(2*H*)-one;
5-chloro-2-[(2,2-dimethyl-1,3-dioxolan-4-yl)methyl]-7,10-bis(phenylmethoxy)anthra[1,9-*cd*]pyrazol-6(2*H*)-one;
5-chloro-2-[2-[(2-hydroxyethyl)amino]ethyl]-7-(phenylmethoxy)anthra[1,9-*cd*]pyrazol-6(2*H*)-one;
5-chloro-2-[2-[(2-hydroxyethyl)amino]ethyl]-10-(phenylmethoxy)anthra[1,9-*cd*]pyrazol-6(2*H*)-one;
5-chloro-2-[2-[(2-hydroxyethyl)amino]ethyl]-7,8,10-tris(phenylmethoxy)anthra[1,9-*cd*]pyrazol-6(2*H*)-one;
2-(3-aminopropyl)-5-chloro-7,10-bis(phenylmethoxy)anthra[1,9-*cd*]pyrazol-6(2*H*)-one;
5-chloro-2-[2-[(2-hydroxyethyl)amino]ethyl]-7,8-bis(phenylmethoxy)anthra[1,9-*cd*]pyrazol-6(2*H*)-one;
5-chloro-2-[2-[[2-(dimethylamino)ethyl]amino]ethyl]-7,10-bis(phenylmethoxy)anthra[1,9-*cd*]pyrazol-6(2*H*)-one;
and the pharmaceutically acceptable salts thereof.

21. A compound having the structural formula IV

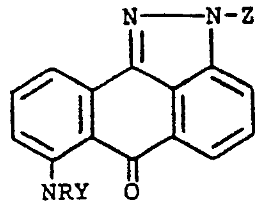

(IV)

wherein R is H or alkyl of from one to six carbon atoms; Y is H, alkyl of from one to six carbon atoms which may be substituted with an $OR_1$ group wherein $R_1$ is H or alkyl of from one to six carbon atoms, or $ANR_2R_3$ wherein A is alkylene of from two to eight carbon atoms, $R_2$ and $R_3$ may be the same or different and are H, alkyl of from one to six carbon atoms which may be substituted with OH or an NRaRa wherein Ra may be the same or different and is H or alkyl of from one to three carbon atoms which may be substituted with OH, or $R_2$ and $R_3$ when taken together may be ethylene or may form

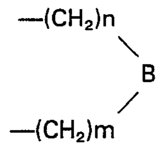

wherein n and m may be the same or different and are one, two, or three, provided that the sum of n and m is an integer of from three to six, and B is a direct bond, O, S, or N—$R_4$ wherein $R_4$ is H or alkyl of from one to six carbon atoms; R and Y when taken together may be ethylene or may form

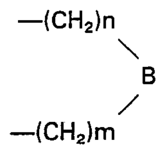

wherein n, m, and B are defined above; Z is H, alkyl of from one to six carbon atoms which may be substituted with an $N(R_1)_2$, $SR_1$, or $OR_1$ group wherein $R_1$ may be the same or different and is defined above, or $DNR_2R_3$ wherein D is alkylene of from two to eight carbon atoms which may be substituted with an OH group and $R_2$ and $R_3$ are as defined above; and the pharmaceutically acceptable salts thereof; with the following provisos, 1) when Z is H, R and Y when taken together do not complete a piperidine ring, 2) when Z is $CH_3$, R and Y when taken together do not complete a piperidine ring or a morpholine ring, and 3) when both R and Y are H, Z is neither H nor alkyl having from 1 to 6 carbon atoms.

22. The chemical compounds defined in Claim 21 having the following names:

2-[2-(diethylamino)ethyl]-7-[2-[(2-hydroxyethyl)amino]ethyl]amino]anthra[1,9-*cd*]pyrazol-6(2*H*)-one;

2-[2-(diethylamino)ethyl-7-[[2-(diethylamino)ethyl]amino]anthra[1,9-*cd*]pyrazol-6(2*H*)-one;

2-[2-[(2-hydroxyethyl)amino]ethyl]-7-[[2-[(2-hydroxyethyl)amino]ethyl]amino]anthra[1,9-*cd*]pyrazol-6(2*H*)-one

and the pharmaceutically acceptable salts thereof.

23. A compound having the structural formula VII

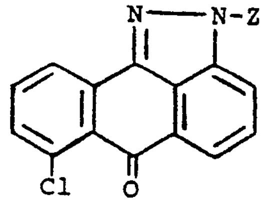

(VII)

wherein Z is defined in Claim 1, provided it is not H or alkyl having from 1 to 6 carbon atoms.

24. The chemical compound defined in Claim 23 having the following name:

7-chloro-2-[2-(diethylamino)ethyl]anthra[1,9-*cd*]pyrazol-6(2*H*)-one;

7-chloro-2-[2-[(2-hydroxyethyl)amino]ethyl]anthra[1,9-*cd*]pyrazol-6(2*H*)-one;

and the pharmaceutically acceptable salts thereof.

25. The compound 2-[(hydrazinoethyl)amino]ethanol and the acid addition salts thereof.

26. 5,8-Dichloro-1,4,9,10-anthracenetetrone.

27. A process for preparing a compound defined in Claim 1 having the structural formula

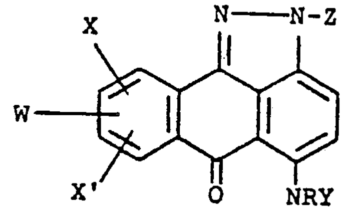

which comprises reacting a compound having the structural formula

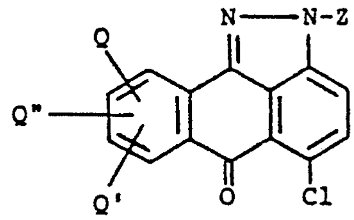

with an amine having the formula NHRY wherein W, X, X′, Q, Q′, Q″, Y, Z, and R are defined in Claim 1 and, if necessary removing any benzyl groups by catalytic hydrogenation or by treatment with boron tribromide or trichloride.

28. A process for preparing a compound defined in Claim 2 having structural formula I

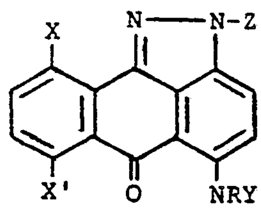

(I)

which comprises reacting a compound having structural formula II

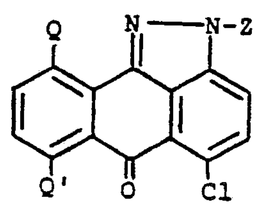

(II)

with an amine having the formula HNRY wherein X, X′, Q, Q′, Y, Z, and R are defined in Claim 2.

29. The process defined in Claim 28 wherein X and X′ are OH.

30. The process defined in Claim 28 wherein X and X′ are H.

31. A process for preparing a compound defined in Claim 16 having structural formula III

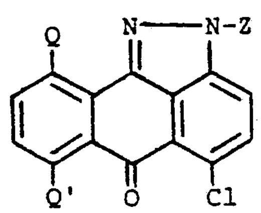

(III)

which comprises reacting a compound having formula V

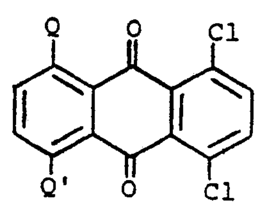

(V)

with a hydrazine having the formula $H_2N$—NHZ, wherein Q, Q' and Z are defined in Claim 15.

32. The process defined in Claim 31 wherein Q and Q' are benzyloxy, *p*-chlorobenzyloxy, or *p*-methoxybenzyloxy.

33. The process defined in Claim 31 wherein Q and Q' are OH.

34. The process defined in Claim 31 wherein Q and Q' are H.

35. A process for preparing a compound defined in Claim 21 having structural formula IV

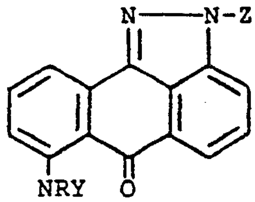

(IV)

which comprises reacting a compound having structural formula VII

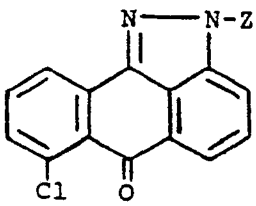

(VII)

with an amine having the formula HNRY, wherein Y, Z, and R are defined in Claim 21.

36. A pharmaceutical composition comprising a compound defined in any one of Claims 1 to 26 or a pharmaceutically acceptable salt thereof in combination with a pharmaceutically acceptable carrier.

37. A compound of the structural formula

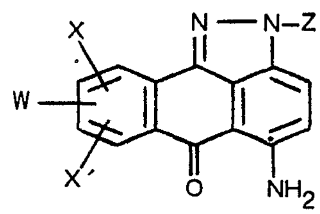

wherein Z is hydrogen or alkyl having from one to six carbon atoms, and X, X' and W are as defined in Claim 1,
or a pharmaceutically acceptable salt thereof, for use in the treatment of microbial infections in a mammal.

38. The use, of a compound as defined in Claim 37, for the preparation of a medicament for treating leukemia in a mammal.

39. The use, of a compound as defined in Claim 37, for the preparation of a medicament for treating solid tumors in a mammal.

40. A compound of the structural formula

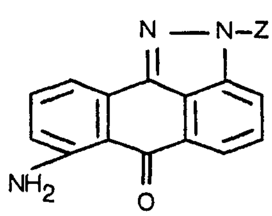

wherein Z is hydrogen or an alkyl having from one to six carbon atoms, or a pharmaceutically acceptable salt thereof, for use in the treatment of microbial infections in a mammal.

41. The use, of a compound as defined in Claim 40, for the preparation of a medicament for treating leukemia in a mammal.

42. The use, of a compound as defined in Claim 40, for the preparation of a medicament for treating solid tumors in a mammal.

**Claims for the Contracting State: AT**

1. A process for producing a compound having the structural formula

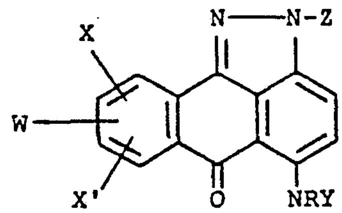

wherein X, X′, and W may be the same or different and are H, OH, alkoxy of one to four carbon atoms or chlorine; R is H or alkyl of from one to six carbon atoms; Y is H, alkyl of from one to six carbon atoms which may be substituted with an $OR_1$ group wherein $R_1$ is H or alkyl of from one to six carbon atoms, or $ANR_2R_3$ wherein A is straight or branched alkylene of from two to eight carbon atoms, $R_2$ and $R_3$ may be the same or different and are H, alkyl of from one to six carbon atoms which may be substituted with OH or an NRaRa wherein Ra may be the same or different and is H or alkyl of from one to three carbon atoms which may be substituted with OH, or $NR_bR_b$ wherein $R_b$ is the same or different and is H or alkyl of from one to three carbon atoms, or $R_2$ and $R_3$ when taken together may be ethylene or may form

—$(CH_2)n$
\
B
/
—$(CH_2)m$

wherein n and m may be the same or different and are one, two, or three provided that the sum of n and m is an integer of from three to six, and B is a direct bond, O, S, or N—$R_4$ wherein $R_4$ is H or alkyl of from one to six carbon atoms; R and Y when taken together may be ethylene or may form

—$(CH_2)n$
\
B
/
—$(CH_2)m$

wherein n, m, and B are defined above; Z is H, alkyl of from one to six carbon atoms which may be substituted with an $N(R_1)_2$, $SR_1$, or $OR_1$ group wherein $R_1$ is the same or different and is defined above, or $DNR_2R_3$ wherein D is straight or branched alkylene of from two to eight carbon atoms which may be substituted with an OH group and $R_2$ and $R_3$ are as defined above; and the pharmaceutically acceptable salts thereof; with the following provisos, 1) when X, X′ and W are H and Z is H, R and Y when taken together do not complete a piperidine ring, 2) when X, X′ and W are H and Z is $CH_3$, R and Y when taken together do not complete a piperidine ring or a morpholine ring, and 3) when both of R and Y are H, Z is neither H or alkyl of from 1 to 6 carbon atoms which comprises reacting a compound having the structural formula

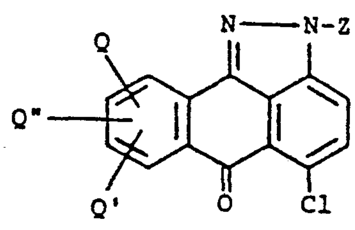

with an amine having the formula HNRY wherein W, X, X′, Q, Q′, Q″, Y, Z, and R are defined above and, if necessary, removing any benzyl groups by catalytic hydrogenation or by treatment with boron tribromide or trichloride.

2. A process for producing a compoound having the structural formula

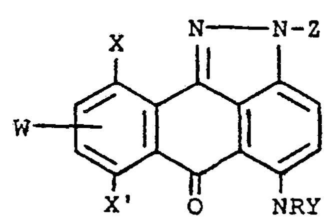

wherein X, X′, and W may be the same or different and are H, OH, alkoxy of one to four carbon atoms or chlorine; R is H or alkyl of from one to six carbon atoms; Y is H, alkyl of from one to six carbon atoms which may be substituted with an $OR_1$ group wherein $R_1$ is H or alkyl of from one to six carbon atoms, or $ANR_2R_3$ wherein A is straight or branched alkylene of from two to eight carbon atoms, $R_2$ and $R_3$ may be the same or different and are H, alkyl of from one to six carbon atoms which may be substituted with OH or an NRaRa wherein Ra may be the same or different is H or alkyl of from one to three carbon atoms which may be substituted with OH, or $NR_bR_b$ wherein $R_b$ is the same or different and is H or alkyl of from one to three carbon atoms, or $R_2$ and $R_3$ when taken together may be ethylene or may form

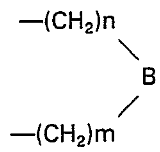

wherein n and m may be the same or different and are one, two, or three provided that the sum of n and m is an integer of from three to six, and B is a direct bond, O, S, or N—$R_4$ wherein $R_4$ is H or alkyl of from one to six carbon atoms; R and Y when taken together may be ethylene or may form

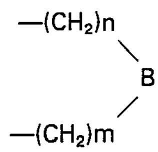

wherein n, m, and B are defined above; Z is H, alkyl of from one to six carbon atoms which may be substituted with an $N(R_1)_2$, $SR_1$, or $OR_1$ group wherein $R_1$ is the same or different and is defined above, or $DNR_2R_3$ wherein D is straight or branched alkylene of from two to eight carbon atoms which may be substituted with an OH group and $R_2$ and $R_3$ are as defined above; and the pharmaceutically acceptable salts thereof; with the following provisos, 1) when X, X′ and are H and Z is H, R and Y when taken together do not complete a piperidine ring, 2) when X and X′ are H and Z is $CH_3$, R and Y when taken together do not complete a piperidine ring or a morpholine ring, and 3) when both of R and Y are H, Z is neither H nor alkyl of from 1 to 6 carbon atoms, which comprises reacting a compound having structural formula II

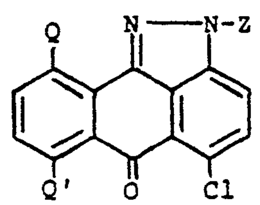

(II)

with an amine having the formula HNRY wherein X, X′, Q, Q′, Y, Z, and R are defined above.

3. The process defined in Claim 2 wherein X and X′ are OH.

4. The process defined in Claim 2 wherein X and X′ are H.

5. A process for producing a chemical compound having the structural formula III

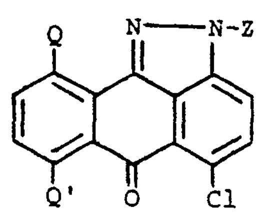

(III)

wherein Q and Q′ may be the same or different and are H, OH, benzyloxy, *p*-chlorobenzyloxy, or *p*-methoxybenzyloxy and Z is defined in Claim 1; and the pharmaceutically acceptable salts thereof; provided that when Q = Q′ = H, Z may not be H or alkyl of from 1 to 6 carbon atoms, which comprises reacting a compound having formula V

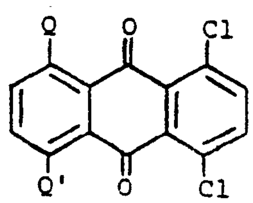

(V)

with a hydrazine having the formula $H_2N—NHZ$, wherein Q and Q′ may be the same or different and are H, OH, benzyloxy, *p*-chlorobenzyloxy and *p*-methoxybenzyloxy; and the pharmaceutically acceptable salts thereof;

Z is defined in Claim 1; and the pharmaceutically acceptable salts thereof; provided that when Q = Q′ = H, Z may not be H or alkyl of from 1 to 6 carbon atoms.

6. A process for producing a compound having the structural formula IV

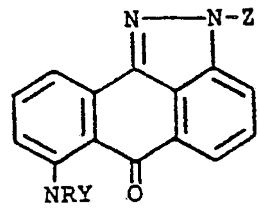

(IV)

wherein R is H or alkyl of from one to six carbon atoms; Y is H, alkyl of from one to six carbon atoms which may be substituted with an $OR_1$ group wherein $R_1$ is H or alkyl of from one to six carbon atoms, or $ANR_2R_3$ wherein A is alkylene of from two to eight carbon atoms, $R_2$ and $R_3$ may be the same or different and are H, alkyl of from one to six carbon atoms which may be substituted with OH or an NRaRa wherein Ra may be the same or different and is H or alkyl of from one to three carbon atoms which may be substituted with OH, or $R_2$ and $R_3$ when taken together may be ethylene or may form

—$(CH_2)n$
\
B
/
—$(CH_2)m$

wherein n and m may be the same or different and are one, two, or three, provided that the sum of n and m is an integer of from three to six, and B is a direct bond, O, S, or $N—R_4$ wherein $R_4$ is H or alkyl of from one to six carbon atoms; R and Y when taken together may be ethylene or may form

—$(CH_2)n$
\
B
/
—$(CH_2)m$

wherein n, m, and B are defined above; Z is H, alkyl of from one to six carbon atoms which may be substituted with an $N(R_1)_2$, $SR_1$, or $OR_1$ group wherein $R_1$ may be the same or different and is defined above, or $DNR_2R_3$ wherein D is alkylene of from two to eight carbon atoms which may be substituted with an OH group and $R_2$ and $R_3$ are as defined above; and the pharmaceutically acceptable salts thereof; with the following provisos, 1) when Z is H, R and Y when taken together do not complete a piperidine ring, 2) when Z is $CH_3$, R and Y when taken together do not complete a piperidine ring or a morpholine ring, and 3) when both R and Y are H, Z is neither H nor alkyl of from 1 to 6 carbon atoms, which comprises reacting a compound having the structural formula VII

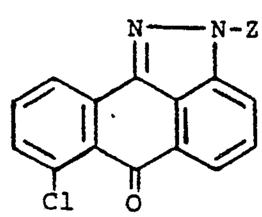

(VII)

with an amine having the formula HNRY, wherein Y, Z, and R are defined above.

7. A process for producing a pharmaceutical composition, comprising combining a compound produced by a process according to any preceding claim or a pharmaceutically acceptable salt thereof, with a pharmaceutically acceptable carrier.

8. A process for producing a medicament, comprising combining a compound having one of the following formulae:

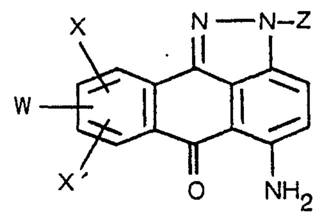 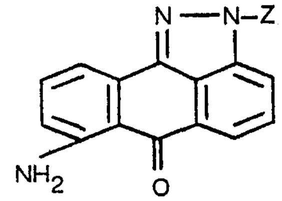

wherein X, X' and W are as defined in Claim 1, and Z is hydrogen or an alkyl having from one to six carbon atoms, or a pharmaceutically acceptable salt thereof, with a pharmaceutically acceptable carrier.

**Patentansprüche für die Vertragsstaaten: BE CH DE FR GB IT LI LU NL SE**

1. Verbindung der Strukturformel

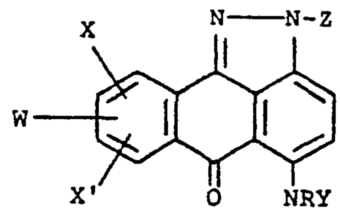

worin X, X' und W gleich oder verschieden sein können und H, OH, Alkoxy mit 1 bis 4 Kohlenstoffatomen oder Chlor bedeuten; R für H oder Alkyl mit 1 bis 6 Kohlenstoffatomen steht; Y für H, Alkyl mit 1 bis 6 Kohlenstoffatomen, welches substituiert sein kann durch eine $OR_1$-Gruppe, worin $R_1$ H oder Alkyl mit 1 bis 6 Kohlenstoffatomen darstellt, oder $ANR_2R_3$, worin A gerades oder verzweigtes Alkylen mit 2 bis 8 Kohlenstoffatomen darstellt, $R_2$ und $R_3$ gleich oder verschieden sein können und H, Alkyl mit 1 bis 6 Kohlenstoffatomen, welches substituiert sein kann durch OH oder ein NRaRa, worin Ra gleich oder verschieden sein kann und für H oder Alkyl mit 1 bis 3 Kohlenstoffatomen, welches durch OH substituiert sein kann, steht, oder $NR_bR_b$, worin $R_b$ gleich oder verschieden ist und H oder Alkyl mit 1 bis 3 Kohlenstoffatomen bedeutet, darstellen, oder $R_2$ und $R_3$, wenn sie zusammengenommen werden, Äthylen bedeuten können oder

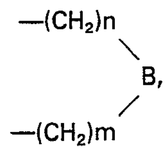

worin n und m gleich oder verschieden sein können und 1, 2 oder 3 bedeuten mit der Maßgabe, daß die Summe von n und m eine ganze Zahl von 3 bis 6 darstellt, und B eine direkte Bindung, O, S oder N—$R_4$, worin $R_4$ für H oder Alkyl mit 1 bis 6 Kohlenstoffatomen steht, darstellt, bilden können, steht; R und Y, wenn sie zusammengenommen werden, Äthylen sein können oder

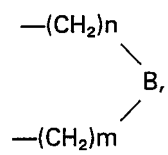

worin n und m und B wie oben definiert sind, bilden können; Z für H, Alkyl mit 1 bis 6 Kohlenstoffatomen, welches substituiert sein kann durch eine $N(R_1)_2$—, $SR_1$— oder $OR_1$-Gruppe, worin $R_1$ gleich oder verschieden ist und wie oben definiert ist, oder $DNR_2R_3$, worin D gerades oder verzweigtes Alkylen mit 2 bis 8 Kohlenstoffatomen, welches durch eine OH-Gruppe substituiert sein kann, darstellt, und $R_2$ und $R_3$ wie oben definiert sind, steht; und die pharmazeutisch akzeptablen Salze davon; mit den folgenden Maßgaben:

1) wenn alle von X, X' und W für H stehen und Z für H steht, vervollständigen R und Y nicht einen Piperidinring, wenn sie zusammengenommen werden,

2) wenn alle von X, X' und W für H stehen und Z $CH_3$ bedeutet, vervollständigen R und Y nicht einen Piperidinring oder einen Morpholinring, wenn sie zusammengenommen werden, und

3) wenn sowohl R als auch Y für H stehen, bedeutet Z weder H noch Alkyl mit 1 bis 6 Kohlenstoffatomen.

2. Verbindung der Strukturformel

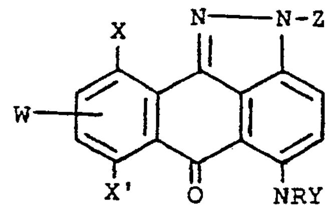

worin X, X′ und W gleich oder verschieden sein können und H oder OH, Alkoxy mit 1 bis 4 Kohlenstoffatomen oder Chlor bedeuten; R für H oder Alkyl mit 1 bis 6 Kohlenstoffatomen steht; Y H, Alkyl mit 1 bis 6 Kohlenstoffatomen, welches substituiert sein kann durch eine $OR_1$-Gruppe, worin $R_1$ für H oder Alkyl mit 1 bis 6 Kohlenstoffatomen steht, oder $ANR_2R_3$, worin A gerades oder verzweigtes Alkylen mit 2 bis 8 Kohlenstoffatomen darstellt, $R_2$ und $R_3$ gleich oder verschieden sein können und H, Alkyl mit 1 bis 6 Kohlenstoffatomen, welches substituiert sein kann durch OH oder ein NRaRa, worin Ra gleich oder verschieden sein kann, H oder Alkyl mit 1 bis 3 Kohlenstoffatomen, welches durch OH substituiert sein kann, darstellt, oder $NR_bR_b$, worin $R_b$ gleich oder verschieden ist und H oder Alkyl mit 1 bis 3 Kohlenstoffatomen darstellt, bedeuten, oder $R_2$ und $R_3$, wenn sie zusammengenommen werden, Äthylen sein können oder

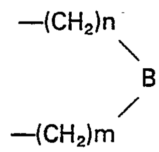

bilden können, worin n und m gleich oder verschieden sein können und 1, 2 oder 3 bedeuten, mit der Maßgabe, daß die Summe von n und m eine ganze Zahl von 3 bis 6 ist und B eine direkte Bindung, O, S oder N—$R_4$, worin $R_4$ für H oder Alkyl mit 1 bis 6 Kohlenstoffatomen steht, ist, bedeutet; R und Y, wenn sie zusammengenommen werden, Äthylen darstellen können oder

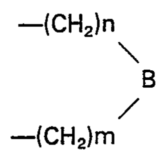

bilden können, worin n, m und B wie oben definiert sind; Z für H, Alkyl mit 1 bis 6 Kohlenstoffatomen, welches substituiert sein kann durch eine $N(R_1)_2$—, $SR_1$—, oder $OR_1$-Gruppe, worin $R_1$ gleich oder verschieden sein kann und wie oben definiert ist, oder $DNR_2R_3$, worin D gerades oder verzweigtes Alkylen mit 2 bis 8 Kohlenstoffatomen, welches durch eine OH-Gruppe substituiert sein kann, und $R_2$ und $R_3$ wie oben definiert sind, steht; und die pharmazeutisch akzeptablen Salze davon; mit den folgenden Maßgaben:

1) wenn X und X′ für H stehen und Z H bedeutet, vervollständigen R und Y nicht einen Piperidinring, wenn sie zusammengenommen werden,

2) wenn X und X′ für H stehen und Z $CH_3$ darstellt, vervollständigen R und Y nicht einen Piperidinring oder einen Morpholinring, wenn sie zusammengenommen werden und

3) wenn sowohl R als auch Y H bedeuten, bedeutet Z weder H noch Alkyl mit 1 bis 6 Kohlenstoffatomen.

3. Verbindung der Strukturformel

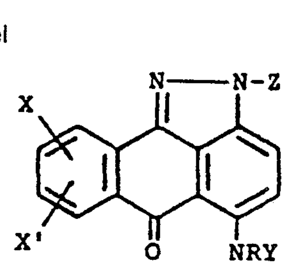

worin X, und X′ gleich oder verschieden sein können und H, OH, Alkoxy mit 1 bis 4 Kohlenstoffatomen oder Chlor bedeuten; R für H oder Alkyl mit 1 bis 6 Kohlenstoffatomen steht; Y für H, Alkyl mit 1 bis 6 Kohlenstoffatomen, welches substituiert sein kann durche eine $OR_1$-Gruppe, worin $R_1$ H oder Alkyl mit 1 bis 6 Kohlenstoffatomen darstellt, oder $ANR_2R_3$, worin A gerades oder verzweigtes Alkylen mit 2 bis 8 Kohlenstoffatomen darstellt, $R_2$ und $R_3$ gleich oder verschieden sein können und H, Alkyl mit 1 bis 6

Kohlenstoffatomen, welches substituiert sein kann durch OH oder ein NRaRa, worin Ra gleich oder verschieden sein kann und für H oder Alkyl mit 1 bis 3 Kohlenstoffatomen, welches durch OH substituiert sein kann, steht, oder $NR_bR_b$, worin $R_b$ gleich oder verschieden ist und H oder Alkyl mit 1 bis 3 Kohlenstoffatomen bedeutet, darstellen, oder $R_2$ und $R_3$, wenn sie zusammengenommen werden, Äthylen bedeuten können oder

$$\begin{matrix} -(CH_2)n \\ \quad\quad \diagdown \\ \quad\quad\quad B, \\ \quad\quad \diagup \\ -(CH_2)m \end{matrix}$$

worin n und m gleich oder verschieden sein können und 1, 2 oder 3 bedeuten mit der Maßgabe, daß die Summe von n und m eine ganze Zahl von 3 bis 6 darstellt, und B eine direkte Bindung, O, S oder N—$R_4$, worin $R_4$ für H oder Alkyl mit 1 bis 6 Kohlenstoffatomen steht, darstellt, bilden können, steht; R und Y, wenn sie zusammengenommen werden, Äthylen sein können oder

$$\begin{matrix} -(CH_2)n \\ \quad\quad \diagdown \\ \quad\quad\quad B, \\ \quad\quad \diagup \\ -(CH_2)m \end{matrix}$$

worin n und m und B wie oben definiert sind, bilden können; Z für H, Alkyl mit 1 bis 6 Kohlenstoffatomen, welches substituiert sein kann durch eine $N(R_1)_2$—, $SR_1$— oder $OR_1$-Gruppe, worin $R_1$ gleich oder verschieden ist und wie oben definiert ist, oder $DNR_2R_3$, worin D gerades oder verzweigtes Alkylen mit 2 bis 8 Kohlenstoffatomen, welches durch eine OH-Gruppe substituiert sein kann, darstellt, und $R_2$ und $R_3$ wie oben definiert sind, steht; und die pharmazeutisch akzeptablen Salze davon; mit den folgenden Maßgaben:

1) wenn X und X' für H stehen und Z für H steht, vervollständigen R und Y nicht einen Piperidinring, wenn sie zusammengenommen werden,

2) wenn X und X' für H stehen und Z $CH_3$ bedeutet, vervollständigen R und Y nicht einen Piperidinring oder einen Morpholinring, wenn sie zusammengenommen werden, und

3) wenn sowohl R als auch Y für H stehen, bedeutet Z weder H noch Alkyl mit 1 bis 6 Kohlenstoffatomen.

4. Verbindung der Strukturformel I

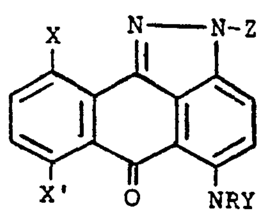

(I)

worin X und X' gleich oder verschieden sein können und H oder OH repräsentieren; R für H oder Alkyl mit 1 bis 6 Kohlenstoffatomen steht; Y für H, Alkyl mit 1 bis 6 Kohlenstoffatomen, welches substituiert sein kann durch eine $OR_1$-Gruppe, worin $R_1$ H oder Alkyl mit 1 bis 6 Kohlenstoffatomen darstellt, oder $ANR_2R_3$, worin A gerades oder verzweigtes Alkylen mit 2 bis 8 Kohlenstoffatomen darstellt, $R_2$ und $R_3$ gleich oder verschieden sein können und H, Alkyl mit 1 bis 6 Kohlenstoffatomen, welches substituiert sein kann durch OH oder ein NRaRa, worin Ra gleich oder verschieden sein kann und für H oder Alkyl mit 1 bis 3 Kohlenstoffatomen, welches durch OH substituiert sein kann, steht, oder $NR_bR_b$, worin $R_b$ gleich oder verschieden ist und H oder Alkyl mit 1 bis 3 Kohlenstoffatomen bedeutet, darstellen, oder $R_2$ und $R_3$, wenn sie zusammengenommen werden, Äthylen bedeuten können oder

$$\begin{matrix} -(CH_2)n \\ \quad\quad \diagdown \\ \quad\quad\quad B, \\ \quad\quad \diagup \\ -(CH_2)m \end{matrix}$$

worin n und m gleich oder verschieden sein können und 1, 2 oder 3 bedeuten, mit der Maßgabe, daß die Summe von n und m eine ganze Zahl von 3 bis 6 darstellt, und B eine direkte Bindung, O, S oder N—$R_4$, worin $R_4$ für H oder Alkyl mit 1 bis 6 Kohlenstoffatomen steht, bilden können, steht; R und Y, wenn sie zusammengenommen werden, Äthylen sein können oder

$$-(CH_2)n \diagdown B, \diagup -(CH_2)m$$

worin n, m und B wie oben definiert sind, bilden können; Z für H, Alkyl mit 1 bis 6 Kohlenstoffatomen, welches substituiert sein kann durch eine $N(R_1)_2$—, $SR_1$— oder $OR_1$-Gruppe, worin $R_1$ gleich oder verschieden ist und wie oben definiert ist, oder $DNR_2R_3$, worin D gerades oder verzweigtes Alkylen mit 2 bis 8 Kohlenstoffatomen, welches durch eine OH-Gruppe substituiert sein kann, darstellt, und $R_2$ und $R_3$ wie oben definiert sind, steht; und die pharmazeutisch akzeptablen Salze davon; mit den folgenden Maßgaben:

1) wenn X und X' für H stehen und Z für H steht, vervollständigen R und Y nicht einen Piperidinring, wenn sie zusammengenommen werden,

2) wenn X, X' für H stehen und Z $CH_3$ bedeutet, vervollständigen R und Y nicht einen Piperidinring oder einen Morpholinring, wenn sie zusammengenommen werden, und

3) wenn sowohl R als auch Y für H stehen, bedeutet Z weder H noch Alkyl mit 1 bis 6 Kohlenstoffatomen.

5. Chemische Verbindungen nach Anspruch 4, worin X und X' Oh bedeuten, und die pharmazeutisch akzeptablen Salze davon.

6. Chemische Verbindungen nach Anspruch 4, worin X und X' H bedeuten; und die pharmazeutisch akzeptablen Salze davon.

7. Chemische Verbindungen nach Anspruch 4, worin A und D gleich oder verschieden sind und Äthylen oder Propylen darstellen; und die pharmazeutisch akzeptablen Salze davon.

8. Verbindungen nach Anspruch 4 der Struturformel I'

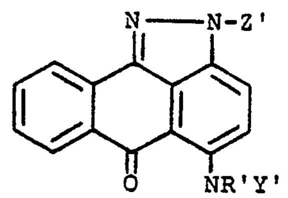

(I')

worin R' für H oder Alkyl mit 1 bis 6 Kohlenstoffatomen steht; Y' $CH_2CH_2NHCH_2CH_2OH$ darstellt, wenn Z' Alkyl mit 1 bis 4 Kohlenstoffatomen, welches substituiert sein kann durch eine $SR_1'$— oder $OR_1'$-Gruppe, worin $R_1'$ für H oder Alkyl mit 1 bis 4 Kohlenstoffatomen steht, oder $D'NR_2'R_3'$, worin D' gerades oder verzweigtes Alkylen mit 2 bis 4 Kohlenstoffatomen, welches durch eine OH-Gruppe substituiert sein kann, bedeutet, und $R_2'$ und $R_3'$ gleich oder verschieden sein können und H, Alkyl mit 1 bis 6 Kohlenstoffatomen, welches durch ein OH substituiert sein kann, repräsentieren oder $R_2'$ und $R_3'$, wenn sie zusammengenommen werden, Äthylen sein können oder

$$-(CH_2)n' \diagdown B' \diagup -(CH_2)m'$$

worin n' und m' gleich oder verschieden sein können und 1 oder 2 darstellen mit der Maßgabe, daß die Summe von n' und m' 3 oder 4 ist, und B' eine direkte Bindung, O, S oder N—$R_4'$, worin $R_4'$ für H oder Alkyl mit 1 bis 4 Kohlenstoffatomen steht, darstellt, bilden können, repräsentiert; oder Z' für $CH_2CH_2NHCH_2CH_2OH$ steht, wenn Y' H, Alkyl mit 1 bis 6 Kohlenstoffatomen, welches substituiert sein kann durch eine $OR_1'$-Gruppe, worin $R_1'$ wie oben definiert ist, oder $A'NR_1'R_2'$, worin A' Alkylen mit 2 bis 4 Kohlenstoffatomen bedeutet, und $R_1'$ und $R_2'$ wie oben definiert sind, repräsentiert; und die pharmazeutisch akzeptablen Salze davon.

9. Verbindung nach Anspruch 4 der Strukturformel I''

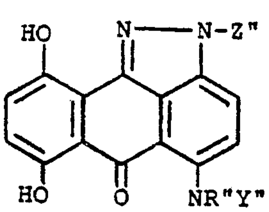

(I'')

worin R'' für H oder Alkyl mit 1 bis 6 Kohlenstoffatomen steht; Y'' H, Alkyl mit 1 bis 6 Kohlenstoffatomen,

welches substituiert sein kann durch eine $OR_1''$-Gruppe, worin $R_1''$ für H oder Alkyl mit 1 bis 4 Kohlenstoffatomen steht, oder $A''NR_2''R_3''$, worin $A''$ Alkylen mit 2 bis 4 Kohlenstoffatomen darstellt, $R_2''$ und $R_3''$ gleich oder verschieden sein können und H, Alkyl mit 1 bis 6 Kohlenstoffatomen, welches substituiert sein kann durch ein OH, oder ein $NRa''Ra''$, worin $Ra''$ gleich oder verschieden sein kann und für H oder Alkyl mit 1 bis 3 Kohlenstoffatomen, welches durch ein OH substituiert sein kann, steht, sind oder $R_2''$ und $R_3''$, wenn sie zusammengenommen werden, Äthylen sein können oder

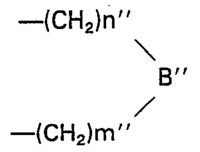

worin $n''$ und $m''$ gleich oder verschieden sein können und 1 oder 2 darstellen mit der Maßgabe, daß die Summe von $n''$ und $m''$ 3 oder 4 ist, und $B''$ eine direkte Bindung, O, S oder $N—R_4''$, worin $R_4''$ für H oder Alkyl mit 1 bis 4 Kohlenstoffatomen steht, darstellt, bilden können, repräsentiert; $Z''$ für Alkyl mit 1 bis 4 Kohlenstoffatomen, welches substituiert sein kann durch eine $SR_1''$— oder $OR_1''$-Gruppe, worin $R_1''$ wie oben definiert ist, oder $D''NR_2''R_3''$, worin $D''$ Alkylen mit 2 bis 4 Kohlenstoffatomen, welches durch eine OH-Gruppe substituiert sein kann, steht, und $R_2''$ und $R_3''$ wie oben definiert sind, bedeutet, steht; und pharmazeutisch akzeptable Salze davon.

10. Verbindungen nach Anspruch 2 der Strukturformel I'''

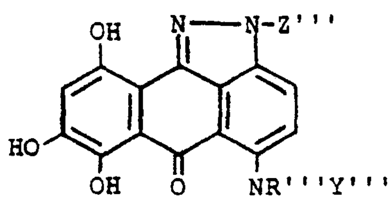

(I''')

worin $R'''$ für H oder Alkyl mit 1 bis 6 Kohlenstoffatomen steht; $Y'''$ H, Alkyl mit 1 bis 6 Kohlenstoffatomen, welches substituiert sein kann durch eine $OR_1'''$-Gruppe, worin $R_1'''$ steht H oder Alkyl mit 1 bis 4 Kohlenstoffatomen bedeutet, oder $A'''NR_2'''R_3'''$, worin $A'''$ Alkylen mit 2 bis 4 Kohlenstoffatomen steht, $R_2'''$ und $R_3'''$ gleich oder verschieden sein können und H, Alkyl mit 1 bis 6 Kohlenstoffatomen, welches substituiert sein kann durch ein OH, oder ein $NRa'''Ra'''$, worin $Ra'''$ gleich oder verschieden sein kann und H oder Alkyl mit 1 bis 3 Kohlenstoffatomen, welches durch ein OH substituiert sein kann, ist, repräsentieren oder $R_2'''$ und $R_3'''$, wenn sie zusammengenommen werden, Äthylen sein können oder

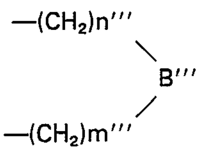

worin $n'''$ und $m'''$ gleich oder verschieden sein können und 1 oder 2 darstellen mit der Maßgabe, daß die Summe von $n'''$ und $m'''$ 3 oder 4 ist, und $B'''$ eine direkte Bindung, O, S oder $N—R_4'''$, worin $R_4'''$ für H oder Alkyl mit 1 bis 4 Kohlenstoffatomen steht, darstellt, bilden können, repräsentiert; $Z'''$ für Alkyl mit 1 bis 4 Kohlenstoffatomen, welches substituiert sein kann durch eine $SR_1'''$— oder $OR_1'''$-Gruppe, worin $R_1'''$ wie oben definiert ist, oder $D'''NR_2'''R_3'''$, worin $D'''$ Alkylen mit 2 bis 4 Kohlenstoffatomen, welches durch eine OH-Gruppe substituiert sein kann, bedeutet, und $R_2'''$ und $R_3'''$ wie oben definiert sind, steht; und die pharmazeutisch akzeptablen Salze davon.

11. Verbindungen nach Anspruch 4 der Strukturformel I''''

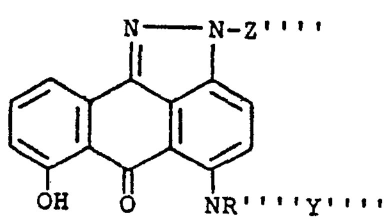

(I'''')

worin $R''''$ für H oder Alkyl mit 1 bis 6 Kohlenstoffatomen steht; $Y''''$ H, Alkyl mit 1 bis 6 Kohlenstoffatomen, welches substituiert sein kann durch eine $OR_1''''$-Gruppe, worin $R_1''''$ H oder Alkyl mit 1 bis 4 Kohlenstoffatomen darstellt, oder $AR''''NR_2''''R_3''''$, worin $A''''$ Alkylen mit 2 bis 4 Kohlenstoffatomen bedeutet, $R_2''''$ und $R_3''''$ gleich oder verschieden sein können und H, Alkyl mit 1 bis 6 Kohlenstoffatomen,

welches substituiert sein kann mit einem OH, oder einem $NRa''''Ra''''$, worin $Ra''''$ gleich oder verschieden sein kann und für H oder Alkyl mit 1 bis 3 Kohlenstoffatomen, welches mit einem OH substituiert sein kann, bedeutet, repräsentieren oder $R_2''''$ und $R_3''''$, wenn sie zusammengenommen werden, Äthylen sein können oder

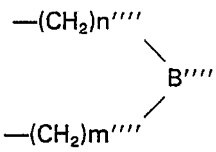

worin $n''''$ und $m''''$ gleich oder verschieden sein können und 1 oder 2 sind mit der Maßgabe, daß die Summe von $n''''$ und $m''''$ 3 oder 4 ist, und $B''''$ eine direkte Bindung, O, S oder $N—R_4''''$, worin $R_4''''$ für H oder Alkyl mit 1 bis 4 Kohlenstoffatom steht, repräsentiert, bilden können, darstellt; $Z''''$ für Alkyl mit 1 bis 4 Kohlenstoffatomen, welches substituiert sein kann durch eine $SR_1''''$— oder $OR_1''''$-Gruppe, worin $R_1''''$ die obige Bedeutung hat, oder $D''''NR_2''''R_3''''$, worin $D''''$ Alkylen mit 2 bis 4 Kohlenstoffatomen, welches durch eine OH-Gruppe substituiert sein kann, bedeutet, und $R_2''''$ und $R_3''''$ die obige Bedeutung haben, steht; und die pharmazeutisch akzeptablen Salze davon.

12. Verbindungen nach Anspruch 4 der Strukturformel $I^v$

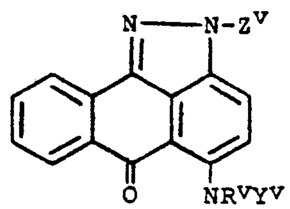

$(I^V)$

worin $R^v$ für H oder Alkyl mit 1 bis 6 Kohlenstoffatomen steht; $Y^v$ H, Alkyl mit 1 bis 4 Kohlenstoffatomen oder $A^vNR_2^vR_3^v$, worin $A^v$ Alkylen mit 2 bis 4 Kohlenstoffatomen darstellt, $R_2^v$ und $R_3^v$ gleich oder verschieden sein können und H oder Alkyl mit 1 bis 6 Kohlenstoffatomen, welches durch ein OH substituiert sein kann, darstellen oder $R_2^v$ und $R_3^v$, wenn sie zusammengenommen werden, Äthylen bedeuten können oder

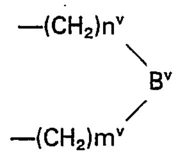

worin $n^v$ und $m^v$ gleich oder verschieden sein können und 1 oder 2 sind mit der Maßgabe, daß die Summe von $n^v$ und $m^v$ 3 oder 4 beträgt, und $B^v$ eine direkte Bindung, O, S oder $NR_4^v$, worin $R_4^v$ für oder Alkyl mit 1 bis 4 Kohlenstoffatomen steht, bedeutet, bilden können, repräsentiert; $Z^v$ Alkyl mit 1 bis 4 Kohlenstoffatomen, welches substituiert sein kann durch eine $SR_1^v$— oder $OR_1^v$-Gruppe, worin $R_1^v$ für H oder Alkyl mit 1 bis 4 Kohlenstoffatomen steht, oder $D^vNR_2^vR_3^v$, worin $D^v$ Alkylen mit 2 bis 4 Kohlenstoffatomen, welches durch eine OH-Gruppe substituiert sein kann, bedeutet, und $R_2^v$ und $R_3^v$ die obige Bedeutung haben, repräsentiert; und die pharmazeutisch akzeptablen Salze davon.

13. Verbindungen nach Anspruch 4 der Strukturformel $I^{vi}$

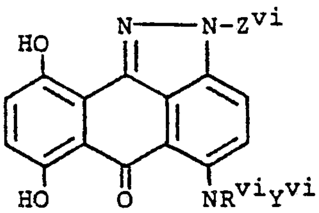

$(I^{vi})$

worin $R^{vi}$ für H oder Alkyl mit 1 bis 6 Kohlenstoffatomen steht; $Y^{vi}$ H, Alkyl mit 1 bis 4 Kohlenstoffatomen, welches substituiert sein kann durch eine $OR_1^{vi}$-Gruppe, worin $R_1^{vi}$ für H oder Alkyl mit 1 bis 4 Kohlenstoffatomen steht, oder $A^{vi}NR_2^{vi}R_3^{vi}$, worin $A^{vi}$ Alkylen mit 2 bis 4 Kohlenstoffatomen darstellt, $R_2^{vi}$ und $R_3^{vi}$ gleich oder verschieden sein können und Alkyl mit 1 bis 6 Kohlenstoffatomen, welches durch ein OH substituiert sein kann, darstellen oder $R_2^{vi}$ und $R_3^{vi}$, wenn sie zusammengenommen werden, Äthylen sein können oder

$$-(CH_2)n^{vi}$$
$$\diagdown$$
$$B^{vi},$$
$$\diagup$$
$$-(CH_2)m^{vi}$$

worin $n^{vi}$ und $m^{vi}$ gleich oder verschieden sein können und 1 oder 2 sind mit der Maßgabe, daß die Summe von $n^{vi}$ und $m^{vi}$ 3 oder 4 ist, und $B^{vi}$ eine direkte Bindung, O, S oder $NR_4{}^{vi}$, worin $R_4{}^{vi}$ für H oder Alkyl mit 1 bis 4 Kohlenstoffatomen steht, darstellt, bilden können, repräsentiert; $Z^{vi}$ $D^{vi}NR_2{}^{vi}R_3{}^{vi}$, worin $D^{vi}$ für Alkylen mit 2 bis 4 Kohlenstoffatomen steht, $R_2{}^{vi}$ und $R_3{}^{vi}$ die obige Bedeutung haben, repräsentiert; und die pharmazeutisch akzeptablen Salze davon.

14. Chemische Verbindungen nach Anspruch 2 mit den folgenden Bezeichnungen:

2-[2-[(2-Hydroxyäthyl)-amino]-äthyl]-5-[[2-[(2-hydroxyäthyl)-amino]-äthyl]-amino]-anthra[1,9-cd]-pyrazol-6(2H)-on;

2-[2-(Diäthylamino)-äthyl]-7,10-dihydroxy-5-[[2-[(2-hydroxyäthyl)-amino]-äthyl]-amino]-anthra[1,9-cd]-pyrazol-6(2H)-on;

2-[2-[[2-(Dimethylamino)-äthyl]-amino]-äthyl]-5-[[2-[(2-hydroxyäthyl)-amino]-äthyl]-amino]-7,10-dihydroxy-anthra[1,9-cd]pyrazol-6(2H)-on;

5-[(2-Aminoäthyl)-amino]-2-[2-(diäthylamino)-äthyl]-7,10-dihydroxyanthra[1,9-cd]pyrazol-6(2H)-on;

2-[2-(Dimethylamino)-äthyl]-7,10-dihydroxy-5-[[2-[(2-hydroxyäthyl)-amino]-äthyl]-amino]-anthra[1,9-cd]-pyrazol-6(2H)-on;

5-[[2-(Dimethylamino)-äthyl]-amino]-7,10-dihydroxy-2-(2-hydroxyäthyl)-anthra[1,9-cd]pyrazol-6(2H)-on;

5-[(2-Aminoäthyl)-amino]-7,10-dihydroxy-2-[2-[(2-hydroxyäthyl)-amino]-äthyl]-anthra[1,9-cd]pyrazol-6(2H)-on;

5-[(3-Aminopropyl)-amino]-7,10-dihydroxy-2-[2-[(2-hydroxyäthyl)-amino]-äthyl]-anthra[1,9-cd]pyrazol-6(2H)-on;

5-[[2-[[2-(Dimethylamino)-äthyl]-amino]-äthyl]-amino]-7,10-dihydroxy-2-[2-[(2-hydroxyäthyl)-amino]-äthyl]-anthra[1,9-cd]pyrazol-6(2H)-on;

5-[[2-[bis-(2-Hydroxyäthyl)-amino]-äthyl]-amino]-7,10-dihydroxy-2-[2-[(2-hydroxyäthyl)-amino]-äthyl]-anthra[1,9-cd]pyrazol-6(2H)-on;

7,10-Dihydroxy-2-[2-[(2-hydroxyäthyl)-amino]-äthyl]-5-[[2-(methylamino)-äthyl]-amino]-anthra[1,9-cd]pyrazol-6(2H)-on;

2-(2-Aminoäthyl)-7,10-dihydroxy-5-[[2-[(2-hydroxyäthyl)-amino]-äthyl]-amino]-anthra[1,9-cd]pyrazol-6(2H)-on;

2-(2,3-Dihydroxypropyl)-7,10-dihydroxy-5-[[2-[(2-hydroxyäthyl)-amino]-äthyl]-amino]-anthra[1,9-cd]-pyrazol-6(2H)-on;

7-Hydroxy-2-[2-[(2-hydroxyäthyl)-amino]-äthyl]-5-[[2-[(2-hydroxyäthyl)-amino]-äthyl]-amino]-anthra-[1,9-cd]pyrazol-6(2H)-on;

7-Hydroxy-2-[2-[(2-hydroxyäthyl)-amino]-äthyl)-5-[[2-(methylamino)-äthyl]-amino]-anthra[1,9-cd]-pyrazol-6(2H)-on;

7,8,10-Trihydroxy-2-[2-[(2-hydroxyäthyl)-amino]-äthyl]-5-[[2-[(2-hydroxyäthyl)-amino]-äthyl]-amino]-anthra[1,9-cd]pyrazol-6-(2H)-on;

7,8,10-Trihydroxy-2-[2-[(2-hydroxyäthyl)-amino)]-äthyl]-5-[[2-(methylamino)-äthyl]-amino]-anthra-[1,9-cd]pyrazol-6(2H)-on;

2-(3-Aminopropyl)-7,10-dihydroxy-5-[[2-[(2-hydroxyäthyl)-amino]-äthyl]-amino]-anthra-[1,9-cd]pyrazol-6(2H)-on;

2-(2-Aminoäthyl)-7,10-dihydroxy-5-[[2-(methylamino)-äthyl]-amino]-anthra-[1,9-cd]pyrazol-6(2H)-on;

5-[(2-Aminoäthyl)-amino]-2-[3-(dimethylamino)-propyl]-7,10-dihydroxyanthra[1,9-cd]pyrazol-(2H)-on;

7,8-Dihydroxy-2-[2-[(2-hydroxyäthyl)-amino]-äthyl]-5-[[2-[(2-hydroxyäthyl)-amino]-äthyl]-amino]-anthra-[1,9-cd]pyrazol-6(2H)-on;

und die pharmazeutisch akzeptablen Salze davon.

15. Chemische Verbindung der Strukturformel:

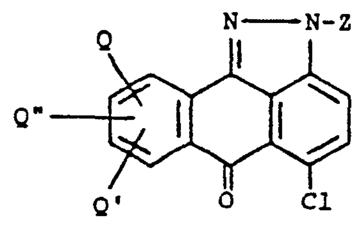

worin Q, Q′ und Q″ gleich oder verschieden sein können und für H, OH, Benzyloxy, p-Chlorbenzyloxy und p-Methoxybenzyloxy stehen; und die pharmazeutisch akzeptablen Salze davon;

Z wie im Anspruch 1 definiert ist; und die pharmazeutisch akzeptablen Salze davon; mit der Maßgabe, daß Z nicht H oder Alkyl mit 1 bis 6 Kohlenstoffatomen sein kann, wenn Q = Q' = O'' = H.

16. Chemische Verbindung der Strukturformel III

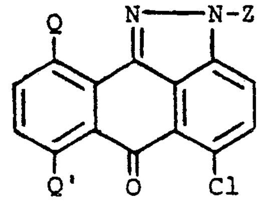

(III)

worin Q und Q' gleich oder verschieden sein können und H, OH, Benzyloxy, p-Chlorbenzyloxy oder p-Methoxybenzyloxy darstellen, und Z wie im Anspruch 1 definiert ist; und die pharmazeutisch akzeptablen Salze davon; mit der Maßgabe, daß Z nicht H oder Alkyl mit 1 bis 6 Kohlenstoffatomen sein kann, wenn Q = Q' = H.

17. Chemische Verbindungen nach Anspruch 16, worin Q und Q' H darstellen; und die pharmazeutisch akzeptablen Salze davon.

18. Chemische Verbindungen nach Anspruch 16, worin Q und Q' für Benzyloxy, p-Chlorbenzyloxy oder p-Methoxybenzyloxy stehen; und die pharmazeutisch akzeptablen Salze davon.

19. Chemische Verbindungen nach Anspruch 16, worin Q und Q' für OH stehen; und die pharmazeutisch akzeptablen Salze davon.

20. Chemische Verbindungen nach Anspruch 15 mit den folgenden Bezeichnungen:

5-Chlor-2-[2-[(2-hydroxyäthyl)-amino]-äthyl]-anthra-[1,9-cd]pyrazol-6(2H)-on;

5-Chlor-2-[2-(diäthylamino)-äthyl]-7,10-dihydroxy-anthra-[1,9-cd]pyrazol-6(2H)-on;

5-Chlor-7,10-dihydroxy-2-(2-hydroxyäthyl)-anthra-[1,9-cd]pyrazol-6(2H)-on;

5-Chlor-2-[2-(diäthylamino)-äthyl]-7,10-bis-(phenylmethoxy)-anthra-[1,9-cd]pyrazol-6(2H)-on;

5-Chlor-2-[2-[[(4-methylphenyl)-sulfonyl]-oxy]-äthyl]-7,10-bis-(phenylmethoxy)-anthra-[1,9-cd]pyrazol-6(2H)-on;

5-Chlor-7,10-dihydroxy-2-[2-[(2-hydroxyäthyl)-amino]-äthyl]-anthra-[1,9-cd]pyrazol-6(2H)-on;

5-Chlor-2-[2-[(2-hydroxyäthyl)-amino]-äthyl]-7,10-bis-(phenylmethoxy)-anthra[1,9-cd]pyrazol-6(2H)-on;

5-Chlor-2-[2-[[2-(dimethylamino)-äthyl]-amino]-äthyl]-7,10-dihydroxyanthra[1,9-cd]pyrazol-6(2H)-on;

5-Chlor-2-[2-(dimethylamino)-äthyl]-7,10-dihydroxy-anthra[1,9-cd]pyrazol-6(2H)-on;

5-Chlor-2-[3-(dimethylamino)-propyl]-7,10-dihydroxy-anthra[1,9-cd]pyrazol-6(2H)-on;

5-Chlor-2-(2-hydroxyäthyl)-7,10-bis-(phenylmethoxy)-anthra[1,9-cd]pyrazol-6(2H)-on;

5-Chlor-2-[3-[[4-methylphenyl)-sulfonyl]-oxy]-propyl]-7,10-bis-(phenylmethoxy)-anthra[1,9-cd]pyrazol-6(2H)-on;

5-Chlor-2-(3-hydroxypropyl)-7,10-bis-(phenylmethoxy)-anthra-[1,9-cd]pyrazol-6(2H)-on;

2-(2-Aminoäthyl)-5-chlor-7,10-dihydroxyanthra[1,9-cd]-pyrazol-6(2H)on;

2-(2-Aminoäthyl)-5-chlor-7,10-bis-(phenylmethoxy)-anthra-[1,9-cd]pyrazol-6(2H)-on;

5-Chlor-2-[(2,2-dimethyl-1,3-dioxolan-4-yl)-methyl]-7,10-bis-(phenylmethoxy)-anthra[1,9-cd]pyrazol-6(2H)-on;

5-Chlor-2-[2-[(2-hydroxyäthyl)-amino]-äthyl]-7-(phenylmethoxy)-anthra[1,9-cd]pyrazol-6(2H)-on;

5-Chlor-2-[2-[(2-hydroxyäthyl)-amino]-äthyl]-10-(phenylmethoxy)-anthra[1,9-cd]pyrazol-6(2H)-on;

5-Chlor-2-[2-[(2-hydroxyäthyl)-amino]-äthyl]-7,8,10-tris-(phenylmethoxy)-anthra[1,9-cd]pyrazol-6(2H)-on;

2-(3-Aminopropyl)-5-chlor-7,10-bis-(phenylmethoxy)-anthra[1,9-cd]pyrazol-6(2H)-on;

5-Chlor-2-[2-[(2-hydroxyäthyl)-amino]-äthyl]-7,8-bis-(phenylmethoxy)-anthra-[1,9-cd]pyrazol-6(2H)-on;

5-Chlor-2-[2-[[2-(dimethylamino)-äthyl]-amino]-äthyl]-7,10-bis-(phenylmethoxy)-anthra-[1,9-cd]pyrazol-6(2H)-on;

und die pharmazeutisch akzeptablen Salze davon.

21. Verbindung der Strukturformel IV

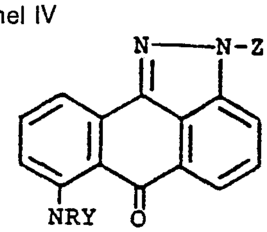

(IV)

worin R für H oder Alkyl mit 1 bis 6 Kohlenstoffatomen steht; Y H, Alkyl mit 1 bis 6 Kohlenstoffatomen, welches substituiert sein kann durch eine $OR_1$-Gruppe, worin $R_1$ für H oder Alkyl mit 1 bis 6 Kohlenstoffatomen steht, oder $ANR_2R_3$, worin A Alkylen mit 2 bis 8 Kohlenstoffatomen bedeutet, $R_2$ und $R_3$ gleich oder verschieden sein können und H, Alkyl mit 1 bis 6 Kohlenstoffatomen, welches substituiert sein

kann durch Oh oder ein NRaRa, worin Ra gleich oder verschieden sein kann und H oder Alkyl mit 1 bis 3 Kohlenstoffatomen, welches durch OH substituiert sein kann, bedeutet, repräsentieren oder $R_2$ und $R_3$, wenn sie zusammengenommen werden, Äthylen sein können oder

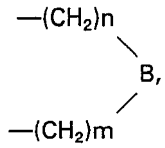

worin n und m gleich oder verschieden sein können und 1, 2 oder 3 sind mit der Maßgabe, daß die Summe von n und m eine ganze Zahl von 3 bis 6 ist, und B eine direkte Bindung, O, S oder N—$R_4$, worin $R_4$ für H oder Alkyl mit 1 bis 6 Kohlenstoffatomen steht, bedeutet, bilden können, repräsentiert; R und Y, wenn sie zusammengenommen werden Äthylen bedeuten können oder

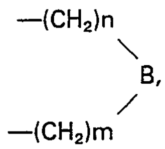

worin n, m und B wie oben definiert sind, bilden können; Z für H, Alkyl mit 1 bis 6 Kohlenstoffatomen, welches substituiert sein kann durch eine $N(R_1)_2$—, $SR_1$— oder $OR_1$-Gruppe, worin $R_1$ gleich oder verschieden sein kann und die obige Bedeutung hat, oder $DNR_2R_3$, worin D Alkylen mit 2 bis 8 Kohlenstoffatomen, welches durch eine OH-Gruppe substituiert sein kann, bedeutet, und $R_2$ und $R_3$ die obige Bedeutung haben, steht; und die pharmazeutisch akzeptablen Salze davon; mit den folgenden Maßgaben:

1) R und Y vervollständigen nicht einen Piperidinring, wenn sie zusammengenommen werden, wenn Z für H steht,

2) R und Y vervollständigen nicht einen Piperidinring oder einen Morpholinring, wenn sie zusammengenommen werden, wenn Z für $CH_3$ steht, und

3) wenn sowohl R als auch Y H bedeuten, repräsentiert Z weder H noch Alkyl mit 1 bis 6 Kohlenstoffatomen.

22. Chemische Verbindungen nach Anspruch 21 mit den folgenden Bezeichnungen:

2-[2-(Diäthylamino)-äthyl]-7-[[2-[(2-hydroxyäthyl)-amino]-äthyl]-amino]-anthra[1,9-cd]pyrazol-6(2H)-on;

2-[2-(Diäthylamino)-äthyl]-7-[[2-(diäthylamino)-äthyl]-amino-anthra-[1,9-cd]pyrazol-6(2H)-on;

2-[2-[(2-Hydroxyäthyl)-amino]-äthyl]-7-[[2-[(2-hydroxyäthyl)-amino]-äthyl]-amino]-anthra]-[1,9-cd]pyrazol-6(2H)-on und die pharmazeutisch akzeptablen Salze davon.

23. Verbindung der Strukturformel VII

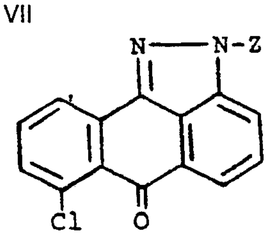

(VII)

worin Z wie im Anspruch 1 definiert ist, mit der Maßgabe, daß es nicht H oder Alkyl mit 1 bis 6 Kohlenstoffatomen bedeutet.

24. Chemische Verbindungen nach Anspruch 23 mit den folgenden Bezeichnungen:

7-Chlor-2-[2-(diäthylamino)-äthyl]-anthra-[1,9-cd]pyrazol-6(2H)-on;

7-Chlor-2-[2-[(2-hydroxyäthyl)-amino]-äthyl]-anthra[1,9-cd]pyrazol-6(2H)-on; und die pharmazeutisch akzeptablen Salze davon.

25. Verbindung 2-[(Hydrazinoäthyl)-amino]-äthanol und die Säureadditionssalze davon.

26. 5,8-Dichlor-1,4,9,10-anthracentetron.

27. Verfahren zur Herstellung einer Verbindung nach Anspruch 1 der Strukturformel

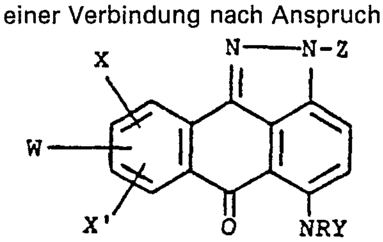

welches die Reaktion einer Verbindung der Strukturformel

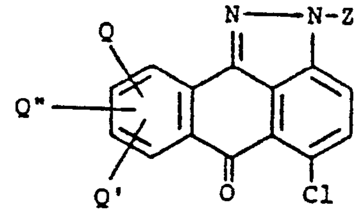

mit einem Amin der Formel HNRY, worin W, X, X', Q, Q', Q'', Y, Z und R wie im Anspruch 1 definiert sind, und nötigenfalls Entfernen irgendwelcher Benzylgruppen durch katalytische Hydrierung oder durch Behandlung mit Bohrtribromid oder -trichchlorid, umfaßt.

28. Verfahren zur Herstellung einer Verbindung nach Anspruch 2 der Strukturformel I

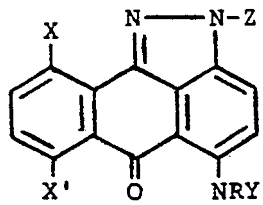

(I)

welches die Reaktion einer Verbindung der Strukturformel II

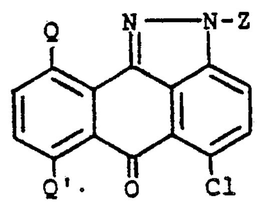

(II)

mit einem Amin der Formel HNRY, worin X, X', Q, Q', Y, Z und R wie im Anspruch 2 definiert sind, umfaßt.

29. Verfahren nach Anspruch 28, worin X und X' für OH stehen.

30. Verfahren nach Anspruch 28, worin X und X' für H stehen.

31. Verfahren zur Herstellung einer Verbindung nach Anspruch 16 der Strukturformel III

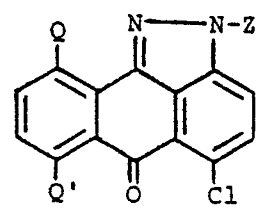

(III)

welches die Umsetzung einer Verbindung der Strukturformel V

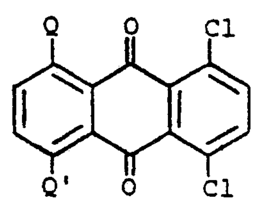

(V)

mit einem Hydrazin der Formel $H_2N—NHZ$, worin Q, Q' und Z wie im Anspruch 15 definiert sind, umfaßt.

32. Verfahren nach Anspruch 31, worin Q und Q' Benzyloxy, p-Chlorbenzyloxy oder p-Methoxybenzyloxy bedeuten.

33. Verfahren nach Anspruch 31, worin Q und Q' für OH stehen.

34. Verfahren nach Anspruch 31, worin Q und Q' für H stehen.

35. Verfahren zur Herstellung einer Verbindung nach Anspruch 21 der Strukturformel IV

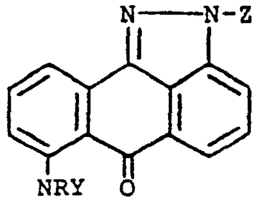

(IV)

welches die Umsetzung einer Verbindung der Strukturformel VII

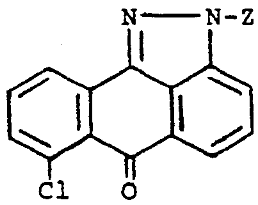

(VII)

mit einem Amin der Formel HNRY, worin Y, Z und R wie im Anspruch 21 definiert sind, umfaßt.

36. Pharmazeutische Zusammensetzung, welche eine in irgendeinem der Ansprüche 1 bis 26 definierte Verbindung oder ein pharmazeutisch akzeptables Salz davon in Kombination mit einem pharmazeutisch akzeptablen Träger umfaßt.

37. Verbindung der Strukturformel

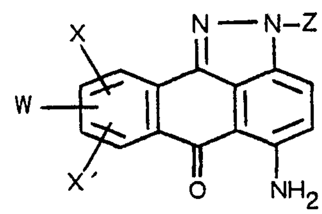

worin Z für Wasserstoff oder Alkyl mit 1 bis 6 Kohlenstoffatomen steht, und X, X' und W wie im Anspruch 1 definiert sind, oder ein pharmazeutisch akzeptables Salz davon zur Verwendung bei der Behandlung von mikrobiellen Infektionen bei Säugetieren.

38. Verwendung einer Verbindung nach Anspruch 37 zur Herstellung eines Arzneimittels zur Behandlung von Leukämie bei Säugetieren.

39. Verwendung einer Verbindung nach Anspruch 37 zur Herstellung eines Arzneimittels zur Behandlung von festen Tumoren bei Säugetieren.

40. Verbindung der Strukturformel

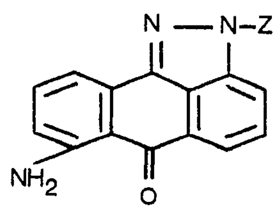

worin Z für Wasserstoff oder ein Alkyl mit 1 bis 6 Kohlenstoffatomen steht, oder ein pharmazeutisch akzeptables Salz davon zur Verwendung bei der Behandlung von mikrobiellen Infektionen bei Säugetieren.

41. Verwendung einer Verbindung nach Anspruch 40 für die Herstellung eines Arzneimittels zur Behandlung von Leukämie bei Säugetieren.

42. Verwendung einer Verbindung nach Anspruch 40 für die Herstellung eines Arzneimittels zur Behandlung von festen Tumoren bei Säugetieren.

**Patentansprüche für den Vertragsstaat: AT**

1. Verfahren zur Herstellung einer Verbindung der Strukturformel

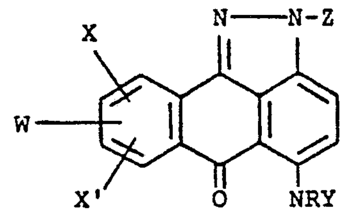

worin X, X′ und W gleich oder verschieden sein können und H, OH, Alkoxy mit 1 bis 4 Kohlenstoffatomen oder Chlor bedeuten; R für H oder Alkyl mit 1 bis 6 Kohlenstoffatomen steht; Y für H, Alkyl mit 1 bis 6 Kohlenstoffatomen, welches substituiert sein kann durch eine $OR_1$-Gruppe, worin $R_1$ H oder Alkyl mit 1 bis 6 Kohlenstoffatomen darstellt, oder $ANR_2R_3$, worin A gerades oder verzweigtes Alkylen mit 2 bis 8 Kohlenstoffatomen darstellt, $R_2$ und $R_3$ gleich oder verschieden sein können und H, Alkyl mit 1 bis 6 Kohlenstoffatomen, welches substituiert sein kann durch OH oder ein NRaRa, worin Ra gleich oder verschieden sein kann und für H oder Alkyl mit 1 bis 3 Kohlenstoffatomen, welches durch OH substituiert sein kann, steht, oder $NR_bR_b$, worin $R_b$ gleich oder verschieden ist und H oder Alkyl mit 1 bis 3 Kohlenstoffatomen bedeutet, darstellen, oder $R_2$ und $R_3$, wenn sie zusammengenommen werden, Äthylen bedeuten können oder

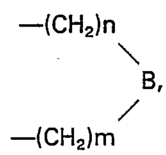

worin n und m gleich oder verschieden sein können und 1, 2 oder 3 bedeuten mit der Maßgabe, daß die Summe von n und m eine ganze Zahl von 3 bis 6 darstellt, und B eine direkte Bindung, O, S oder N—$R_4$, worin $R_4$ für H oder Alkyl mit 1 bis 6 Kohlenstoffatomen steht, darstellt, bilden können, steht; R und Y, wenn sie zusammengenommen werden, Äthylen sein können oder

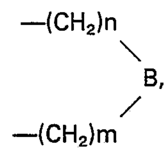

worin n, m und B wie oben definiert sind, bilden können; Z für H, Alkyl mit 1 bis 6 Kohlenstoffatomen, welches substituiert sein kann durch eine $N(R_1)_2$—, $SR_1$— oder $OR_1$-Gruppe, worin $R_1$ gleich oder verschieden ist und wie oben definiert ist, oder $DNR_2R_3$, worin D gerades oder verzweigtes Alkylen mit 2 bis 8 Kohlenstoffatomen, welches durch eine OH-Gruppe substituiert sein kann, darstellt, und $R_2$ und $R_3$ wie oben definiert sind, steht; und der pharmazeutisch akzeptablen Salze davon; mit den folgenden Maßgaben:

1) wenn X, X′ und W für H stehen und Z für H steht, vervollständigen R und Y nicht einen Piperidinring, wenn sie zusammengenommen werden,

2) wenn X, X′ und W für H stehen und Z $CH_3$ bedeutet, vervollständigen R und Y nicht einen Piperidinring oder einen Morpholinring, wenn sie zusammengenommen werden, und

3) wenn sowohl R als auch Y für H stehen, bedeutet Z weder H noch Alkyl mit 1 bis 6 Kohlenstoffatomen, und welches Verfahren die Umsetzung einer Verbindung der folgenden Strukturformel

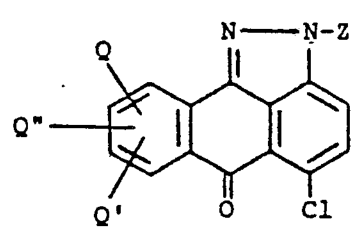

mit einem Amin der Formel HNRY, worin W, X, X′, Q, Q′, Q″, Y, Z und R wie oben definiert sind, und nötigenfalls Entfernen irgendwelcher Benzylgruppen mittels katalytischer Hydrierung oder mittels Behandlung mit Bortribromid oder -trichlorid umfaßt.

2. Verfahren zur Herstellung einer Verbindung der Strukturformel

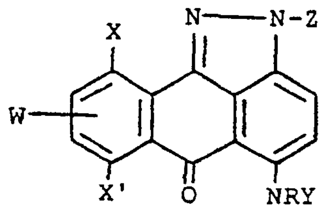

worin X, X' und W gleich oder verschieden sein können und H oder OH, Alkoxy mit 1 bis 4 Kohlenstoffatomen oder Chlor bedeuten; R für H oder Alkyl mit 1 bis 6 Kohlenstoffatomen steht; Y H, Alkyl mit 1 bis 6 Kohlenstoffatomen, welches substituiert sein kann durch eine $OR_1$-Gruppe, worin $R_1$ für H oder Alkyl mit 1 bis 6 Kohlenstoffatomen steht, oder $ANR_2R_3$, worin A gerades oder verzweigtes Alkylen mit 2 bis 8 Kohlenstoffatomen darstellt, $R_2$ und $R_3$ gleich oder verschieden sein können und H, Alkyl mit 1 bis 6 Kohlenstoffatomen, welches substituiert sein kann durch OH oder ein NRaRa, worin Ra gleich oder verschieden sein kann, H oder Alkyl mit 1 bis 3 Kohlenstoffatomen, welches durch OH substituiert sein kann, darstellt, oder $NR_bR_b$, worin $R_b$ gleich oder verschieden ist und H oder Alkyl mit 1 bis 3 Kohlenstoffatomen darstellt, bedeuten, oder $R_2$ und $R_3$, wenn sie zusammengenommen werden, Äthylen sein können oder

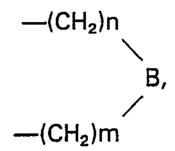

worin n und m gleich oder verschieden sein können und 1, 2 oder 3 bedeuten mit der Maßgabe, daß die Summe von n und m eine ganze Zahl von 3 bis 6 ist, und B eine direkte Bindung, O, S oder N—$R_4$, worin $R_4$ für H oder Alkyl mit 1 bis 6 Kohlenstoffatomen steht, ist, bilden können, bedeutet; R und Y, wenn sie zusammengenommen werden, Äthylen darstellen können oder

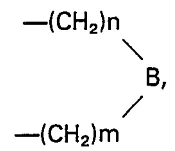

worin n, m und B wie oben definiert sind, bilden können; Z für H, Alkyl mit 1 bis 6 Kohlenstoffatomen, welches substituiert sein kann durch eine $N(R_1)_2$—, $SR_1$— oder $OR_1$-Gruppe, worin $R_1$ gleich oder verschieden sein kann und wie oben definiert ist, oder $DNR_2R_3$, worin D gerades oder verzweigtes Alkylen mit 2 bis 8 Kohlenstoffatomen, welches durch eine OH-Gruppe substituiert sein kann, darstellt, und $R_2$ und $R_3$ wie oben definiert sind, steht; und pharmazeutisch akzeptabler Salze davon; mit den folgenden Maßgaben:

1) wenn X und X' für H stehen und Z H bedeutet, vervollständigen R und Y nicht einen Piperidinring, wenn sie zusammengenommen werden,

2) wenn X und X' für H stehen und Z $CH_3$ darstellt, vervollständigen R und Y nicht einen Piperidinring oder einen Morpholinring, wenn sie zusacen Morpholinring, wenn sie zusammengenommen werden, und

3) wenn sowohl R als auch Y H darstellen, bedeutet Z weder H noch Alkyl mit 1 bis 6 Kohlenstoffatomen, welches Verfahren die Umsetzung einer Verbindung der Strukturformel II

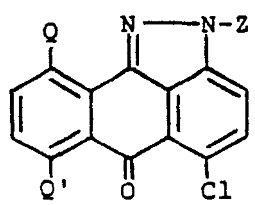

(II)

mit einem Amin der Formel HNRY, worin X, X', Q, Q', Y, Z und R oben definiert sind, umfaßt.

3. Verfahren nach Anspruch 2, worin X und X' für OH stehen.

4. Verfahren nach Anspruch 2, worin X und X' H bedeuten.

5. Verfahren zur Herstellung einer chemischen Verbindung der Strukturformel III

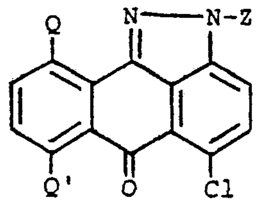

(III)

worin Q und Q′ gleich oder verschieden sein können und H, OH, Benzyloxy, p-Chlorbenzyloxy oder p-Methoxybenzyloxy darstellen, und Z wie im Anspruch 1 definiert ist; und der pharmazeutisch akzeptablen Salze davon; mit der Maßgabe, daß, wenn Q = Q′ = H, Z nicht H oder Alkyl mit 1 bis 6 Kohlenstoffatomen bedeuten kann, welches die Umsetzung einer Verbindung der Strukturformel V

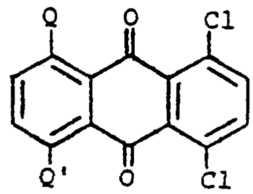

(V)

mit einem Hydrazin der Formel $H_2N—NHZ$, worin Q und Q′ gleich oder verschieden sein können, und H, OH, Benzyloxy, p-Chlorbenzyloxy und p-Methoxybenzyloxy darstellen, und der pharmazeutisch akzeptablen Salze davon;

Z im Anspruch 1 definiert ist; und der pharmazeutisch akzeptablen Salze davon; mit der Maßgabe, daß, wenn Q = Q′ = H, Z nicht oder Alkyl mit 1 bis 6 Kohlenstoffatomen sein kann, umfaßt.

6. Verfahren zur Herstellung einer Verbindung der Strukturformel IV

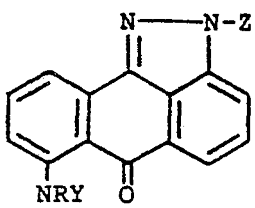

(IV)

worin R für H oder Alkyl mit 1 bis 6 Kohlenstoffatomen steht; Y H, Alkyl mit 1 bis 6 Kohlenstoffatomen, welches substituiert sein kann durch eine $OR_1$-Gruppe, worin $R_1$ H oder Alkyl mit 1 bis 6 Kohlenstoffatomen bedeutet, oder $ANR_2R_3$, worin A für Alkylen mit 2 bis 8 Kohlenstoffatomen steht, $R_2$ und $R_3$ gleich oder verschieden sein können und H, Alkyl mit 1 bis 6 Kohlenstoffatomen, welches substituiert sein kann durch OH oder ein NRaRa, worin Ra gleich oder verschieden sein kann und H oder Alkyl mit 1 bis 3 Kohlenstoffatomen, welches durch OH substituiert sein kann, bedeutet, darstellen, oder $R_2$ und $R_3$, wenn sie zusammengenommen werden, Äthylen bedeuten können oder

$—(CH_2)n$
\
B,
/
$—(CH_2)m$

worin n und m gleich oder verschieden sein können und 1, 2 oder 3 repräsentieren mit der Maßgabe, daß die Summe von n und m eine ganze Zahl von 3 bis 6 ist, und B eine direkte Bindung, O, S oder $N—R_4$, worin $R_4$ H oder Alkyl mit 1 bis 6 Kohlenstoffatomen darstellt, ist, bilden können, bedeutet; R und Y, wenn sie zusammengenommen werden, Äthylen sein können oder

$—(CH_2)n$
\
B,
/
$—(CH_2)m$

worin n, m und B wie oben definiert sind, bilden können; Z für H, Alkyl mit 1 bis 6 Kohlenstoffatomen, welches substituiert sein kann durch eine $N(R_1)_2$—, $SR_1$— oder $OR_1$-Gruppe, worin $R_1$ gleich oder verschieden sein kann und wie oben definiert ist, oder $DNR_2R_3$, worin D Alkylen mit 2 bis 8 Kohlenstoffatomen, welches durch eine OH-Gruppe substituiert sein kann, bedeutet, und $R_2$ und $R_3$ wie oben definiert sind, steht; und der pharmazeutisch akzeptablen Salze davon; mit den folgenden Maßgaben:

1) wenn Z für H steht, vervollständigen R und Y nicht einen Piperidinring, wenn sie zusammengenommen werden,

2) wenn Z für $CH_3$ steht, vervollständigen R und Y nicht einen Piperidinring oder einen Morpholinring, wenn sie zusammengenommen werden, und

3) wenn sowohl R als auch Y für H stehen, bedeutet Z weder H noch Alkyl mit 1 bis 6 Kohlenstoffatomen,

welches die Umsetzung einer Verbindung der Strukturformel VII

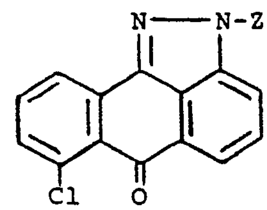

(VII)

mit einem Amin der Formel HNRY, worin Y, Z und R wie oben definiert sind, umfaßt.

7. Verfahren zur Herstellung einer pharmazeutischen Zusammensetzung, welches die Kombination einer gemäß eines Verfahrens nach irgendeinem der vorhergehenden Ansprüche hergestellten Verbindung oder eines pharmazeutisch akzeptablen Salzes derselben mit einem pharmazeutisch akzeptablen Träger umfaßt.

8. Verfahren zur Herstellung eines Arzneimittels, welches die Kombination einer Verbindung mit einer der folgenden Formeln

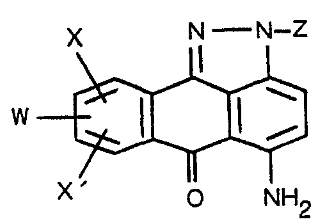

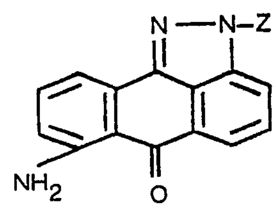

worin X, X' und W wie in Anspruch 1 definiert sind, und Z für Wasserstoff oder ein Alkyl mit 1 bis 6 Kohlenstoffatomen steht, oder eines pharmazeutisch akzeptablen Salzes derselben mit einem pharmazeutisch akzeptablen Träger umfaßt.

**Revendications pour les Etats Contractants: BE CH DE FR GB IT LI LU NL SE**

1. Un composé présentant la formule structurelle:

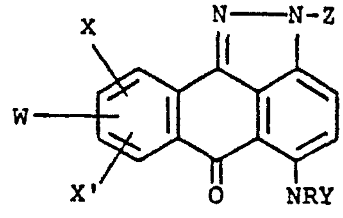

dans laquelle: X, X' et W peuvent être identiques ou différents et consistent en hydrogène, hydroxy, alkoxy comprenant de 1 à 4 atomes de carbone et chlore; R est un atome d'hydrogène ou un radical alkyle comprenant de 1 à 6 atomes de carbone; Y est un atome d'hydrogène ou un radical alkyle, comprenant de 1 à 6 atomes de carbone, susceptible d'être substitué par un groupe $OR_1$ dans lequel $R_1$ est un atome d'hydrogène ou un radical alkyle comprenant de 1 à 6 atomes de carbone, ou $ANR_2R_3$ dans lequel A est un alkylène linéaire ou ramifié comprenant de 2 à 8 atomes de carbone, $R_2$ et $R_3$ pouvant être identiques ou différents et consister en hydrogène, alkyle comprenant de 1 à 6 atomes de carbone susceptible d'être

substitué par un groupe OH ou NRaRa dans lequel les groupes Ra peuvent être identiques ou différents et consistent en hydrogène ou alkyle comprenant de 1 à 3 atomes de carbone, susceptible d'être substitué par OH ou NRbRb, les groupes Rb identiques ou différents consistant en H ou alkyle comprenant de 1 à 3 atomes de carbone, ou $R_2$ et $R_3$ forment ensemble un groupe éthylène ou peuvent former un groupe:

—$(CH_2)_n$
\
B
/
—$(CH_2)_m$

dans lequel n et m peuvent être identiques ou différents et sont égaux à 1, 2 ou 3, étant entendu que la somme de n et m est nombre entier comprise entre 3 et 6 et que B est une liaison directe, un atome d'oxygène ou de soufre ou un groupe N—$R_4$ dans lequel $R_4$ est formé de'atome d'hydrogène ou d'un radical alkyle comprenant de 1 à 6 atomes de carbone, R et Y pris ensemble pouvant être un radical éthylène ou pouvant former un groupe:

—$(CH_2)_n$
\
B
/
—$(CH_2)_m$

dans lequel n et m et B sont tels que définis ci-dessus; Z est un atome d'hydrogène, un radical alkyle comprenant de 1 à 6 atomes de carbone, susceptible d'être substitué par un groupe $N(R_1)_2$, $SR_1$ ou $OR_1$ dans lesquels $R_1$ est identique ou différent et est tel que défini ci-dessus, ou $DNR_2R_3$ avec D consistant en un radical alkylène linéaire ou ramifié comprenant de 2 à 8 atomes de carbone, qui peut être substitué par un groupe OH et, $R_2$ et $R_3$ étant tels que définis ci-dessus; ainsi que les sels pharmaceutiquement acceptables en dérivant, à la condition cependant, que: 1) lorsque X, X' et W sont H et Z est H, R et Y lorsqu'ils sont pris ensemble ne forment pas un cycle pipéridine; 2) lorsque X, X' et W sont H et Z est $CH_3$, R et Y pris ensemble ne forment pas un noyau pipéridine ou un noyau morpholine; et 3) lorsque R et Y tous deux sont H, Z ne soit ni H, ni un radical alkyle comprenant de 1 à 6 atomes de carbone.

2. Un composé présentant la formule structurelle:

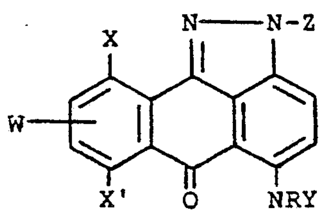

dans laquelle X, X' et W peuvent être identiques ou différents et consistent en H ou OH, un radical alkoxy comprenant de 1 à 4 atomes de carbone ou chlore; R est un atome d'hydrogène ou un radical alkyle comprenant de 1 à 6 atomes dè carbone; Y est un atome d'hydrogène, un radical alkyle comprenant de 1 à 6 atomes de carbone, susceptible d'être substitué par un groupe $OR_1$ dans lequel $R_1$ est H ou alkyle comprenant de 1 à 6 atomes de carbone, ou $ANR_2R_3$ dans lequel A est un radical alkylène linéaire ou ramifié comprenant de 2 à 8 atomes de carbone, $R_2$ et $R_3$ pouvant être identiques ou différents et consister en H, alkyle comprenant de 1 à 6 atomes de carbone susceptible d'être substitué par un groupe OH ou NRaRa dans lequel les groupes Ra peuvent être identiques ou différents et consister en H ou un radical alkyle comprenant de 1 à 3 atomes de carbone, susceptible d'être substitués par un groupe OH, ou NRbRb dans lequel les groupes Rb sont identiques ou différents et consistent en atome d'hydrogène ou un radical alkyle comprenant de 1 à 3 atomes de carbone, ou $R_2$ et $R_3$ pris ensemble peuvent former un radical éthylène ou un groupe:

—$(CH_2)_n$
\
B
/
—$(CH_2)_m$

dans lequel n et m, qui peuvent être identiques ou différents, sont égaux à 1, 2 ou 3, étant entendu que la somme de n et m forme un nombre entier compris entre 3 et 6 et que B est une liaison directe formée un atome d'oxygène, de soufre ou un groupe N—$R_4$ dans lequel $R_4$ est un atome d'hydrogène ou un radical alkyle comprenant de 1 à 6 atomes de carbone; R et Y lorsqu'ils sont pris ensemble pouvant être un radical éthylène ou pouvant former un groupe:

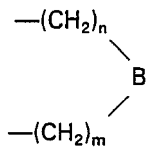

dans lequel n, m et B sont tels que définis ci-dessus; Z est un atome d'hydrogène, un radical alkyle comprenant de 1 à 6 atomes de carbone, susceptible d'être substitué par un groupe $N(R_1)_2$, $SR_1$ ou $OR_1$ dans lesquels $R_1$ est identique ou différent et est tel que défini ci-dessus, ou $DNR_2R_3$ dans lequel D est un radical alkylène linéaire ou ramifié comprenant de 2 à 8 atomes de carbone susceptible d'être substitué par un groupe OH et, $R_2$ et $R_3$ sont tels que définis ci-dessus, ainsi que les sels pharmaceutiquement acceptables en dérivant, aux conditions suivantes cependant, que: 1) lorsque X, X' et W sont H et Z est H, R et Y pris ensemble ne forment pas un groupe pipéridine; 2) lorsque X, X' et W sont H et Z est $CH_3$, R et Y lorsqu'ils sont pris ensemble ne forment pas un noyau pipéridine ou un noyau morphline; et 3) lorsque R et Y tous deux sont H, Z ne soit ni H, ni un radical alkyle comprenant de 1 à 6 atomes de carbone.

3. Un composé présentant la formule structurelle:

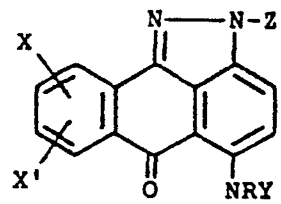

dans laquelle: X et X' peuvent être identiques ou différents et consistent en H, OH, alkoxy comprenant de 1 à 4 atomes de carbone ou chlore; R est un atome d'hydrogène ou un radical alkyle comprenant de 1 à 6 atomes de carbone; Y est un atome d'hydrogène ou un radical alkyle comprenant de 1 à 6 atomes de carbone, qui peut être substitué par un groupe $OR_1$ dans lequel $R_1$ est un atome d'hydrogène ou un groupe alkyle comprenant de 1 à 6 atomes de carbone, ou $ANR_2R_3$ dans lequel A est un radical alkylène comprenant 2 à 11 atomes de carbone, $R_2$ et $R_3$ pouvant être identiques ou différents et consister en un atome d'hydrogène ou un radical alkyle comprenant de 1 à 6 atomes de carbone susceptible d'être substitué par un groupe OH ou un groupe NRaRa dans lequel Ra sont des atomes d'hydrogène ou un radical alkyle comprenant de 1 à 3 atomes de carbone susceptible d'être substitué par un groupe OH, ou NRbRb dans lequel Rb sont identiques ou différents et consistent en un atome d'hydrogène ou un radical alkyle comprenant de 1 à 3 atomes de carbone, ou $R_2$ et $R_3$ pris ensemble peuvent former un radical éthylène ou un groupe:

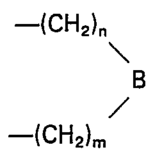

dans lequel n et m sont identiques ou différents et sont égaux à 1, 2 ou 3, étant entendu que la somme de n et m est égal à un nombre entier compris entre 3 et 6 et que B soit une liaison directe, un atome d'oxygène, de soufre ou un groupe N—$R_4$ dans lequel $R_4$ est un atome d'hydrogène ou un radical alkyle comprenant de 1 à 6 atomes de carbone, R et Y lorsqu'ils sont pris ensemble pouvant former un radical éthylène ou pouvant former un groupe:

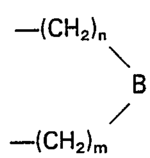

dans lequel n, m et B sont tels que définis ci-dessus; Z est un atome d'hydrogène, un radical alkyle comprenant de 1 à 6 atomes de carbone qui peut être substitué par un groupe $N(R_1)_2$, $SR_1$ ou $OR_1$ dans lesquels $R_1$ est tel que défini ci-dessus ou $DNR_2R_3$ dans lequel D est un radical alkylène comprenant de 2 à 11 atomes de carbone susceptible d'être substitué par un groupe OH et, $R_2$ et $R_3$ sont tels que définis ci-dessus, ainsi que les sels pharmaceutiquement acceptables en dérivant, aux conditions suivantes cependant, que: 1) lorsque X est H et Z est H, R et Y pris ensemble ne forment pas un noyau pipéridine; 2) lorsque X est H et Z est $CH_3$, R et Y pris ensemble ne forment pas un noyau pipéridine ou un noyau morpholine; et 3) lorsque R et Y sont tous deux égaux à H, Z ne soit ni un atome d'hydrogène, ni un radical alkyle comprenant de 1 à 6 atomes de carbone.

4. Un composé présentant la formule structurelle I:

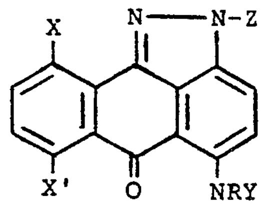

(I)

dans laquelle: X et X′ peuvent être identiques ou différents et consistent en un atome d'hydrogène ou un groupe OH; R est un atome d'hydrogène ou un radical alkyle comprenant de 1 à 6 atomes de carbone; Y est un atome d'hydrogène ou un radical alkyle comprenant de 1 à 6 atomes de carbone, qui peut être substitué par un groupe $OR_1$ dans lequel $R_1$ est un atome d'hydrogène ou un radical alkyle comprenant de 1 à 6 atomes de carbone ou $ANR_2R_3$ dans lequel A est un radical alkylène linéaire ou ramifié comprenant de 2 à 8 atomes de carbone, $R_2$ et $R_3$ pouvant être identiques ou différents et consister en un atome d'hydrogène, un radical alkyle comprenant de 1 à 6 atomes de carbone susceptible d'être substitué par un groupe OH ou NRaRa dans lequel Ra sont identiques ou différents et consistent en un atome d'hydrogène ou un radical alkyle comprenant de 1 à 3 atomes de carbone qui peut être substitué par OH, ou NRbRb dans lequel Rb sont identiques ou différents et consistent en un atome d'hydrogène ou un radical alkyle comprenant de 1 à 3 atomes de carbone, ou $R_2$ et $R_3$ pris ensemble peuvent former un radical éthylène ou un groupe:

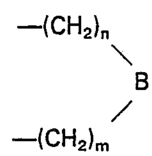

dans lequel n et m sont identiques ou différents et sont égaux à 1, 2 ou 3, étant entendu que la somme de n et m forme un nombre entier comprise entre 3 et 6 et que B soit une liaison directe, un atome d'oxygène, de soufre ou un groupe N—$R_4$ dans lequel $R_4$ est un atome d'hydrogène ou un radical alkyle comprenant de 1 à 6 atomes de carbone; R et Y lorsqu'ils sont pris ensemble peuvent former un radical éthylène ou un groupe

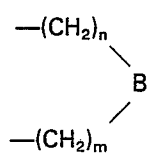

dans lequel n, m et B sont tels que définis ci-dessus; Z est un atome d'hydrogène, un radical alkyle comprenant de 1 à 6 atomes de carbone qui peut être substitué par un groupe $N(R_1)_2$, $SR_1$ ou $OR_1$ dans lesquels $R_1$ est identique ou différent et est tel que défini ci-dessus ou $DNR_2R_3$ dans lequel D est un radical alkylène linéaire ou ramifié comprenant de 2 à 8 atomes de carbone qui peut être substitué par un groupe OH et, $R_2$ et $R_3$ sont tels que définis ci-dessus, ainsi que les sels pharmaceutiquement acceptables en dérivant, aux conditions suivantes cependant, que: 1) lorsque X et X′ sont H et Z est H, R et Y pris ensemble ne forment par un noya pipéridine; 2) lorsque X et X′ sont H et Z est $CH_3$, R et Y pris ensemble ne forment pas un noyau pipéridine ou un noyau morpholine; et 3) lorsque R et Y sont tous deux égaux à H, Z ne soit ni un atome d'hydrogène, ni un radical alkyle comprenant de 1 à 6 atomes de carbone.

5. Un composé chimique tel que défini dans la revendication 4, dans laquelle X et X′ sont OH; ainsi que les sels pharmaceutiquement acceptables en dérivant.

6. Un composé chimique tel que défini dans la revendication 4, dans laquelle X et X′ sont H; ainsi que les sels pharmaceutiquement acceptables en dérivant.

7. Un composé chimique tel que défini dans la revendication 4, dans laquelle A et D sont identiques ou différents et consistent en un groupe éthylène ou propylène, ainsi que les sels pharmaceutiquement acceptables en dérivant.

8. Un composé tel que défini dans la revendication 4, présentant la formule structurelle I′

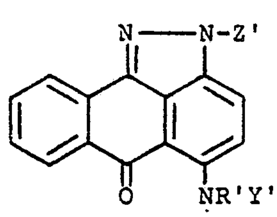

(I′)

dans laquelle R′ est un atome d'hydrogène ou un radical alkyle comprenant de 1 à 6 atomes de carbone; Y′ est $CH_2CH_2NHCH_2CH_2OH$ lorsque Z′ est un radical alkyle comprenant de 1 à 4 atomes de carbone qui peut être substitué par un groupe $SR_1'$ ou $OR_1'$ dans lesquels $R_1'$ est un atome d'hydrogène ou un radical alkyle comprenant de 1 à 4 atomes de carbone, ou $D'NR_2'R_3'$ dans lequel D′ est un radical alkylène linéaire ou ramifié comprenant de 2 à 4 atomes de carbone qui peut être substitué par un groupe OH et, $R_2'$ et $R_3'$ peuvent être identiques ou différents et consister en un atome d'hydrogène ou un radical alkyle comprenant de 1 à 6 atomes de carbone qui peut être substitue par un radical OH, ou $R_2'$ et $R_3'$ peuvent former ensemble un radical éthylène ou peuvent former le groupe:

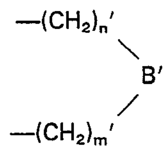

dans lequel n′ et m′ peuvent être identiques ou différents et sont égaux à 1 ou 2, étant entendu que la somme de n′ et m′ est égal à 3 ou 4, et B′ est une liaison directe, un atome d'oxygène, de soufre ou un groupe N—$R_4'$ dans lequel $R_4'$ est un atome d'hydrogène ou un radical alkyle comprenant de 1 à 4 atomes de carbone; ou Z′ est $CH_2CH_2NHCH_2CH_2OH$ lorsque Y′ est H, un radical alkyle comprenant de 1 à 6 atomes de carbone qui peut être substitué par un groupe $OR_1'$ dans lequel $R_1'$ est tel que défini ci-dessus, ou $A'NR_1'R_2'$ dans lequel A′ est un radical alkylène comprenant de 2 à 4 atomes de carbone et $R_1'$ et $R_2'$ sont tels que définis ce-dessus, ainsi que les sels pharmaceutiquement acceptables en dérivant.

9. Un composé tel que défini dans la revendication 4, présentant la formule structurelle I″,

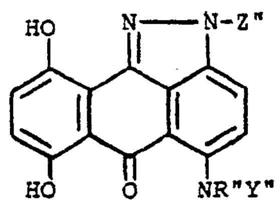

(I″)

dans laquelle: R″ est un atome d'hydrogène ou un radical alkyle comprenant de 1 à 6 atomes de carbone; Y″ est un atome d'hydrogène ou un radical alkyle comprenant de 1 à 6 atomes de carbone qui peut être substitué par un groupe $OR_1''$ dans lequel $R_1''$ est un atome d'hydrogène ou un radical alkyle comprenant de 1 à 4 atomes de carbone, ou $A''NR_2''R_3''$ dans lequel A″ est un radical alkylène comprenant de 2 à 4 atomes de carbone, $R_2''$ et $R_3''$ peuvent être identiques ou différents et consistent en un atome d'hydrogène, un radical alkyle comprenant de 1 à 6 atomes de carbone qui peut être substitué par un groupe OH ou un groupe NRa″Ra″ dans lequel Ra″ sont identiques ou différents et consistent en un atome d'hydrogène, un radical alkyle comprenant de 1 à 3 atomes de carbone qui peut être substitué par un group OH, ou $R_2''$ et $R_3''$ lorsqu'ils sont pris ensemble peuvent former un groupe éthylène ou un groupe:

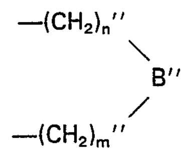

dans lequel n″ et m″ peuvent être identiques ou différents et sont égaux à 1 ou 2, étant entendu que la somme de n″ et m″ est égal à 3 ou 4, et B″ est une liaison directe, un atome d'oxygène, de soufre ou un groupe R—$R_4''$ dans lequel $R_4''$ est un atome d'hydrogène, un radical alkyle comprenant de 1 à 4 atomes de carbone; Z″ est un radical alkyle comprenant de 1 à 4 atomes de carbone qui peut être substitué par un groupe $SR_1''$ ou $OR_1''$ dans lesquels $R_1''$ est tel que défini ci-dessus, ou $D''NR_2''R_3''$ dans lequel D″ est un radical alkylène comprenant de 2 à 4 atomes de carbone qui peut être substitué par un groupe OH, et $R_2''$ et $R_3''$ sont tels que définis ci-dessus, ainsi que les sels pharmaceutiquement acceptables en dérivant.

10. Un composé tel que défini dans la revendication 2, présentant la formule structurelle I‴:

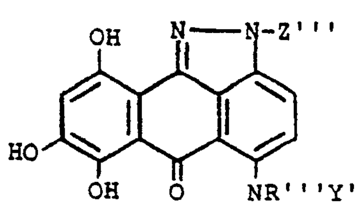

(I‴)

dans laquelle; R''' est un atome d'hydrogène ou un radical alkyle comprenant de 1 à 6 atomes de carbone; Y''' est un atome d'hydrogène ou un radical alkyle comprenant de 1 à 6 atomes de carbone qui peut être substitué par un groupe $OR_1'''$ dans lequel $R_1'''$ est un atome d'hydrogène ou un radical alkyle comprenant de 1 à 4 atomes de carbone, ou $A'''NR_2'''R_3'''$ dans lequel A''' est un radical alkyle comprenant de 2 à 4 atomes de carbone, $R_2'''$ et $R_3'''$ peuvent être identiques ou différents et consister en un atome d'hydrogène ou un radical alkyle comprenant de 1 à 6 atomes de carbone qui peut être substitué par un groupe OH ou un groupe NRa'''Ra''' dans lequel Ra''' sont identiques ou différents et consistent en un atome d'hydrogène ou un radical alkyle comprenant de 1 à 3 atomes de carbon qui peut être substitué par un groupe OH, ou $R_2'''$ et $R_3'''$ lorsqu'ils sont pris ensemble peuvent former un groupe éthylène ou peuvent former un groupe:

$$\begin{array}{l} —(CH_2)_n''' \\ \quad\quad\quad\quad \diagdown \\ \quad\quad\quad\quad\quad B''' \\ \quad\quad\quad\quad \diagup \\ —(CH_2)_m''' \end{array}$$

dans lequel n''' et m''' peuvent être identiques ou différents et sont égaux à 1 ou 2, étant entendu que la somme de n''' et m''' est égal à 3 ou 4, et B''' est une liaison directe, un atome d'oxygène, de soufre ou un groupe $R—R_4'''$ dans lequel $R_4'''$ est un atome d'hydrogène, un radical alkyle comprenant de 1 à 4 atomes de carbone; Z''' est un radical alkyle comprenant de 1 à 4 atomes de carbone qui peut être substitué par un groupe $SR_1'''$ ou $OR_1'''$ dans lesquels $R_1'''$ est tel que défini ci-dessus, ou $D'''NR_2'''R_3'''$ dans lequel D''' est un radical alkylène comprenant de 2 à 4 atomes de carbone qui peut être substitué par un groupe OH, et $R_2'''$ et $R_3'''$ sont tels que définis ci-dessus, ainsi que les sels pharmaceutiquement acceptables en dérivant.

11. Un composé tel que défini dans la revendication 4, présentant la formule structurelle I'''':

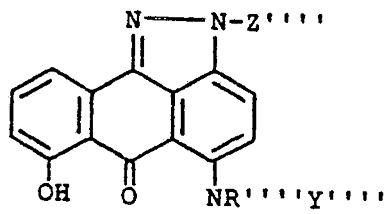

(I'''')

dans laquelle: R'''' est un atome d'hydrogène ou un radical alkyle comprenant de 1 à 6 atomes de carbone; Y'''' est un atome d'hydrogène ou un radical alkyle comprenant de 1 à 6 atomes de carbone qui peut être substitué par un groupe $OR_1''''$ dans lequel $R_1''''$ est H ou un radical alkyle comprenant de 1 à 4 atomes de carbone ou $R''''NR_2''''R_3''''$ dans lequel A'''' est un radical alkylène comprenant de 2 à 4 atomes de carbone, $R_2''''$ et $R_3''''$ peuvent être identiques ou différents et consister en un atome d'hydrogène ou un radical alkyle comprenant de 1 à 6 atomes de carbone, qui peut être substitué par un groupe OH ou un groupe NRa''''Ra'''' dans lequel Ra'''' sont identiques ou différents et consistent en un atome d'hydrogène ou un radical alkyle comprenant de 1 à 3 atomes de carbone qui peut être substitué par un groupe OH, ou $R_2''''$ et $R_3''''$ lorsqu'ils sont pris ensemble peuvent former un groupe éthylène ou peuvent former le groupe:

$$\begin{array}{l} —(CH_2)_n'''' \\ \quad\quad\quad\quad \diagdown \\ \quad\quad\quad\quad\quad B'''' \\ \quad\quad\quad\quad \diagup \\ —(CH_2)_m'''' \end{array}$$

dans lequel n'''' et m'''' peuvent être identiques ou différents et sont égaux à 1 ou 2, étant entendu que la somme de n'''' et m'''' est égal à 3 ou 4, et B'''' est une liaison directe, un atome d'oxygène, de soufre ou un groupe $N—R_4''''$ dans lequel $R_4''''$ est un atome d'hydrogène ou un radical alkyle comprenant de 1 à 4 atomes de carbone; Z'''' est un radical alkyle comprenant de 1 à 4 atomes de carbone qui peut être substitué par un groupe $SR_1''''$ ou $OR_1''''$ dans lesquels $R_1''''$ est tel que défini ci-dessus, ou $D''''NR_2''''R_3''''$ dans lequel D'''' est un radical alkylène comprenant de 2 à 4 atomes de carbone qui peut être substitué par un groupe OH, et $R_2''''$ et $R_3''''$ sont tels que définis ci-dessus, ainsi que les sels pharmaceutiquement acceptables en dérivant.

12. Un composé tel que défini dans la revendication 4, présentant la formule structurelle $I^V$:

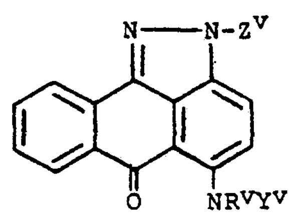

($I^V$)

dans laquelle: $R^v$ est un atome d'hydrogène ou un radical alkyle comprenant de 1 à 6 atomes de carbone; $Y^v$ est un atome d'hydrogène ou un radical alkyle comprenant de 1 à 4 atomes de carbone ou $A^vNR_2^vR_3^v$ dans lequel $A^v$ est un radical alkylène comprenant de 2 à 4 atomes de carbone, $R_2^v$ et $R_3^v$ peuvent être identiques et différents et consister en un atome d'hydrogène ou un radical alkyle comprenant de 1 à 6 atomes de carbone qui peut être substitué par un groupe OH, ou $R_2^v$ et $R_3^v$ lorsqu'ils sont pris ensemble peuvent former un groupe éthylène ou peuvent former le groupe:

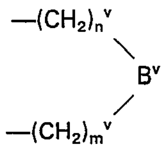

dans lequel $n^v$ et $m^v$ peuvent être identiques ou différents et sont égaux à 1 ou 2, étant entendu que la somme de $n^v$ et $m^v$ est égal à 3 ou 4, et $B^v$ est une liaison directe, un atome d'oxygène, de soufre ou un groupe $N—R_4^v$ dans lequel $R_4^v$ est un atome d'hydrogène ou un radical alkyle comprenant de 1 à 4 atomes de carbone; $Z^v$ est un radical alkyle comprenant de 1 à 4 atomes de carbone qui peut être substitué par un groupe $SR_1^v$ ou $OR_1^v$ dans lesquels $R_1^v$ est un atome d'hydrogène ou un radical alkyle comprenant de 1 à 4 atomes de carbone, ou $D^vNR_2^vR_3^v$ dans lequel $D^v$ est un radical alkylène comprenant de 2 à 4 atomes de carbone qui peut être substitué par un groupe OH, et $R_2^vR_3^v$ sont tels que définis ci-dessus, ainsi que des sels pharmaceutiquement acceptables en dérivant.

13. Un composé tel que défini dans la revendication 4, présentant la formule structurelle $I^{vi}$:

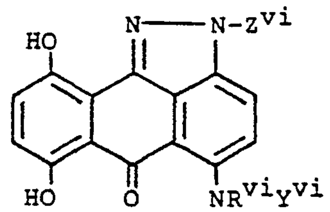

$(I^{vi})$

dans laquelle: $R^{vi}$ est un atome d'hydrogène ou un radical alkyle comprenant de 1 à 6 atomes de carbone; $Y^{vi}$ est un atome d'hydrogène ou un radical alkyle comprenant de 1 à 4 atomes de carbone qui peut être substitué par un groupe $OR_1^{vi}$ dans lequel $R_1^{vi}$ est un atome d'hydrogène, ou un radical alkyle comprenant de 1 à 4 atomes de carbone, ou $A^{vi}NR_2^{vi}R_3^{vi}$ dans lequel $A^{vi}$ est un radical alkylène comprenant de 2 à 4 atomes de carbone, $R_2^{vi}$ et $R_3^{vi}$ peuvent être identiques et différents et consister en un radical alkyle comprenant de 1 à 6 atomes de carbone qui peut être substitué par un groupe OH, ou $R_2^{vi}$ et $R_3^{vi}$ lorsqu'ils sont pris ensemble peuvent former un groupe éthylène ou peuvent former le groupe:

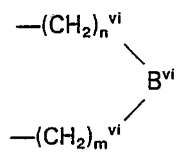

dans lequel $n^{vi}$ et $m^{vi}$ peuvent être identiques ou différents et sont égaux à 1 ou 2, étant entendu que la somme de $n^{vi}$ et $m^{vi}$ est égal à 3 ou 4, et $B^v$ est une liaison directe, un atome d'oxygène, de soufre ou un groupe $N—R_4^{vi}$ dans lequel $R_4^{vi}$ est un atome d'hydrogène ou un radical alkyle comprenant de 1 à 4 atomes de carbone; $Z^{vi}$ est $D^{vi}NR_2^{vi}R_3^{vi}$ dans lequel $D^{vi}$ est un groupe alkylène comprenant de 2 à 4 atomes de carbone, $R_2^{vi}$ et $R_3^{vi}$ sont tels que définis ci-dessus, ainsi que des sels pharmaceutiquement acceptables en dérivant.

14. Les composés chimiques tels que définis dans la revendication 2, ayant les dénominations suivantes:

2-[2-[(2-hydroxyéthyl)amino]éthyl]-5-{[2-[(2-hydroxyéthyl)amino]éthyl]amino]anthra(1,9-cd)}pyrazol-6(2H)-one;

2-[2-(diéthylamino)éthyl]-7,10-dihydroxy-5-{[2-[(2-hydroxyéthyl)amino]éthyl]amino}anthra(1,9-cd)pyrazol-6(2H)-one;

2-{2-[[2-(diméthylamino)éthyl]amino]éthyl}-5-{[2-[(2-hydroxyéthyl)amino]éthyl]amino}-7,10-dihydroxyanthra(1,9-cd)pyrazol-6(2H)-one;

5-[(2-aminoéthyl)amino]-2-[2-(diéthylamino)éthyl]-7,10-dihydroxyanthra(1,9-cd)pyrazol-6(2H)-one;

2-[2-(diméthylamino)éthyl]-7,10-dihydroxy-5-{[2-[(2-hydroxyéthyl)amino]éthyl]amino}anthra(1,9-cd)pyrazol-6(2H)-one;

5-{[2-(diméthylamino)éthyl]amino}-7,10-dihydroxy-2-(2-hydroxyéthyl)anthra(1,9-cd)pyrazol-6(2H)-one;

5-[(2-aminoéthyl)amino]-7,10-dihydroxy-2-[2-[(2-hydroxyéthyl)amino]éthyl]anthra(1,9-cd)pyrazol-6(2H)-one;

5-[(3-aminopropyl)amino]-7,10-dihydroxy-2-[2-[(2-hydroxyéthyl)amino]éthyl]anthra(1,9-cd)pyrazol-6(2H)-one;

5-{[2-[(2-aminoéthyl)amino]éthyl]amino}-7,10-dihydroxy-2-[2-[(2-hydroxyéthyl)amino]éthyl]anthra(1,9-cd)pyrazol-6(2H)-one;

5-{[2-[bis(2-hydroxyéthyl)amino]éthyl]amino}-7,10-dihydroxy-2-[2-[(2-hydroxyéthyl)amino]éthyl]anthra(1,9-cd)pyrazol-6(2H)-one;

7,10-dihydroxy-2-[2-[(2-hydroxyéthyl)amino]éthyl]-5-{[2-(méthylamino)éthyl]amino}anthra(1,9-cd)pyrazol-6(2H)-one;

2-(2-aminoéthyl)-7,10-dihydroxy-5-{[2-[(2-hydroxyéthyl)amino]éthyl]amino}anthra(1,9-cd)pyrazol-6(2H)-one;

2-(2,3-dihydroxypropyl)-7,10-dihydroxy-5-{[2-[(2-hydroxyéthyl)amino]éthyl]amino}anthra(1,9-cd)pyrazol-6(2H)-one;

7-hydroxy-2-[2-[(2-hydroxyéthyl)amino]éthyl]-5-{[2-[(2-hydroxyéthyl)amino]éthyl]amino}anthra(1,9-cd)pyrazol-6(2H)-one;

7-hydroxy-2-[2-[(2-hydroxyéthyl)amino]éthyl]-5-{[2-(méthylamino)éthyl]amino}anthra(1,9-cd)pyrazol-6(2H)-one;

7,8,10-trihydroxy-2-[2-[(2-hydroxyéthyl)amino]éthyl]-5-{[2-[(2-hydroxyéthyl)amino]éthyl]amino}anthra(1,9-cd)pyrazol-6(2H)-one;

7,8,10-trihydroxy-2-[2-[(2-hydroxyéthyl)amino]éthyl]-5-{[2-(méthylamino)éthyl]amino}anthra(1,9-cd)pyrazol-6(2H)-one;

2-(3-aminopropyl)-7,10-dihydroxy-5-{[2-[(2-hydroxyéthyl)amino]éthyl]amino}anthra(1,9-cd)pyrazol-6(2H)-one;

2-(2-aminoéthyl)-7,10-dihydroxy-5-{[2-(méthylamino)éthyl]amino}anthra(1,9-cd)pyrazol-6(2H)-one;

5-[(2-aminoéthyl)amino]-2-[3-(diméthylamino)propyl]-7,10-dihydroxy-anthra(1,9-cd)pyrazol-6(2H)-one;

7,8-dihydroxy-2-[2-[(2-hydroxyéthyl)amino]éthyl]-5-{[2-[(2-hydroxyéthyl)amino]éthyl]amino}anthra-(1,9-cd)pyrazol-6(2H)-one;

ainsi que les sels pharmaceutiquement acceptables en dérivant.

15. Un composé chimique présentant la formule structurelle:

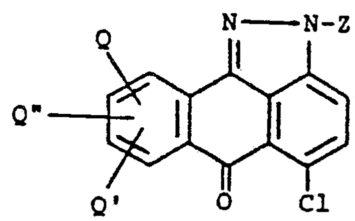

dans laquelle: Q, Q′ et Q″ peuvent être identiques ou différents et consistent en un atome d'hydrogène, un groupe OH, alkoxy comprenant de 1 à 4 atomes de carbone, chlore, benzyloxy, p-chlorobenzyloxy et p-méthoxybenzyloxy, ainsi que les sels pharmaceutiquement acceptables en dérivant; Z est tel que défini dans la revendication 1, ainsi que les sels pharmaceutiquement acceptables en dérivant, étant entendu que lorsque Q=Q′=Q″=H, Z ne peut pas être H ni un radical alkyle comprenant de 1 à 6 atomes de carbone.

16. Un composé chimique présentant la formule structurelle III:

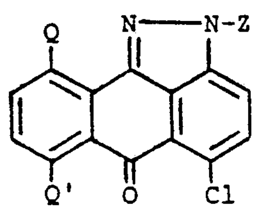

(III)

dans laquelle Q et Q′ peuvent être identiques ou différents et consistent en un atome d'hydrogène, un groupe OH, benzyloxy, p-chlorobenzyloxy ou p-méthoxybenzyloxy; et Z est tel que défini dans la revendication 1, ainsi que les sels pharmaceutiquement acceptables en dérivant; étant entendu que lorsque Q=Q′=H, Z ne doit être ni H, ni un radical alkyle comprenant de 1 à 6 atomes de carbone.

17. Les composés chimiques tels que définis dans la revendication 16, dans laquelle Q et Q′ sont un atome d'hydrogène, ainsi que les sels pharmaceutiquement acceptables en dérivant.

18. Les composés chimiques tels que définis dans la revendication 16, dans laquelle Q et Q′ sont des radicaux benzyloxy, p-chlorobenzyloxy ou p-méthoxybenzyloxy, ainsi que les sels pharmaceutiquement acceptables en dérivant.

19. Les composés chimiques tels que définis dans la revendication 16, dans laquelle Q et Q′ sont un groupe OH, ainsi que les sels pharmaceutiquement acceptables en dérivant.

20. Les composés chimiques tels que définis dans la revendication 15, ayant les dénominations suivantes:

5-chloro-2-[2-[(2-hydroxyéthyl)amino]éthyl]anthra(1,9-cd)pyrazol-6(2H)-one;

5-chloro-2-[2-(diéthylamino)éthyl]-7,10-dihydroxy-anthra(1,9-cd)pyrazol-6(2H)-one;

5-chloro-7,10-dihydroxy-2-(2-hydroxyéthyl)anthra(1,9-cd)pyrazol-6(2H)-one;

5-chloro-2-[2-(diéthylamino)éthyl]-7,10-bis(phénylméthoxy)antha(1,9-cd)pyrazol-6(2H)-one;

5-chloro-2-[2-[[(4-méthylphényl)sulfonyl]oxy]éthyl]-7,10-bis(phénylméthoxy)anthra(1,9-cd)pyrazol-6(2H)-one;

5-chloro-7,10-dihdyroxy-2-[2-[(2-hydroxyéthyl)amino]éthyl]anthra(1,9-cd)pyrazol-6(2H)-one;

5-chloro-2-[2-[(2-hydroxyéthyl)amino]éthyl]-7,10-bis(phénylméthoxy)anthra(1,9-cd)pyrazol-6(2H)-one;

5-chloro-2-[2-[[2-(diméthylamino)éthyl]amino]éthyl]-7,10-dihydroxy-anthra(1,9-cd)pyrazol-6(2H)-one;

5-chloro-2-[3-(diméthylamino)propyl]-7,10-dihydroxyanthra(1,9-cd)pyrazol-6(2H)-one;

5-chloro-2-(2-hydroxyéthyl)-7,10-bis(phénylméthoxy)anthra(1,9-cd)pyrazol-6(2H)-one;

5-chloro-2-[3-[[4-méthylphényl)sulfonyl]oxy]propyl]-7,10-bis(phénylméthoxy)anthra(1,9-cd)pyrazol-6(2H)-one;

5-chloro-2-(3-hydroxypropyl)-7,10-bis(phénylméthoxy)anthra(1,9-cd)pyrazol-6(2H)-one;

2-(2-aminoéthyl)-5-chloro-7,10-dihydroxyanthra(1,9-cd)pyrazol-6(2H)-one;

2-(2-aminoéthyl)-5-chloro-7,10-bis(phénylméthoxy)anthra(1,9-cd)pyrazol-6(2H)-one;

5-chloro-2-[(2,2-diméthyl-1,3-dioxolan-4-yl)méthyl]-7,10-bis(phénylméthoxy)anthra(1,9-cd)pyrazol-6(2H)-one;

5-chloro-2-[2-[(2-hydroxyéthyl)amino]éthyl]-7-(phénylméthoxy)anthra(1,9-cd)pyrazol-6(2H)-one;

5-chloro-2-[2-[(2-hydroxyéthyl)amino]éthyl]-10-(phénylméthoxy)anthra(1,9-cd)pyrazol-6(2H)-one;

5-chloro-2-[2-[(2-hydroxyéthyl)amino]éthyl]-7,8,10-tris(phénylméthoxy)anthra(1,9-cd)pyrazol-6(2H)-one;

2-(3-aminopropyl)-5-chloro-7,10-bis(phénylméthoxy)anthra(1,9-cd)pyrazol-6(2H)-one;

5-chloro-2-[2-[(2-hydroxyéthyl)amino]éthyl]-7,8-bis(phénylméthoxy)anthra(1,9-cd)pyrazol-6(2H)-one;

5-chloro-2-[2-[[2-(diméthylamino)éthyl]amino]éthyl]-7,10-bis(phénylméthoxy)anthra(1,9-cd)pyrazol-6(2H)-one;

ainsi que les sels pharmaceutiquement acceptables en dérivant.

21. Un composé présentant la formule structurelle IV:

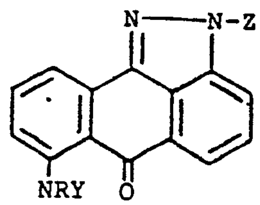

(IV)

dans laquelle: R est un atome d'hydrogène ou un radical alkyle comprenant de 1 à 6 atomes de carbone; Y est un atome d'hydrogène, un radical alkyle comprenant de 1 à 6 atomes de carbone qui peut être substitué par un groupe $OR_1$ dans lequel $R_1$ est un atome d'hydrogène ou un radical alkyle comprenant de 1 à 6 atomes de carbone, ou $AnR_2R_3$ dans lequel A est un radical alkylène comprenant de 2 à 8 atomes de carbone, $R_2$ et $R_3$ pouvant être identiques ou différents et consistant en un atome hydrogène ou un radical alkyle comprenant de 1 à 6 atomes de carbone qui peut être substitué par OH ou un groupe NRaRa dans lequel Ra identique ou différent consiste en un atome d'hydrogène ou un radical alkyle comprenant de 1 à 3 atomes de carbone, qui peut être substitué par OH, ou $R_2$ et $R_3$ peuvent former ensemble un radical éthylène ou un groupe:

$$\begin{matrix} -(CH_2)_n & \\ & \diagdown \\ & B \\ & \diagup \\ -(CH_2)_m & \end{matrix}$$

dans lequel n et m peuvent être identiques ou différents et égaux à 1, 2 ou 3, étant entendu que la somme de n et m est nombre entier comprise entre 3 et 6 et que B est une liaison directe, un atome d'oxygène, de soufre ou un groupe $N—R_4$ dans lequel $R_4$ est un atome d'hydrogène, un radical alkyle comprenant 1 à 6 atomes de carbone; R et Y pris ensemble forment un radical éthylène ou peuvent encore former un groupe:

$$\begin{matrix} -(CH_2)_n & \\ & \diagdown \\ & B \\ & \diagup \\ -(CH_2)_m & \end{matrix}$$

dans lequel n, m et B sont tels que définis ci-dessus; Z est un atome d'hydrogène, un radical alkyle

comprenant 1 à 6 atomes de carbone qui peut être substitué par un groupe $N(R_1)_2$, $SR_1$ ou $OR_1$ dans lesquels $R_1$, identique ou différent, sont tels que définis ci-dessus, ou un groupe $DnR_2R_3$ dans lequel D est un radical alkylène comprenant 2 à 8 atomes de carbone, qui peut être substitué par un groupe OH, et $R_2$ et $R_3$ sont tels que définis ci-dessus, ainsi que les sels pharmaceutiquement acceptables en dérivant, à la condition cependant, que: 1) lorsque Z est un H, R et Y pris ensemble ne forment pas le cycle pipéridine; 2) lorsque Z est $CH_3$, R et Y pris ensemble ne forment pas le noyau pipéridine ou le noyau morpholine; et 3) lorsque R et Y sont tous deux égaux à H, Z n'est ni un atome d'hydrogène, ni un radical alkyle comprenant 1 à 6 atomes de carbone.

22. Les composés chimiques tels que définis dans la revendication 21, ayant les dénominations ci-dessous:

2-[2-(diéthylamino)éthyl]-7-{[2-[(2-hydroxyéthyl)amino]éthyl]amino}anthra(1,9-cd)pyrazol-6(2H)one;

2-[2-(diéthylamino)éthyl]-7-{[2-(diéthylamino)éthyl]amino}anthra(1,9-cd)pyrazol-6(2H)-one;

2-[2-[(2-hydroxyéthyl)amino]éthyl]-7-{[2-[(2-hydroxyéthyl)amino]éthyl]amino}anthra(1,9-cd)pyrazol-6(2H)one;

ainsi que les sels pharmaceutiquement acceptables en dérivant.

23. Un composé présentant la formule structurelle VII:

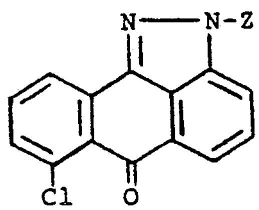

(VII)

dans laquelle Z est tel que défini ci-dessus, à condition qu'il ne soit ni H, ni un radical alkyle comprenant de 1 à 6 atomes de carbone.

24. Les composés chimiques tels que définis dans la revendication 23, présentant les dénominations suivantes:

7-chloro-2-[2-(diéthylamino)éthyl]anthra(1,9-cd)pyrazol-6(2H)-one;

7-chloro-2-[2-[(2-hydroxyéthyl)amino]éthyl]anthra(1,9-cd)pyrazol-6(2H)-one;

ainsi que les sels pharmaceutiquement acceptables en dérivant.

25. Le composé 2-[(hydrazinoéthyl)amino]éthanol et les sels d'addition d'acides en dérivant.

26. 5,8-dichloro-1,4,9,10-anthracènetérone.

27. Un procédé de préparation d'un composé tel que défini dans la revendication 1 présentant la formule structurelle:

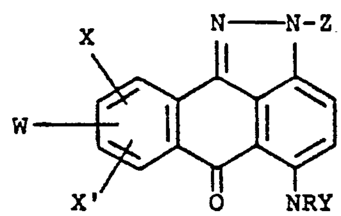

qui consiste à faire réagir un composé ayant la formule structurelle:

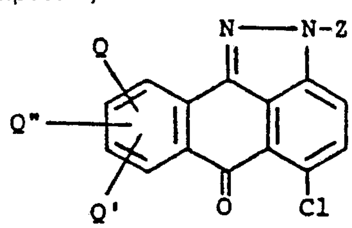

avec une amine ayant la formule HNRY dans laquelle W, X, X', Q, Q', Q'', Y, Z et R sont tels que définis dans la revendication 1, et les cas échéant, éliminer par hydrogénation catalytique ou par traitement par trichlorure ou tribromure de bore tous groupes benzyliques présents.

28. Un procédé de préparation d'un composé tel que défini dans la revendication 2 présentant la formule structurelle I:

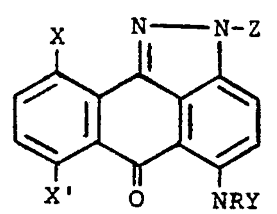

(I)

qui consiste à faire réagir un composé présentant la formule structurelle II:

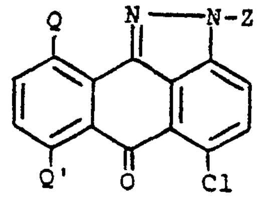

(II)

avec une amine ayant la formule HNRY dans laquelle X, X', Q, Q', Y, Z et R sont tels que définis la revendication 2.

29. Un procédé tel que défini dans la revendication 28, dans lequel X et X' sont OH.

30. Un procédé tel que défini dans la revendication 28, dans lequel X et X' sont H.

31. Un procédé de préparation d'un composé tel que défini dans la revendication 16, présentant la formule structurelle III:

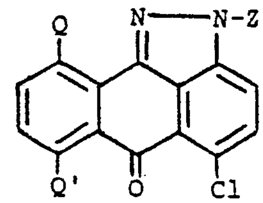

(III)

qui consiste à faire réagir un composé ayant la formule V:

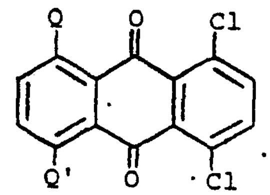

(V)

avec une hydrazine ayant la formule $H_2N—\ddot{N}HZ$ dans laquelle Q, Q' et Z sont tels que définis dans la revendication 15.

32. Le procédé tel que défini dans la revendication 31, dans lequel Q et Q' sont des radicaux benzyloxy, p-chlorobenzyloxy ou p-méthoxybenzyloxy.

33. Le procédé tel que défini dans la revendication 31, dans lequel Q et Q' sont OH.

34. Le procédé tel que défini dans la revendication 31, dans lequel Q et Q' sont H.

35. Un procédé de préparation d'un composé tel que défini dans la revendication 21, présentant la formule structurelle IV:

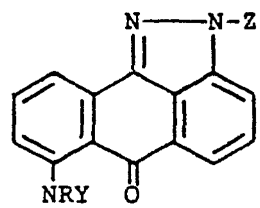

(IV)

qui consite à faire réagir un composé ayant la formule structurelle VII;

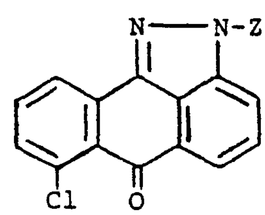

(VII)

avec une amine ayant la formule HNRY, dans laquelle Y, Z et R sont tels que définis dans la revendication 21.

36. Une composition pharmaceutiquement comprenant un composé tel que défini dans l'une quelconque des revendications 1 à 26 ou un des sels pharmaceutiquement acceptables en dérivant, en association avec un support acceptable du point de vue pharmaceutique.

37. Un composé de formule structurelle:

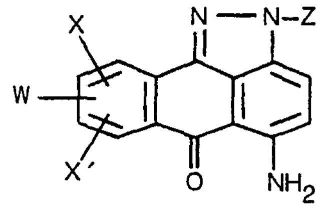

dans laquelle: Z est un atome d'hydrogène ou un radical alkyle comprenant de 1 à 6 atomes de carbone; et X, X' et W sont tels que définis dans la revendication 1, ou un des sels pharmaceutiquement acceptables en dérivant, convenant au traitement d'infections microbiennes chez les mammifières.

38. L'emploi d'un composé tel que défini dans la revendication 37, pour la préparation d'un médicament convenant au traitement de la leucémie chez les mammifères.

39. L'emploi d'un composé tel que défini dans la revendication 37, pour la préparation d'un médicament convenant au traitement des tumeurs solides chez les mammifères.

40. Un composé de formule structurelle:

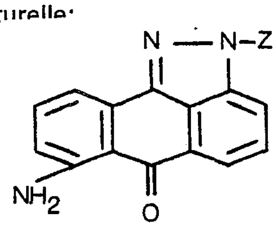

dans laquelle: Z est un atome d'hydrogène ou un radical alkyle comprenant de 1 à 6 atomes de carbone, ou un des sels pharmaceutiquement acceptables en dérivant, convenant au traitement d'infections microbiennes chez les mammifières.

41. L'emploi d'un composé tel que défini dans la revendication 40, pour la préparation d'un médicament convenant au traitment de la leucémie chez les mammifères.

42. L'emploi d'un composé tel que défini dans la revendication 40, pour la préparation d'un médicament convenant au traitment des tumeurs solides chez les mammifères.

**Revendications pour l'Etat contractant: AT**

1. Un procédé de préparation d'un composé présentant la formule structurelle:

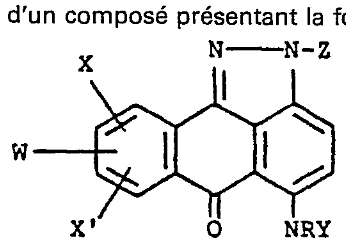

dans laquelle: X, X' et W peuvent être identiques ou différents et consistent en un hydrogène, hydroxy, alkoxy comprenant de 1 à 4 atomes de carbone et chlore; R est un atome d'hydrogène ou un radical alkyle comprenant de 1 à 6 atomes de carbone; Y est un atome d'hydrogène ou un radical alkyle comprenant de 1 à 6 atomes de carbone, susceptible d'être substitué par un groupe $OR_1$ dans lequel $R_1$ est un atome d'hydrogène ou un radical alkyle comprenant de 1 à 6 atomes de carbone, ou $ANR_2R_3$ dans lequel A est un alkylène linéaire ou ramifié comprenant de 2 à 8 atomes de carbone, $R_2$ et $R_3$ pouvant être identiques ou différents et consister en hydrogène, alkyle comprenant de 1 à 6 atomes de carbone susceptible d'être substitué par un groupe OH ou NRaRa dans lequel les groupes Ra peuvent être identiques ou différents et consistent en hydrogène ou alkyle comprenant de 1 à 3 atomes de carbone, susceptibles d'être substitué par OH ou NRbRb dans lequel les groupes Rb identiques ou différents consistant en H ou alkyle comprenant de 1 à 3 atomes de carbone, ou $R_2$ et $R_3$ forment ensemble un groupe éthylène ou peuvent former un groupe:

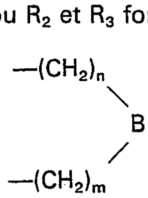

dans lequel n et m peuvent être identiques ou différents et sont égaux à 1, 2 ou 3, étant entendu que la somme de n et m est nombre entier comprise entre 3 et 6 et que B est une liaison directe, un atome d'oxygène ou de soufre ou un groupe N—$R_4$ dans lequel $R_4$ est formé d'atome d'hydrogène ou d'un radical alkyle comprenant de 1 à 6 atomes de carbone, R et Y pris ensemble pouvant être un radical éthylène ou pouvant former un groupe:

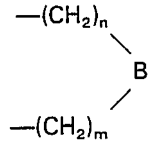

dans lequel n et m et B sont tels que définis ci-dessus; Z est un atome d'hydrogène, un radical alkyle comprenant de 1 à 6 atomes de carbone, susceptible d'être substitué par un groupe $N(R_1)_2$, $SR_1$ ou $OR_1$ dans lesquels $R_1$ est identique ou différent et est tel que défini ci-dessus, ou $DNR_2R_3$ avec D consistant en un radical alkylène linéaire ou ramifié comprenant de 2 à 8 atomes de carbone, qui peut être substitué par un groupe OH et, $R_2$ et $R_3$ sont tels que définis ci-dessus; ainsi que les sels pharmaceutiquement acceptables en dérivant, à la condition cependant, que: 1) lorsque X, X' et W sont H et Z est H, R et Y lorsqu'ils sont pris ensemble ne forment pas un cycle pipéridine; 2) lorsque X, X' et W sont H et Z est $CH_3$, R et Y pris ensemble ne forment pas un noyau pipéridine ou un noyau morpholine; et 3) lorsque R et Y tous deux sont H, Z ne soit ni H, ni un radical alkyle comprenant de 1 à 6 atomes de carbone, et qui consiste à faire réagir un composé ayant la formule structurelle:

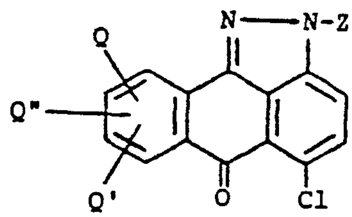

avec une amine ayant la formule HNRY dans laquelle W, X, X', Q, Q', Q'', Y, Z et R sont tels que définis ci-dessus et le cas échéant, éliminer par hydrogénation catalytique ou par traitement par trichlorure ou tribromure de bore tous groupes benzyliques présents.

2. Un procédé de préparation d'un composé présentant la formule structurelle:

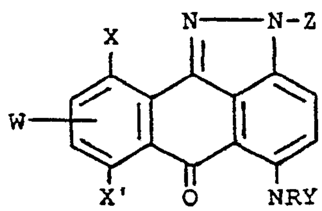

dans laquelle: X, X' et W peuvent être identiques ou différents et consistent en H ou OH, un radical alkoxy comprenant de 1 à 4 atomes de carbone ou chlore; R est un atome d'hydrogène ou un radical alkyle comprenant de 1 à 6 atomes de carbone; Y est un atome d'hydrogène, un radical alkyle comprenant de 1 à 6 atomes de carbone, susceptible d'être substitué par un groupe $OR_1$ dans lequel $R_1$ est H ou alkyle comprenant de 1 à 6 atomes de carbone, ou $ANR_2R_3$ dans lequel A est un radical alkylène linéaire ou ramifié comprenant de 2 à 8 atomes de carbone, $R_2$ et $R_3$ pouvant être identiques ou différents et consistent en H, alkyle comprenant de 1 à 6 atomes de carbone susceptible d'être substitué par un groupe OH ou NRaRa dans lequel les groupes Ra peuvent être identiques ou différents et consister en H ou un radical alkyle comprenant de 1 à 3 atomes de carbone, susceptible d'être substitués par un groupe OH, ou NRbRb dans lequel les groupes Rb sont identiques ou différents et consistent en atome d'hydrogène ou un radical alkyle comprenant de 1 á 3 atomes de carbone, ou $R_2$ et $R_3$ pris ensemble peuvent former un radical éthylène ou peuvent former un groupe:

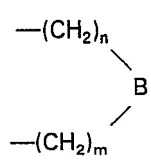

dans lequel n et m, qui peuvent être identiques ou différents, sont égaux à 1, 2 ou 3, étant entendu que la somme de n et m forme un nombre entier compris entre 3 et 6 et que B est une liaison directe formée un atome d'oxygène, de soufre ou un groupe N—$R_4$ dans lequel $R_4$ est un atome d'hydrogène ou un radical

alkyle comprenant de 1 à 6 atomes de carbone; R et Y lorsqu'ils sont pris ensemble pouvant être un radical éthylène ou pouvant former un groupe:

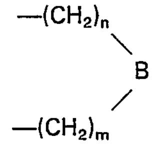

dans lequel n, m et B sont tels que définis ci-dessus; Z est un atome d'hydrogène, un radical alkyle comprenant de 1 à 6 atomes de carbone, susceptible d'être substitué par un groupe $N(R_1)_2$, $SR_1$ ou $OR_1$ dans lesquels $R_1$ est identique ou différent et est tel que défini ci-dessus, ou $DNR_2R_3$ dans lequel D est un radical alkylène linéaire ou ramifié comprenant de 2 à 8 atomes de carbone susceptible d'être substitué par un groupe OH et, $R_2$ et $R_3$ sont tels que définis ci-dessus, ainsi que les sels pharmaceutiquement acceptables en dérivant, aux conditions suivantes cependant, que: 1) lorsque X, X' et W sont H et Z est H, R et Y pris ensemble ne forment pas un groupe pipéridine; 2) lorsque X, X' et W sont H et Z est $CH_3$, R et Y lorsqu'ils sont pris ensemble ne forment pas un noyau pipéridine ou un noyau morphline; et 3) lorsque R et Y tous deux sont H, Z ne soit ni H, ni un radical alkyle comprenant de 1 à 6 atomes de carbone, et qui consiste à faire réagir un composé ayant la formule structurelle II:

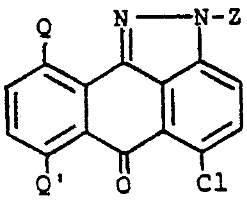

(II)

avec une amine ayant la formule HNRY dans laquelle W, X, X', Q, Q', Y, Z et R sont tels que défini ci-dessus.

3. Le procédé tel que défini dans la revendication 2, dans lequel X et X' sont OH.

4. Le procédé tel que défini dans la revendication 2, dans lequel X et X' sont H.

5. Un procédé de préparation d'un composé chimique présentant la formule structurelle III:

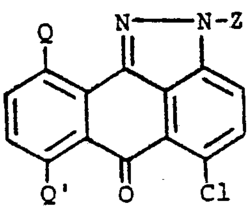

(III)

dans laquelle: Q et Q' peuvent être identiques ou différents et consistent en un atome d'hydrogène, un groupe OH, benzyloxy, p-chlorobenzyloxy ou p-méthoxybenzyloxy; et Z est tel que défini dans la revendication 1, ainsi que les sels pharmaceutiquement acceptables en dérivant; étant entendu que lorsque Q=Q'=H, Z ne doit être ni H, ni un radical alkyle comprenant de 1 à 6 atomes de carbone qui consiste à faire réagir un composé ayant la formule V:

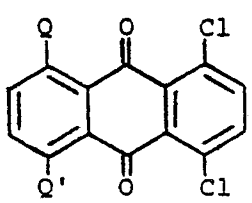

(V)

avec une hydrazine ayant la formule $H_2N—NHZ$ dans laquelle: Q, Q' et Z sont identiques ou différents et sont un atome d'hydrogène, un groupe OH, benzyloxy, p-chlorobenzyloxy et p-méthoxybenzyloxy; ainsi que les sels pharmaceutiquement acceptables en dérivant; Z est tel que défini dans la revendication 1, ainsi que les sels pharmaceutiquement acceptables en dérivant; étant entendu que lorsque Q=Q'=H, Z ne doit être ni H, ni un radical alkyle comprenant de 1 à 6 atomes de carbone.

6. Un procédé de préparation d'un composé présentant la formule structurelle IV:

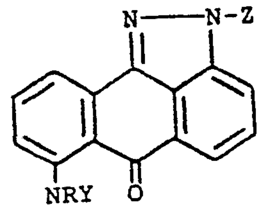

(IV)

dans laquelle: R est un atome d'hydrogène ou un radical alkyle comprenant de 1 à 6 atomes de carbone; Y est un atome d'hydrogène, un radical alkyle comprenant de 1 à 6 atomes de carbone qui peut être substitué par un groupe $OR_1$ dans lequel $R_1$ est un atome d'hydrogène ou un radical alkyle comprenant de 1 à 6 atomes de carbone, ou $AnR_2R_3$ dans lequel A est un radical alkylène comprenant de 2 à 8 atomes de carbone, $R_2$ et $R_3$ pouvant être identiques ou différents et consistant en un atome hydrogène ou un radical alkyle comprenant de 1 à 6 atomes de carbone qui peut être substitué par OH ou un groupe NRaRa dans lequel Ra identique ou différents consistent en un atome d'hydrogène ou un radical alkyle comprenant de 1 à 3 atomes de carbone, qui peut être substitué par OH, ou $R_2$ et $R_3$ peuvent former ensemble un radical éthylène ou un groupe:

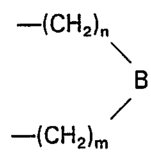

dans lequel n et m peuvent être identiques ou différents et égaux à 1, 2 ou 3, étant entendu que la somme de n et m est nombre entier comprise entre 3 et 6 et que B est une liaison directe, un atome d'oxygène, de soufre ou un groupe N—$R_4$ dans lequel $R_4$ est un atome d'hydrogène, un radical alkyle comprenant 1 à 6 atomes de carbone; R et Y pris ensemble forment un radical éthylène ou peuvent encore former un groupe:

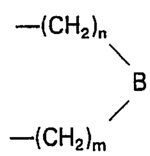

dans lequel n, m et B sont tels que définis ci-dessus; Z est un atome d'hydrogène, un radical alkyle comprenant 1 à 6 atomes de carbone qui peut être substitué par un groupe $N(R_1)_2$, $SR_1$ ou $OR_1$ dans lesquels $R_1$, identique ou différent, sont tels que définis ci-dessus, ou un groupe $DnR_2R_3$ dans lequel D est un radical alkylène comprenant 2 à 8 atomes de carbone, qui peut être substitué par un groupe OH, et $R_2$ et $R_3$ sont tels que définis ci-dessus, ainsi que les sels pharmaceutiquement acceptables en dérivant, à la condition cependant, que: 1) lorsque Z est un H, R et Y pris ensemble ne forment pas le cycle pipéridine; 2) lorsque Z est $CH_3$, R et Y pris ensemble ne permettant pas d'achever le noyau pipéridine ou le noyau morpholine; et 3) lorsque R et Y sont tous deux égaux à H, Z n'est ni un atome d'hydrogène, ni un radical alkyle comprenant 1 à 6 atomes de carbone, qui consiste à faire réagir un composé présentant la formule structurelle VII:

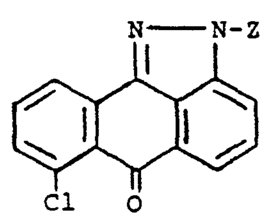

(VII)

avec une amine ayant la formule HNRY dans laquelle Y, Z et R sont tels que définis ci-dessus.

7. Un procédé de préparation d'une composition pharmaceutique comprenant la combinaison d'un composé produit par un procédé selon l'une quelconque des revendications précédentes ou un de ses sels pharmaceutiquement acceptables en dérivant, avec un support pharmaceutiquement acceptable.

8. Un procédé de préparation d'un médicament comprenant la combinaison d'un composé présentant une des formules suivantes:

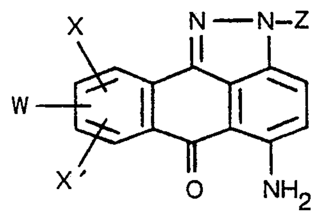
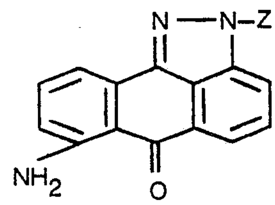

dans lesquelles: X, X' et W sont tels que définis dans la revendication 1; et Z est un atome d'hydrogène, ou un radical alkyle comprenant de 1 à 6 atomes de carbone, ou les sels pharmaceutiquement acceptables en dérivant, avec un support pharmaceutiquement acceptable.